# EUROPEAN PATENT SPECIFICATION

(11) **EP 1 953 145 B1**
(45) Date of publication and mention of the grant of the patent: **04.11.2015**
(21) Application number: 06832946.5
(22) Date of filing: 20.11.2006
(51) Int. Cl.: C07D 231/18, A61K 31/415, A61K 31/4152, A61K 31/4155, A61K 31/4178, A61K 31/4439, A61K 31/454, A61K 31/4709, A61K 31/496, A61K 31/662, C07D 403/06, C07D 403/12, C07D 405/12, C07D 409/12

(54) **HETEROCYCLIC COMPOUND HAVING INHIBITORY ACTIVITY ON 11-B-HYDROXYSTEROID DEHYDROGENASE TYPE I**
HETEROCYCLISCHE VERBINDUNG MIT HEMMENDER WIRKUNG AUF 11-B-HYDROXYSTEROIDDEHYDROGENASE VOM TYP I
COMPOSE HETEROCYCLIQUE PRESENTANT UNE ACTIVITE INHIBITRICE DE LA 11-B-HYDROXYSTEROIDE DESHYDROGENASE DE TYPE I

(30) Priority: 21.11.2005 JP 2005335995
(43) Date of publication of application: 06.08.2008
(73) Proprietor: Shionogi & Co., Ltd., Osaka-shi, Osaka 541-0045 (JP)
(72) Inventor: KUROSE, Noriyuki, Toyonaka-shi, Osaka 5610825 (JP); HAYASHI, Mikayo, Naruto-shi, Tokushima 7720003 (JP); OGAWA, Tomoyuki, Toyonaka-shi, Osaka 5610825 (JP); MASUDA, Koji, Toyonaka-shi, Osaka 5610825 (JP); KOJIMA, Eiichi, Toyonaka-shi, Osaka 5610825 (JP)
(74) Representative: Hoffmann Eitle
(86) International application number: PCT/JP2006/323096
(87) International publication number: WO 2007/058346

(56) References cited:
- WO-A1-01/23358
- WO-A1-98/41519
- WO-A1-03/104208
- WO-A1-2006/106052
- WO-A1-2006/132436
- WO-A2-2004/089470
- WO-A2-2005/016877
- WO-A2-2005/061462
- JP-A- 2001 516 361
- Michael O'Riordan: "RIO-Diabetes: Rimonabant effective in patients with type 2 diabetes", Medscape News , 13 June 2005 (2005-06-13), XP002646525, Retrieved from the Internet: URL:http://www.medscape.com/viewarticle/53 8697 [retrieved on 2011-06-29]
- "RIMONABANT-STUDIE BESTAETIGT SIGNIFIKANTE VERBESSERUNGEN DER HBA1C-WERTE UND METABOLISCHEN RISIKOFAKTOREN BEI PATIENTEN MIT TYP-2-DIABETES", INTERNET CITATION, 4 August 2005 (2005-08-04), XP002339554, Retrieved from the Internet: URL:http://www.diabetes-news.de/news/nachr ichten-2005/pm050617.htm [retrieved on 2005-08-04]

## Description

### Field of the invention

This invention relates to a pharmaceutically usuful compound with an inhibitory activity to 11βhydroxysteroid dehydrogenase type 1, hereinafter referred to as 11β-HSD-1

### Background Art

11β-HSD-1 is an enzyme that converts inactive steroids, 11β-dehydrosteroid into its active steroids and is considered to be important in the basal metabolic rate in the living body (Non-patent Document 1). Moreover, 11β-HSD-1 knockout mice have the resistance to hyperglycemia induced by obesity or stress (Non-patent Document 2). In addition, a similar phenomenon was observed in human on administration of 11β-HSD-1 inhibitor, carbenoxolone (Non-patent Document 3). These facts suggest that the 11β-HSD-1 inhibitors could be useful as drugs for the treatment of insulin independent diabetes or obesity (Non-patent Document 4).

Patent document 1 describes that pyrazole derivatives are useful as herbicide. Patent document 2 describes that pyrazole derivatives are useful as pesticide. Patent document 3 describes that pyrazole derivatives are useful as herbicide. Patent document 4 describes that pyrazole derivatives are useful as insecticide. Patent document 5 describes that pyrazole derivatives are useful as insecticide. Patent document 6 describes that pyrazole derivatives are useful as pesticide. Patent document 7 describes that pyrazole derivatives are useful as herbicide. The compounds disclosed in these patents have the carbamoyl group that is substituted with a substituent selected from a group consisting of substituted aryl, substituted arylalkyl, substituted heteroaryl and alkyl at 4-position of the pyrazole ring, and they are different from the compounds in the present invention.

Furthermore the compounds having straight alkyloxy at 5-position on the pyrazole ring are disclosed in patent document 8 and useful for the treatment of schizophrenia, and they are different from the present invention.

Moreover patent document 9 describes that the pyrazole derivatives shown below are useful as a cannabinoid receptor agonist, but does not describe the inhibitory activity to 11β-HSD-1.

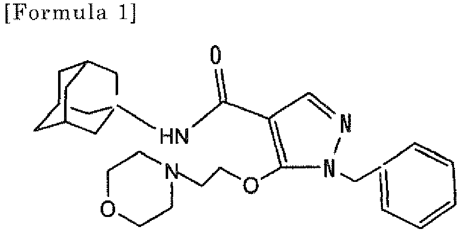

[Non-patent Document 1] Clin.Endocrinol, 1996, 44, 493
[Non-patent Document 2] Proc.Nat.Acad.Sci.USA, 1997, 94, 14924
[Non-patent Document 3] J.Clin.Endocrinol.Metab. 1995, 80, 3155
[Non-patent Document 4] Lancet, 1997, 349, 1210
[Patent Document 1] WO05/070889
[Patent Document 2] WO02/096882
[Patent Document 3] WO93/25535
[Patent Document 4] JP06-025199
[Patent Document 5] JP03-223256
[Patent Document 6] JP01-207289
[Patent Document 7] JP60-214785
[Patent Document 8] US4226877
[Patent Document 9] WO98/41519

### Disclosure of Invention

### Problems to be solved by the Invention

The present invention provides usuful compounds having an inhibitory activity to 11βhydroxysteroid dehydrogenase type 1.

### Means for Solving the Problem

The present invention provides;

Michael O'Riordan, Medscape News, 13 June 2005 (2005-06-13) (available under http://www.medscape.com/viewarticle/538697) discusses a clinical trial evaluating the effects of rimonabant, a pyrazole derivative acting as a cannabinoid receptor CB1 inverse agonist, in the treatment of diabetes.

This trial was also discussed in under "Diabetes-News" under http://www.diabetes-news.de/news/nachrichten-2005/pm05.0617.htm.

WO 2005/016877 describes pyrazole carboxamides of a specific structural formula, which act as selective inhibitors of the 11-beta-hydroxysteroid dehydrogenase-1. The compounds are useful for the treatment of diabetes, such as noninsulin-dependent diabetes (NIDDM), hyperglycemia, obesity, insulin resistance, dyslipidemia, hyperlipidemia, hypertension, Metabolic Syndrome, and other symptoms associated with NIDDM.
[1] A compound represented by formula (I): 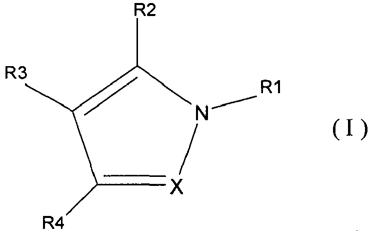 a pharmaceutically acceptable salt or a solvate thereof,
   wherein
   R¹ is optionally substituted alkyl, optionally substituted alkenyl, optionally substituted alkynyl, optionally substituted cycloalkyl, optionally substituted cycloalkenyl, optionally substituted aryl, optionally substituted heteroaryl or optionally substituted heterocycle,
   one of R² and R⁴ is a group of formula: -Y-R⁵,
   wherein Y is -O- or -S -, and
   R⁵ is substituted straight alkyl wherein the substituent of said substituted straight alkyl is cycloalkyl or cycloalkenyl,
   branched alkyl optionally substituted with hydroxy, carboxy or halogen, alkenyl optionally substituted with hydroxy, carboxy or halogen, alkynyl optionally substituted with hydroxy, carboxy or halogen, cycloalkyl optionally substituted with hydroxy, carboxy or halogen, cycloalkenyl optionally substituted with hydroxy, carboxy or halogen, aryl optionally substituted with hydroxy, carboxy or halogen, heteroaryl optionally substituted with hydroxy, carboxy or halogen or heterocycle optionally substituted with hydroxy, carboxy or halogen,
   the other of R² and R⁴ is hydrogen, optionally substituted alkyl, optionally substituted alkenyl, optionally substituted alkynyl, optionally substituted cycloalkyl, optionally substituted cycloalkenyl, optionally substituted aryl, optionally substituted heteroaryl or optionally substituted heterocycle,
   R³ is a group of formula: -C(=O)-Z-R⁶,
   wherein Z is -NR⁷- or -NR⁷-W-, and
   R⁶ is cycloalkyl optionally substituted with hydroxy, carboxy, halogen, cyano, -CH₃, - CH₂OH, -CH₂OCONH₂, -CH₂NH₂, -OCONH₂, -NHCOCH₃, -OCH₃, -NH₂, - NHSO₂CH₃, -CONHCH₃, -OSO₂NH₂, -NHSO₂NH₂, -NHCOOCH₃, -CONH₂, -COOCH₃ or -CONHSO₂CH₃, said cycloalkyl is selected from 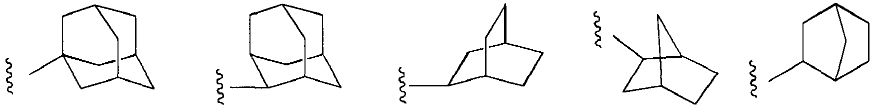 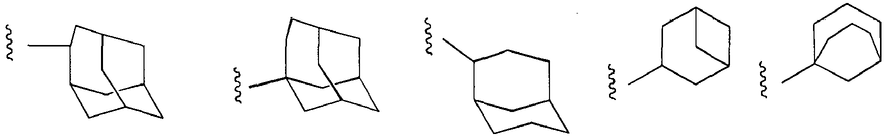 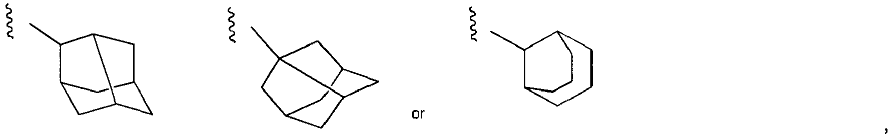 R⁷ is hydrogen or optionally substituted alkyl,
   W is optionally substituted alkylene,
   X is =N- or =CR⁸-, and
   R⁸ is hydrogen or optionally substituted alkyl,
   wherein "alkyl" means a C1 to C10 straight or branched alkyl group;
   "alkenyl" means a C2 to C8 straight or branched alkenyl group;
   "alkynyl" means a C2 to C8 straight or branched alkynyl group;
   "cycloalkyl" means a C3 to C15 saturated cyclic hydrocarbon group;
   "cycloalkenyl" means a C3 to C7 unsaturated aliphatic hydrocarbon group; "aryl" means phenyl or naphthyl;
   "heteroaryl" means furyl, thienyl, pyrrolyl, imidazolyl, pyrazolyl, triazolyl, tetraz olyl, oxazolyl, isoxazolyl, thiazolyl, thiadiazolyl, isothiazolyl, pyridyl, pyridazinyl, pyrimidinyl, furazanyl, pyrazinyl, oxadiazolyl, benzofuryl, benzothienyl, benzimi dazolyl, dibenzofuryl, benzoxazolyl, quinoxalyl, cinnolyl, quinazolyl, quinolyl, pht halazinyl, isoquinolyl, puryl, pteridinyl, carbazolyl, phenanthridinyl, acridinyl, in dolyl, isoindolyl, phenazinyl or phenothiazinyl;
   "heterocycle" means 1-pyrrolinyl, 2-pyrrolinyl, 3-pyrrolinyl, 1-pyrrolidinyl, 2-pyrr olidinyl, 3-pyrrolidinyl, 1-imidazolinyl, 2-imidazolinyl, 4-imidazolinyl, 1-imidazoli dinyl, 2-imidazolidinyl, 4-imidazolidinyl, 1-pyrazolinyl, 3-pyrazolinyl, 4-pyrazoliny l, 1-pyrazolidinyl, 3-pyrazolidinyl, 4-pyrazolidinyl, piperidino, 2-piperidinyl, 3-pip eridinyl, 4-piperidinyl, 1-piperazinyl, 2-piperazinyl, 2-morpholinyl, 3-morpholinyl, morpholino, tetrahydropyranyl or the following groups 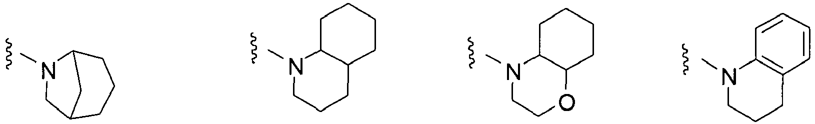 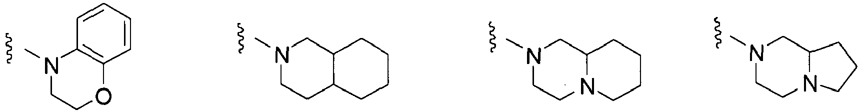 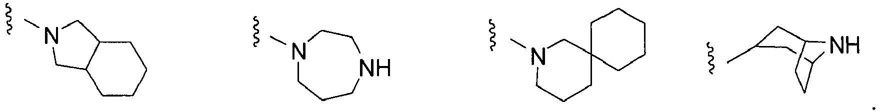
[2] The compound according to Item 1, a pharmaceutically acceptable salt or a solvate thereof, wherein R¹ is substituted alkyl wherein the substituent of said substituted alkyl is optionally substituted amino or optionally substituted heterocycle.
[3] The compound according to Item 1, a pharmaceutically acceptable salt or a solvate thereof, wherein R¹ is substituted ethyl wherein the substituent of said substituted ethyl is optionally substituted amino or optionally substituted heterocycle.
[4] The compound according to Item 1, a pharmaceutically acceptable salt or a solvate thereof, wherein R¹ is unsubstituted alkyl.
[5] The compound according to Item 1, a pharmaceutically acceptable salt or a solvate thereof, wherein R² is a group of formula: -Y-R⁵,
   wherein Y and R⁵ have the same meaning as defined in claim 1.
[6] The compound according to Item 5, a pharmaceutically acceptable salt or a solvate thereof, wherein Y is -O-.
[7] The compound according to Item 5 or 6, a pharmaceutically acceptable salt or a solvate thereof, wherein R⁵ is substituted straight alkyl wherein the substituent of said substituted straight alkyl is optionally substituted cycloalkyl.
[8] The compound according to claim 7, a pharmaceutically acceptable salt or a solvate thereof, wherein R⁵ is substituted methyl wherein the substituent of said substituted methyl is optionally substituted cycloalkyl.
[9] The compound according to Item 7 or 8, a pharmaceutically acceptable salt or a solvate thereof, wherein said optionally substituted cycloalkyl is optionally substituted cyclohexyl.
[10] The compound according to Item 5 or 6, a pharmaceutically acceptable salt or a solvate thereof, wherein R⁵ is branched alkyl.
[11] The compound according to Item 1, a pharmaceutically acceptable salt or a solvate thereof, wherein Z is -NR⁷-, and R⁷ has the same meaning as defined in claim 1.
[12] The compound according to Item 11, a pharmaceutically acceptable salt or a solvate thereof, wherein R⁷ is hydrogen.
[13] The compound according to Item 1, a pharmaceutically acceptable salt or a solvate thereof, wherein R⁶ is adamantyl.
[14] The compound according to Item 1, a pharmaceutically acceptable salt or a solvate thereof,
   wherein R³ is a group of formula ( II ): 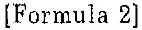 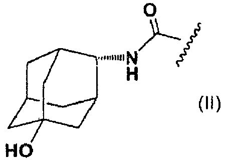
[15] The compound according to any one of Items 1 to 14, a pharmaceutically acceptable salt or a solvate thereof, wherein X is =N-.
[16] The compound according to Item 1, a pharmaceutically acceptable salt or a solvate thereof, wherein R¹ is a group of formula: -CH=CH-C(R⁹R¹⁰)-R¹¹-R¹²,
   wherein R⁹ and R¹⁰ are each independently hydrogen, optionally substituted alkyl or halogen, or R⁹ and R¹⁰ taken together with the carbon atom to which they are attached may form an optionally substituted ring,
   R¹¹ is -(CH₂)n-, wherein n is an integer of 0 to 3, and
   R¹² is hydrogen, hydroxy, carboxy, cyano, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted alkyloxycarbonyl, optionally substituted arylalkylcarbonyl, optionally substituted carbamoyl, optionally substituted thiocarbamoyl, optionally substituted alkylsulfonyl, optionally substituted arylsulfonyl, optionally substituted sulfamoyl, optionally substituted amino, optionally substituted carbamoyloxy, optionally substituted alkyloxy or optionally substituted alkylthio,
   a group of formula: -C(=O)-NR¹³R¹⁴,
   wherein R¹³ and R¹⁴ are each independently hydrogen, optionally substituted amino, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted alkylsulfonyl, optionally substituted arylsulfonyl, optionally substituted heteroarylsulfonyl or optionally substituted heterocyclesulfonyl, or R¹³ and R¹⁴ taken together with the nitrogen atom to which they are attached may form an optionally substituted ring or,
   a group of formula: -NR¹⁵R¹⁶,
   wherein R¹⁵ and R¹⁶ are each independently hydrogen, carboxy, hydroxy, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted acyl, optionally substituted carbamoyl, optionally substituted thiocarbamoyl, optionally substituted alkylsulfonyl, optionally substituted arylsulfonyl, optionally substituted alkyloxycarbonyl or optionally substituted sulfamoyl, or R¹⁵ and R¹⁶ taken together with the nitrogen atom to which they are attached may form optionally substituted ring.
[17] The compound according to Item 1, a pharmaceutically acceptable salt or a solvate thereof, wherein R¹ is a group of formula: -CH₂-CH₂-C(R⁹R¹⁰)-R¹¹-R¹²,
   wherein R⁹, R¹⁰, R¹¹ and R¹² have the same meaning as defined in Item 16.
[18] The compound according to Item 16 or 17, a pharmaceutically acceptable salt or a solvate thereof, wherein R⁹ and R¹⁰ are each independently optionally substituted alkyl or R⁹ and R¹⁰ taken together with the carbon atom to which they are attached may form optionally substituted ring.
[19] The compound according to any one of Items 16 to 18, a pharmaceutically acceptable salt or a solvate thereof, wherein R¹¹ is -(CH₂)n-, wherein n is an integer of 0 to 1.
[20] The compound according to any one of Items 16 to 19, a pharmaceutically acceptable salt or a solvate thereof, wherein R¹² is carboxy, cyano or heterocycle.
[21] The compound according to any one of Items 16 to 19, a pharmaceutically acceptable salt or a solvate thereof, wherein R¹² is a group of formula: -C(=O)-NR¹³R¹⁴,
   wherein R¹³ and R¹⁴ are each independently hydrogen, optionally substituted alkyl, optionally substituted aryl, optionally substituted cycloalkyl, optionally substituted alkylsulfonyl, optionally substituted arylsulfonyl, optionally substituted heteroarylsulfonyl, optionally substituted heterocyclesulfonyl or optionally substituted heterocycle, or R¹³ and R¹⁴ taken together with the nitrogen atom to which they are attached may form an optionally substituted ring.
[22] The compound according to any one of Items 16 to 19, a pharmaceutically acceptable salt or a solvate thereof, wherein R¹² is a group of formula: -NH¹⁵R¹⁶,
   wherein R¹⁵ and R¹⁶ have the same meaning as defined in Item 16.
[23] The compound according to Item 22, a pharmaceutically acceptable salt or a solvate thereof, wherein R¹⁵ is a group of formula: -C(=O)R', wherein R' is optionally substituted alkyl, optionally substituted alkenyl, optionally substituted cycloalkyl, optionally substituted aryl, optionally substituted amino or optionally substituted alkyloxy.
[24] A pharmaceutical composition which comprises the compound according to any one of Items 1 to 23, a pharmaceutically acceptable salt or a solvate thereof as an active ingredient.
[25] The pharmaceutical composition according to item [24], for treating and/or preventing diabetes.

### Effect of the Invention

The compounds of the present invention possess an inhibitory activity to 11β hydroxysteroid dehydrogenase type 1 and the pharmaceutical compositions comprising them are very useful for a medicament, especially a medicament for treating and/or preventing hyperlipidemia, diabetes, obesity, arteriosclerosis, atherosclerosis, hyperglycemia and/or syndrome X. Moreover, the compounds of the present invention selectively inhibit 11βhydroxysteroid dehydrogenase type 1. The preferable compounds in the present compounds have a high metabolic stability, a weak drug metabolizing enzyme induction, a weak drug metabolizing enzyme inhibition or a high oral absorption, and they are especially useful for a medicament. In addition, the present invention includes compounds having a low clearance and a long half-life period for exhibiting the drug activity.

### Best Mode for Carrying Out the Invention

Terms used in the present specification are explained below. Each term has the following meanings alone or together with other terms.
"Alkyl" means a C1 to C10 straight or branched alkyl group, for example, methyl, ethyl, n-propyl, isopropyl, n-butyl, isobutyl, sec-butyl, tert-butyl, n-pentyl, isopentyl, neopentyl, tert-pentyl, n-hexyl, isohexyl, n-heptyl, n-octyl, n-nonyl, n-decyl or the like. Preferred is a C1 to C6 alkyl or a C1 to C4 alkyl, for example, methyl, ethyl, n-propyl, isopropyl, n-butyl, isobutyl, sec-butyl, tert-butyl, n-pentyl, isopentyl, neopentyl, tert-pentyl, n-hexyl, isohexyl.
"Straight alkyl" means a C1 to C10 straight alkyl group, for example, methyl, ethyl, n-propyl, n-butyl, n-pentyl, n-hexyl, n-heptyl, n-octyl, n-nonyl, n-decyl or the like. Preferred is a C1 to C6 or a C1 to C4 straight alkyl.
"Branched alkyl" means a C3 to C10 branched alkyl group, for example, isopropyl, isobutyl, sec-butyl, tert-butyl, isopentyl, neopentyl, tert-pentyl, isohexyl or the like. Preferred is a C3 to C6 branched alkyl.
"Alkylene" means a di-valent group derived from the above "alkyl", which includes a C1 to C10 straight or branched alkylene. Preferred is methylene, ethylene, propylene, trimethylene, tetramethylene, ethylethylene, pentamethylene, hexamethylene or the like.
"Alkenyl" means a C2 to C8 straight or branched alkenyl group, which includes a group having one or more double bond(s), for example 1 to 3 double bond(s) in the above "alkyl". Exemplified is vinyl, 1-propenyl, 2-propenyl, 1-butenyl, 2-butenyl, 3-butenyl, 1,3-butadienyl, 3-methyl -2-butenyl or the like.
"Alkynyl" means a C2 to C8 straight or branched alkynyl group, which includes a group having one or more triple bond(s), for example 1 to 3 triple bond(s) in the above "alkyl". Exemplified is ethynyl or the like. Moreover, "alkynyl" can possess 1 to 3 double bond(s).
"Cycloalkyl" means a C3 to C15 saturated cyclic hydrocarbon group. Bridged cyclic hydrocarbons group is also included. Exemplified is cyclopropyl, cyclobutyl, cyclopentyl, cyclohexyl, cycloheptyl, cyclooctyl or bridged cyclic hydrocarbon, exemplified as follows. 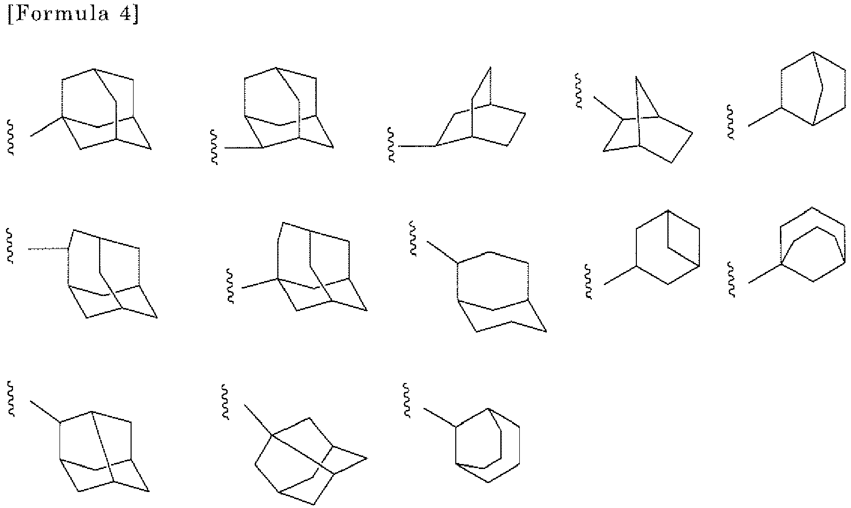 "Cycloalkenyl" means a C3 to C7 unsaturated aliphatic hydrocarbon group, including bridged cyclic hydrocarbon group. Exemplified is cyclopropenyl, cyclobutenyl, cyclopentenyl, cyclohexenyl or cycloheptenyl. Preferred is cyclopropenyl, cyclobutenyl, cyclopentenyl or cyclohexenyl. Furthermore, cycloalkenyl means a group that has unsaturated bond in the above exemplified bridged cyclic hydrocarbon group.
"Aryl" means a monocyclic aromatic hydrocarbon group (e.g., phenyl) or a fuzed aromatic hydrocarbon group (e.g., 1-naphthyl, 2-naphthyl, 1-anthryl, 2-anthryl, 9-anthryl, 1-phenanthryl, 2- phenanthryl, 3- phenanthryl, 4-phenanthryl, 9- phenanthryl etc.). Preferred is phenyl or naphthyl (1-naphthyl, 2-naphthyl) or the like.
"Heteroaryl" means a monocyclic aromatic heterocyclic group or fused aromatic heterocyclic group.

The monocyclic aromatic heterocyclic group means a group derived from 5 to 8-membered aromatic heterocycle which may contain 1 to 4 oxygen, sulfur and/or nitrogen atom(s) in the ring. The binding bond can be at any substitutable position.

The fused aromatic heterocyclic group means a group derived from 5 to 8-membered aromatic heterocycle which may contain 1 to 4 oxygen, sulfur and/or nitrogen atom(s) in the ring fused with one to four of 5 to 8-membered aromatic carbocycle(s) or other 5 to 8-membered aromatic heterocycle(s). The binding bond can be at any substitutable position.

For example, it is furyl (e.g., furan-2-yl or furan-3-yl), thienyl (e.g., thiophene-2-yl or thiophene-3-yl), pyrrolyl (e.g., pyrrole-1-yl, pyrrole-2-yl or pyrrole-3-yl), imidazolyl (e.g., imidazole-1-yl, imidazole-2-yl or imidazole-4-yl), pyrazolyl (e.g., pyrazole-1-yl, pyrazole-3-yl or pyrazole-4-yl), triazolyl (e.g., 1,2,4-triazole-1-yl, 1,2,4-triazole-3-yl or 1,2,4-triazole-4-yl), tetrazolyl (e.g., tetrazole-1-yl, tetrazole-2-yl or tetrazole-5-yl), oxazolyl (e.g., oxazole-2-yl, oxazole-4-yl or oxazole-5-yl), isoxazolyl (e.g., isoxazole-3-yl, isoxazole-4-yl or isoxazole-5-yl), thiazolyl (e.g., thiazole-2-yl, thiazole-4-yl or thiazole-5-yl), thiadiazolyl, isothiazolyl (e.g., isothiazole-3-yl, isothiazole-4-yl or isothiazole-5-yl), pyridyl (e.g., pyridine-2-yl, pyridine-3-yl or pyridine-4-yl), pyridazinyl (e.g., pyridazine-3-yl or pyridazine-4-yl), pyrimidinyl (e.g., pyrimidine-2-yl, pyrimidine-4-yl or pyrimidine-5-yl), furazanyl (e.g., furazan-3-yl), pyrazinyl (e.g., pyrazine-2-yl), oxadiazolyl (e.g., 1,3,4-oxadiazole-2-yl), benzofuryl (e.g., benzo[b]furan-2-yl, benzo[b]furan-3-yl, benzo[b]furan-4-yl, benzo[b]furan-5-yl, benzo[b]furan-6-yl or benzo[b]furan-7-yl)', benzothienyl (e.g., benzo[b]thiophene-2-yl, benzo[b]thiophene-3-yl, benzo[b]thiophene-4-yl, benzo[b]thiophene-5-yl, benzo[b]thiophene-6-yl or benzo[b]thiophene-7-yl), benzimidazolyl (e.g., benzimidazole-I-yl, benzimidazole-2-yl, benzimidazole-4-yl or benzimidazole-5-yl), dibenzofuryl, benzoxazolyl, quinoxalyl (e.g., quinoxaline-2-yl, quinoxaline-5-yl or quinoxaline-6-yl), cinnolyl (e.g., cinnoline-3-yl, cinnoline-4-yl, cinnoline-5-yl, cinnoline-6-yl, cinnoline-7-yl or cinnoline-8-yl), quinazolyl (e.g., quinazoline-2-yl, quinazoline-4-yl, quinazoline-5-yl, quinazoline-6-yl, quinazoline-7-yl or quinazoline-8-yl), quinolyl (e.g., quinoline-2-yl, quinoline-3-yl, quinoline-4-yl, quinoline-5-yl, quinoline-6-yl, quinoline-7-yl or quinoline-8-yl), phthalazinyl (e.g., phthalazine-1-yl, phthalazine-5-yl or phthalazine-6-yl), isoquinolyl (e.g., isoquinoline-1-yl, isoquinoline-3-yl, isoquinoline-4-yl, isoquinoline-5-yl, isoquinoline-6-yl, isoquinoline-7-yl or isoquinoline-8-yl), puryl, pteridinyl (e.g., pteridine-2-yl, pteridine-4-yl, pteridine-6-yl or pteridine-7-yl), carbazolyl, phenanthridinyl, acridinyl (e.g., acridine-1-yl, acridine-2-yl, acridine-3-yl, acridine-4-yl or acridine-9-yl), indolyl (e.g., indole-1-yl, indole-2-yl, indole-3-yl, indole-4-yl, indole-5-yl, indole-6-yl or indole-7-yl), isoindolyl, phenazinyl (e.g., phenazine-1-yl or phenazine-2-yl), phenothiazinyl (e.g., phenothiazine-1-yl, phenothiazine-2-yl, phenothiazine-3-yl or phenothiazine-4-yl) or the like.

"Heterocycle" means a 5 to 8-membered nonaromatic heterocycle group which may contain 1 to 4 oxygen, sulfur and/or nitrogen atom(s) in the ring. The binding bond can be at any substitutable position. Moreover, the nonaromatic heterocycle group can be substituted with a C1 to C5 alkylene chain or a C2 to C5 alkenylene chain to form fused ring(including bicyclic ring) or spiro ring, or can be fused with cycloalkane(preferred is 5 to 6-membered ring) or benzene ring. Heterocycle can be saturated or unsaturated, as long as it is nonaromatic. Preferred is 5 to 8-membered ring. Exemplified is 1-pyrrolinyl, 2-pyrrolinyl, 3-pyrrolinyl, 1-pyrrolidinyl, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidinyl, 3-piperidinyl, 4-piperidinyl, 1-piperazinyl, 2-piperazinyl, 2-morpholinyl, 3-morpholinyl, morpholino, tetrahydropyranyl, or the following groups. Each ring can be optionally substituted at any substitutable position.

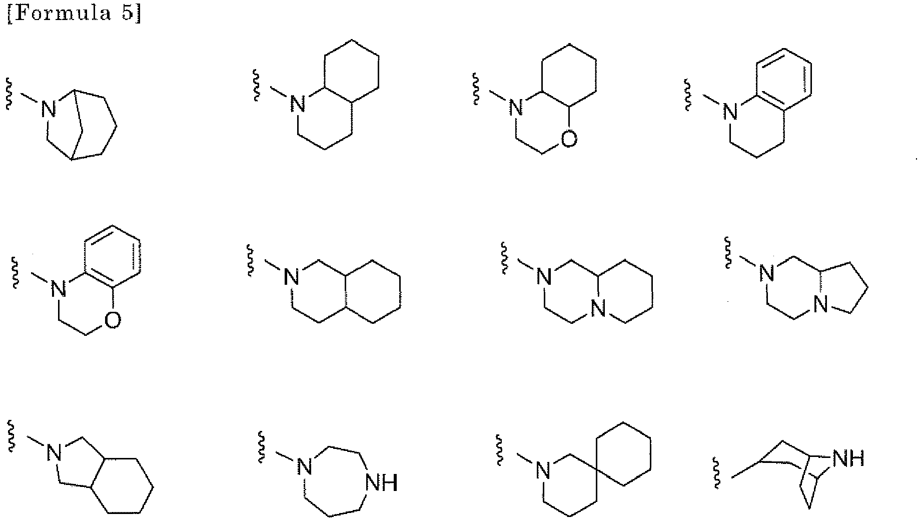

"A ring formed by taking together R⁶ and R⁷" means 5 to 8-membered ring including the nitrogen atom attached to R⁷ in the ring. The above ring is attached to the carbon atom of the carbonyl group through the binding bond from the nitrogen atom attached to R⁷. The ring is composed of carbon, oxygen, sulfer atom(s) or the like, besides the above nitrogen atom. The ring can contain 1 to 4 oxygen, sulfer and/or nitrogen atom(s) in the ring. Moreover, the ring can be substituted with C1 to C5 alkylene chain or C2 to C5 alkenylene chain to form fused ring(including bicycle ring), spiro ring or can be fused with cycloalkane(preferred is 5 to 6-membered ring) or benzene ring. The ring can be saturated or unsaturated. Preffered is 5 to 8-membered ring, for example, 1-pyrrolinyl, 1-pyrrolidinyl, 1-imidazolinyl, 1-imidazolidinyl, 1-pyrazolinyl, 1-pyrazolidinyl, piperidino, 1-piperadinyl, morpholino, or the following groups. Each ring can be optionally substituted.

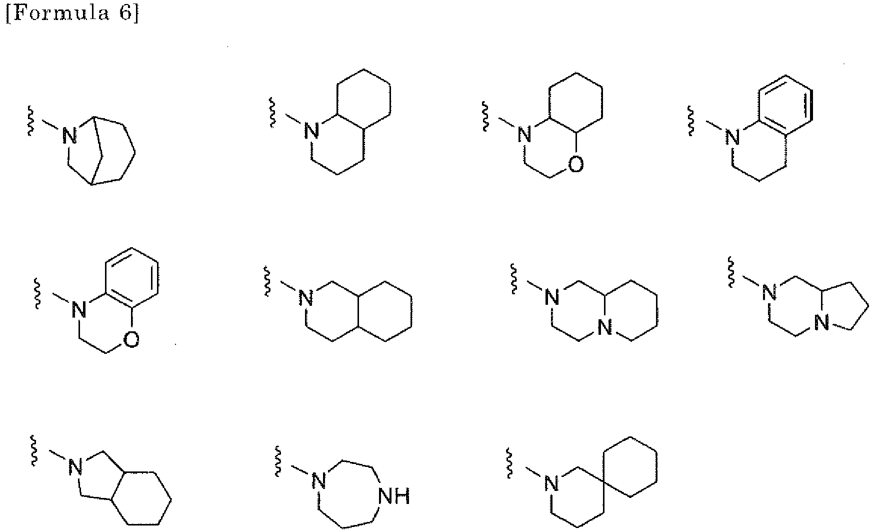

"A ring formed by taking together R⁹ and R¹⁰ with the carbon atom to which they are attached" means 3 to 15-membered saturated or unsaturated hydrocarbon ring or 3 to 15-membered saturated or unsaturated hetero ring containing 1 to 4 oxygen, sulfer, and/or nitrogen atom(s) in said hydrocarbon ring. Preffered is nonaromatic ring, for example, cyclopropane, cyclobutane, cyclopentane, cyclohexane, cycloheptane, cyclooctane, cyclopropene, cyclobutene, cyclopentene, cyclohexene, cycloheptene, or the like. Exemplified is saturated or unsaturated hetero ring containing 1 to 4 oxygen, sulfer, and/or nitrogen atom(s) in the hydrocarbon ring.

For example, a group of formula: -C(R⁹R¹⁰)-, wherein R⁹ and R¹⁰ taken together with the carbon atom to which they are attached may form an optionally substituted ring, is exemplified as follows. Each ring can be optionally substituted.

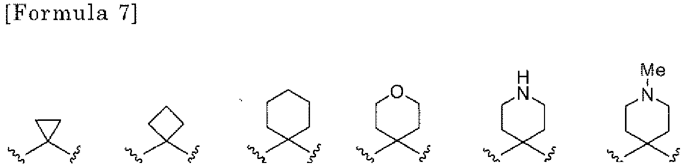

"A ring formed by taking together R¹³ and R¹⁴ with the nitrogen atom to which they are attached" and "a ring formed by taking together R¹⁶ and R¹⁶ with the nitrogen atom to which they are attached" mean 3 to 15-membered nonaromatic hetero ring which may contain 1 to 4 oxygen, sulfer, and/or nitrogen atom(s) besides the above nitrogen atom in the ring. The nonaromatic hetero ring can be bridged with C1 to C4 alkyl chain and be fused with cycloalkane (preffered is 5 to 6-membered ring) or benzene ring. The ring can be saturated or unsaturated, as long as it is nonaromatic. Preffered is 5 to 8-membered ring. For example, a group of formula: -NR¹³R¹⁴ wherein R¹³ and R¹⁴ taken together with the nitrogen atom to which they are attached may form an optionally substituted ring and a group of formula:-NR¹⁵R¹⁶ wherein R¹⁵ and R¹⁶ taken together with the nitrogen atom to which they are attached may form an optionally substituted ring are exemplified as follows. 1-Pyrrolinyl, 1-pyrrolidinyl, 1-imidazolinyl, 1-imidazolidinyl, 1-pyrazolinyl, 1-pyrazolidinyl, piperidino, morpholino and the following group are exemplified. Each ring can be optionally substituted.

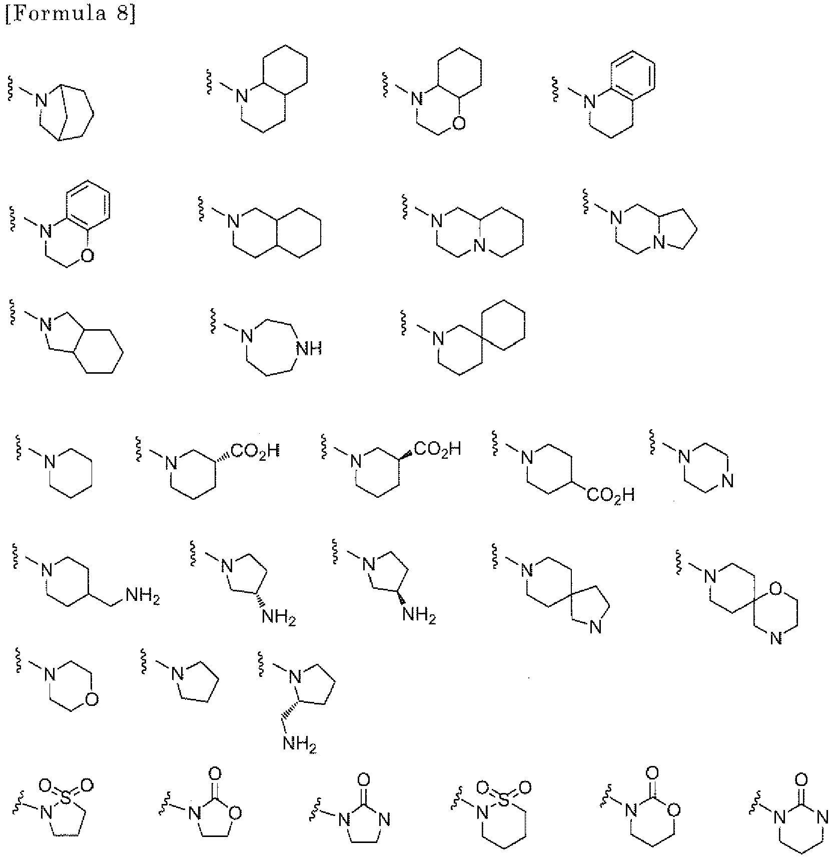

"Optionally substituted cycloalkyl", "optionally substituted cycloalkenyl", "optionally substituted aryl", "optionally substituted heteroaryl", "optionally substituted heterocycle", "optionally substituted alkyl", "optionally substituted alkylene", "optionally substituted alkenyl", "optionally substituted alkynyl", "substituted straight alkyl", "optionally substituted branched alkyl", "a ring formed by taking together R⁶ and R^{7"}, "optionally substituted imino", "a ring formed by taking together R⁹ and R¹⁰ with the carbon atom to which they are attached", "a ring formed by taking together R¹³ and R¹⁴ with the nitrogen atom to which they are attached", "a ring formed by taking together R¹⁵ and R¹⁶ with the nitrogen atom to which they are attached" and "optionally substituted methylene" may be substituted with 1 to 4 substituent(s) selected from a group consisting of, for example, hydroxy, carboxy, halogen (e.g.: F, Cl, Br, I),
optionally substituted alkyl (e.g.: methyl, ethyl, isopropyl, tert-butyl, halogenated alkyl (e.g.: -CF₃, -CH₂CF₃, -CH₂CCl₃),
optionally substituted alkylthio (e.g.: methylthio),
optionally substituted alkylsulfonyl (e.g.: methansulfonyl, ethansulfonyl),
optionally substituted carbamoyl (e.g.: optionally substituted alkylcarbamoyl (e.g.: methylcarbamoyl, ethylcarbamoyl, dimethylcarbamoyl), optionally substituted alkylsulfonylcarbamoyl),
optionally substituted alkenyl (e.g.: vinyl),
optionally substituted alkenyloxy (e.g.: vinyloxy, allyloxy), alkynyl (e.g.: ethynyl),
optionally substituted cycloalkyl (e.g.: cyclopropyl),
optionally substituted cycloalkenyl (e.g.: cyclopropenyl),
optionally substituted alkyloxy (e.g.: methoxy, ethoxy, propoxy, butoxy, carboxymethyloxy),
optionally substituted alkyloxycarbonyl (e.g.: methoxycarbonyl, ethoxycarbonyl, tert-butoxycarbonyl), nitro, nitroso,
optionally substituted amino (e.g.: alkylamino (e.g.: methylamino, ethylamino, diethylamino), acylamino (e.g.: optionally substituted alkylcarbonylamino,
optionally substituted arylcarbonylamino, optionally substituted heteroarylcarbonylamino, optionally substituted heterocyclecarbonylamino),
optionally substituted arylalkylamino (e.g.: benzylamino, tritylamino), hydroxyamino, optionally substituted alkyloxycarbonylamino, optionally substituted alkylsulfonylamino, optionally substituted carbamoylamino,
optionally substituted arylsulfonylamino, optionally substituted arylamino), azide,
optionally substituted aryl (e.g.: phenyl),
optionally substituted arylalkyl (e.g.: benzyl),
optionally substituted heteroaryl,
optionally substituted heterocycle,
cyano, isocyano, isocyanate, thiocyanate, isothiocyanate, mercapto, optionally substituted sulfamoyl,
acyl (e.g.: formyl, optionally substituted alkylcarbonyl, optionally substituted alkenylcarbonyl, optionally substituted arylcarbonyl, optionally substituted heteroarylcarbonyl, optionally substituted heterocyclecarbonyl),
formyloxy, haloformyl, oxalo, thioformyl, thiocarboxy,
dithiocarboxy, optionally substituted thiocarbamoyl,
sulfino, sulfo, sulfoamino, hydrazino, azide, ureido, amidino,
guanidino, phthalimide, oxo, alkylene,
alkylenedioxy (-O-CH₂-O-, -O-CH₂-CH₂-O-, -O-CH₂-CH₂-CH₂-O-, or the like),
optionally substituted heterocyclecarbonyl, phosphoester (e.g., -P(=O)(OEt)₂),
optionally substituted cycloalkylthio, optionally substituted arylthio,
optionally substituted heteroarylthio,
optionally substituted heteroaryloxy, optionally substituted aryloxy,
optionally substituted heterocycleoxy, optionally substituted imino,
optionally substituted alkylsulfonyl, optionally substituted arylsulfonyl,
optionally substituted alkylsulfinyl, optionally substituted arylsulfinyl,
optionally substituted alkylcarbonyloxy, optionally substituted arylcarbonyloxy, optionally substituted heteroarylcarbonyloxy, optionally substituted heterocyclcarbonyloxy,
optionally substituted alkylcarbonyl,
optionally substituted arylcarbonyl,
optionally substituted heteroarylcarbonyl,
optionally substituted aryloxycarbonyl,
optionally substituted heteroaryloxycarbonyl,
optionally substituted heterocycleoxycarbonyl,
optionally substituted methylene.

The alkyl part of "optionally substituted alkylsulfonyl", "optionally substituted alkylsulfinyl", "optionally substituted alkyloxy", "optionally substituted alkylsulfonylamino", "optionally substituted alkylcarbonyloxy", "optionally substituted alkylcarbonyl", "optionally substituted alkyloxycarbonyl", "optionally substituted alkyloxycarbonylamino", "optionally substituted alkylthio", "optionally substituted alkylcarbamoyl" and "optionally substituted alkylsulfonylcarbamoyl" is the same as the above "alkyl". The alkyl part can be optionally substituted with the same substituent as the above "optionally substituted alkyl".

The alkenyl part of "optionally substituted alkenylcarbonyl", "optionally substituted alkenyloxy" is the same as the above "alkenyl". The alkenyl part can be optionally substituted with the same substituent as the above "optionally substituted alkenyl".

The cycloalkyl part of "optionally substituted cycloalkylthio" is the same as the above "cycloalkyl". The cycloalkyl part can be optionally substituted with the same substituent as the above "optionally substituted. cycloalkyl".

The aryl part of "optionally substituted arylsulfonyl", "optionally substituted arylsulfinyl", "optionally substituted arylsulfonylamino", "optionally substituted arylcarbonyloxy", "optionally substituted aryloxycarbonyl", "optionally substituted arylcarbonyl", "optionally substituted arylamino", "optionally substituted arylthio", "optionally substituted aryloxy" is the same as the above "aryl". The aryl part can be optionally substituted with the same substituent as the above "optionally substituted aryl".

The heteroaryl part of "optionally substituted heteroarylcarbonyloxy", "optionally substituted heteroarylcarbonyl", "optionally substituted heteroaryloxycarbonyl", "optionally substituted heteroarylthio" and "optionally substituted heteroaryloxy" is the same as the above "heteroaryl". The heteroaryl part can be optionally substituted with the same substituent as the above "optionally substituted heteroaryl".

The heterocycle part of "optionally substituted heterocyclecarbonyloxy", "optionally substituted heterocycleoxycarbonyl", "optionally substituted heterocyclecarbonyl" and "optionally substituted heterocycleoxy" is the same as the above "heterocycle". The heterocycle part can be optionally substituted with the same substituent as the above "optionally substituted heterocycle".

The aryl part of "optionally substituted arylalkylamino" is the same as the above "aryl" and the alkyl part is the same as the above "alkyl". The aryl part can be optionally substituted with the same substituent as the above "optionally substituted aryl" and the alkyl part can be optionally substituted with the same substituent as the above "optionally substituted alkyl".

A substituent of "optionally substituted amino", "optionally substituted carbamoylamino", "optionally substituted carbamoyl", "optionally substituted thiocarbamoyl", "optionally substituted sulfamoyl", "optionally substituted imino" includes optionally substituted alkyl, optionally substituted alkenyl, optionally substituted aryl, optionally substituted heteroaryl, acyl, hydroxy, optionally substituted alkylsulfonyl, optionally substituted alkylsulfinyl, optionally substituted arylsulfonyl, optionally substituted arylsulfinyl, optionally substituted amino or the like.

A substituent of "optionally substituted alkylsulfonyl", "optionally substituted alkylsulfinyl", "optionally substituted arylsulfonyl," "optionally substituted arylsulfinyl" includes the same substituent as optionally substituted alkyl or optionally substituted aryl.

"Acyl" means formyl, optionally substituted alkylcarbonyl, optionally substituted alkenylcarbonyl, optionally substituted arylcarbonyl, optionally substituted heteroarylcarbonyl or optionally substituted heterocyclecarbonyl.

A substituent of "optionally substituted alkylcarbonyl", "optionally substituted alkenylcarbonyl", "optionally substituted arylcarbonyl", "optionally substituted heteroarylcarbonyl", "optionally substituted heterocyclecarbonyl" includes the same substituent as optionally substituted alkyl, optionally substituted alkenyl, optionally substituted aryl, optionally substituted heteroaryl, optionally substituted heterocycle.

"Arylalkyl" means the above alkyl which is substituted with 1 to 3 of the above aryl.

R¹ is optionally substituted alkyl, optionally substituted alkenyl, optionally substituted alkynyl, optionally substituted cycloalkyl, optionally substituted cycloalkenyl, optionally substituted aryl, optionally substituted heteroaryl or optionally substituted heterocycle. Preferably R¹ is substituted alkyl wherein the substituent of said substutited alkyl is optionally substituted amino or optionally substituted heterocycle, unsubstituted alkyl, a group of formula: -CH=CH-C(R⁹R¹⁰)-R¹¹-R¹², or a group of formula: -CH₂-CH₂-C(R⁹R¹⁰)-R¹¹-R¹², wherein R⁹ and R¹⁰ are each independently hydrogen, optionally substituted alkyl or halogen, or R⁹ and R¹⁰ taken together with the carbon atom to which they are attached may form an optionally substituted ring,
R¹¹ is -(CH₂)n- wherein n is an integer of 0 to 3,
R¹² is hydrogen, hydroxy, carboxy, cyano, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted alkyloxycarbonyl, optionally substituted arylalkylcarbonyl, optionally substituted carbamoyl, optionally substituted thiocarbamoyl, optionally substituted alkylsulfonyl, optionally substituted arylsulfonyl, optionally substituted sulfamoyl, optionally substituted amino, optionally substituted carbamoyloxy, optionally substituted alkyloxy or optionally substituted alkylthio,
a group of formula: -C(=O)-NR¹³R¹⁴
wherein R¹³ and R¹⁴ are each independently hydrogen, optionally substituted amino, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted alkylsulfonyl, optionally substituted arylsulfonyl, optionally substituted heteroarylsulfonyl,
optionally substituted heterocyclesulfonyl, or R¹³ and R¹⁴ taken together with the nitrogen atom to which they are attached may form an optionally substituted ring or,
a group of formula; -NR¹⁵R¹⁶
wherein R¹⁵ and R¹⁶ are each independently hydrogen, carboxy, hydroxy, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted acyl, optionally substituted carbamoyl, optionally substituted thiocarbamoyl, optionally substituted alkylsulfonyl, optionally substituted arylsulfonyl, optionally substituted alkyloxycarbonyl, optionally substituted sulfamoyl, or R¹⁵ and R¹⁶ taken together with the nitrogen atom to which they are attached may form optionally substituted ring.

One of R² and R⁴ is a group of formula: -Y-R⁵,
wherein Y is -O-or -S-, and
R⁵ is substituted straight alkyl wherein the substituent of said substituted straight alkyl is cycloalkyl or cycloalkenyl,
branched alkyl optionally substituted with hydroxy, carboxy or halogen, alkenyl optionally substituted with hydroxy, carboxy or halogen, alkynyl optionally substituted with hydroxy, carboxy or halogen, cycloalkyl optionally substituted with hydroxy, carboxy or halogen, cycloalkenyl optionally substituted with hydroxy, carboxy or halogen, aryl optionally substituted with hydroxy, carboxy or halogen, heteroaryl optionally substituted with hydroxy, carboxy or halogen or heterocycle optionally substituted with hydroxy, carboxy or halogen,
the other of R² and R⁴ is hydrogen, optionally substituted alkyl, optionally substituted alkenyl, optionally substituted alkynyl, optionally substituted cycloalkyl, optionally substituted cycloalkenyl, optionally substituted aryl, optionally substituted heteroaryl or optionally substituted heterocycle.

Preferably R² is a group of formula: -Y-R⁵ wherein R⁵ has the same meaning as defined in the above (1). More Preferable as R² is a a group of formula: -Y-R⁵ wherein Y is -O-, and R⁵ is substituted straight alkyl wherein the substituent of said substituted straight alkyl is optionally substituted cycloalkyl. As to said alkyl, methyl, ethyl, propyl(especially, methyl) are preferred. As to said cycloalkyl, cyclopentyl, cyclohexyl, cycloheptyl, cyclooctyl(especially, cyclohexyl) are preferred.

R³ is a group of formula: -C(=O)-Z-R⁶,
wherein Z is -NR⁷- or -NR⁷-W-, and
R⁶ is optionally substituted cycloalkyl as defined above.
R⁷ is hydrogen or optionally substituted alkyl.
W is optionally substituted alkylene.

Preferable as Z is -NR⁷- wherein R⁷ has the same meaning as defined in the above (1). More preferable as Z is -NH-.

Moreover, as to R⁶, optionally substituted cycloalkyl is preferable. Adamantyl(especially, 2-adamantyl) is more preferable as R⁶.

X is =N- or =CR⁸- wherein R⁸ is hydrogen or optionally substituted alkyl. Preferable is =N-.

As to R¹, for example, the following groups are preferable. 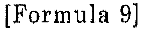 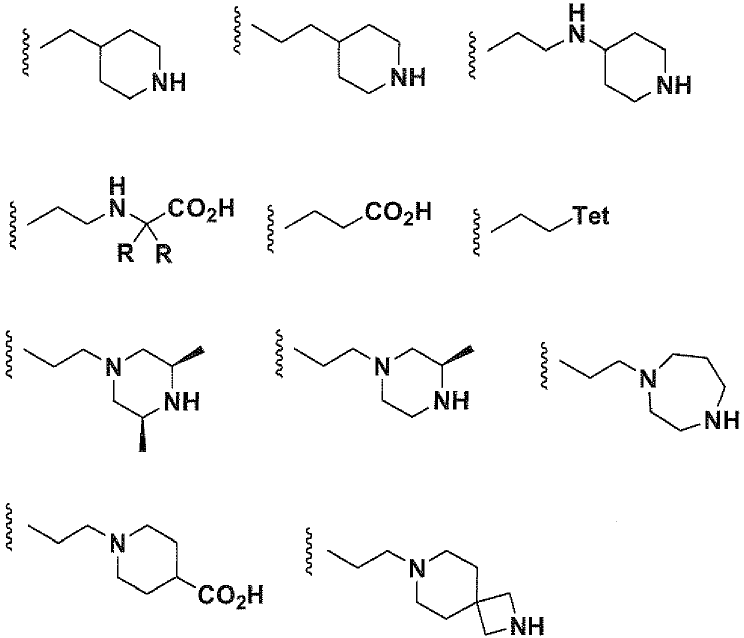 wherein R are each independently optionally substituted alkyl, and "Tet" means tetrazoryl. 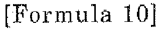 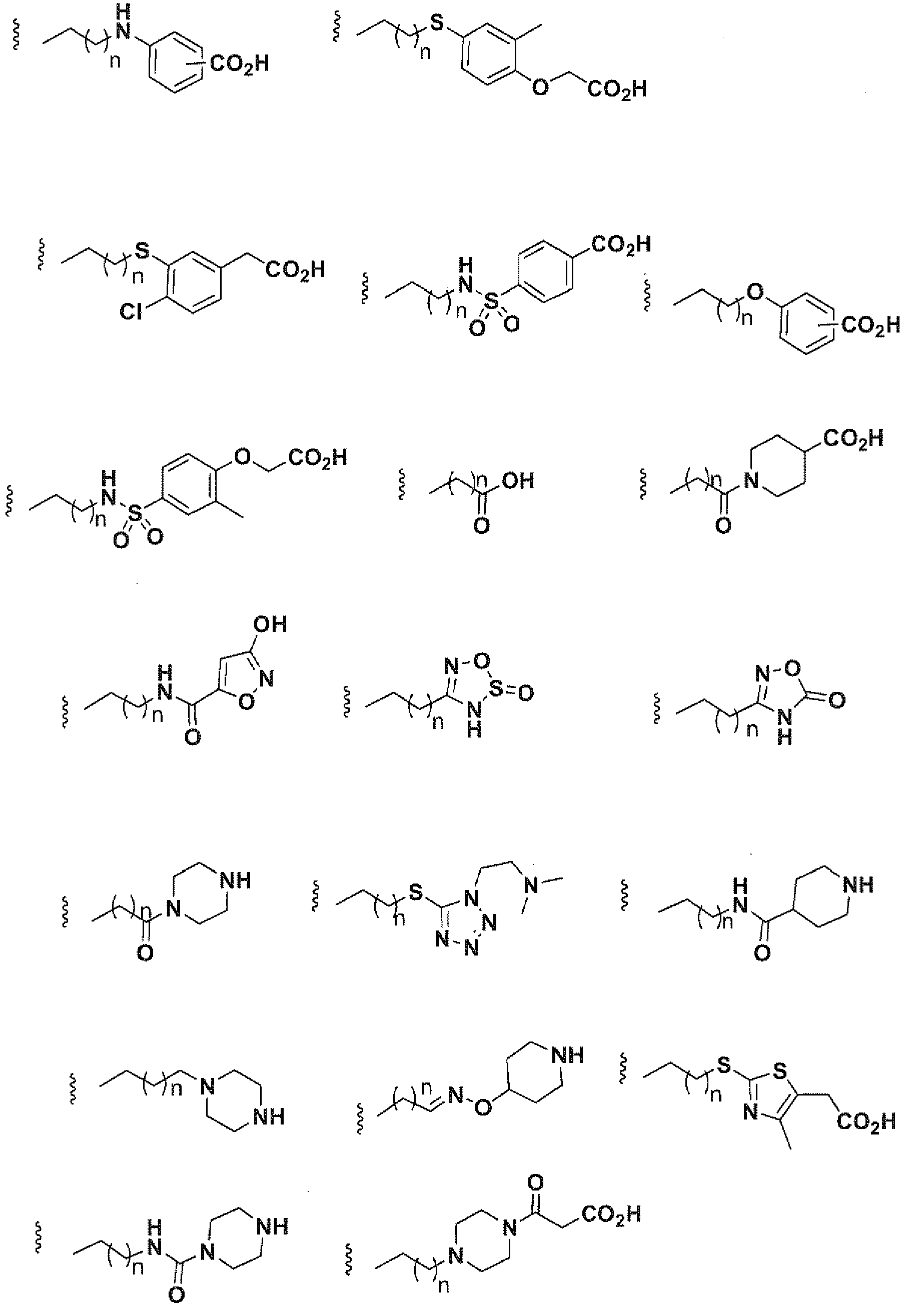 wherein n is an integer of 1 to 3. 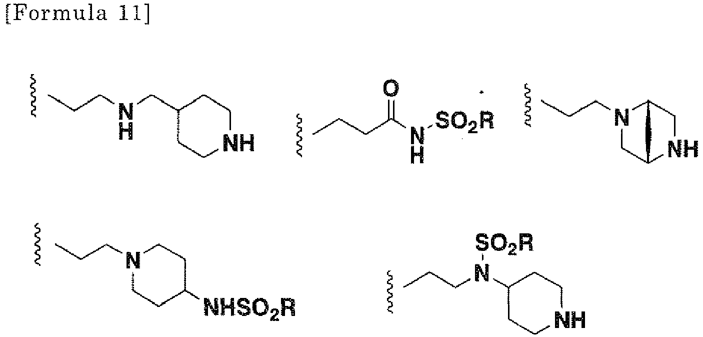 wherein R is optionally substituted alkyl or optionally substituted aryl.

As to a group of formula: -V-R⁵, for example, the following groups are preferable.

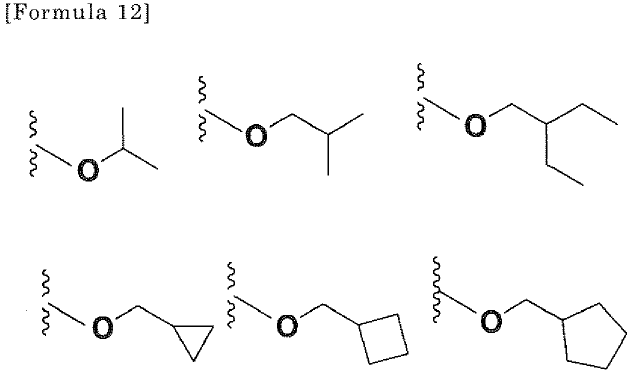

As to the substituent of substituted alkyl of R⁵, the followings are preferable:
A) optionally substituted cycloalkyl (e.g., cyclopentyl, cyclohexyl, cycloheptyl, cyclooctyl),
B) optionally substituted cycloalkenyl (e.g., cyclopentenyl, cyclohexenyl, cycloheptenyl, cyclooctenyl),
C) optionally substituted aryl (phenyl, naphthyl),
D) heteroaryl (e.g., pyridyl, imidazolyl),
E) optionally substituted heterocycle(e.g., 4-piperidinyl, 2-pyrrolidinyl, morpholino, 2-morpholinyl, piperidino, 3,5-dimethylmorpholino, piperazinyl, N-tert-butoxycarbonyl-3-piperidinyl, 1-pyrrolidinyl, tetrahydropyranyl).

As to optionally substituted cycloalkyl of R⁶, for example, the following groups are preferable.

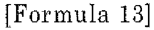

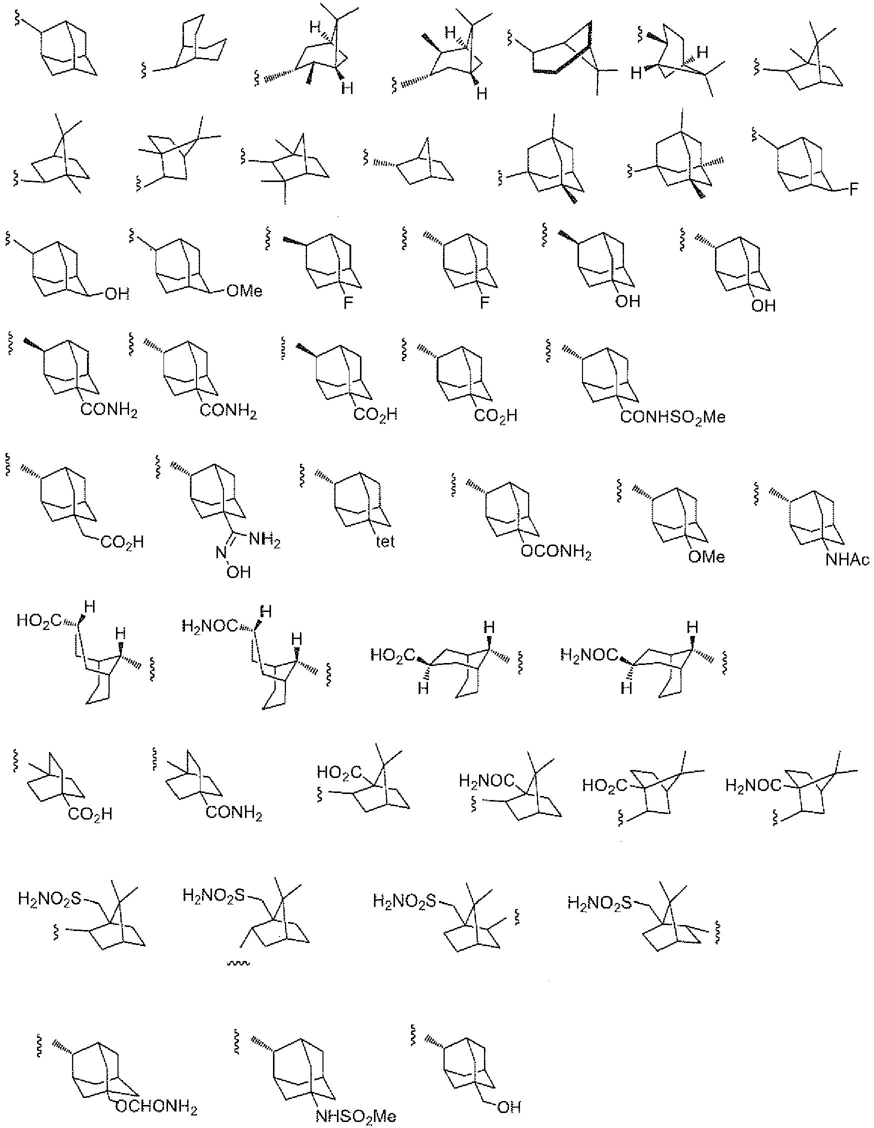

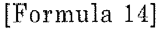

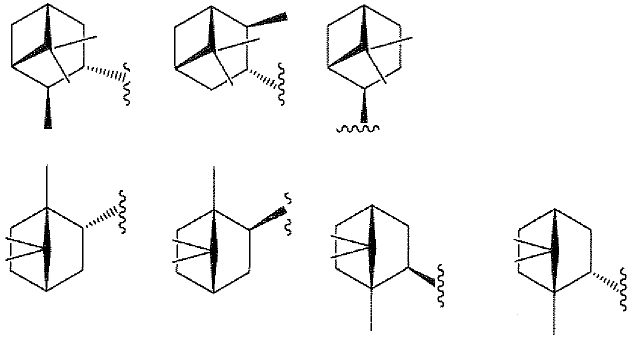

Pharmaceutically acceptable salts of the compounds of the present invention are exemplified as follows. Basic salts, for example, are salts of alkali metal such as sodium, potassium or the like; salts of alkaline-earth metal such as calcium, magnesium or the like; salts of ammonium; salts of aliphatic amine such as trimethylamine, triethylamine, dicyclohexylamine, ethanolamine, diethanolamine, triethanolamine, procaine, meglumine, diethanol amine, ethylenediamine or the like; salts of arylalkyl amine such as N,N-dibenzylethylenediamine, benetamine or the like; salts of hetero aromatic amine such as pyridine, picoline, quinoline, isoquinoline or the like; salts of quaternary ammonium such as tetramethylammonium, tetraethylammonium, benzyltrimethylammonium, benzyltriethylammonium, benzyltributylammonium, methyltrioctylammonium, tetrabutylammonium or the like; salts of basic amino acid such as arginine, lysine or the like.

Acidic salts, for example, are salts of inorganic acid such as hydrochloric acid, sulfuric acid, nitric acid, phosphoric acid, carbonic acid, hydrogencarbonic acid, perchloric acid or the like; salts of organic acid such as acetic acid, propionic acid, lactic acid, maleic acid, fumaric acid, tartaric acid, malic acid, citric acid or ascorbic acid; salts of sulfonic acid such as methansulfonic acid, isethionic acid, benzenesulfonic acid, p-toluenesulfonic acid or the like; salts of acidic amino acid such as aspartic acid, glutamic acid or the like.

Solvate means a solvate of a compound of the present invention or a pharmaceutically acceptable salt thereof, for example, alcohol(e.g., ethanol) solvate, hydrate or the like. As to hydrate, monohydrate, dihydrate or the like are exemplified.

A general method for producing a compound of the present invention is explained below. Each symbol is the same as the above (1). In addition, the treatment of the conventional organic synthesis such as extraction, purification and the like can be used for the synthesis of a compound of the present invention. 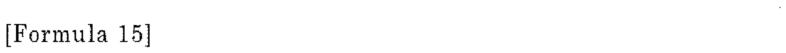 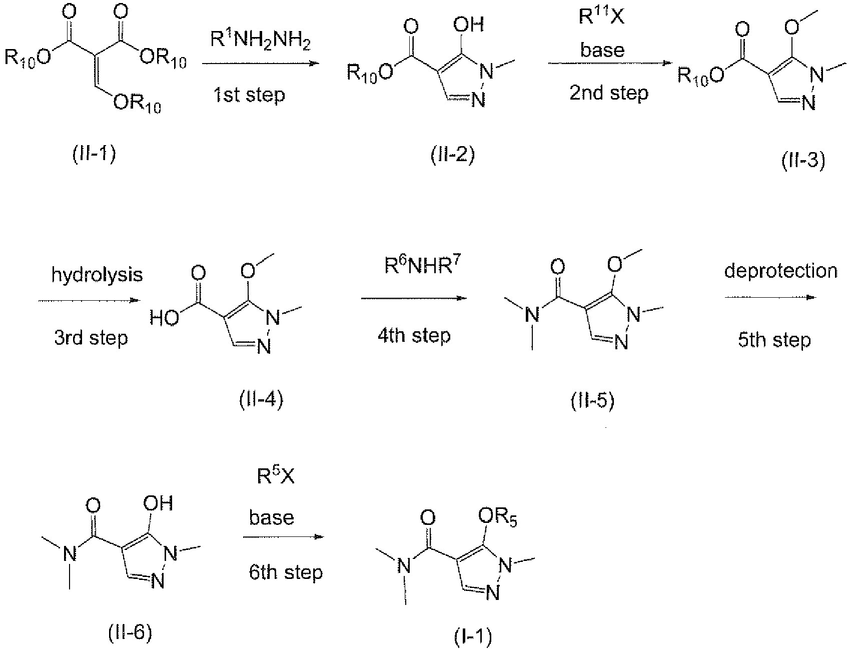 wherein R¹, R⁵ and R⁶ are the same as the above, R¹⁰ is a protecting group(e.g., alkyl or the like), R¹¹ is a protecting grouop(e.g.,benzyl or the like), and X is a leaving group (e.g., halogen or the like).

### 1^{st} step

1^{st} step is a process for manufacturing a compound of formula (II-2) which comprises reacting a compound of formula (II-1) with R¹NH₂NH₂.

R¹⁰OH can be used as a reaction solvent. This reaction can be performed at room temperature or under refluxing temperature.

### 2^{nd} step

2^{nd} step is a process of manufacturing a compound of formula (II-3) which comprises reacting a compound of formula (II-2) with R¹¹X.

Benzylhalide can be as R¹¹X.

This reaction is preferably performed in the presence of a base and can be carried out at room temperature or under refluxing temperature. Acetone, dimethylformamide or the like can be used as a reaction solvent.

### 3^{rd} step

3^{rd} step is a process of manufacturing a compound of formula (II-4) which comprises hydrolyzing a compound of formula (II-3).

This reaction can be performed in a hydrous solvent and in the presense of a base. A hydrous solvent includes hydrous alcohol, hydrous tetrahydrofuran or the like. The mixed solvent of the above can be used. Sodium hydroxide, lithium hydroxide or the like can be used as a base. This reaction can be carried out at room temperature or under refluxing temperature. The preferable reaction temperature is room temperature.

### 4^{th} step

4^{th} step is a process of manufacturing a compound of formula (II-5) which comprises reacting a compound of formula (II-4) with R⁶NHR⁷.

This reaction can be performed with the reaction condition known as the conditon used for the condensation reaction of carboxylic acid and amine. For example, a condensing agent such as 1,3-dicyclohexylcarbodiimide (DCCD), 1-(3-dimethylaminopropyl)-3-ethylcarbodiimide (WSCI) or the like can be used. 1-Hydroxybenzotriazole (HOBt), 3,4-Dihydro-hydroxy-4-oxo-1,2,3-benzotriazine(HOOBt) or the like can be used as an additive.

Dimethylformamide can be used as a solvent. This reaction can be performed at room temperature.

Additionally, after the amidation reaction with R⁶NH₂, R⁷ group can be introduced by reacting the obtained compound with R⁷X in the presence of a base. Moreover, R⁶WNHR⁷ can be used, instead of R⁶NHR⁷.

### 5^{th} step

5^{th} step is a process of manufacturing a compound of formula (II-6) which comprises deprotecting a protective group of a compound of formula (II-5).

When R¹¹ is benzyl group, the deprotection reaction can be performed by catalytic reduction.

Alcohol can be used as a solvent. This reaction can be carried out by using palladium-carbon(5~10%) as a catalyst under hydrogen atmosphere.

### 6^{th} step

6^{th} step is a process of manufacturing a compound of formula (I-1) which comprises reacting a compound of formula (II-6) with R⁵X.

This reaction can be performed in the presence of a base. Potassium carbonate, sodium carbonate, sodium hydroxide, lithium hydroxide or the like can be used as a base.

This reaction can be carried out at room temperature or under refluxing temperature. Dimethylformamide can be used as a solvent.

A compound of the present invention wherein X is =CR⁸- can be synthesized by using a compound having a pyrrole ring instead of a pyrazole ring described in the above formula (II-2) in accordance with the above explained scheme.

Various substituent of a compound of the present invention can be introduced referring to (1) Alan R. Katriszly et al., Comprehensive Heterocyclic Chemistry, (2) Alan R. Katriszly et al., Comprehensive Heterocyclic Chemistry II, (3) RODD'S CHEMISTRY OF CARBON COMPOUNDS VOLUME IV HETEROCYCLIC COMPOUNDS, or the like.

A compound of the present invention has a high inhibitory activity to 11β hydroxysteroid dehydrogenase type 1. Therefore, a compound of the present invention can be used for treating and/or preventing a disease concerning 11 β hydroxysteroid dehydrogenase type 1, especially, hyperlipidemia, diabetes, obesity, arteriosclerosis, atherosclerosis, hyperglycemia and/or syndrome X. Especially, a compound of the present invention is useful for treating and/or preventing diabetes.

A compound of the present invention can be administrated via oral or parenteral. When the present compound is administrated via oral, the present compound can be used for in any form of the conventional pharmaceutical formulations, for example, solid formulations such as tablets, powders, granules, capsules or the like; aqueous formulations; oleaginous suspensions; or solution formulations such as syrup or elixir. When the present compound is administrated via parenteral, the present compound can be used as an aqueous or oleaginous suspensions injection or nose droops. In the preparation of such formulations, the conventional pharmaceutical excipients, binding agents, lubricants, aqueous solvents, oleaginous solvents, emulsifying agents, suspending agents, preservatives, stabilizers, and the like can be optionally used. Especially, a compound of the present invention is preferably used as oral agents.

A formulation according to the present invention can be manufactured by combining (e.g., admixing) a curatively effective amount of a compound of the present invention with a pharmaceutically acceptable carrier or diluent. The formulation can be manufactured by using of well-known and easily available ingredients in accordance with a known method.

A dosage of a compound of the present invention depends on the administration route, age, body weight, conditions of the patient, and kind of disease, but in case of oral administration, the daily dosage for an adult can be between approximately 0.05mg~3000mg, preferably approximately 0.1 mg~1000mg. The daily dosage can be administered in divisions. When a compound of the present invention is administrated via parenteral, the daily dosage for an adult can be between approximately 0.01mg~1000mg, preferably approximately 0.05mg~500mg. Moreover, a compound of the present invention can be administrated with other curative agents.

Examples are show below for further detail explanation of the present invention, but are not intended to limit the scope of the present invention.

### Example 1

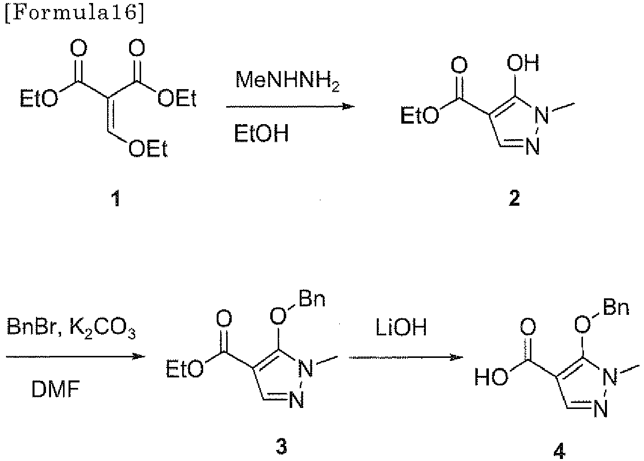

To a solution of Compound **1**(50.0g) in ethanol was added methylhydrazine(13.5ml) dropwisely under ice-cooloing, then the reaction solution was stirred at room temperature for one hour and refluxed for 4 hours. The solvent was removed under reduced pressure to give a solid. The solid was washed with hexane to give Compound **2**(34.2g).

To a solution of Compound **2**(20.0g) in dimethyl formamide(200ml) were added potassium carbonate(48.7g) and benzylbromide(15.4ml), then the resulting mixture was stirred at room temperature for 4hrs. The insoluble was removed by filtration and the filtrate was poured into a solution of ethyl acetate and 0.1N HCl aquesous soln. and extracted with ethyl acetate. The extraction was washed with 0.1N HCl aqueous soln., H₂O and brine, successively, then dried with sodium sulfate and concentrated *in vacuo*. The residue was purified by silicagel columnchromatography to give Compound **3**(24.0g).

To a solution of Compound **3**(24.0g) in methanol(150ml)-tetrahydrofuran(30ml)-H₂O(130ml) was added 4N lithium hydroxide aqueous soln.(100ml) under ice-cooling. The resulting solution was stirred at room temperature for 30 min. and at 60 °C for 3hrs. The solution was neutralized with 2N HCl aqueous soln. under ice-cooling and extracted with ethyl acetate. The extraction was washed with H₂O, brine and dried with magnesium sulfate, and concentrated *in vacuo* to give Compound **4**(18.9g) as a crystal.

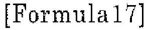

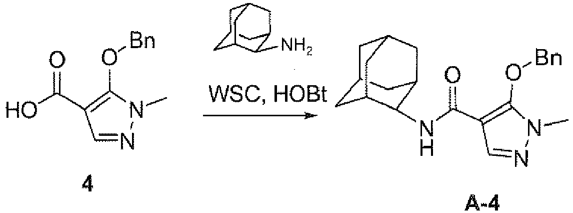

To a solution of Compound **4**(2.32g), 2-aminoadamantane hydrochloride(2.25g) and 1-hydroxybenztriazole (405mg) in dimethyl formamide(25ml) were added triethylamine (3.35ml) and 1-(3-dimethylaminopropyl)-3-ethylcarbodiimide hydrochloride (2.30 g) successively, then the resulting mixture was stirred at room temperature overnight. The reaction mixture was poured into a solution of 0.1N HCl aqueous soln. and ethyl acetate and extracted with ethyl acetate. The extraction was washed with H₂O, brine and dried with magnesium sulfate and concentrated *in vacuo*. The residue was purified by silicagel columnchromatography to give Compound **A-4**(3.12g).

### Example 2

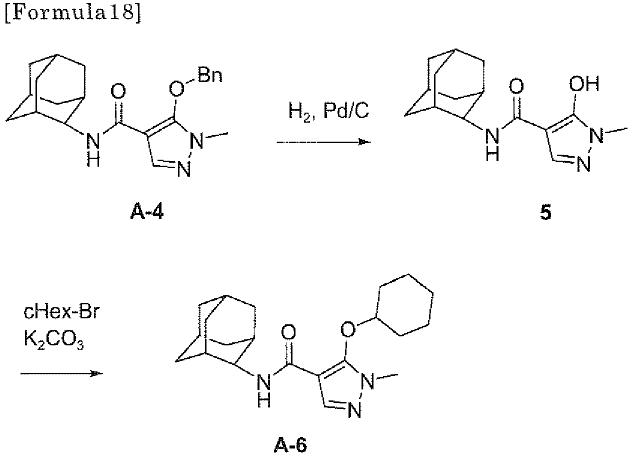

To a solution of Compound **A-4**(1.00g) in ethanol(10ml) was added 5%Pd-C(174mg), then the resulting mixture was stirred under H₂ atomosphere(latm) for four hours. The insoluble was removed by filtration using Celite, then the filtrate was concentrated *in vacuo* to give Compound 5(711mg) as a solid.

To a solution of Compound **5**(110mg) in dimethyl formamide(1.5ml) were added potassium carbonate(165mg) and bromocyclohexane(59µl), then the resulting solution was stirred at 150 °C for 1.5hrs by using microwave. The reaction solution was poured into a solution of 0.1N HCl aquesous soln. and ethyl acetate and extracted with ethyl acetate. The extraction was washed with H₂O, brine and dried with magnesium sulfate and concentrated *in vacuo*. The residue was purified by silicagel columnchromatography to give Compound **A-6**(16mg).

### Example 3

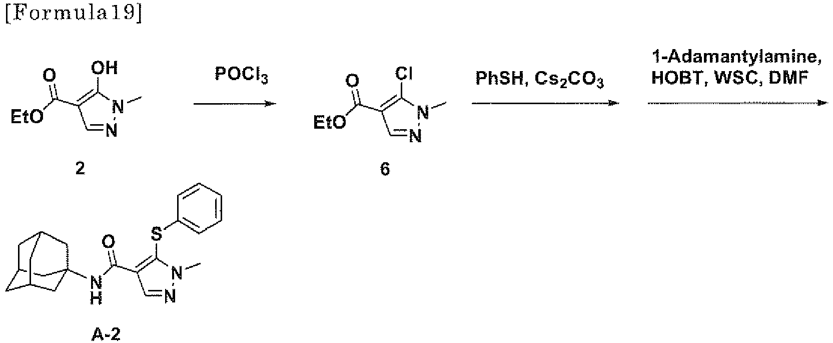

The reaction of Compound **2** with phosphorus oxychloride gave Compound **6**. The reaction of Compound **6** with thiophenol in the presence of cesium carbonate gave Compound **A-2**.

### Example 4

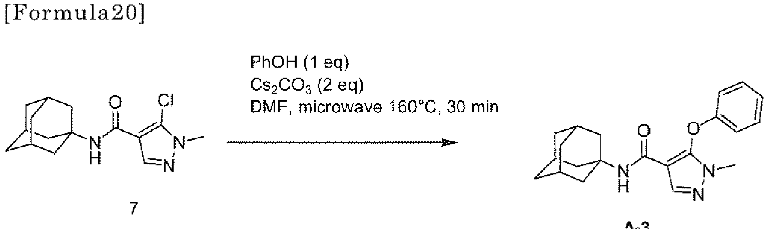

Compound **A-3** was obtained from the reaction with phenol instead of thiophenol described in Example 3.

### Example 5

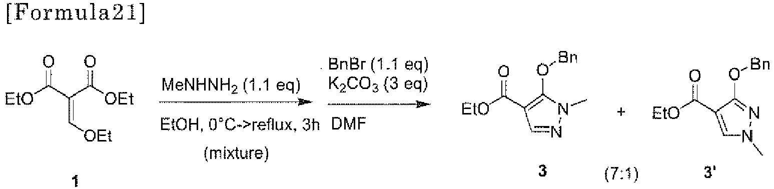

Compound **3** and the regioisomer 3' were synthesized from the same method described in Example 1.

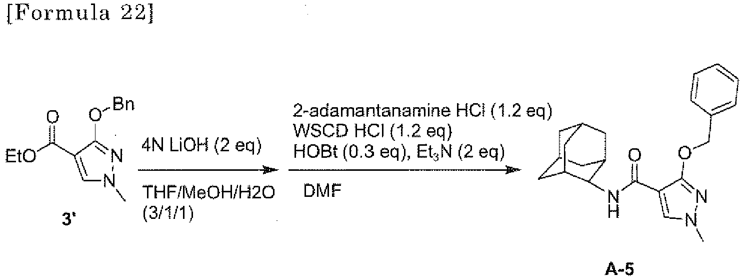

Compound **A-5** was synthesized by using Compound 3'.

### Example 6

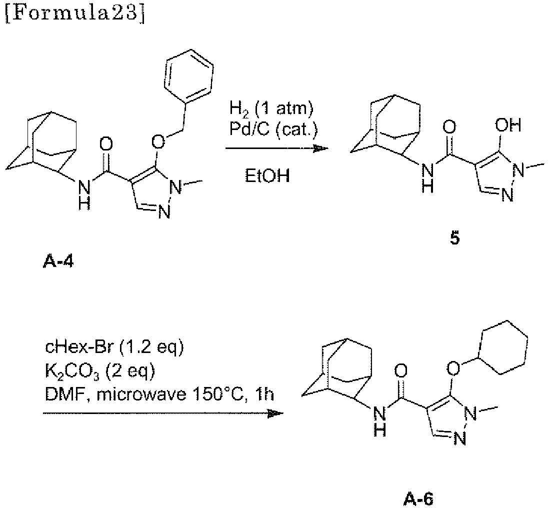

A reaction of catalytic reduction of Compound **A-4** gave Compound **5**. The obtained compound was reacted with cyclohexylbromide in the presence of potassium carbonate to afford Compound **A-6**.

### Example 7

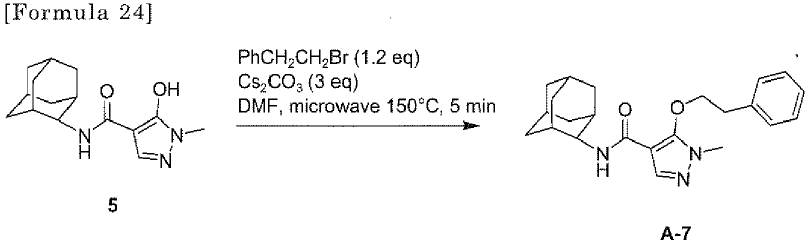

Compound 5 obtained in Example 6 was reacted with phenethylbromide to give Compound **A-7**. Additionally, a compound of the present invention can be obtained by using various halides as well as the halides shown in Examples 6 and 7. Compounds **C-36~38**, **41** were synthesized in accordance with the method shown in the above Examples.

### Example 8

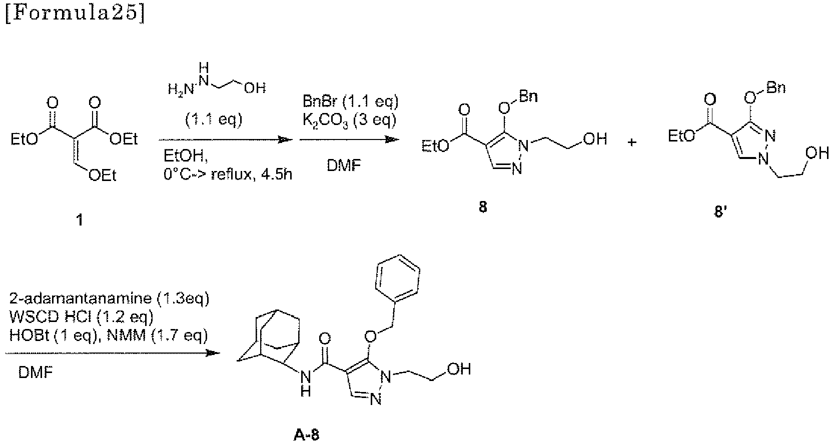

Compounds **8** and **8'** were synthesized by using Compound **1** as a starting material and HOCH₂CH₂NHNH₂ instead of MeNHNH₂ in Example 1. The obtained 8 was reacted with 2-adamantanamine to give Compound **A-8**.

### Example 9

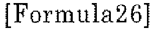

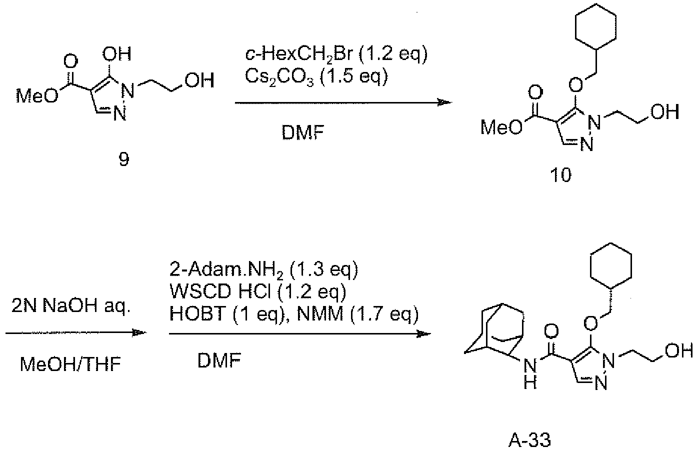

According to the above scheme, Compound **A-33** was prepared. Additionally, Compound **C-70** was synthesized by using hydroxy adamantanamine instead of adamantanamine.

### Example 10

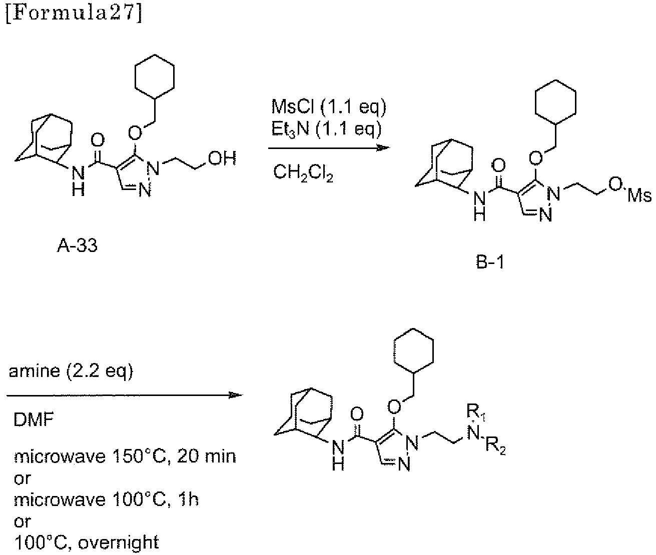

The obtained **A-33** was reacted with mesyl chloride in the presence of triethylamine to give Compound **B-1.** Compound **B-1** was reacted with various amines to afford Compounds **A-40, A-41, A-42, A-44, A-45** and **A-46.** Moreover, Compounds **C-1, 2, 12 to 28, 51 to 53, 84, 101, 102, 108** to **110** were synthesized from Compound **A-33.** Moreover, Compounds **C-3** to **6,** 11 were synthesized from Compound **A-44.**

### Example 11

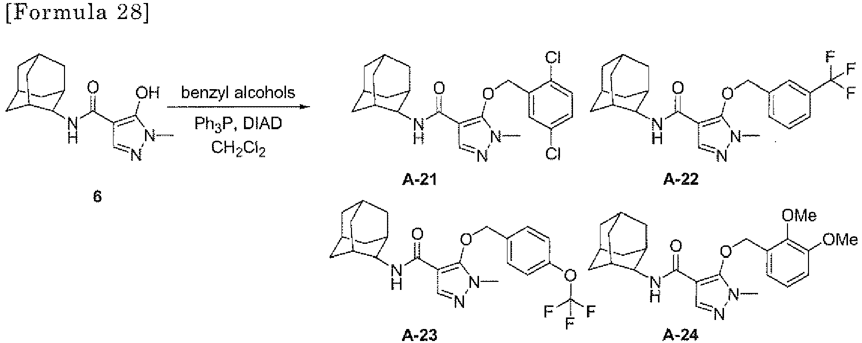

Compound **6** was reacted with various benzylalcohols to give Compounds **A-21, A-22, A-23** and **A-24** via Mitsunobu reaction.

### Example 12

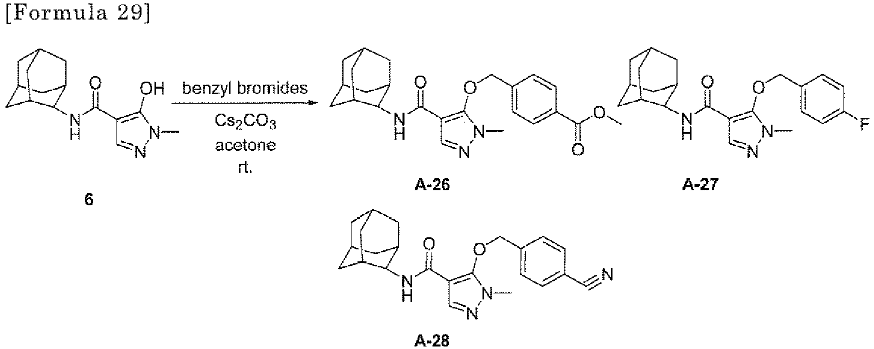

Compound **6** was reacted with various benzylbromides in the presence of cesium carbonate to give Compounds **A-26, A-27** and **A-28**.

### Example 13

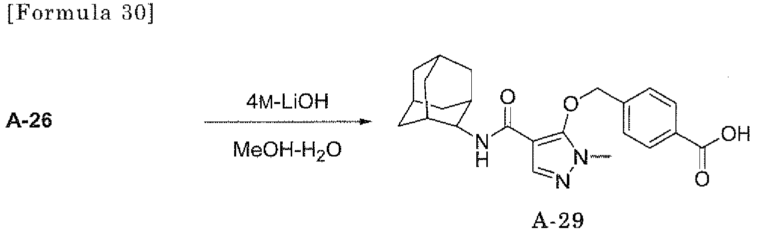

Compound **A-26** was hydrolyzed by alkali to afford Compound **A-29**.

### Example 14

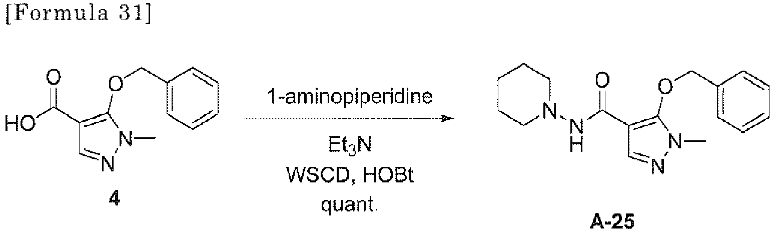

Compound 4 was reacted with 1-aminopiperidine to give Compound **A-25**. Compounds **A-13, A-14, A-19, A-20, A-29, A-34** and **A-35** were synthesized in accordance with the above Example.

### Example 15

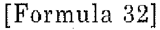

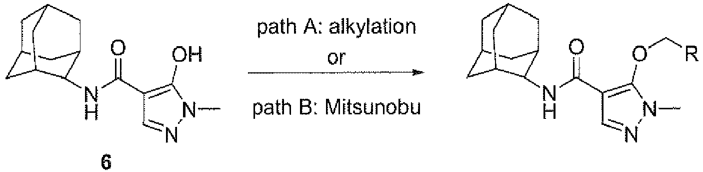

Compounds **A-7, A-13, A-14, A-19, A-20, A-22, A-23, A-24, A-26, A-27**, **A-28**, **A-29**, **A-34**, **A-35**, **A-51**, **A-52** and **A-59** to **65** were synthesized from Compound **6** via Mitsunobu reaction or alkylation reaction.

### Example 16

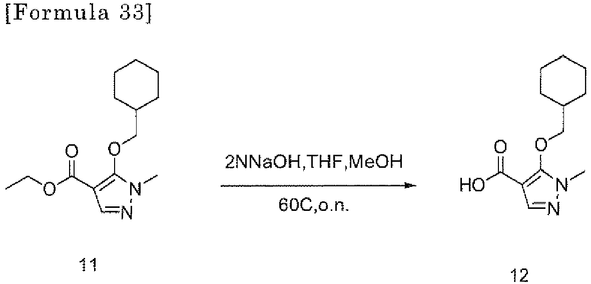

Compound **11** was reacted with sodium hydroxide to give Compound **12**.

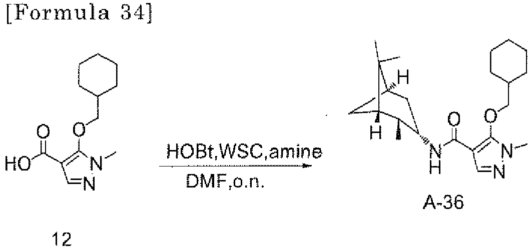

Compound **12** was reacted with various amines in the presence of HOBt and WSC to afford Compound **A-36**. Compounds **A-37**, **A-38**, **A-47** to **50**, **A-53**, **A-55**, **A-57**, **A-58**, **A-66**, **A-67**, **C-7** to **10**, **45** and **54** to **58** were synthesized as well as the above Example.

### Example 17

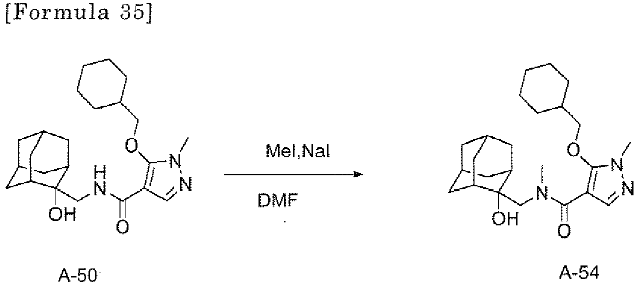

Compound **A-50** was reacted with methyliodide and sodiumiodide to give Compounds **A-54** and **A-55**. According to the above method, Compounds **A-47** to **49, 57, 58, 66** and 67 were synthesized.

### Example 18

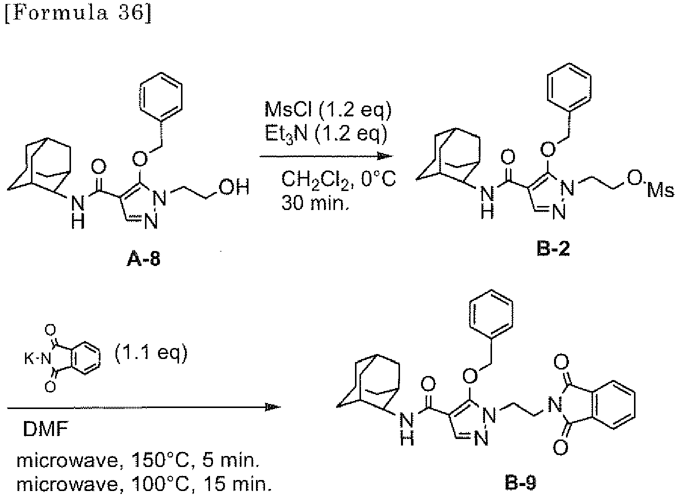

Compound **A-8** was reacted with mesylchloride in the presence of triethylamine to give Compound **B-2.** Compound **B-2** was reacted with potassium phthalimide to afford Compound **B-9**.

### Example 19

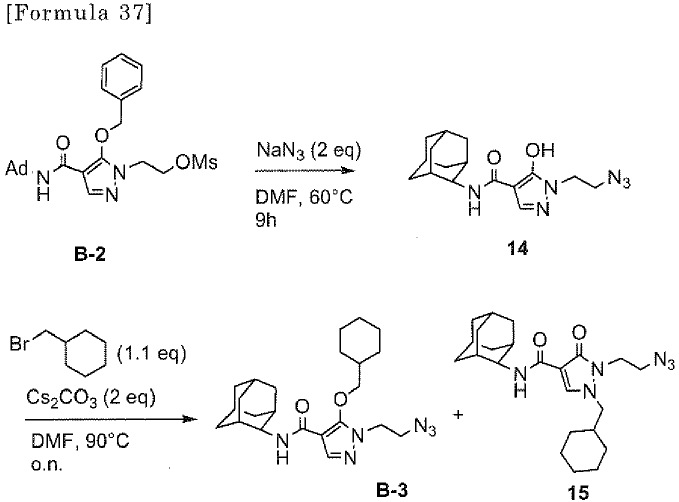

Compound **B-2** was reacted with sodium azide to give Compound **14**. Compound **14** was reacted with cyclohexylmethylbromide to afford Compound **B-3** and Compound **15**.

### Example 20

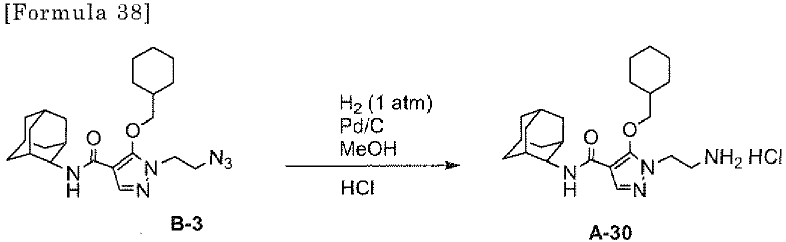

The catalytic reduction of **B-3**, followed by treatment with hydrochloric acid to give Compound A-30(HCl salt). The amidation reaction of the obtained product gave Compounds **C-61** and **62**.

### Example 21

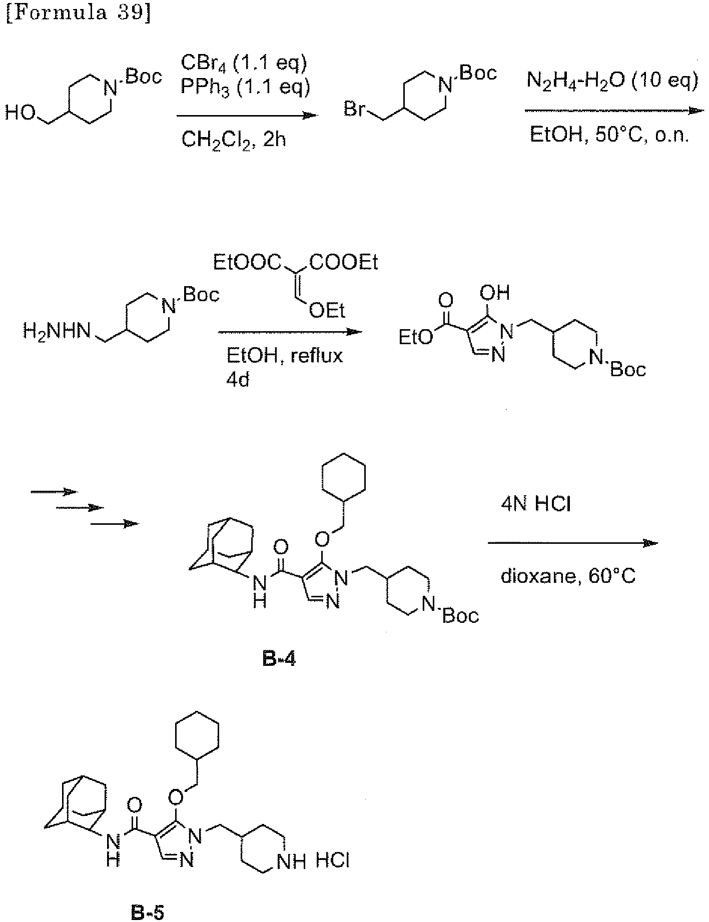

### Example 22

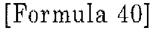

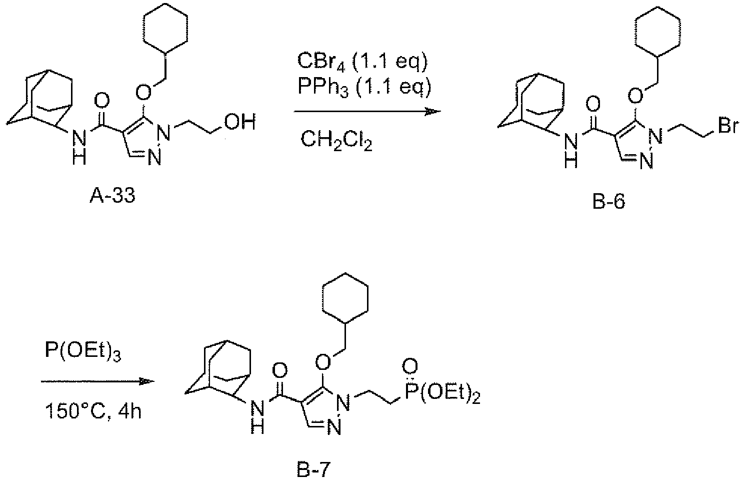

According to the above scheme, Compounds **B-6** and **B-7** were synthesized from Compound **A-33**.

### Example 23

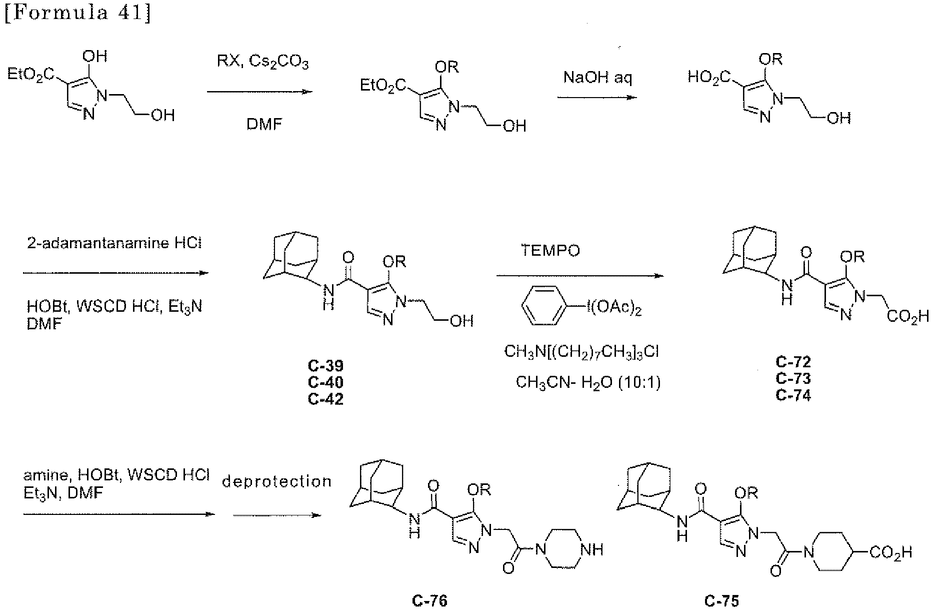

According to the above scheme, Compounds **C-39, 40, 42, 72, 73, 74, 75** and **76** were synthesized. Compounds **C-79, 81,** 151 to **153** were synthesized as well as the above Example.

### Example 24

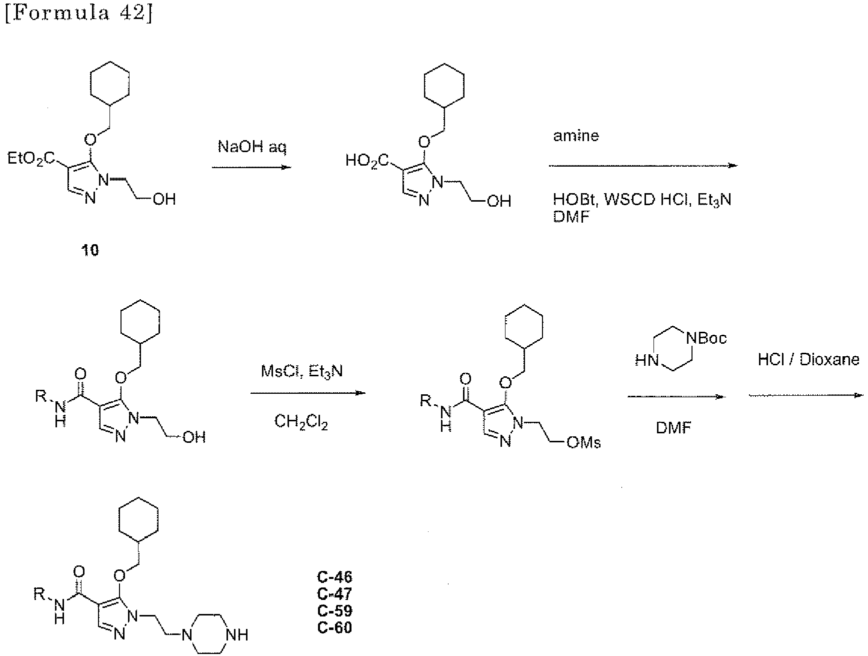

According to the above scheme, Compounds **C-46, 47, 59** and **60** were synthesized.

### Example 25

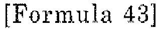

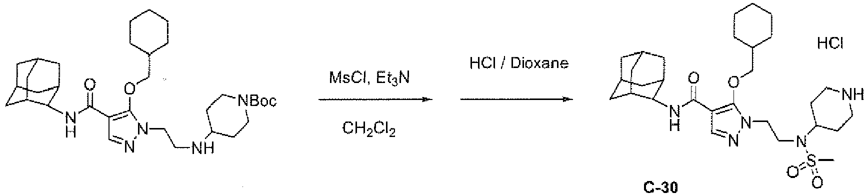

According to the above scheme, Compound **C-30** was synthesized.

### Example 26

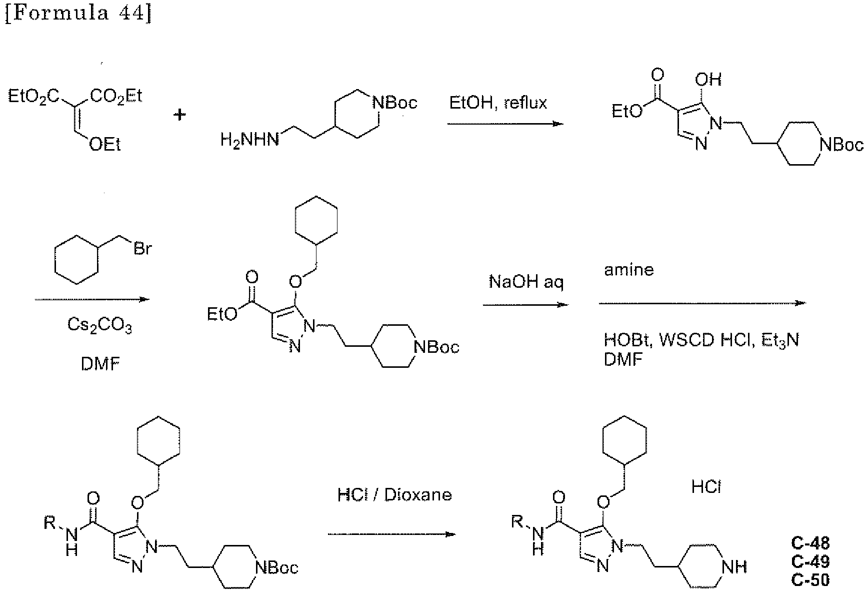

According to the above scheme, Compounds **C-48**, **49** and **50** were synthesized. Compounds **C-126** to **128** were synthesized as well as the above Example.

### Example 27

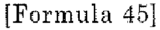

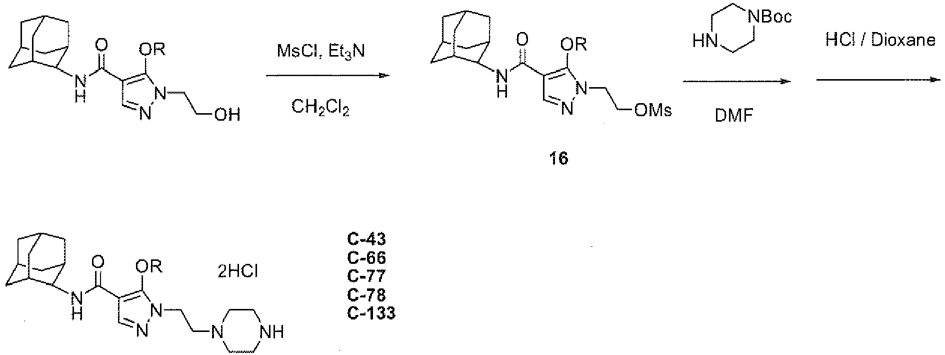

According to the above scheme, Compounds **C-43, 66, 77, 78** and **133** were synthesized.

### Example 28

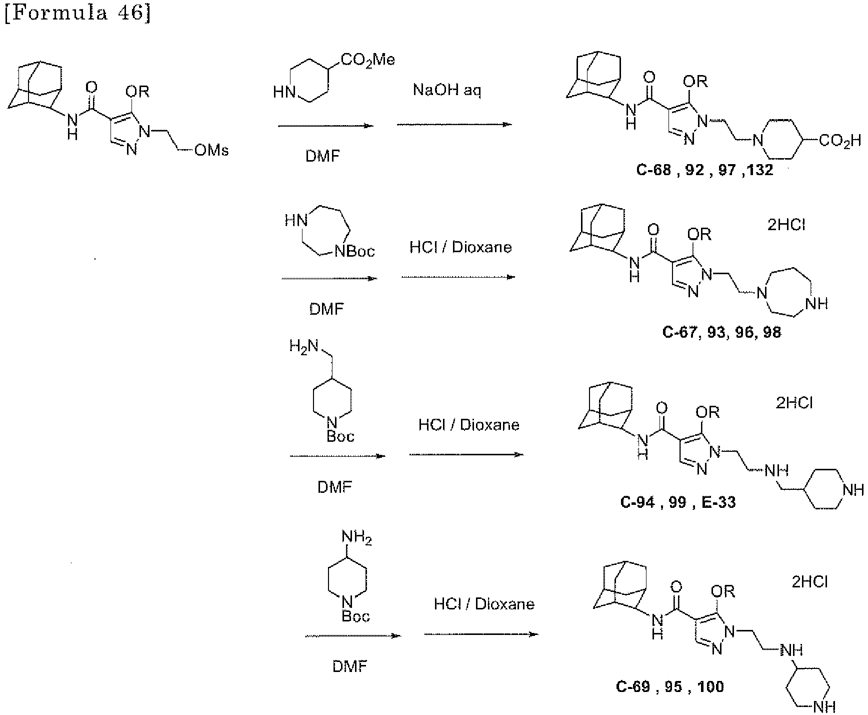

According to the above scheme, Compounds **C-67, 68, 69, 92, 93, 94, 95**, **96, 97, 98, 99, 100, 132** and **E-33** were synthesized.

### Example 29

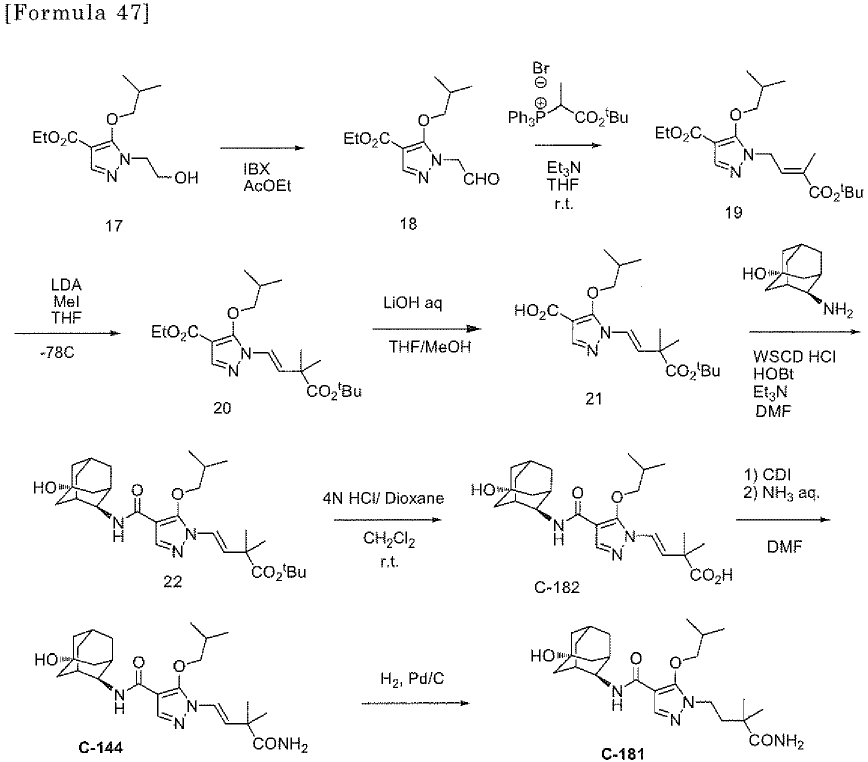

To a solution of Compound 17(5.7g) in ethyl acetate(160ml) was added IBX(7.5g), then the resulting mixture was refluxed for 6hrs. After termination of the reaction, the insoluble was removed by filtration and the filtrate was concentrated to give Compound **18**(5.6g). The obtained product was used for the next reaction without further purification.

To a solution of Compound **18** in tetrahydrofuran(40ml) was added phosphonium salt(13.5g). Triethylamine(3.4g) was added dropwisely to the solution over 20 min, then the whole mixture was stirred at room temperature for 3 hrs. After termination of the reaction, H₂O(40ml) was added to the mixture, then extracted with AcOEt. The organic layer was washed with brine and dried with magnesium sulfate and concentrated. The residue was purified by silicagel columnchromatography to give Compound 19(5.2g).

A solution of diisopropylamine(1.3ml) in tetrahydrofuran(60ml) was cooled to -78 °C, then n- butyllithium(3.25ml, 2.8M in hexane) was added dropwisely to the solution. After stirring at -78 °C for 30min, Compound 19(2.8g) in tetrahydrofuran(40ml) was added to the solution and the whole mixture was stirred for 30min. Iodomethane(1.4ml) was added to the mixture, then the whole mixture was allowed to gradually warm up to 0 °C. After 3hrs, the mixture was diluted with sat. ammonium chloride aqueous soln. and extracted with ethyl acetate. The organic layer was washed with brine, dried with magnesium sulfate and concentrated. The residue was purified by silicagel columnchromatography to give Compound 20(2.42g).

To a solution of Compound 20(173mg) in tetrahydrofuran(4ml). methanol(2ml) was added 4N lithium hydroxide aqueous soln.(0.9ml), then the resulting solution was stirred at room temperature for 24hrs. After termination of the reaction, the solution was acidified with 2N HCl aqueous soln. and extracted with ethyl acetate. The organic layer was washed with brine and dried with magnesium sulfate and concentrated to give Compound 21(181mg). The obtained product was used for the next reaction without further purification.

To a solution of Compound 21(181mg) in dimethyl formamide were added hydroxy adamantanamine(94mg), 1-(3-dimethylaminopropyl)-3-ethylcarbodiimide hydrochloride(118mg), 1-hydroxybenzotriazole(21mg) and triethylamine(121µl), then the resulting solution was stirred at room temperature for 24hrs. After termination of the reaction, the solution was diluted with 2N HCl aqueous soln. and extracted with ethyl acetate. The organic layer was washed with sat. sodium hydrogencarbonate soln. and brine successively, and dried with magnesium sulfate. The residue was purified by silicagel columnchromatography to give Compound **22**(176mg). To a solution of Compound **22**(176mg) in methylene chloride(3ml) was added 4N HCl/dioxane(2ml), then the resulting solution was stirred at room temperature for 26hrs. After termination of the reaction, the solution was diluted with diisopropylether to give crystal. The obtained crystal was collected by filtration and washed with diisopropylether, then dried to give Compound **C-182**(120mg).

To a solution of Compound **C-182**(55mg) in dimethyl formamide(1ml) was added 1,1'-carbonyldiimidazole(30mg), then the resulting solution was stirred at room temperature for 45min. 28% Ammonia aqueous soln.(0.2ml) was added to the solution and the whole solution was stirred for 1.5hrs. After termination of the reaction, the solution was diluted with water and extracted with ethyl acetate. The organic layer was washed with 0.1N HCl aqueous soln. and brine successively, and dried with magnesium sulfate and concentrated. The obtained crystal was washed with diisopropylether to afford Compound **C-144**(35mg).

To a solution of Compound **C-244**(118mg) in tetrahydrofuran(4.4ml)-methanol(0.4ml) was added 10% Pd-C(40mg), then the resulting mixture was stirred for 3.5hrs. under H₂ atomosphere. After termination of the reaction, Pd-C was removed by filtration and the solvent was removed to give Compound **C-181**(117mg).

Compounds **C-147**, **160**, **163**, **187** and **195** were synthesized as well as the above Example.

### Example 30

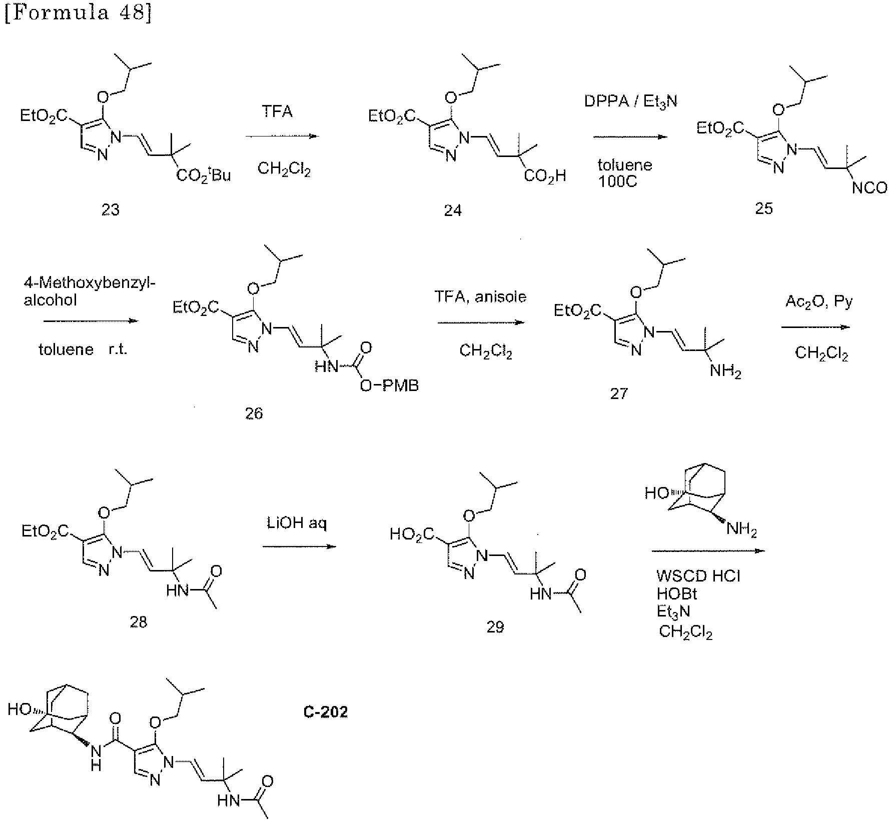

To a solution of Compound **23** in methylene chloride(100ml) was added trifluoroacetic acid(50ml), the resulting solution was stirred at room temperature for 3hrs. After termination of the reaction, the solvent was removed and the residue was diluted with H₂O(100ml) and extracted with ethyl acetate. The organic layer was washed with brine and dried with magnesium sulfate and concentrated to give Compound **24**(16.6g).

To a solution of Compound **24**(16.6g) in toluene(70ml) were added triethylamine(5.67g), diphenylphosphoryl azide (14.7g), then the resulting solution was stirred at 100 °C for 3hrs. After termination of the reaction, the solution was diluted with toluene(70ml) and the organic layer was washed with sat. sodium hydrogencarbonate soln. and brine successively, and dried with magnesium sulfate and concentrated. The obtained product was used for the next reaction without further purification.

According to the above procedure, the obtained 25 was dissolved in toluene(30ml) and 4-methoxybenzylalcohol(10.6g) was added to the solution. The solution was stirred at 50 °C for 24hrs. After termination of the reaction, the solvent was removed and the residue was purified by silicagel columnchromatography to give Compound **26**(19.2g).

To a solution of Compound **26** in methylene chloride(100ml) were added anisole(13.6g) and trifluoroacetic acid(20ml), then the resulting solution was stirred at room temperature for 2hrs. After termination of the reaction, the solvent was removed and the residue was diluted with 1N HCl aqueous soln. (50ml) and H₂O(60ml). The aqueous layer was washed with hexane and alkalified with 2N NaOH aqueous soln.(30ml). The solution was extracted with ethyl acetate and the organic layer was washed with brine and dried with magnesium sulfate and concentrated to give Compound **27**(9.6g).

To a solution of Compound **27**(9.6g) in methylene chloride(50ml) were added pyridine(3.9g) and acetic anhydride(4.0g), then the resulting solution was stirred at room temperature for 2hrs. After termination of the reaction, the solution was diluted with chloroform and the organic layer was washed with 2N HCl aqueous soln., sat. sodium hydrogencarbonate soln. and brine successively. The organic layer was dried with magnesium sulfate and concentrated. The residue was purified by silicagel columnchromatography to give Compound **28**(7.1g).

To a solution of Compound **28**(7.1g) in tetrahydrofuran(20ml)-methanol(20ml) was added 2N LiOH aqueous soln.(21ml), then the resulting solution was stirred at room temperature for 16hrs. After termination of the reaction, the solution was acidified with 2N HCl aqueous soln. and extracted with ethyl acetate. The organic layer was washed with brine and dried with magnesium sulfate and concentrated to give Compound **29**(6.3g).

To a solution of Compound **29**(102mg) in methylene chloride(2ml) were added hydroxy adamantanamine(81mg), 1-(3-dimethylaminopropyl)-3-ethylcarbodiimide hydrochloride(82mg), 1-hydroxybenzotriazole(14mg) and triethylamine(115µl), then the resulting solution was stirred at room temperature for 13hrs. After termination of the reaction, the solution was acidified with 2N HCl aqueous soln. and extracted with methylene chloride. The organic layer was washed with sat. sodium hydrogencarbonate soln. and brine successively, and dried with sodium sulfate. The residue was purified by silicagel columnchromatography to afford Compound **C-202**(123mg).

Compounds **C-194** and **204** were synthesized as well as the above Example.

### Example 31

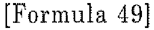

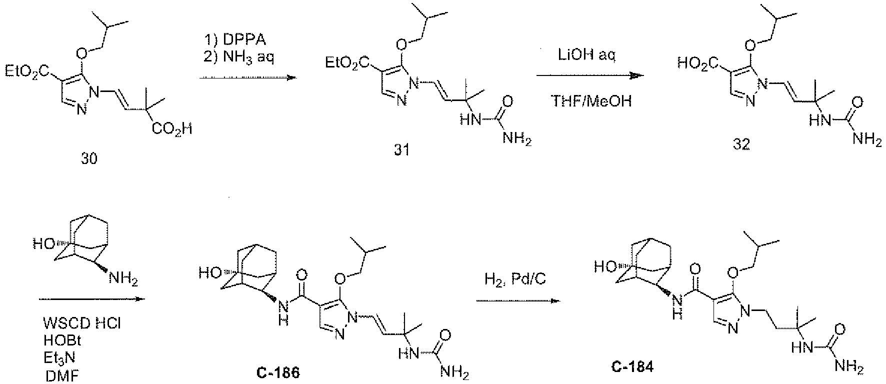

To a solution of Compound **30**(400mg) in toluene(8ml) were added triethylamine(180µl) and diphenylphosphoryl azide(279µl), then the resulting solution was stirred at 100 °C for 2hrs. After cooling to 0 °C, 28% ammonia aqueous soln.(2ml) was added to the solution and the whole solution was stirred at room temperature for 80min. After termination of the reaction, H₂O was added to the solution and extracted with ethyl acetate. The organic layer was washed with sat. sodium hydrogencarbonate soln. and brine, and dried with sodium sulfate and concentrated. The residue was purified by silicagel columnchromatography to give Compound 31(303mg).

To a solution of Compound 31(303mg) in tetrahydrofuran(3ml)-methanol(1.5ml) was added 2N LiOH aqueous soln.(0.68ml), then the resulting solution was stirred at room temperature for 19hrs. After termination of the reaction, the solution was diluted with H₂O and the organic layer was washed with diethylether. The mixture was acidified with 2N HCl aqueous soln., and extracted with ethyl acetate-tetrahydrofuran. The organic layer was washed with brine and dried with sodium sulfate and concentrated. The obtained crystal was washed with ethyl acetate-hexane to afford Compound **32**(193mg).

To a solution of Compound **32**(193mg) in dimethyl formamide(4ml) were added hydroxy adamantanamine(152mg), 1-(3-dimethylaminopropyl)-3-ethylcarbodiimide hydrochloride(155mg), 1-hydroxybenzotriazole(26mg) and triethylamine(217µl), then the resulting solution was stirred at room temperature for 18hrs. After termination of the reaction, the solution was acidified with 2N HCl aqueous soln. and extracted with methylene chloride. The organic layer was washed with sat. sodium hydrogencarbonate soln. and brine successively, and dried with sodium sulfate. The residue was purified by silicagel columnchromatography to give Compound **C-186**(36mg).

To a solution of Compound **C-186**(25mg) in tetrahydrofuran(lml)-methanol(0.1ml) was added 10%Pd-C(12mg), then the resulting mixture was stirred for 24hrs under H₂ atmosphere. After termination of the reaction, Pd-C was removed by filtration and the solvent was removed to afford Compound **C-184**(25mg).

Compounds **C-183**, **185**, **198** and **199** were synthesized as well as the above Example.

### Example 32

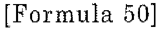

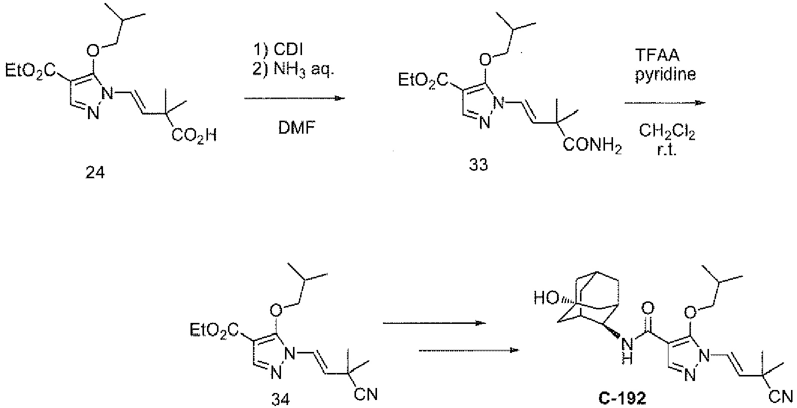

According to Example 29, Compound 33 was synthesized from Compound **24**. To a solution of Compound 33(149mg) in methylene chloride(3ml) were added pyridine(74µl) and anhydrous trifluoroacetic acid(98µl), then the resulting solution was stirred at room temperature for 45min. After termination of the reaction, HCl aqueous soln. was added to the solution. The mixture was extracted with ethyl acetate and the organic layer was washed with sat. sodium hydrogencarbonate soln. and brine successively, and dried with sodium sulfate and concentrated to give Compound **34**(141mg).

According to the above procedure, Compound **C-192** was synthesized.

### Example 33

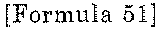

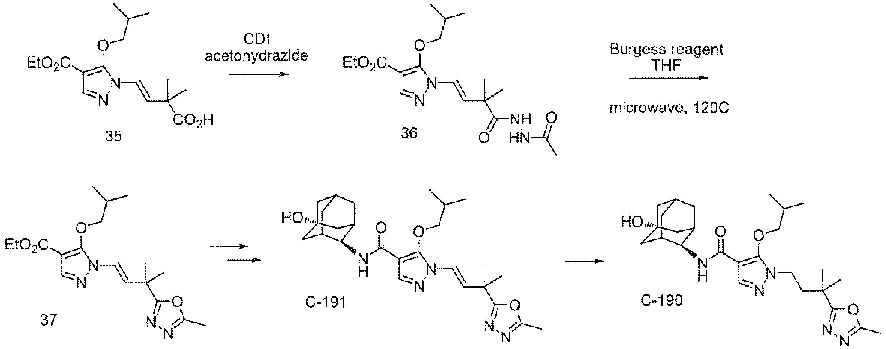

To a solution of Compound **35** in methylene chloride(4ml) was added 1,1'-carbonyldiimidazole(200mg), then the resulting solution was stirred at room temperature for 2hrs and cooled to 0 °C. Acetohydrazide(69mg) in methylene chloride(2ml) was added to the solution and the whole mixture was stirred at room temperature for 2hrs. After termination of the reaction, HCl aqueous soln. was added to the mixture. The extraction was carried with ethyl acetate and the organic layer was washed with sat. sodium hydrogencarbonate soln. and brine, successively and dried with sodium sulfate. The solvent was removed to give a crystal. The obtained crystal was washed with hexane to give Compound **36**(190mg).

To a solution of Compound **36**(140mg) in tetrahydrofuran(2.8ml) was added Burgess Reagent(259mg), then the resulting solution was stirred at 120 °C for 15min. by using microwave. After termination of the reaction, the solvent was removed and the residue was purified by silicagel columnchromatography to afford Compound 37(126mg).

According to the above procedure, Compounds **C-190** and **191** were synthesized.

### Example 34

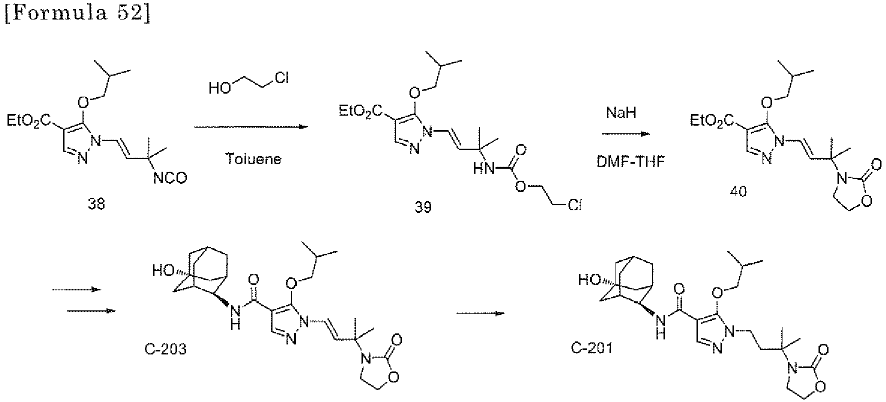

To a solution of Compound **38** in toluene(2ml) were added 2-chloroethanol(103µl), triethylamine(2 drops), then the resulting solution was stirred at room temperature for 24hrs. After termination of the reaction, the solvent was removed and the residue was purified by silicagel columnchromatography to give Compound **39**(215mg).

To a solution of Compound **39**(211mg) in tetrahydrofuran(4ml)-dimethyl formamide(4ml) was added sodium hydride(32mg, 60% oil suspension), then the resulting solution was stirred at room temperature for 140min. After termination of the reaction, HCl aqueous soln. was added to the solution. The extraction was carried out with ethyl acetate and the organic layer was washed with brine and dried with sodium sulfate and concentrated. The residue was purified by silicagel columnchromatography to give Compound **40**(191mg).

According to the above procedure, Compounds **C-201** and **203** were synthesized.

### Example 35

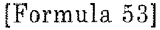

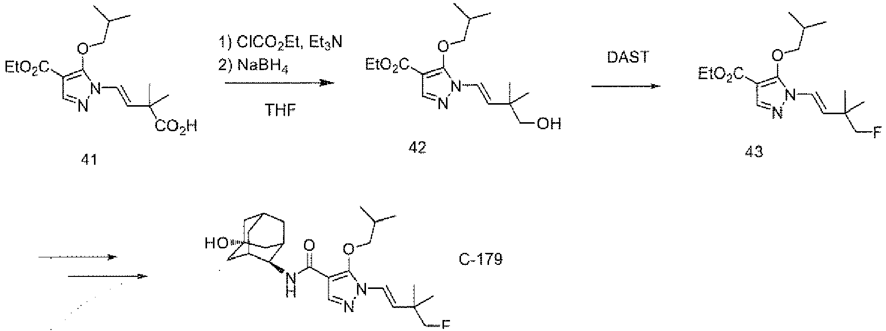

To a solution of Compound **41**(237mg) in tetrahydrofuran(3ml) were added triethylamine(152µl) and ethyl chlorocarbonate(84µl) at 0 °C, then the resulting solution was stirred at room temperature for lhr. Sodium borohydride(69mg) and H₂O(1ml) were added to the solution at 0 °C and the whole mixture was stirred for 20min. After termination of the reaction, HCl aqueous soln. was added to the mixture. The extraction was carried out with ethyl acetate and the organic layer was washed with brine and dried with sodium sulfate and concentrated. The residue was purified by silicagel columnchromatography to afford **42**(185mg).

To a solution of **42**(185mg) in methylene chloride(5ml) was added DAST(102µl) at -78 °C, then the resulting solution was stirred at the same temperature for 30min. After termination of the reaction, sat.ammonium chloride soln. was added to the solution. The extraction was carried out with ethyl acetate and the organic layer was dried with sodium sulfate and concentrated. The residue was purified by silicagel columnchromatography to afford Compound **43**(62mg).

According to the above procedure, Compound **C-179** was synthesized.

### Example 36

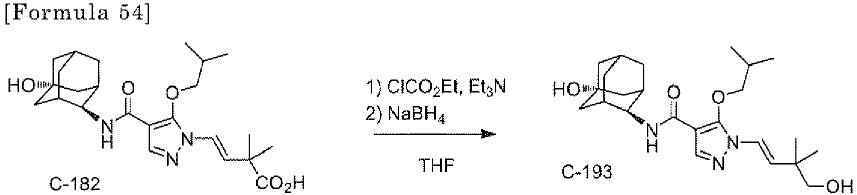

According to the above procedure, the reduction of carboxylic acid, Compound **C-182** gave Compound **C-193.**

Compound **C-162** was synthesized as well as the above Example. According to Example 29, Compound **C-164** was synthesized from Compound **C-162.**

### Example 37

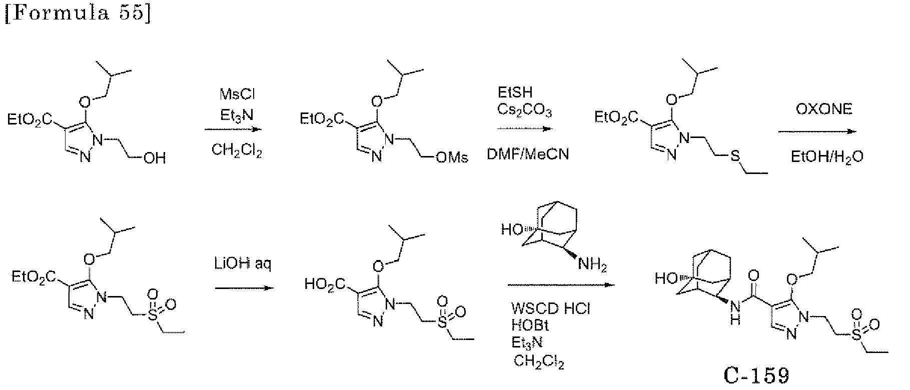

According to the above scheme, Compound C-159 was synthesized.

### Example 38

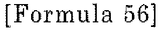

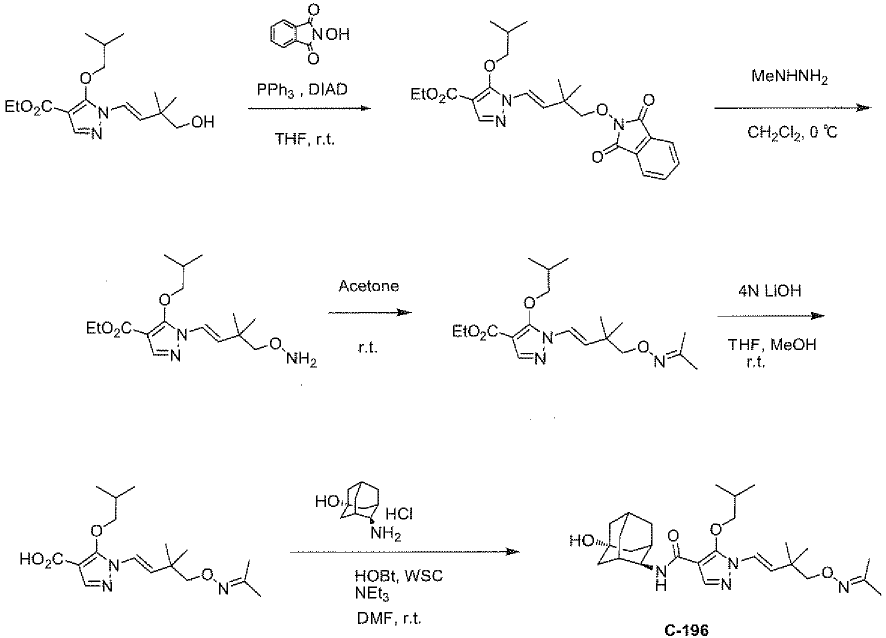

According to the above scheme, Compound **C-196** was synthesized.

### Example 39

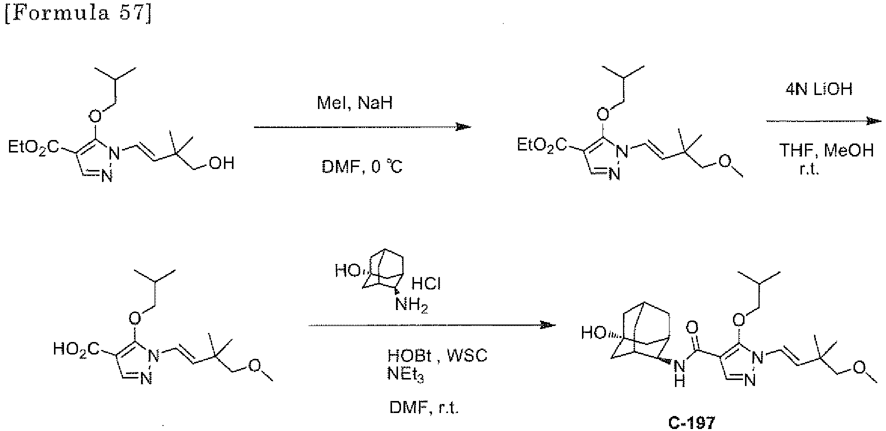

According to the above scheme, Compound **C-197** was synthesized. The reaction conditions, such as reagent, reaction temperature, reaction time, solvent, can be applied to the conventional condition.

### Example 40

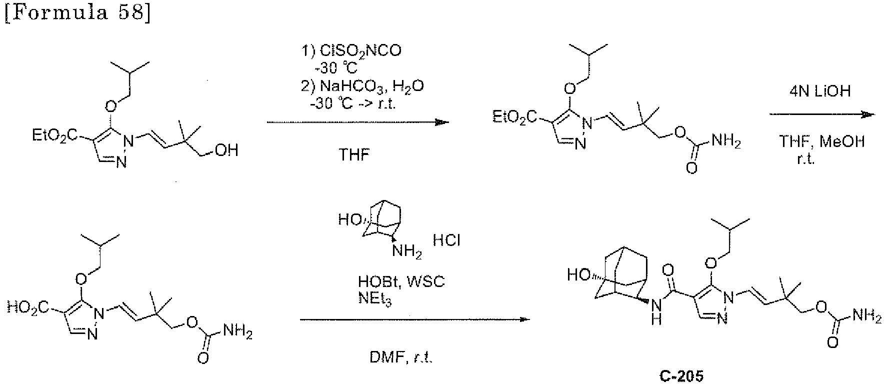

According to the above scheme, Compound **C-205** was synthesized. The reaction conditions, such as reagent, reaction temperature, reaction time, solvent, can be applied to the conventional condition.

### Example 41

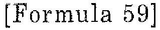

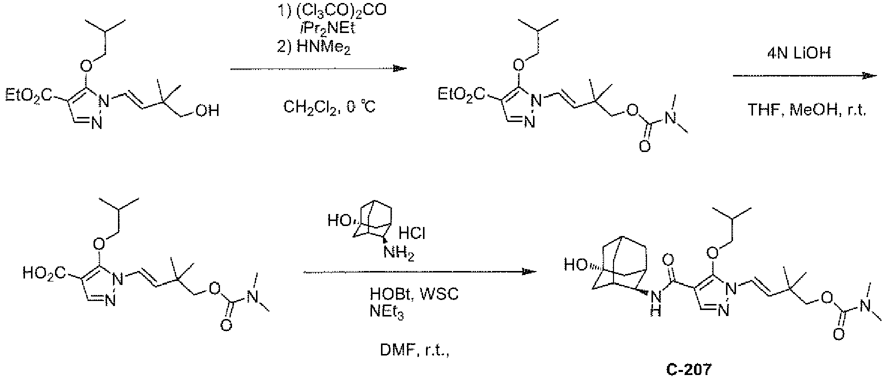

According to the above scheme, Compound **C-207** was synthesized. The reaction conditions, such as reagent, reaction temperature, reaction time, solvent, can be applied to the conventional condition.

### Example 42

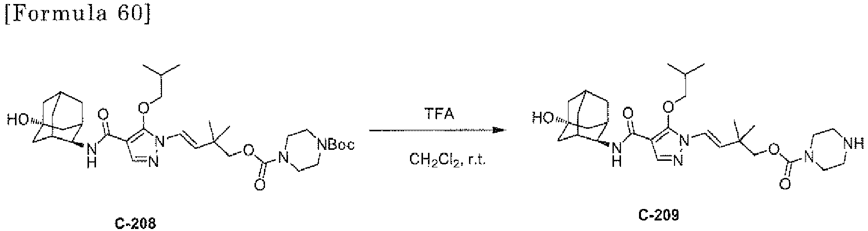

According to the above scheme, Compound **C-209** was synthesized from Compound **C-208**. The reaction conditions, such as reagent, reaction temperature, reaction time, solvent, can be applied to the conventional condition.

### Example 43

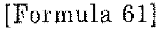

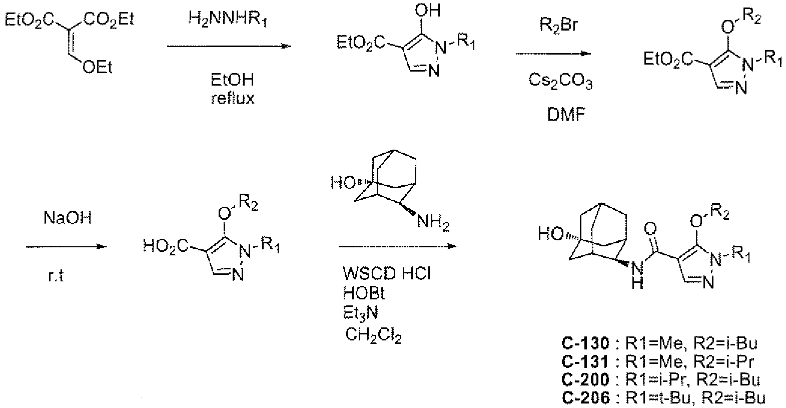

According to the above scheme, Compound **C-130**, **131**, **200** and **206** were synthesized. The reaction conditions, such as reagent, reaction temperature, reaction time, solvent, can be applied to the conventional condition.

### Example 44

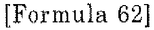

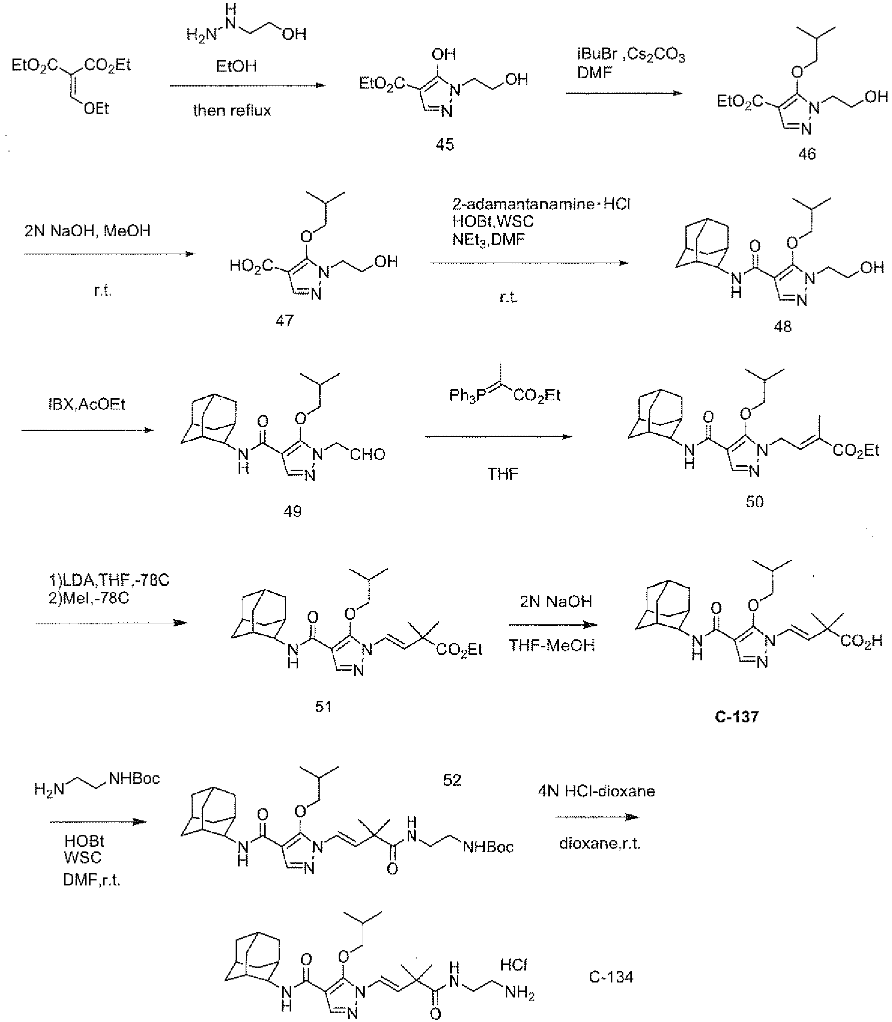

To a solution of diethyl ethoxymethylenemalonate(21.6g) in ethanol(80ml) was added dropwise hydrazine ethanol(8g) in ethanol(20ml) over 30min at -4 °C. The resulting solution was stirred at 40 °C for 1hr and the solvent was removed. The residue was dissolved in chloroform and the organic layer was washed with sat. sodium hydrogencarbonate soln. and dried with magnesium sulfate and concentrated. The residue was dissolved in ethanol(80ml) and the solution was refluxed for 18hrs. After the termination of the reaction, the solvent was removed to give Compound **45**(18.5g).

To a solution of Compound **45**(6.3g) in dimethyl formamide(30ml) were added cesium carbonate(15.4g) and isobutyl bromide(5.6g), then the resulting mixture was stirred at 70 °C for 3hrs. After termination of the reaction, H₂O(60ml) was added to the mixture. The extraction was carried out with ethyl acetate and the organic layer was washed with brine and dried with magnesium sulfate and concentrated to give Compound **46**(5.7g).

To a solution of Compound **46**(8.0g) in methanol(70ml) was added 2N NaOH aqueous soln. (60ml), then the resulting solution was stirred at room temperature for 24hrs. After termination of the reaction, the solution was acidified with 2N HCl aqueous soln.(65ml) and extracted with ethyl acetate. The organic layer was washed with brine and dried with magnesium sulfate and concentrated. The obtained crystal was washed with diisopropylether to afford Compound **47**(6.0g).

To a solution of Compound **47**(5.0g) in dimethyl formamide(50ml) were added 2-adamantanamine hydrochloride(5.35g), 1-(3-dimethylaminopropyl)-3-ethylcarbodiimide hydrochloride(5.04g), 1-hydroxybenzotriazole(3.55g) and triethylamine(7.6ml), then the resulting solution was stirred at room temperature for 24hrs. After termination of the reaction, the solution was diluted with 2N HCl aqueous soln. and extracted with ethyl acetate. The organic layer was washed with sat. sodium hydrogencarbonate soln. and brine, and dried with sodium sulfate and concentrated. The residue was purified by silicagel columnchromatography to give Compound **48**(4.0g).

To a solution of Compound **48**(4.0g) in ethyl acetate(80ml) was added IBX(6.2g), then the resulting mixture was refluxed for 6hrs. After termination of the reaction, the insoluble matter was removed by filtration and the solvent was concentrated to give Compound **49**(4.0g). The obtained product was used for the next reaction without further purification.

To a solution of Compound **49**(4.0g) in tetrahydrofuran(50ml) was added (carbethoxyethylidene)triphenylphosphorane(5.27g), then the resulting solution was stirred at room temperature for 4hrs. After termination of the reaction, the solution was diluted with H₂O and extracted with ethyl acetate. The organic layer was washed with brine and dried with sodium sulfate and concentrated. The residue was purified by silicagel columnchromatography to give Compound **50**(3.9g).

To a solution of diisopropylamine(2.72ml) in tetrahydrofuran(40ml) was added dropwise n-BuLi(12.2ml, 1.59M in hexane) at -78 °C. After stirring at the same temperature for 45min, **50**(3.9g) in tetrahydrofuran(40ml) was added to the solution and the whole solution was stirred for 1h. Iodomethane(0.6ml) was added to the solution, then the solution was stirred for 1.5hrs. After termination of the reaction, the solution was diluted with 2N HCl aqueous soln. and extracted with ethyl acetate. The organic layer was washed with brine and dried with sodium sulfate and concentrated. The residue was purified by silicagel columnchromatography to afford Compound **51**(2.4g).

To a solution of Compound **51**(280mg) in tetrahydrofuran(2.5ml)-methanol(2.5ml) was added 2N NaOH aqueous soln. (2.5ml), then the resulting solution was stirred at room temperature for 1h. After termination of the reaction, the solution was acidified with 2N HCl aqueous soln. and extracted with ethyl acetate. The organic layer was washed with brine and dried with sodium sulfate. The solvent was removed to give Compound **C-137**(259mg).

To a solution of Compound **C-137**(141mg) in dimethyl formamide(3ml) were added tert-butyl- 2-aminoethylcarbamate(68mg), 1-(3-dimethylaminopropyl)-3-ethylcarbodiimide hydrochloride(76mg) and 1-hydroxybenzotriazole(53mg), then the resulting solution was stirred at room temperature for 24hrs. After termination of the reaction, the solution was diluted with 2N HCl aqueous soln. and extracted with ethyl acetate. The organic layer was washed with sat. sodium hydrogencarbonate soln. and brine successively, and dried with sodium sulfate. The solvent was removed and the residue was purified by silicagel columnchromatography to give Compound **52**(146mg).

To a solution of Compound **52**(146mg) in dioxane(1.5ml) was added 4N HCl/dioxane(1.5ml), then the resulting solution was stirred at room temperature for 3hrs. After termination of the reaction, the solution was diluted with diisopropylether to give crystal. The crystal was collected by filtration and washed with diisopropylether and dried to afford Compound **C-134**(94mg).

Compounds **C-129**, **D-13** to **35** were synthesized from the same method.

### Example 45

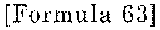

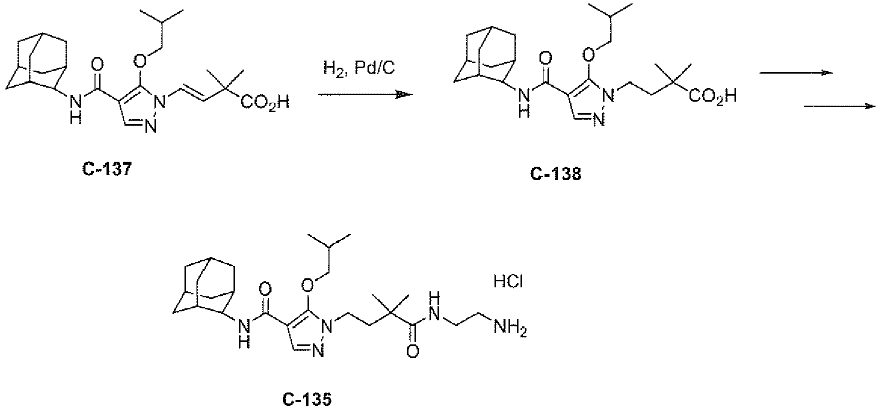

To a solution of Compound **C-137**(118mg) in ethanol(3ml) was added 10%Pd-C(12mg), then the resulting mixture was stirred for 5hrs under H₂ atomosphere. After termination of the reaction, Pd-C was removed by filtration. The filtrate was concentrated to give Compound **C-138**(116mg). According to the conventional procedure, Compound **C-135** was synthesized.

Additiionally, Compounds **C-139**, **D1** to **12** were synthesized as well as the above Example.

### Example 46

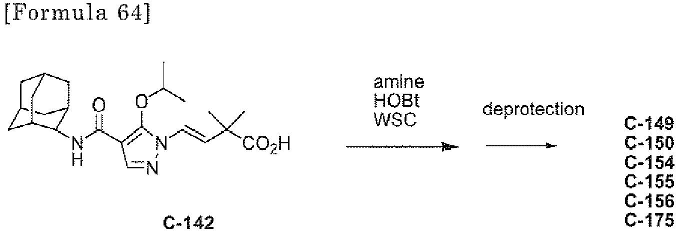

According to the synthetic procedure of Compounds **C-137** and **C-142** were synthesized. Compound **C-142** was converted to Compounds **C-149, 150, 154** to **156** and **175** by the conventional procedure.

### Example 47

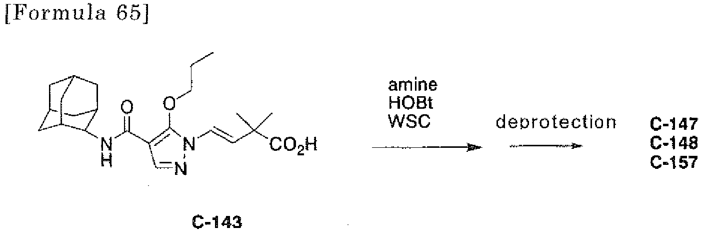

According to the synthetic procedure of Compounds **C-137** and **C-143** was synthesized. Compound **C-143** was converted to Compounds **C-147, 148** and 157 by the conventional procedure.

### Example 48

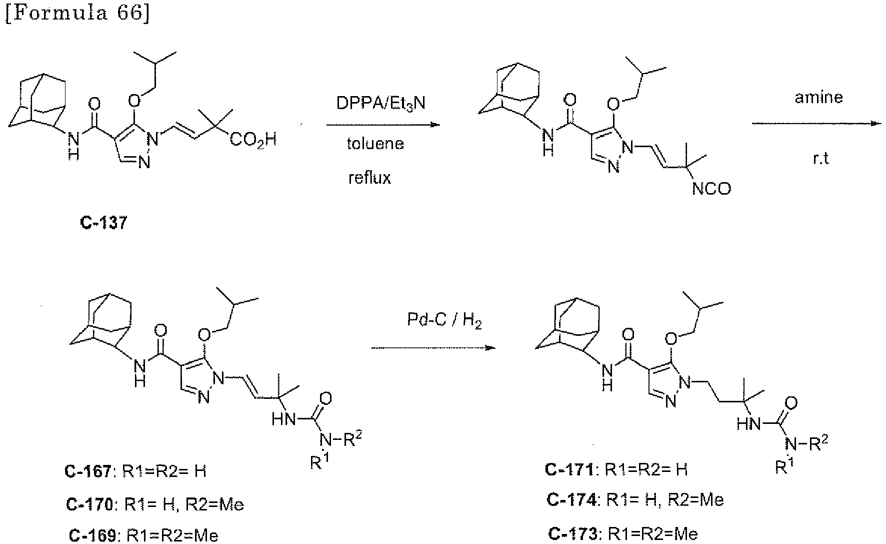

According to the synthetic procedure of Compound **C-184**(Example 31), the above compounds were synthesized from Compound **C-137.**

### Example 49

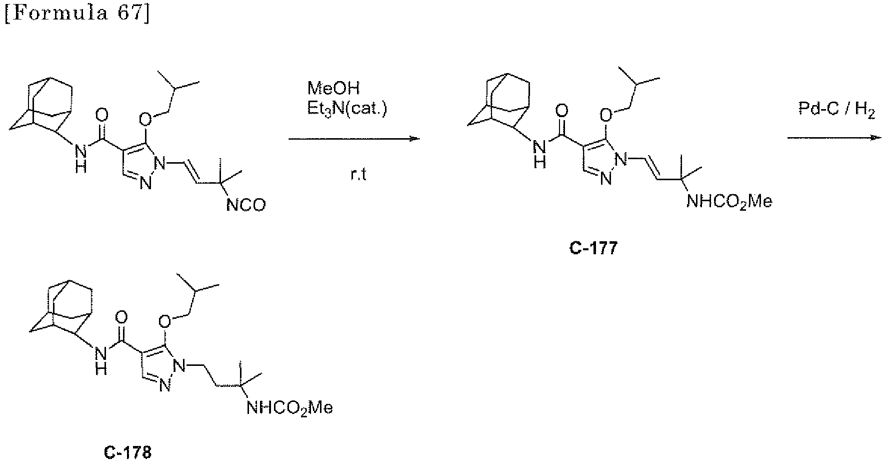

According to the above scheme, Compounds **C-177** and **178** were synthesized.

### Example 50

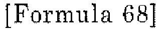

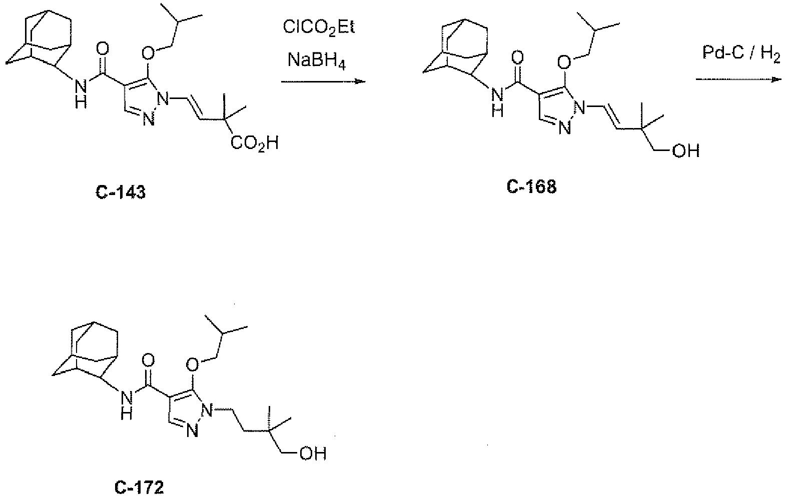

According to the synthetic procedure of Compound **C-193**(Example 36), Compound **C-168** was synthesized from Compound **C-137.** Additionally, Compound **C-168** was converted to Compound **C-272** by the above procedure.

### Example 51

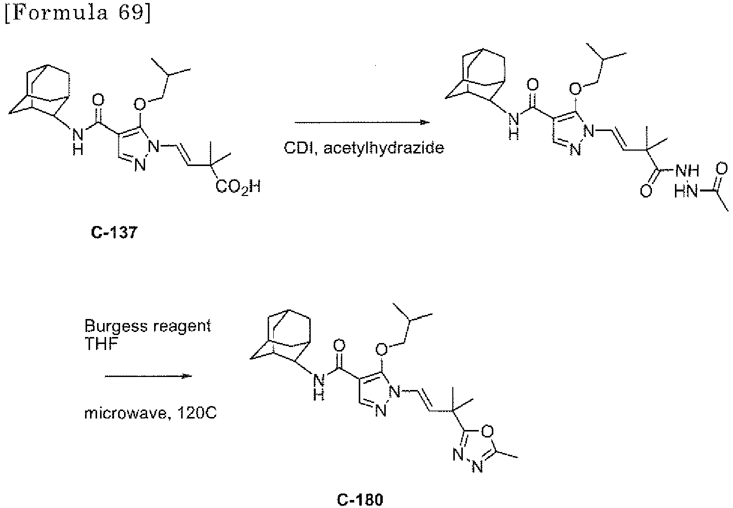

According to the synthetic procedure of Compound **C-191**(Example 33), Compound **C-180** was synthesized from Compound **C-137**.

### Example 52

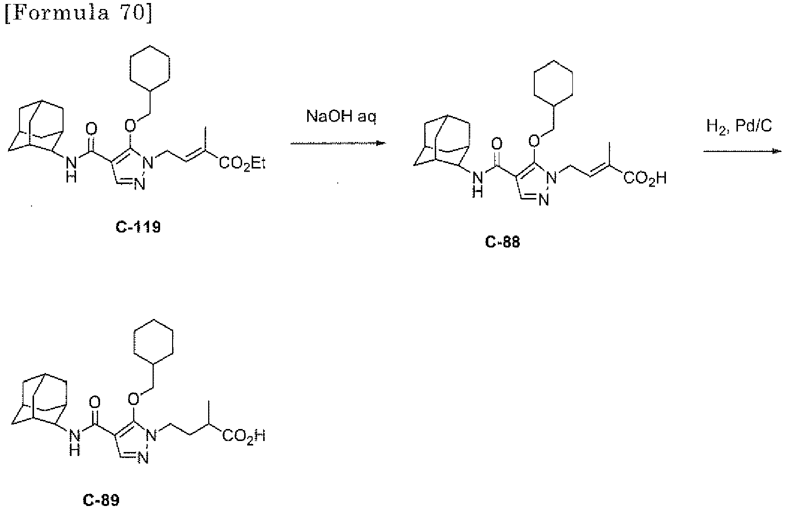

Hydrolysis of Compound **C-119** gave Compound **C-88,** followed by the catalytic reduction of Compound **C-88** gave Compound **C-89.**

### Example 53

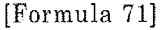

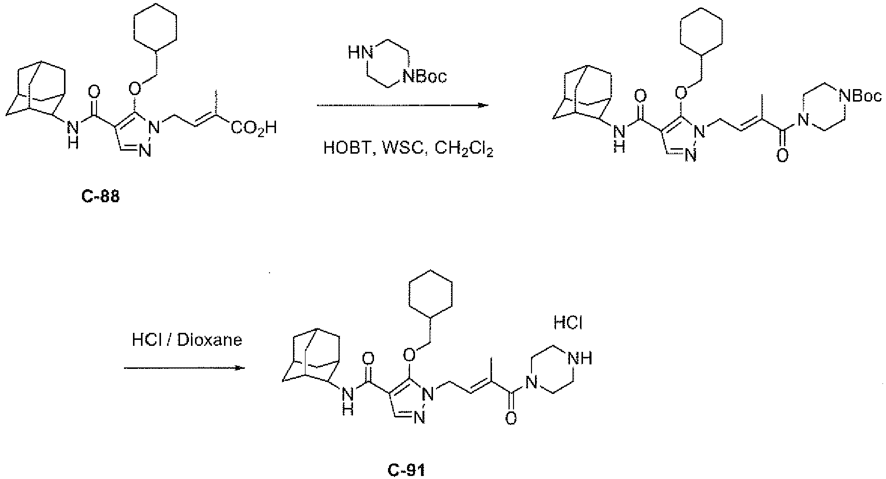

According to the above scheme, Compound **C-91** was synthesized from Compound **C-88.**

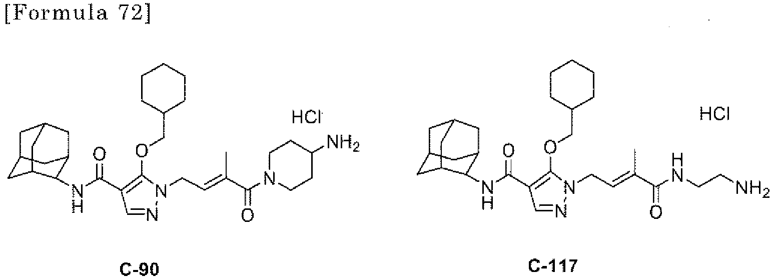

According to the above procedure, the following compounds were synthesized.

### Example 54

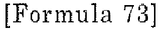

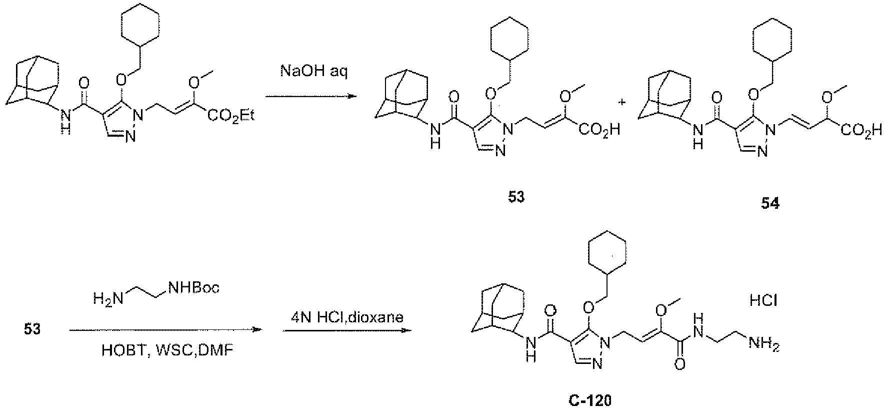

According to the above scheme, Compounds **53** and **54** were synthesized. Compound **C-120** was synthesized from Compound **53**.

### Example 55

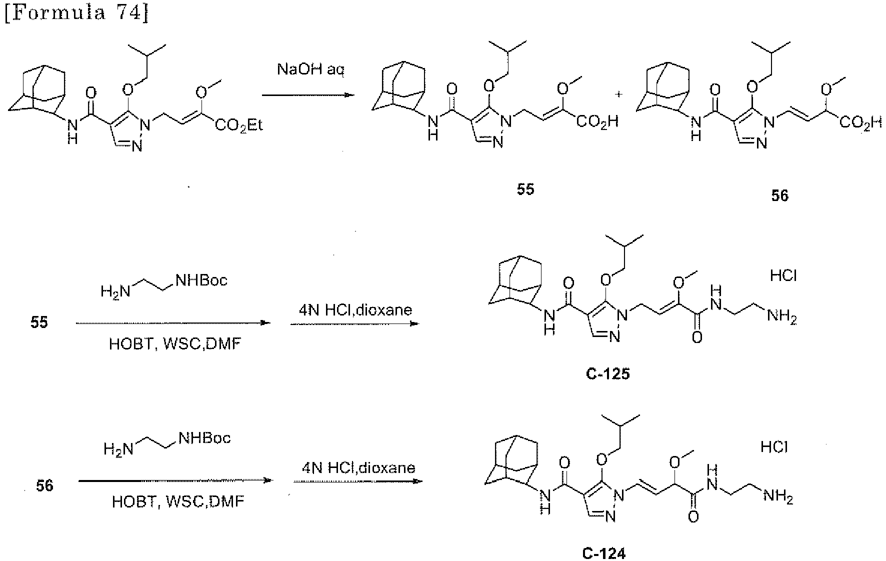

The above starting material was hydrolyzed to afford Compounds **55** and **56**. Compound **55** was condensed with amine, followed by deprotection by HCl to give Compound **C-125.** Compound **56** was condensed with amine, followed by deprotection by HCl to give Compound **C-124.**

### Example 56

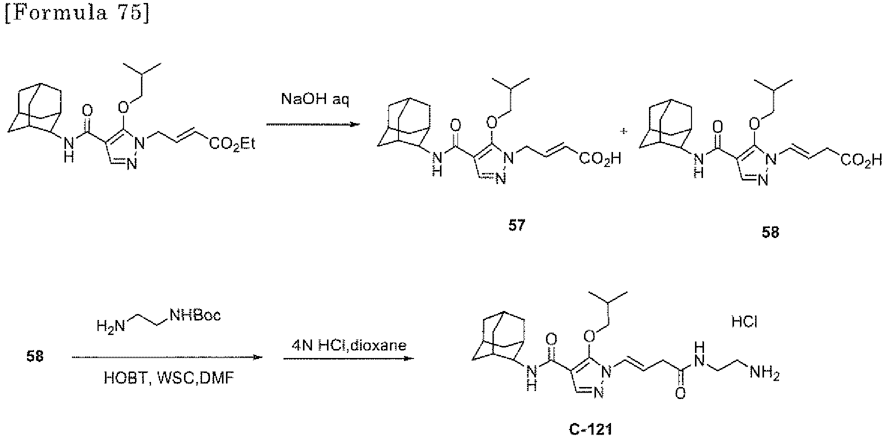

According to the above scheme, Compound **C-121** was obtained.

### Example 57

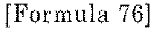

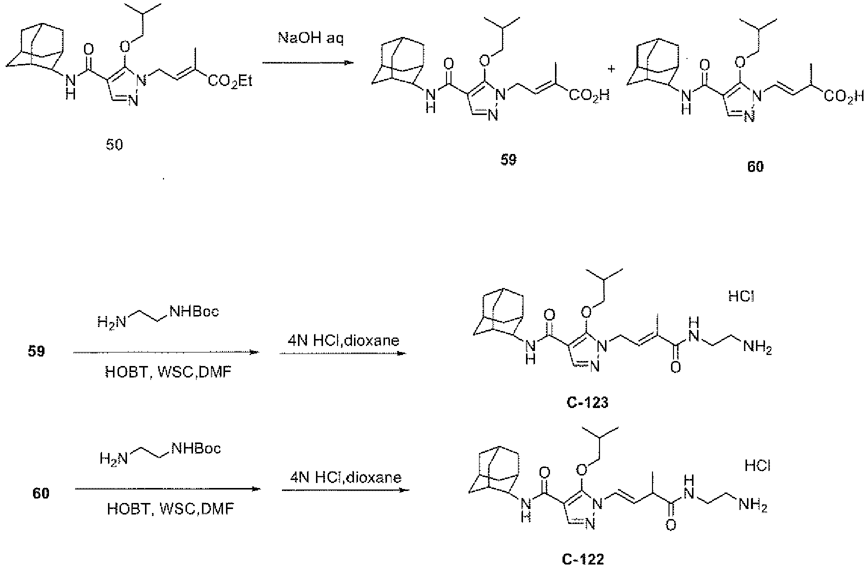

According to the above scheme, Compounds **C-123** and **122** were obtained.

### Example 58

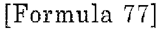

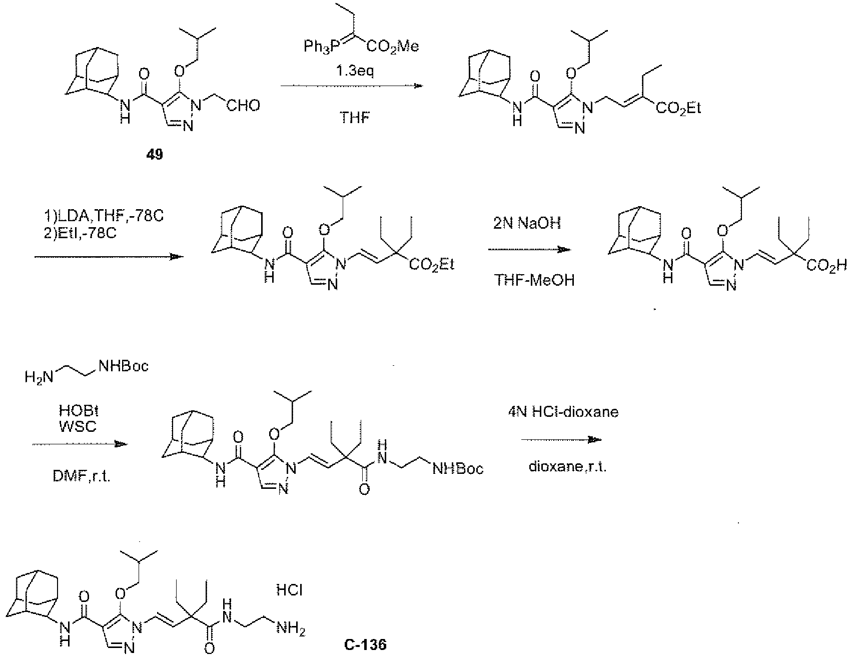

According to the above scheme, Compound **C-136** was obtained.

### Example 59

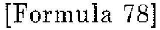

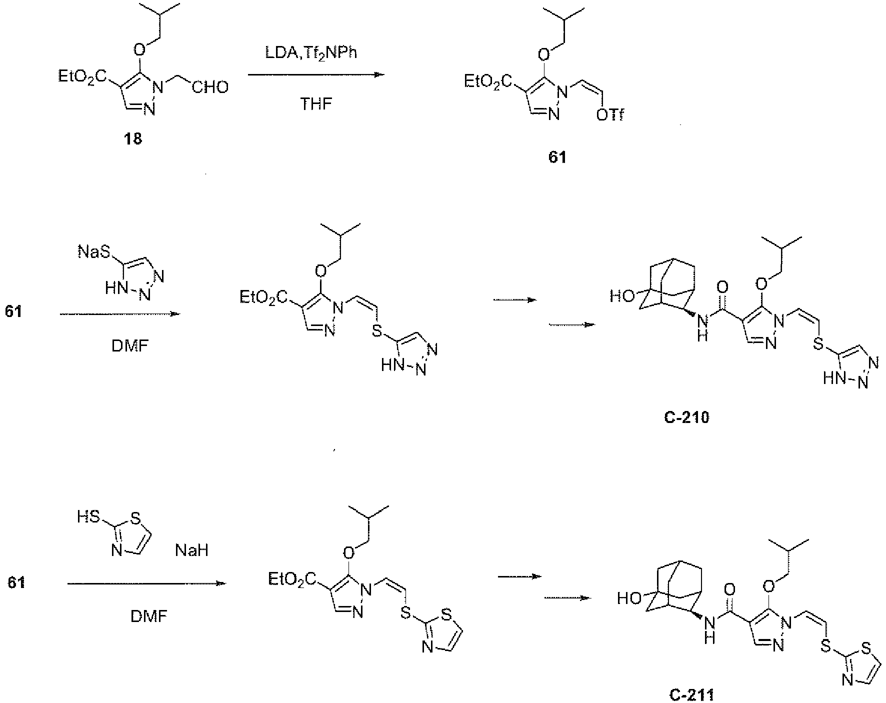

According to the above scheme, Compounds **C-210** and **211** were obtained.

### Example 60

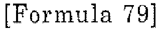

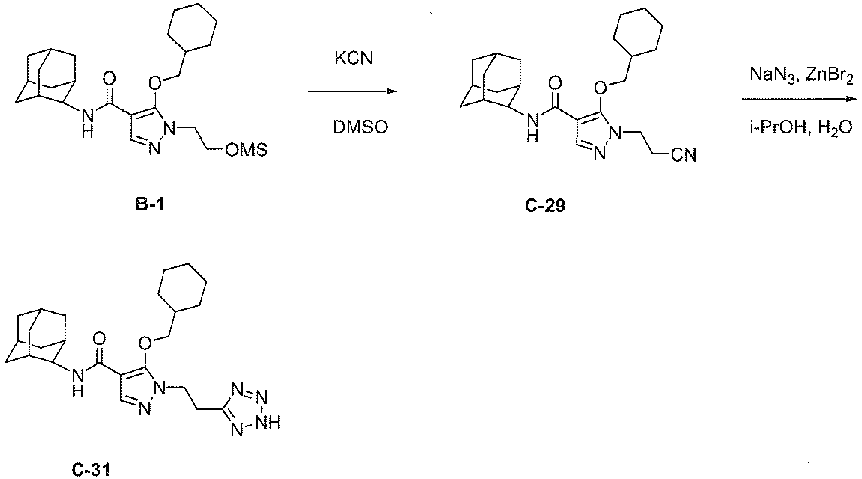

According to the above scheme, Compounds **C-29** and **31** were obtained. Compound **C-104** was synthesized as well as the above-mentioned execution example.

### Example 61

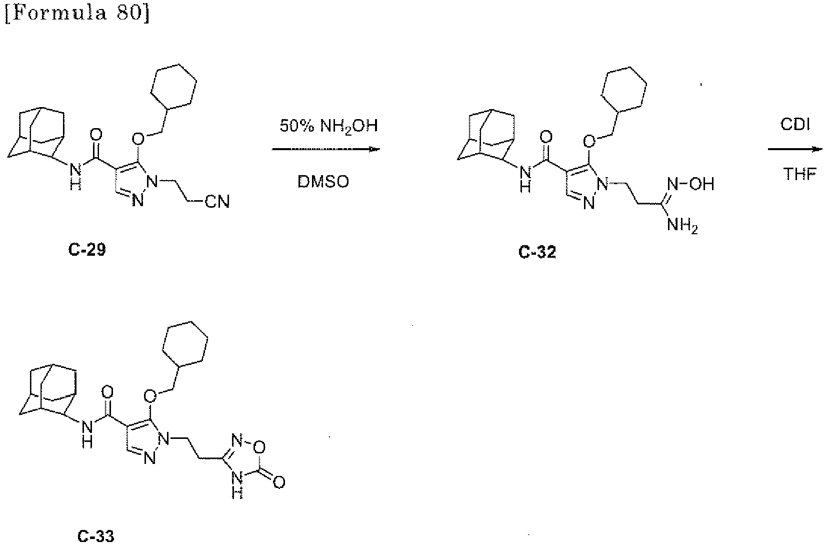

According to the above scheme, Compound **C-32** was synthesized, followed by reaction with 1,1'-carbonyldiimidazole to give Compound **C-33**.

### Example 62

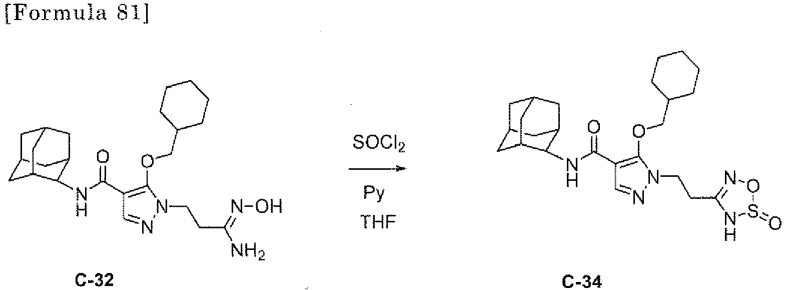

Compound **C-32** was reacted with thionyl chloride in the presence of pyridine to afford Compound **C-34.**

### Example 63

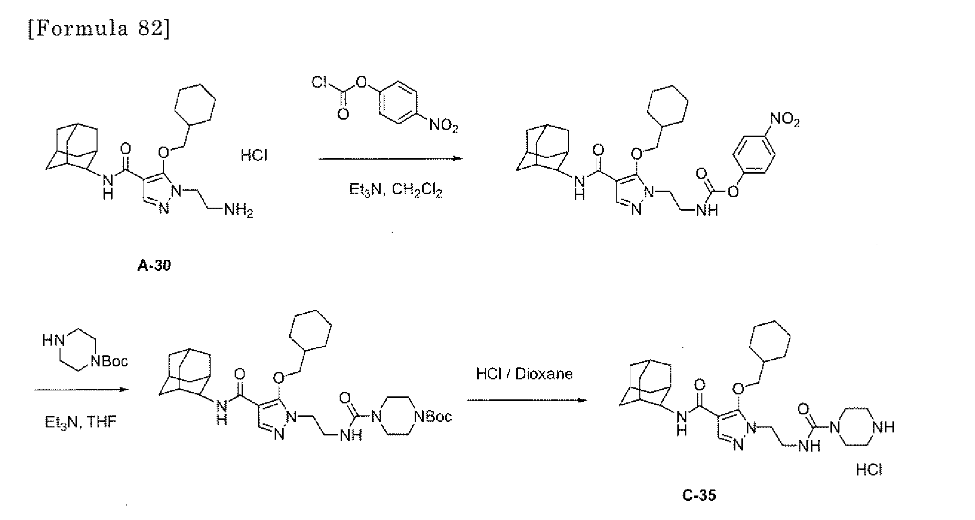

According to the above scheme, Compound **C-35** was synthesized.

### Example 64

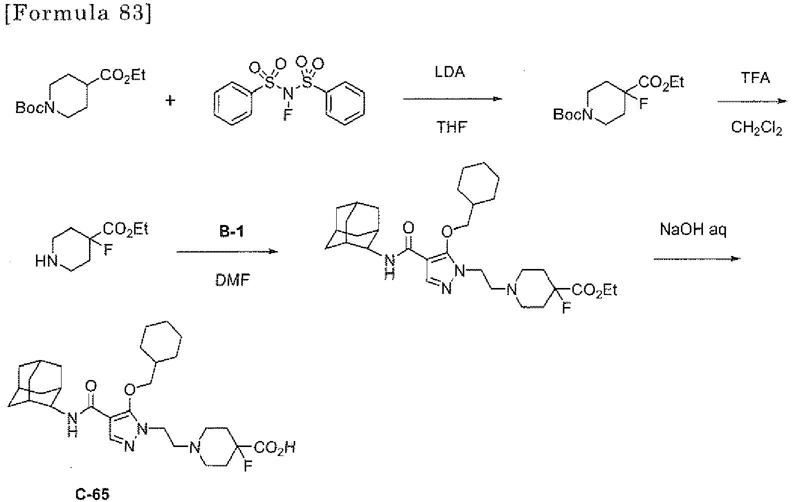

According to the above scheme, Compound **C-65** was synthesized.

### Example 65

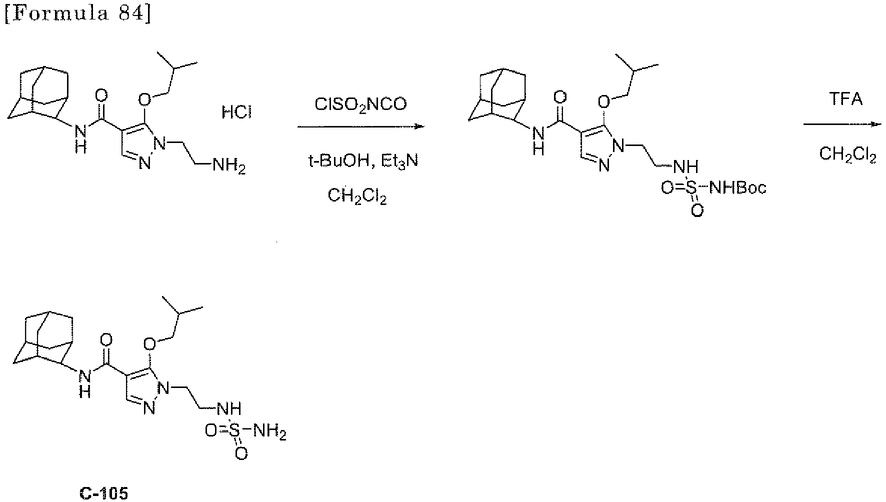

According to the above scheme, Compound **C-105** was synthesized.

### Example 66

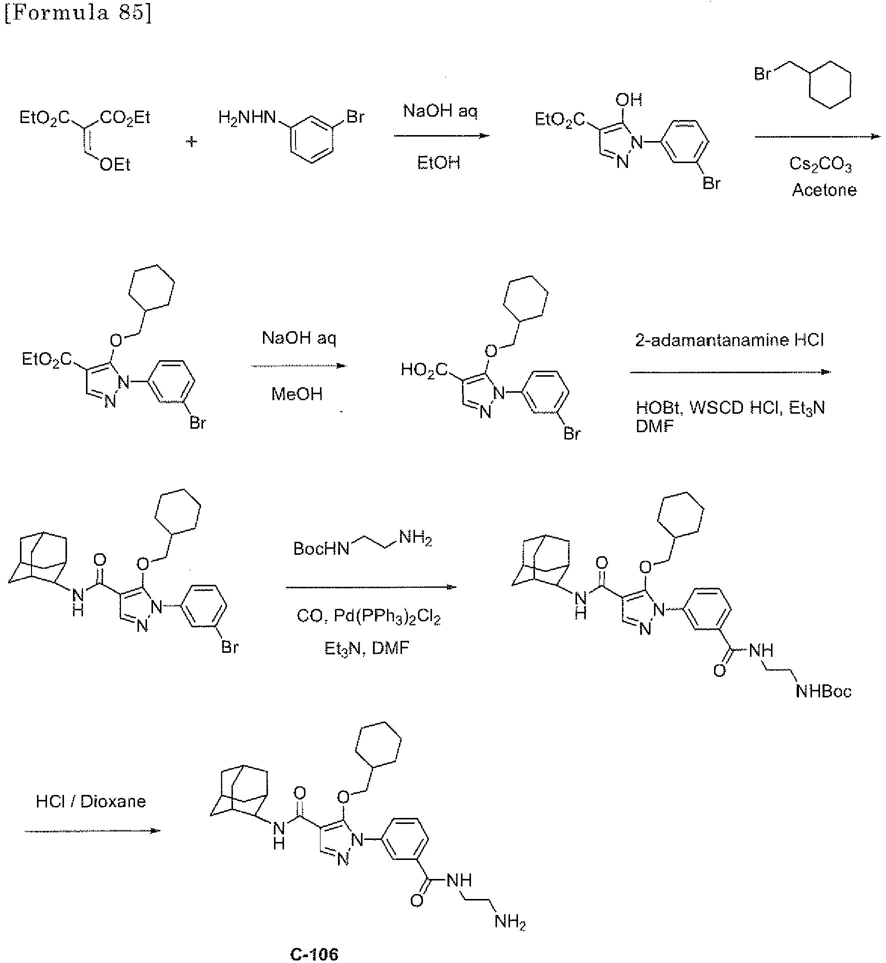

According to the above scheme, Compound **C-106** was synthesized.

### Example 67

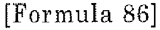

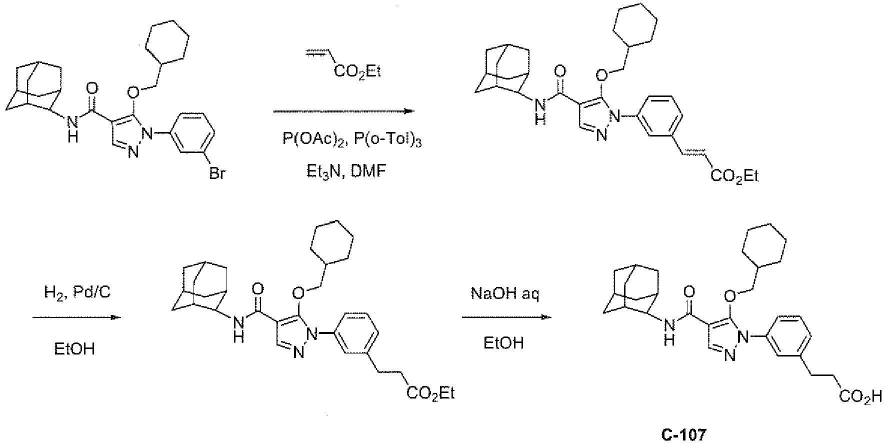

According to the above scheme, Compound C-107 was synthesized.

### Example 68

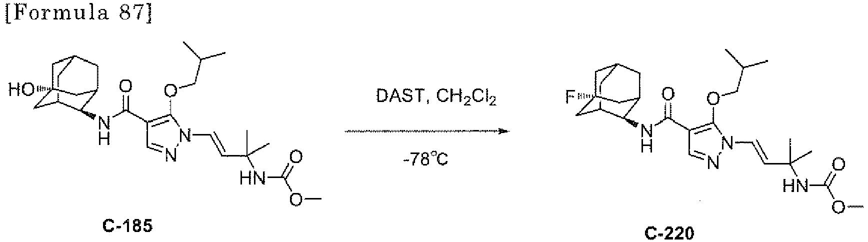

To a solution of Compound **C-185**(300mg) in methylene chloride(20ml) was added DAST(168µl) at -78 °C, then the resulting solution was at the same temperature for 1h. After termination of the reaction, the solution was poured into sat. sodium hydrogencarbonate soln. ane extracted with ethyl acetate. The organic layer was washed with brine and dried with magnesium sulfate and concentrated. The redidue was purified by silicagel columnchromatography to give Compound **C-220**(270mg).

Compound **C-219** was synthesized from Compound **C-144** by the same procedure.

### Example 69

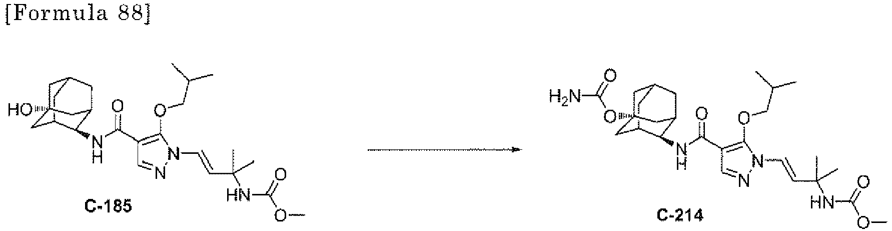

To a solution of Compound **C-185**(250mg) in tetrahydrofuran(2.5ml) was added chlorosulfonyl isocyanate(69µl) at -45 °C, then the resulting solution was stirred at -25 °C for 3hrs. Sodium hydrogencarbonate(221mg) and H₂O(50µl) were added to the solution and the whole mixture was stirred at room temperature for 1h. The mixture was extracted with ethyl acetate and the organic layer was washed with brine and dried with sodium sulfate and concentrated. The residue was purified by silicagel columnchromatography to give Compound **C-214**(220mg).

Compound **C-231** was synthesized from Compound **C-202,** and Compound **C-236** was obtained from Compound **C-216** as well as the above Example.

The following compounds were synthesized as well as the above Example. "CIH" has the same meaning as "HCl" in the following table. The measurement results of NMR, MS and m.p. were disclosed.

**[Table 1]**

| No. | Structure | NMR (CDCl3 or d6-DMSO), MS or m.p. |
|---|---|---|
| A-1 | 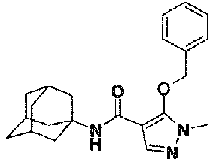 | 1H-NMR (DMSO-d6) δ: 1.64 (br s,6H ), 2.00-2.02 (br m, 10H), 3.48 ( s, 3H), 5.34 ( s, 2H), 6.86 ( s, 1H), 7.40-7.41 (m, 5H), 7.69 (s, 1H). |
| A-2 | 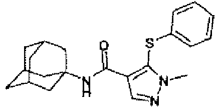 | 1H-NMR (CDCl3) δ : 1.57 (br s, 2H), 1.64-1.67 (br m, 5H), 1.98-2.04 (br m, 8H), 3.88 ( s, 3H), 6.99 (s, 1H), 7.06 (d, J = 6.9 Hz, 2H), 7.27-7.29 ( m, 9H), 8.12 (s, 1H). |
| A-3 | 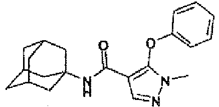 | 1H-NMR (DMSO-d6) δ : 1.57-1.62 ( br m, 7H), 1.82 (br s, 6H), 1.95 (br s, 3H), 2.02 (br s, 1H), 3.59 ( s, 3H), 6.62 (s, 1H), 6.92 (d, J = 8.1 Hz, 2H), 7.14 (t, J = 7.3 Hz, 1H), 7.39 (t, J = 8.0 Hz, 2H), 7.86 (s, 1H). |
| A-4 | 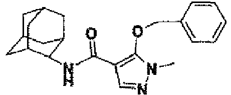 | 1H-NMR (DMSO-d6) δ: 1.48 (d, J = 12.1 Hz, 2H), 1.81 ( dt, J = 56.8, 20.7 Hz, 12H), 3.52 ( s, 3H), 3.99 (s, 1H), 5.36 (s, 2H), 7.23 (d, J = 7.1 Hz, 1H), 7.39-7.41 (m, 5H), 7.82 (s, 1H). |
| A-5 | 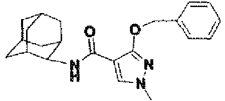 | 1H-NMR (DMSO-d6) δ: 1.39-1.70 (m, 14H), 3.74 (s, 3H), 3.95 (d, J = 8.3 Hz, 1H), 5.26 (s, 2H), 7.10 (d, J = 9.1 Hz, 1H), 7.39-7.40 ( m, 3H), 7.50 (d, J = 7.1 Hz, 2H), 7.99 ( s, 1H). |
| A-6 | 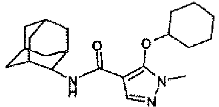 | 1H-NMR (CDCl3) δ : 1.25-1.28 (m, 2H), 1.48-1.52 ( m, 2H), 1.71-1.95 (m, 20H), 3.70 (s, 3H), 4.19-4.22 ( m, 1H), 4.31-4.41 (m, 1H), 6.42 (d, J = 7.0 Hz, 1H), 7.27 (s, 1H), 7.74 (s, 1H). |

**[Table 2]**

| No. | Structure | NMR (CDCl3 or d6-DMSO), MS or m.p. |
|---|---|---|
| A-7 | 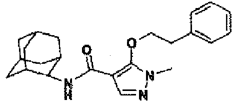 | 1H-NMR (CDCl3) δ: 1.72-2.04 (m, 16H), 3.15 (t, J = 6.8 Hz, 2H), 3.49 (s, 3H), 4.24-4.26 (m, 1H), 4.51 (t, J = 6.8 Hz, 2H), 6.46 (d, J = 7.6 Hz, 1H), 7.28-7.40 (m, 5H), 7.73 (s, 1H). |
| A-8 | 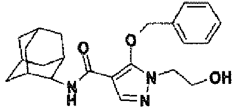 | 1H-NMR (CDCl3) δ: 1.61-1.98 (m, 16H), 2.72 (br s, 1H), 3.80 (t, J = 4.5 Hz, 2H), 3.88 (t, J = 4.6 Hz, 2H), 4.20-4.22 (m, 1H), .5.34 ( s, 2H), 6.49 ( d, J = 8.1 Hz, 1H), 7.27. (s, 1H), 7.35-7.40 (m, 5H), 7.78 (s, 1H). |
| A-9 | 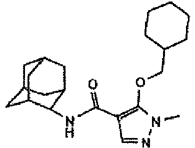 | 1 H-NMR (CDCl3) δ: 1.05-1.28 ( m, 4H), 1.74-1.92 (br m, 26H), 3.69 (t, J = 6.8 Hz, 3H), 3.98 (t, J = 5.3 Hz, 2H), 3.99 (d, J = 6.3 Hz, 2H), 6.46 ( d, J = 7.0 Hz, 1H), 7.73 (s, 1H). |
| A-10 | 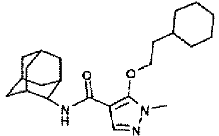 | 1H-NMR (DMSO-d6) δ: 0.92-0.94 (m, 2H), 1.15-1.20 (m, 2H), 142-1.99 (m, 20H), 3.61 (s, 3H), 3.96 ( s, 1H), 4.29 (t, J = 6.9 Hz, 2H), 7.20 (d, J = 7.1 Hz, 1H), 7.76 (s, 1H). |
| A-11 | 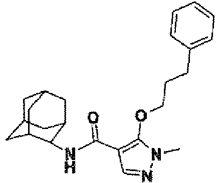 | 1H-NMR (CDCl3) δ: 1.66-1.98 ( br m, 14H), 2.17 (tt, J = 7.2, 2.9 Hz, 2H), 2.79 (q, J = 7.2 Hz, 2H), 3.39 (t, J = 10.5 Hz, 2H), 3.67 ( s, 3H), 4.17-4.27 ( m, 3H), 6.43 (d, J = 8.4 Hz, 1H), 7.13-7.32 (m, 5H), 7.71 ( s, 1 H). |
| A-12 | 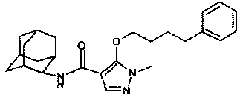 | 1H-NMR (DMSO-d6) δ: 1.50 (d, J = 13.4 Hz, 2H), 1.75-1.90 (m, 16H), 2.61-2.64 (br m, 2H), 3.60 ( s, 3H), 3.94-3.96 (br m, 1H), 4.25-4.27 (br m, 2H), 7.19-7.21 (m, 3H), 7.28 (t, J = 7.6 Hz, 2H), 7.77 ( s, 1H). |

**[Table 3]**

| No. | Structure | NMR (CDCl3 or d6-DMSO), MS or m.p. |
|---|---|---|
| A-13 | 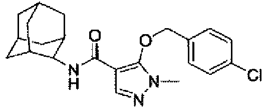 | DMSO-d6 δ 1.42-1.95 (m, 14H), 3.55(s, 3H), 3.97-4.00 (m, 1H), 5.36 (s, 2H), 7.25 (d, J=7.2 hz, 1H), 7.42-7.52 (m, 4H), 7.85 (s, 1H). |
| A-14 | 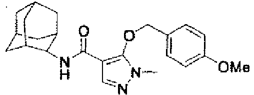 | DMSO-d6 δ 1.50-1.98 (m, 14H), 3.03(s, 3H), 3.13 (d, 1H, J=9.2), 3.31 (d, J=13.6, 1H), 3.71 (s, 3H), 3.79-3.88 (m, 1H), 6.80 (d, J=8.8 Hz, 2H), 7.04 (d, J=8.8 Hz, 2H), 7.52 (d, J=7.6 Hz, 1H), 7.73 (s, 1H). |
| A-15 | 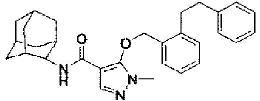 | DMSO-d6 δ 1.39-2.00 (m, 14H), 2.77-2.86 (m, 2H), 2.94-3.01 (m, 2H), 3.48(s, 3H), 3.98-4.07 (m, 1H), 5.43 (s, 2H), 7.14-7.44 (m, 10H), 7.87 (s, 1H). |
| A-16 | 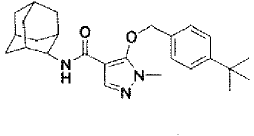 | DMSO-d6 δ 1.28 (s, 9H), 1.41-1.92 (m, 14H), 3.53(s, 3H), 3.91-4.04 (m, 1H), 5.34 (s, 2H), 7.18 (d, J= 7.2 Hz, 1H), 7.32-7.43 (m, 4H), 7.81 (s, 1H). |
| A-17 | 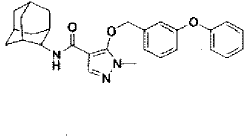 | DMSO-d6 δ 1.42-1.99 (m, 14H), 3.51(s, 3H), 3.94-3.96 (m, 1H), 5.35 (s, 2H), 6.94-7.08 (m, 4H), 7.12-7.22 (m, 3H), 7.37-7.43 (m, 3H), 7.84 (s, 1H). |
| A-18 | 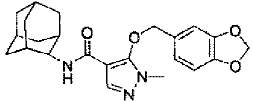 | DMSO-d6 δ 1.50-1.88 (m, 14H), 3.06 (s, 3H), 3.13 (d, J=13.2 Hz, 1H), 3.29 (d, J=13.2 Hz, 1H), 3.78-3.83 (m, 1H), 5.97 (d,J=4.8 Hz, 2H), 6.56-6.80 (m, 3H), 7.50 (d, J=7.6 Hz, 1H), 7.76 (s, 1H). |

**[Table 4]**

| No. | Structure | NMR (CDCl3 or d6-DMSO), MS or m.p. |
|---|---|---|
| A-19 | 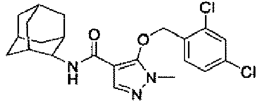 | DMSO-d6 δ 1.40-2.00 (m, 14H), 3.55 (s, 3H), 3.95-4.02 (m, 1H), 5.47 (s, 2H), 7.31 (d, J=6.8 Hz, 1H), 7.46-7.72 (m, 3H), 7.88 (s, 1H). |
| A-20 | 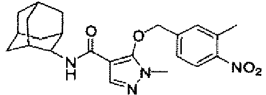 | DMSO-d6 δ 1.50-1.85 (m, 14H), 2.46 (s, 3H), 3.08 (s, 3H), 3.32 (s. 2H), 3.79-3.83 (m, 1H), 7.18-7.49 (m, 3H), 7.79-7.98 (m, 2H). |
| A-21 | 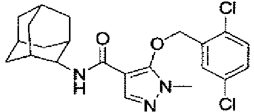 | DMSO-d6 δ 1.39-2.01 (m, 14H), 3.58 (s, 3H), 3.97-4.05 (m, 1H), 5.47 (s, 2H), 7.27-7.32 (m, 1H), 7.45-7.66 (m, 3H), 7.88 (s, 1 H). |
| A-22 | 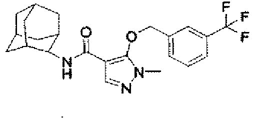 | DMSO-d6 δ 1.40-1.99 (m, 14H), 3.58 (s, 3H), 3.96-4.04 (m, 1H), 5.47 (s, 2H), 7.26 (d, J=6.8 Hz, 1 H), 7.61-7.87 (m, 5H). |
| A-23 | 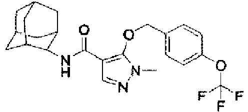 | DMSO-d6 δ 1.39-1.97 (m, 14H), 3.56 (s, 3H), 3.96-4.04 (m, 1H), 5.38 (s, 2H), 7.19-7.28 (m, 1H), 7.32-7.44 (m, 2H), 7.55-7.63 (m, 2H), 7.86 (s, 1H). |
| A-24 | 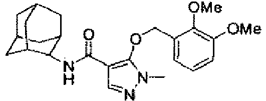 | DMSO-d6 δ 1.52-1.96 (m, 15H), 3.07 (s, 3H), 3.31 (s, 3H), 3.72 (s, 3H), 3.78 (s, 2H), 6.55-6.63 (m, 1H), 6.85-6.93 (m, 2H), 7.52-7.58 (m, 2H). |

**[Table5]**

| No. | Structure | NMR (CDCl3 or d6-DMSO), MS or m.p. |
|---|---|---|
| A-25 | 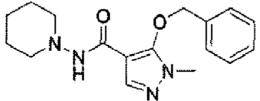 | DMSO-d6 δ 1.04-1.32 (m, 10H), 2.32 (s, 3H), 2.61-2.72 (m, 2H), 4.65-4.89 (m, 2H), 7.39-7.55 (m, 3H), 7.84-7.95 (m, 2H), 9.37 (s, 1 H). |
| A-26 | 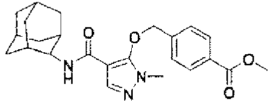 | DMSO-d6 δ 1.41-2.00 (m, 14H), 3.56 (s, 3H), 3.86 (s, 3H), 3.96-4.02 (m, 1H), 5.46 (s, 2H), 7.27 (d, J=7.2 Hz, 1 H), 7.59 (d, J=8.0 Hz, 2H), 7.86 (s, 1H), 7.98 (d, J=8.0 Hz, 2H). |
| A-27 | 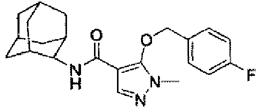 | mp 110-111°C |
| A-28 | 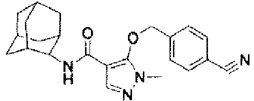 | mp 173-174°C |
| A-29 | 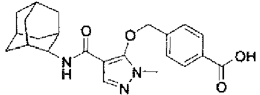 | mp 202-203°C |
| A-30 | 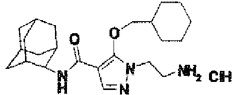 | 1 H-NMR (DMSO-d6) δ: 1.02-1.25 (m, 4H), 1.53 (d, J = 11.9 Hz, 2H), 1.74-1.95 ( br m, 19H), 3.18 (t, J = 5.9 Hz, 2H), 3.97 ( s, 1H), 4.11 (d, J = 6.1 Hz, 2H), 4.18 (t, J = 6.1 Hz, 2H), 7.30 (d, J = 7.6 Hz, 1H), 7.89 (s, 1H). |

**[Table6]**

| No. | Structure | NMR (CDCl3 or d6-DMSO), MS or m.p. |
|---|---|---|
| A-31 | 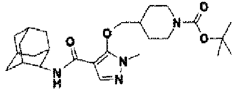 | 1H-NMR (CDCl3) δ : 1.07-1.11 (m, 2H), 1.41 (s, 13H), 1.55-1.81 (m, 16H), 2.55 (t, J = 12.8 Hz, 2H), 3.51 (s, 3H), 3.91-3.99 (m, 4H), 6.10 (d, J = 7.5 Hz, 1H), 7.48 (s, 1 H). |
| A-32 | 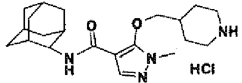 | 1H-NMR (DMSO-d6) δ : 2.03-1.50 (m, 17H), 2.93-2.84 (q, 4H), 3.30-3.27 (m, 2H), 3.61 (s, 3H), 3.96 (m, 1H), 4.16-4.15 (d J = 7.5 Hz, 2H), 7.30-7.28 (d, J = 8.0 Hz, 1H), 7.86 (s, 1H), 8.98 (br s, 1H), 9.06 (br s, 1H), |
| A-33 | 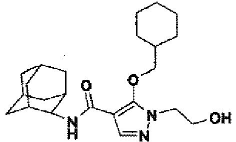 | 1 H-NMR (CDCl3) δ: 1.04-1.40 (m, 4H), 1.72-2.21 (m, 21H), 4.04-4.15 (m, 5H), 4.23-4.26 (m, 1H), 6.48 (d, J = 7.8 Hz, 1H), 7.80 (s, 1 H). |
| A-34 | 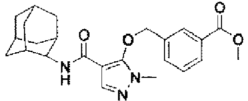 | mp 128-129°C |
| A-35 | 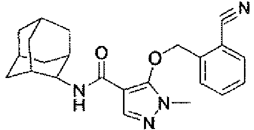 | mp 107-108°C |
| A-36 | 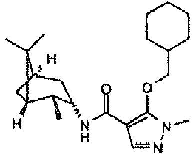 | 300MHz(CDCl3)1.08(s,3H),1.15(d, J=7.2Hz,3H)1.24(3H,s),1.22-2.05(m,16H)2.42-2.49(m,1H),2.63-2.73(m,1H),3.72(s,3H),4.00(d,J=6.3Hz,2H),4. 38-4.49(m,1H)6.04(d,J=8.4Hz,1H),7.76(s,1H) |

**[Table 7]**

| No. | Structure | NMR (CDCl3 or d6-DMSO), MS or m.p. |
|---|---|---|
| A-37 | 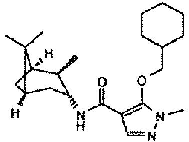 | 300MHz(CDCl3)1.08(s,3H),1.15(d, J=7.2Hz,3H)1.24(3H,s),1.22-2.10(m, 16H)2.41-2.49(m,1H),2.63-2.72(m, 1H),3.71(s,3H),4.00(d,J=6.3Hz,2H),4. 38-4.52(m,1H)6.02(d,J=8.4Hz,1H),7.75(s, 1H) |
| A-38 | 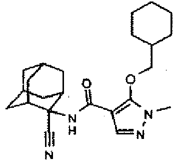 | 300MHz(CDCl3)1.07-1.99(m,23H)2.33(m,1H)2.62(s,1H)3.72(s,3H) 4.01 (d,J=6.3Hz,2H)6.25(s,1H)7.74(s, 1H) |
| A-39 | 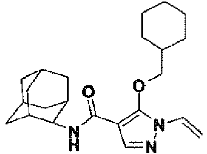 | 1H-NMR (CDCl3) δ : 1.40-1.04 (m, 4H), 2.21-1.72 (m, 21 H), 4.03-4.01 (m, 2H), 4.25-4.16 (m, 1H), 4.96-4.93 (d, J = 8.1 Hz, 1H), 5.80-5.76 (d, J = 11.2 Hz, 1H), 7.00-6.92 (dd, J = 11.3 Hz, J = 8.2 Hz, 1H), 7.83 (s, 1H). |
| A-40 | 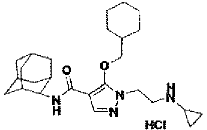 | 1H-NMR (DMSO-d6) δ: 0.73 (q, J = 6.6 Hz, 2H), 1.00-1.04 (m, 4H), 1.14-1.25 (m, 3H), 1.51-2.03 (m, 20H), 2.51 (s, 2H), 2.74-2.77 (m, 1H), 3.36 (d, J = 5.6 Hz, 2H), 3.97 (s, 1H), 4.12 (d, J = 6.1 Hz, 2H), 4.33 (t, J = 6.9 Hz, 2H), 7.32 (d, J = 6.6 Hz, |
| A-41 | 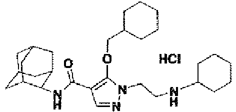 | 1H-NMR (DMSO-d6) δ: 1.03-1.39 (m, 10H), 1.51-2.03 (m, 27H), 3.03 (s, 1H), 3.27 (s, 2H). 3.97 (1H, s, 1H), 4.13 (d, J = 5.8 Hz, 2H), 4.34 (t, J = 6.8 Hz, 2H), 7.32 (d, J = 6.8 Hz, 1H), 7.89 (s, 1 H), 9.46 (s, 2H). |
| A-42 | 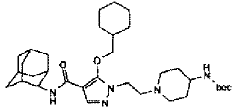 | 1H-NMR (DMSO-d6) δ: 1.03-1.41 (m, 14H), 1.52 (d, J = 13.1 Hz, 2H), 1.63-2.09 (m, 21 H), 2.50 (s, 3H), 2.62 (t, J = 6.4 Hz, 2H), 2.77 (dd, J = 11.7, 6.7 Hz, 2H), 3.17 ( s, 1H), 3.97 (t, J = 6.2 Hz, 3H), 4.08 (d, J = 6.3 Hz, 2H), 6.73 (d, J = 7.1 Hz, 1 |

**[Table 8]**

| No. | Structure | NMR (CDCl3 or d6-DMSO), MS or m.p. |
|---|---|---|
| A-43 | 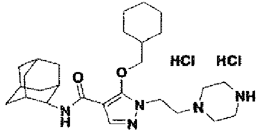 | 1H-NMR (DMSO-d6) δ: 1.14 (dt, J = 56.3, 21.5 Hz, 4H), 1.77 (tt, J = 95.5, 27.9 Hz, 18H), 3.51-3.58 (m, 14H), 3.98 (d, J = 6.3 Hz, 1H), 4.14 (d, J = 5.8 Hz, 2H), 4.46 (t, J = 6.6 Hz, 2H), 7.37 (d, J = 6.8 Hz, 1H), 7.89 (s, 1H), 10.08 (s, 2H). |
| A-44 | 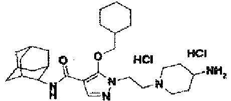 | 1H-NMR (DMSO-d6) δ : 1.00-1.29 (m, 4H), 1.50-2.18 (m, 19H), 3.08-3.57 (m, 8H), 3.97-4.01 (m, 1H), 4.20-4.40 (m, 5H), 7.37 (d, J = 6.8 Hz, 1H), 7.90 (s, 1H), 8.64 (br s, 2H), 11.42 (br s, 1H). |
| A-45 | 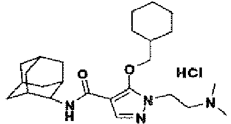 | 1H-NMR (DMSO-d6) δ : 1.03-1.26 (m, 8H), 1.63-1.91 (m, 22H), 2.80 (d, J = 4.2 Hz, 6H), 3.48 (d, J = 5.7 Hz, 2H), 3.97-3.98 (m, 1H), 4.14 (d, J = 6.0 Hz, 2H), 4.34 (t, J = 6.5 Hz, 2H), 7.35 (d, J = 7.0 Hz, 1H), 7.92 (s, 1H). |
| A-46 | 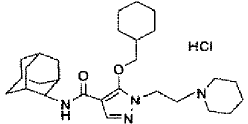 | 1H-NMR (DMSO-d6) δ: 1.05 (t, J = 12.1 Hz, 2H), 1.18-1.38 (m, 4H), 1.77 (ddd, J = 122.7, 76.0, 22.0 Hz, 27H), 2.91-2.93 (br m, 2H), 3.42-3.45 (m, 4H), 3.97 (s, 1H), 4.14 (d, J = 6.1 Hz, 2H), 4.42 (t, J = 6.9 Hz, 2H), 7.35 (d, J = 6.3 Hz, 1H), 7.90 ( |
| A-47 | 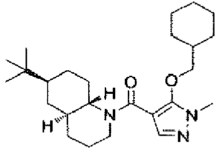 | 300MHz (CDCl3) 0.86 (s, 9H), 1.00-1.82 (m, 23H), 2.28-2.32 (1H), 3.32-3.47 (m, 2H) |
| A-48 | 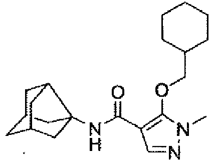 | 400MHz (DMSO) 1.01-1.29 (m, 5H), 1.50-.211 (m, 16H), 2.21 (br, 2H), 2.43 (t, J = 6.4Hz, 1H), 3.59 (s, 3H), 4.07 (d, J = 6.0Hz, 2H), 7.50 (s, 1H), 7.68 (s, 1H) |

**[Table 9]**

| No. | Structure | NMR (CDCl3 or d6-DMSO), MS or m.p. |
|---|---|---|
| A-49 | 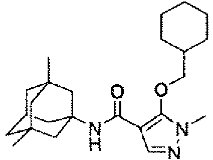 | 400MHz (DMSO) 0.82 (s, 6H), 1.01-1.35 (m, 11H), 1.56-1.84 (m, 12H), 2.09 (brs, 1H), 3.58 (s, 3H), 4.04 (d, J = 6.4Hz, 2H), 6.82 (s, 1H), 7.63 (s, 1H) |
| A-50 | 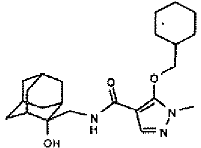 | 300MHz(CDCl3)1.05-2.15(m,25H)3.67(d,J=6.0Hz,2H)3.70(s.3H)3. 98(d,J=6.0Hz,2H)6.52(s,1H)7.74(s,1H) |
| A-51 | 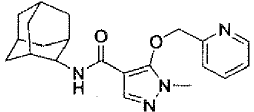 | DMSO-d6 δ 1.42-1.96 (m, 14H), 3.60 (s, 3H), 3.95-3.99 (m, 1H), 5.41 (s, 2H), 7.24-7.30 (m, 1H), 7.32-7.40 (m, 1H), 7.50-7.56 (m, 1H) 7.78-7.88 (m, 2H), 8.56 (d, J=4.4 Hz, 1H). |
| A-52 | 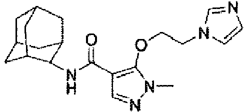 | DMSO-d6 δ 1.42-2.10 (m, 14H), 3.34 (s, 3H), 3.91-4.03 (m, 1H), 4.30-4.41 (m, 2H), 4.47-4.62 (m, 2H), 6.92 (s, 1H), 7.24-7.34 (m, 2H), 7.71 (s, 1H), 7.86 (s, 1H). |
| A-53 | 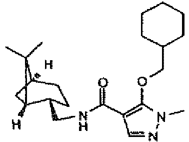 | 300MHz(CDCl3)1.07(s,3H)1.20(s,3H)1.12-1.99(m,18H)2.23-2.29(m,1H)2.34-2.42(m, 1H)3.30-3.75(m,2H)3.71(s,3H)3.96(d,J=6.3Hz,2H)6.1 2(s,1H)7.73(s,1H) |
| A-54 | 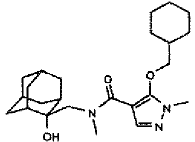 | 300MHz(CDCl3)1.00-1.88(m,24H),2.27-2.31(m,1H)3.22(s,3H)3.66(s,3H)3.80(s,2H)3. 99(d,J=6.3Hz,2H)7.43(s,1H) |

**[Table 10]**

| No. | Structure | NMR (CDCl3 or d6-DMSO), MS or m.p. |
|---|---|---|
| A-55 | 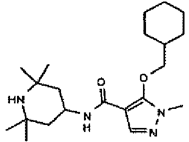 | 300MHz(CDCl3)0.99-2.00(m, 15H)1.20(s,6H)1.32(s,6H)3.70(s,3H) 3.96(d,J=5.7Hz,2H)4.40-4.43(m,1H)5.86(d,J=7.8Hz,1H)7.70(s,1H) |
| A-56 | 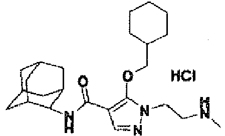 | 1H-NMR (CDCl3) δ : 1.71-2.02 (m, 22H), 2.47 (s, 3H), 3.05 (t, J = 6.0 Hz, 2H), 4.02 (d, J = 6.3 Hz, 2H), 4.10 (t, J = 5.9 Hz, 2H), 4.18-4.21 (m, 1H), 6.41 (d, J = 8.9 Hz, 1H), 7.73(s, 1H). |
| A-57 | 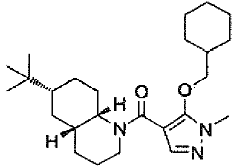 | 400MHz (DMSO) 0.81 (s, 9H), 0.90-2.03 (m, 23H), 2.99 (t, J = 10.8 Hz, 1H), 3.58 (s, 3H), 3.82 (d, J = 10.8Hz, 1H), 3.91 (d, J = 6.4Hz, 2H), 4.33 (br, 1H), 7.31 (s, 1 H) |
| A-58 | 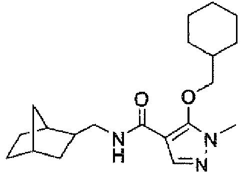 | 400MHz (DMSO) 0.64-0.68 (m, 0.4H), 0.99-2.17 (m, 20.6H), 2.89-3.02 (m, 1.2H), 3.08-3.23 (m, 0.8H), 3.58 (s, 3H), 4.09 (d, J = 7.2Hz, 2H), 7.59 (t, J = 4.8Hz, 0.4H), 7.68 (s, 0.6H), 7.69 (s, 0.4H), 7.74 (t, J = 5.6Hz, 0.6H) |
| A-59 | 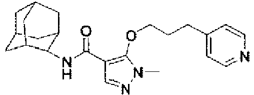 | DMSO-d6 δ 1.51-2.01 (m, 15H), 2.41-2.66 (m, 3H), 3.33 (s, 3H), 3.93-4.11 (m, 3H), 7.19-7.26 (m, 2H), 8.21 (s, 1H), 8.45 (s, 2H), 8.83 (d, J=8.8 Hz, 1H). |
| A-60 | 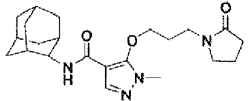 | DMSO-d6 δ 1.41-2.01 (m, 20H), 2.19-2.36 (m, 2H), 3.22-3.48 (m, 2H), 3.63 (s, 3H), 3.96-3.98 (m, 1H), 4.25-4.26 (m, 2H), 7.24 (d, J=6.8 Hz, 1H), 7.81 (s, 1H). |

**[Table 11]**

| No. | Structure | NMR (CDCl3 or d6-DMSO), MS or m.p. |
|---|---|---|
| A-61 | 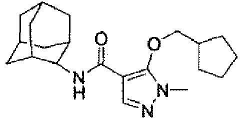 | mp 116-117°C |
| A-62 | 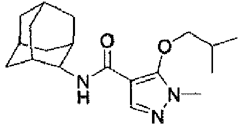 | mp 119-120°C |
| A-63 | 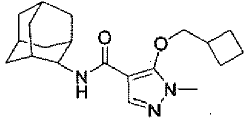 | mp 84-85°C. |
| A-64 | 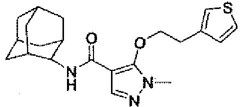 | mp 68-69°C |
| A-65 | 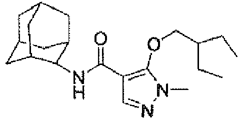 | mp 89-90°C |
| A-66 | 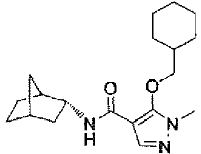 | 400MHz (DMSO) 0.99-1.90 (m, 19H), 2.16 (brs, 1H), 2.35 (brs, 1H), 3.59 (s, 3H), 4.02-4.15 (m, 3H), 7.49 (d, J = 6.8Hz, 1H), 7.75 (s, 1H) |
| A-87 | 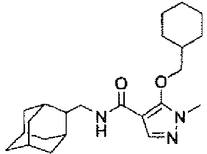 | 400MHz (DMSO) 0.98-1.28 (m, 5H), 1.46-1.93 (m, 21H), 3.29-3.33 (m, 2H), 3.58 (s, 3H), 4.08 (d, J = 6.4Hz, 2H), 7.67 (br, 2H) |

**[Table 12]**

| No. | Structure | No. | Structure |
|---|---|---|---|
| B-1 | 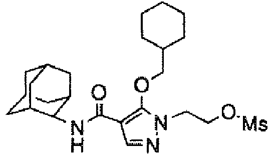 | B-2 | 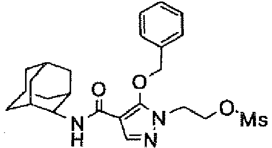 |
| B-3 | 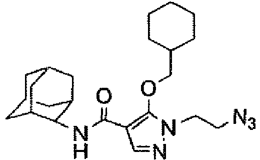 | B-4 | 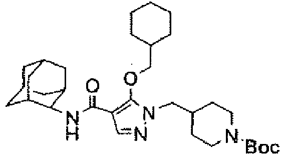 |
| B-5 | 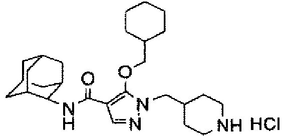 | B-6 | 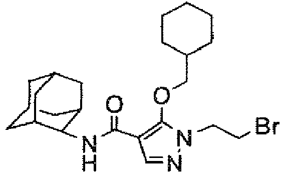 |
| B-7 | 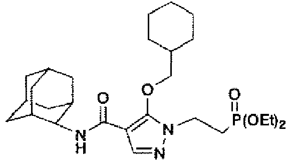 | B-8 | 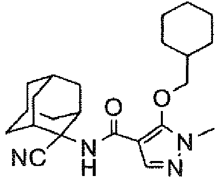 |
| B-9 | 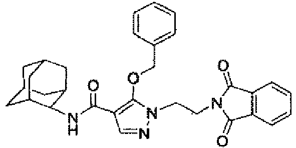 | | |

**[Table 13]**

| No. | Structure | NMR(CDCl3 or d6-DMSO) |
|---|---|---|
| C-1 | 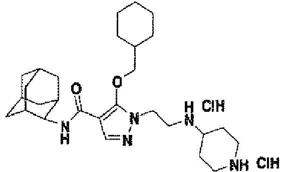 | (DMSO-d6): 0.97-2.26(m,29H), 2.83-2.96(m,2H), 3.25-3.44(m,5H), 3.93-4.01(m,1H), 4.13(d,J=6.4Hz,2H), 4.35(t,J=7.2Hz,2H), 7.32(d,J=6.8Hz,1H), 7.89(s,1H), 9.15(br.s,1H), 9.37(br.s,1H), 9.84(br.s,2H) |
| C-2 | 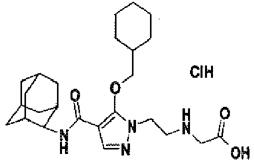 | (DMSO-d6): 0.96-2.06(m,25H), 3.30-3.39(m,2H), 3.87-4.03(m,3H), 4.12(d,J=6.0Hz,2H), 4.31(t,J=6.0Hz,2H), 7.33(d,J=6.8Hz,1H), 7.91(s,1H), 9.42(br.s,2H) |
| C-3 | 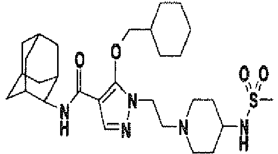 | (DMSO-d6): 0.99-2.11(m,32H), 2.59-2.67(m,2H), 2.72-2.80(m,2H), 2.89(s,3H), 3.93-4.00(m,3H), 4.09(d,J=4.2Hz,2H), 7.01(d,J=7.2Hz,1H), 7.26(d,J=6.0Hz,1H), 7.79(s,1H) |
| C-4 | 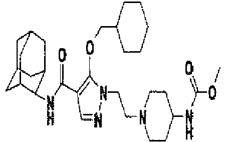 | (DMSO-d6): 1.01-2.08(m,32H), 2.60-2.67(m,2H), 2.73-2.80(m,2H), 3.50(s,3H), 3.94-4,00(m,3H), 4.08(d,J=6.0Hz,2H), 7.04-7.08(m,1H), 7.26(d,J=6.8Hz,1H), 7.79(s,1H) |
| C-5 | 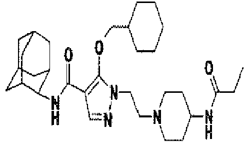 | (DMSO-d6): 0.97(t.J=7.6Hz.3H), 1.01-2.10(m,32H), 2.60-2.68(m,2H), 2.74-2.81 (m,2H), 3.42-3.53(m,2H), 3.94-4.02(m,3H), 4.09(d,J=6.0Hz,2H), 7.26(d,J=6.8Hz,1H), 7.61(d,J=7.2Hz,1H), 7.80(s,1H) |
| C-6 | 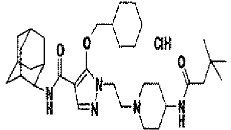 | (DMSO-d6): 0.91-2.08(m,41H), 3.01-3.13(m,2H), 3.32-4.00(m,1H), 4.41-4.49(m,2H), 7.36(d,J=7.2Hz,1H), 7.89(s,1H), 7.99(d,J=7.2Hz,1H), 11.32(br.s,1H) |

**[Table 14]**

| No. | Structure | NMR(CDCl3 or d6-DMSO) |
|---|---|---|
| C-7 | 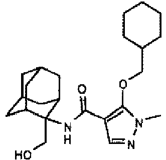 | (CDCl3); 1.05-1.32(m,11H),1.70-2.33(m, 14H),3.15(bs, 1H),3. 75(s,3H),4.0 (d,J =3.0Hz,2H),4.05(s.2H),6.39(s,1H).7.79(s,1H) |
| C-8 | 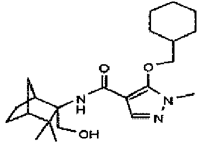 | (CDCl3); 1.07(s,3H),1.14(s,3H),1.23-1.90(m,18 H),2.51-2.52(m,1H),3.62(bs,1 H),3.73(s,3H),3.82(d,J= 11.7Hz,1H),3.95-4.09(m,2H),4.36(d,J=11.7 Hz,1H),6.15(s,1 H), 7.72(s,1H) |
| C-9 | 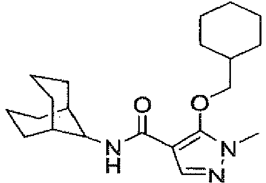 | (DMSO-d6); 1.00-1.92 (m, 25H), 3.62 (s, 3H), 3.79 (brs, 1H), 4.06 (d, J = 6.0Hz, 2H), 7.12 (d, J = 6.4Hz, 1H), 7.73 (s, 1H) |
| C-10 | 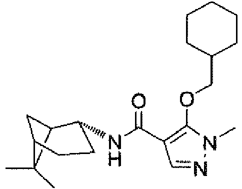 | (DMSO-d6): 0.96-2.28 (m, 25H), 3.59 (s, 3H), 4.00-4.12 (m, 2H), 4.27 (brs, 1H), 7.19 (d, J = 6.0Hz, 1 H), 7.69 (s, 1H) |
| C-11 | 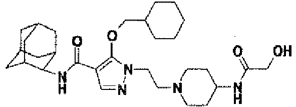 | (DMSO-d6):0.99-2.11(m,32H), 2.61-2.69(m,2H), 2.74-2.81 (m,2H), 3.76(d,J=6.0Hz,2H), 3.95-4.02(m,3H), 4.09(d,J=6.0Hz,2H), 5.37(t,J=6.0Hz,1H), 7.26(d,J=6.4Hz,1H), 7.49(d,J=7.6Hz,1H), 7.79(s,1H) |
| C-12 | 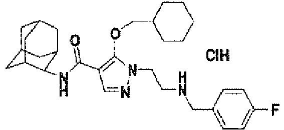 | (DMSO-d6): 0.93-2.03(m,25H), 2.82(t,J=6.4Hz,2H), 3.66(s,2H), 3.94-4.00(m,3H), 4.04(d,J=6.0Hz,2H), 7.10(t,J=8.8Hz,2H), 7.22(d,J=7.2Hz,1H), 7.30(t,J=6.8Hz,2H), 7.78(s,1H) |

**[Table 15]**

| No. | Structure | NMR(CDCl3 or d6-DMSO) |
|---|---|---|
| C-13 | 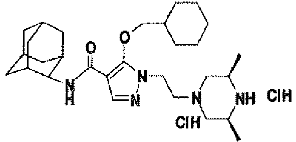 | (DMSO-d6): 0.96-2.07(m,31 H), 2.32-2.56(m,1H), 2.87-3.66(m,6H), 3.93-4.21 (m,6H), 7.31(d,J=7.2Hz,1H), 7.84(s,1H), 9.78(br.s,1H) |
| C-14 | 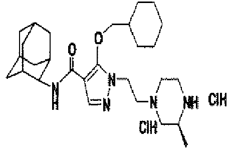 | (DMSO-d6): 0.80-2.17(m,28H), 2.27-2.38(m,1 H), 2.65-2.93(m,5H), 2.99-3.18(m,2H), 3.93-4.13(m,6H), 7.27(d,J=6.8Hz,1H), 7.82(s,1H), 8.32(s, 1H) |
| C-15 | 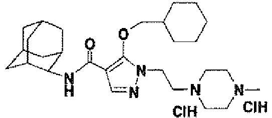 | (DMSO-d6): 0.96-2.07(m,27H), 2.57-3.20(m,11H), 3.93-4.15(m,5H), 7.29(d,J=6.8Hz, H), 7.83(s,1H) |
| C-16 | 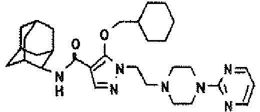 | (DMSO-d6): 1.01-2.04(m,27H), 2.43-2.49(m,2H), 2.69-2.75(m,2H), 3.65-3.71(m,4H), 3.94-4.12(m,5H), 6.61 (t,J=4.8Hz,1H), 7.27(d,J=7.6Hz,1H), 7.80(s, 1H), 8.34(d,J=4.8Hz,1H) |
| C-17 | 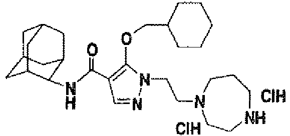 | (DMSO-d6): 0.99-2.23(m,29H), 3.16-3.78(m,8H), 3.93-4.01(m,1H), 4,13(d,J=6.0Hz,2H), 4.43(m,2H), 7.36(d,J=6.8Hz,1H), 7.89(s,1H) |
| C-18 | 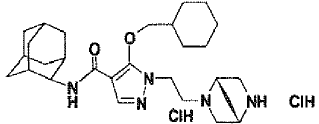 | (DMSO-d6): 0.97-2.46(m,27H), 3.26-4.58(m,13H), 7.37(d,J=6.8Hz,1H), 7.89(s, 1H), 9.83-10.02(br,1H), 10.26-10.39(br,1H) |

**[Table 16]**

| No. | Structure | NMR(CDCl3 or d6-DMSO) |
|---|---|---|
| C-19 | 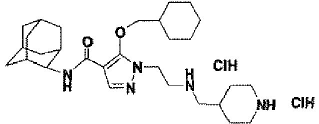 | (DMSO-d6):0.97-2.12(m,30H), 2.77-2.96(m,4H), 3.19-3.35(m,4H), 3.93-4.01 (m,1H), 4.12(d,J=6.0Hz,2H), 4.31-4.40(m,2H), 7.33(d,J=6.8Hz,1H), 7.89(s,1H), 8.92-9.05(br,1H), 9.08-9.19(br,1H), 9.36-9.51(br,1H) |
| C-20 | 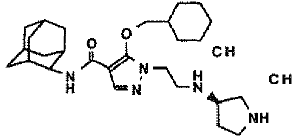 | (DMSO-d6): 0.97-2.39(m,27H), 3.16-3.62(m,6H), 3.91-4.01 (m,4H), 4.13(d,J=6.0Hz.2H), 4.32(t,J=6.8Hz,2H), 7.33(d,J=7.2Hz, 1H), 7.89(s,1H), 9.39-9.53(br,1H), 9.66-9.79(br,1H), 9.88-10.16(br,1H) |
| C-21 | 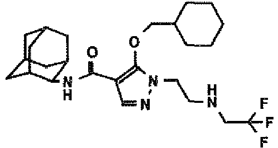 | (CDCl3): 1.04-2.01 (m,27H), 3.99-4.05(m,4H), 4.07-4.12(m,2H), 4.17-4.24(m,1H), 6.44(d,J=7.2Hz, 1H), 7.77(s,1H) |
| C-22 | 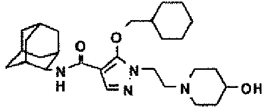 | (DMSO-d6): 0.98-2.14(m,29H), 2.59-3.46(m,7H), 3.92-4.01 (m,3H), 4.09(d,J=5.6Hz,2H), 4.52(s,1H). 7.25(d,J=7.2Hz,1H), 7.79(s,1H) |
| C-23 | 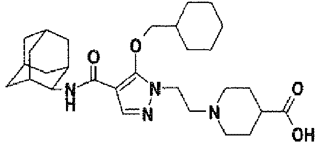 | (DMSO-d6): 0.96-2.33(m,30H), 2.65-^{.} 3.62(m,6H), 3.93-4.16(m,5H), 7.27(d,J=6.4Hz,1H), 7.81 (s,1H) |
| C-24 | 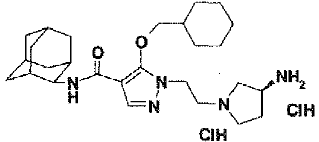 | (DMSO-d6): 0.93-2.07(m,27H), 3.47-3.76(m,7H), 3.91-4.17(m,3H), 4.28-4.41(m,2H), 7.30-7.39(m,1H), 7.87(s,1H), 8.63-8.90(br,2H) |

**[Table 17]**

| No. | Structure | NMR(CDCl3 or d6-DMSO) |
|---|---|---|
| C-25 | 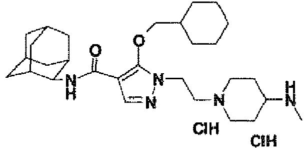 | (DMSO-d6): 0.98-2.32(m,29H), 3.00-4.29(m,13H), 4.41-4.49(m,2H), 7.36(d,J=6.4Hz,1H), 7.90(s,1H), 9.48-9.59(br,1H) |
| C-26 | 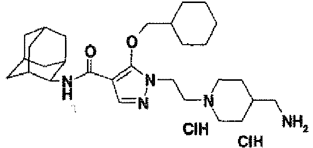 | (DMSO-d6): 0.97-2.07(m,30H), 2.65-4.00(m,9H), 4.13(d,J=7.2Hz,2H). 4.41-4.5 (m,2H), 7.37(d,J=6.4Hz,1H), 7.89(s,1H), 8.26-8.44(br,3H), 11.21-11.37(br,1H) |
| C-27 | 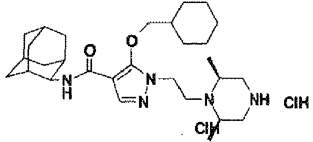 | (DMSO-d6): 0.96-2.08(m,31H), 3.21-3.34(m,2H), 3.44-4.00(m,7H), 4.18(d,J=6.0Hz,2H), 4.30-4.39(m,2H), 7.42(d,J=6.8Hz,1H), 7.90(s,1H), 9.91-10.03(br,1H), 10.12-10,26(br, 1H) |
| C-28 | 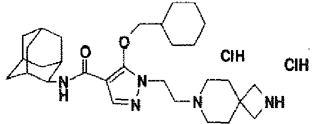 | (DMSO-d6): 0.99-2.26(m,29H), 2.93-3.05(m,2H), 3.35-3.47(m,4H), 3.67-3.81 (m,4H), 3.93-4.00(m,1H), 4.13(d,J=6.0Hz,2H), 4.40-4.47(m,2H), 7.36(d,J=6.4Hz,1H), 7.36(d,J=6.4Hz,1H), 7.89(s,1H), 9.39-9.50(br,2H), 11.20-11.30(br,1H) |
| C-29 | 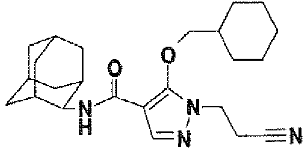 | (DMSO-d6): 0.97-2.09(m,25H), 3.02(t,J=6.0Hz,2H), 3.95-4.00(m,1H), 4.14(d,J=6.0Hz,2H), 4.17(t,J=6.0Hz,2H), 7.38Ed,J=6.8Hz,1H), 7,88(s,1H) |
| C-30 | 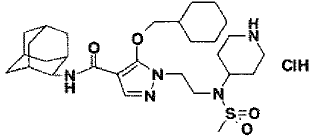 | (CDCl3): 1.06-2.04(m,31H), 2.62-2.76(m,2H), 2.63-2.76(m,2H), 2.91 (s,3H). 3.51 (t,J=6.9Hz,2H), 3.62-3.74(m,1H), 4.03(d,J=6.3Hz,2H), 4.14-4.24(m,3H), 6.44(d,J=7.8Hz, 1H), 7.75(s,1H) |

**[Table 18]**

| No. | Structure | NMR(CDCl3 or d6-DMSO) |
|---|---|---|
| C-31 | 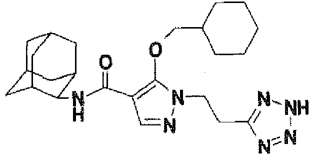 | (CDCl3):1.03-2.04(m,25H), 3.56(t,J=6.0Hz,2H), 3.96(d,J=6.0Hz,2H), 4.16-4.22(m, 1H), 4.40(t,J=6.0Hz,2H), 6.41(d,J=7.5Hz,1H), 7.78(s,1H) |
| C-32 | 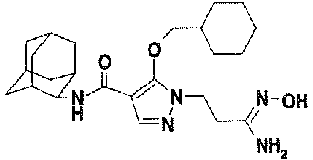 | (CDCl3): 1.03-2.05(m,25H), 2.78(t,J=6.3Hz,2H), 3.89(d,J=6.3Hz,2H), 4.15-4.25(m,3H), 5,25-5.38(br,1H), 6.4(d,J=7.5Hz, 1H), 7.77(s,1H) |
| C-33 | 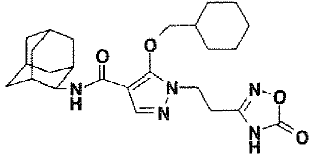 | (DMSO-d6): 0.94-2.06(m,25H), 2.98(t,J=6.4Hz,2H), 3.93-4.00(m,1H), 4.08(d,J=6.0Hz,2H), 4.24(t,J=6.4Hz,2H), 7.31(d,J=6.8Hz,1H), 7.81 (s,1H) |
| C-34 | 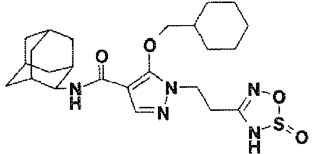 | (DMSO-d6): 0.96-2.07(m,25H), 2.85(t,J=6.4Hz,2H), 3.94-4.00(m, 1H), 4.10(d,J=6.0Hz,2H), 4.29(t,J=6.4Hz,2H), 7.33(d,J=6.8Hz,1H), 7.86(s,1H), 10.99(s,1H) |
| C-35 | 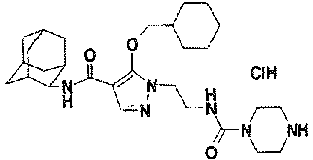 | (DMSO-d6): 0.91-2.06(m,25H), 2.88-3.05(m,4H), 3.28-3.38(m,2H), 3.46-3.61 (m,4H), 3.90-4.11 (m,5H), 7.23(d,J=6.4Hz,1H), 7.86(s,1H), 9.48-9.66(m,2H) |
| C-36 | 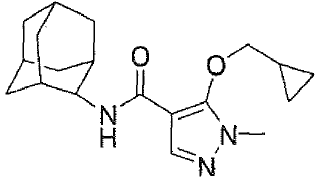 | (DMSO-d6): 1.41-2.08 (m, 14H), 2.38-2.47 (m, 2H), 3.60 (s, 3H), 3.93-4.02 (m, 1H). 4.25-4.38 (m, 2H), 5.00-5.21 (m, 2H), 5.76-5.32 (m, 1H), 7.22 (d, J= 10.0 Hz, 1H), 7.81 (s, 1H). |

**[Table 19]**

| No. | Structure | NMR(CDCl3 or d6-DMSO) |
|---|---|---|
| C-37 | 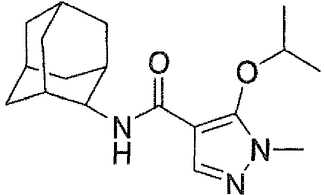 | (DMSO-d6): 1.26 (s, 3H), 1.28 (s, 3H), 1.53 (d, J= 12.4 Hz, 2H), 1.60-2.04 (m, 12H), 3.81 (s, 3H), 3.92-4.03 (m, 1H), 4.80-4.94 (m, 1H), 7.15 (d, J= 6.8 Hz, 1H), 7.83 (s, 1H). |
| C-38 | 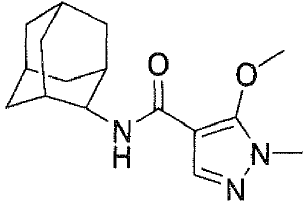 | (DMSO-d6); 1.41-2.11 (m, 14H), 3.37 (s, 3H), 3.64 (s, 1H), 3.89-4.04 (m, 1H), 8.10 (s, 1H), 8.83 (d, J= 8.8 Hz, 1H). |
| C-39 | 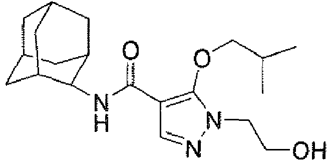 | (DMSO-d6); 0.97 (s, 3H), 0.99 (s, 3H), 1.53 (d, J= 12.4 Hz, 2H), 1.61-2.10 (m, 13H), 3.63-3.78 (m, 2H), 3.85-4.01 (m, 3H), 4.02-4.09 (m, 2H), 4.79-4.92 (m, 1H), 7.19 (d, J= 6.8 Hz, 1H), 7.82 (s, 1H). |
| C-40 | 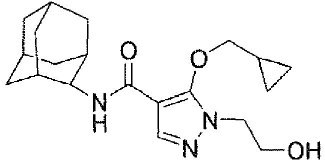 | (DMSO-d6); 0.23-0.37 (m, 2H), 0.46-0.59 (m, 2H), 1.12-1.28 (m, 1H), 1.42-2.06 (m, 14H), 3.64-3.79 (m, 2H), 3.90-4.06 (m, 3H), 4.14 (d, J= 7.6 Hz, 2H), 4.80-4.91 (m, 1H), 7.18 (d, J= 7.2 Hz, 1H), 7.85 (s, 1H). |
| C-41 | 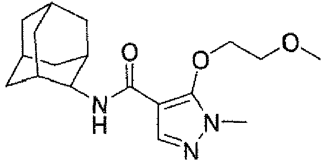 | (DMSO-d6); 1.18 (t, J= 7.2 Hz, 2H), 1.45-2.06 (m, 14H), 3.12-3.40 (m, 7H), 3.92-4.08 (m, 2H), 8.09 (s, 1H), 8.83 (d, J= 8.0 Hz, 1H). |
| C-42 | 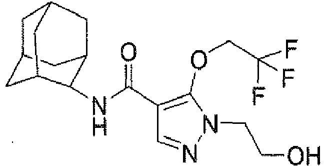 | (DMSO-d6); 1.51 (d, J= 12.4 Hz, 2H), 1.60-2.12 (m, 12H), 3.60-3.73 (m, 2H), 3.90-4.03 (m, 3H), 4.82-4.96 (m, 1H), 4.98-5.12 (m, 2H), 7.44 (d, J= 6.8 Hz, 1H), 8.30 (s, 1H). |

**[Table 20]**

| No. | Structure | NMR(CDCl3 or d6-DMSO) |
|---|---|---|
| C-43 | 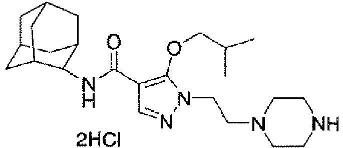 | (DMSO-d6); 0.97 (s, 3H), 0.99 (s, 3H), 1.51 (d, J= 12.4 Hz, 2H), 1.60-2.11 (m, 12H), 3.25-3.62 (m, 12H), 3.90-4.01 (m, 1H), 4.11 (d, J= 6.8 Hz, 2H), 4.35-4.49 (m, 2H), 7.39 (d, J= 6.8 Hz, 1H), 7.91 (s, 1H), 9.98 (brs, 2H). |
| C-44 | 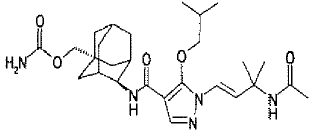 | (DMSO-d6); 0.97(d, J = 6.0Hz, 6H), 1.40-1.58(m, 14H), 1.79(s, 3H), 1.89-2.02(m, 6H), 3.56(s, 2H), 3.91 (brs, 1H), 4.08(d, J = 6.4Hz, 2H), 6.30-6.53(brm, 3H), 6.77(d, J = 14.4Hz, 1H), 7.44(d, J = 6.0Hz, 1H), 7.78(s, 1H), 7.96(s, 1H) |
| C-45 | 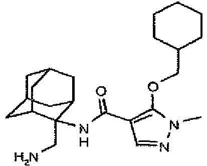 | (CDCl3); 1.06-1.32(m,11H), 1.68-2.46(m,14H)2.22(bs,2H),3.61 (s,2H),3.73(s,3 H),4.09-4.11(m,2H),,6.39(s,1H),7.79(s, 1H) |
| C-46 | 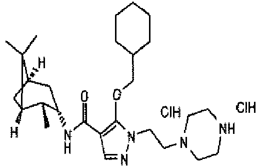 | (DMSO-d6); 1.00(s,3H),1.04(d,J=4.5Hz,3H), 1.22(s,3H),1. 10-2.42(m,18H),3.46-3.71 (m,8H),4.13-4.23(m,2H),4.26-4.38(m,2H),4.32(d,J=6.9Hz,2H)7.78(d,J=8.7 Hz,1H),7.87(s,1H),9.52(bs,2H) |
| C-47 | 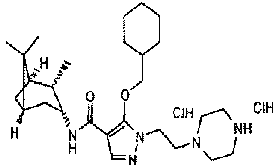 | (DMSO-d6); 1.00(s,3H),1.04(d,J=4.5Hz,3H),1.22(s,3H),1. 09-2.42(m,18H),3.46-3.71(m,8H),4.13-4.23(m,2H),4.26-4.38(m,2H),4.33(d,J=6.9Hz,2H)7.78(d,J=8.4 Hz,1H),7.87(s,1H),9.58(bs,2H) |
| C-48 | 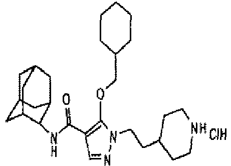 | (DMSO-d6); 1.011-2.03(m,32H),2.73-2.84(m,2H),3.19-3.23(m,2H),3.92-3.97(m,3H),4.08(d,J=6.3Hz,2H),7.30(d,J=7.2 Hz,1H),7.82(s,1H).8.61-9.00(m,2H) |

**[Table 21]**

| No. | Structure | NMR(CDCl3 or d6-DMSO) |
|---|---|---|
| C-49 | 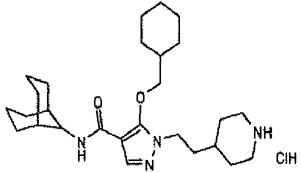 | (DMSO-d6); 1.02-1.93(m,32H),2.77-2.84(m,2H),3.20-3.24(m,2H),3.78-3.90(m,1H)3.95(t,J=7.2Hz,2H),4.08(d,J=6.0 Hz,2H),7.23(d,J=6.6Hz,1H),7.80(s, 1H),8.61-9.00(m,2H) |
| C-50 | 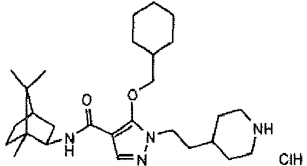 | (DMSO-d6); 0.77(s,3H),0.78(s,3H),0.94(s,3H),0.94-1.83(m.25H),2.74-2.85(m,2H),3.17-3.24(m,2H),3.83-3.96(m,3H),4.01-4.15(m,2H),6.92(d,J=7.8Hz, 1H),7.73(s,2H),8. 48-8.73(m,2H) |
| C-51 | 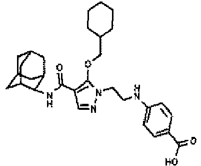 | (DMSO-d6);0.77-1.25(m, 1H),1.50-1.99(m,14H),3.51-3.54(m,2H),3.89(d,J=6.3Hz,2H),3.95-3.97(m,1H),4.01-4.06(m,2H),6.59(d,J=8.7Hz,2H),7.17(d,J=6.9 Hz,1H),7.68(d,J=8.7Hz,2H),7.87(s,1H),12.00 (bs,1H) |
| C-52 | 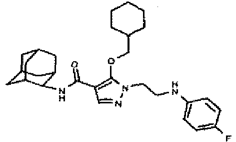 | (CDCl3);0.86-1.31(m,11H),1.67-2.04(m,14H),3.64-3.68(m,2H),3,95(d,J=6.0Hz,2H),4.16-4.21(m,1H),4.26-4.29(m,2H),6.35(d,J=8.4Hz,1H),6.91-7.06(m,4H),7.77(s,1H) |
| C-53 | 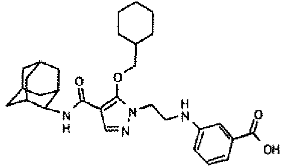 | (DMSO-d6);0.76-1.17(m,11H),1.50-2.00(m,14H),3.47-3.52(m,2H),3.90(d,J=6.3Hz,2H),3.94-3.98(m,1H),4.01-4.06(m,2H),6.06(t,J=6.0Hz,1H),6.78-6.81(m,1H),7.12-7.21 (m,3H),7.86(s,1H),12.62(bs,1H) |
| C-54 | 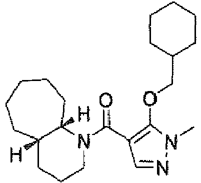 | (DMSO-d6); 0.92-1.99 (m, 24H), 2.50-2.92 (m, 1H), 3.59 (s, 3H), 3.65-4.58 (m, 4H), 7.29 (s, 1H) |

**[Table 22]**

| No. | Structure | NMR(CDCl3 or d6-DMSO) |
|---|---|---|
| C-55 | 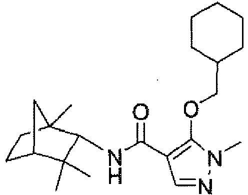 | (DMSO-d6); 0.77 (s, 3H), 1.00-1.79 (m, 24H), 3.62 (s, 3H), 3.67 (d, J = 9.2Hz, 1 H), 4.07 (d, J = 6.0Hz, 2H), 6.78 (d, J = 9.6Hz, 1 H), 7.73 (s, 1 H) |
| C-56 | 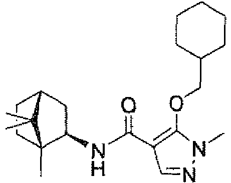 | (DMSO-d6); 0.78 (s, 6H), 0.94 (s, 3H), 1.00-1.80 (m, 18H), 3.60 (s, 3H), 3.89-4.11 (m, 3H), 6.80 (d, J = 8.0Hz, 1H), 7.68 (s, 1H) |
| C-57 | 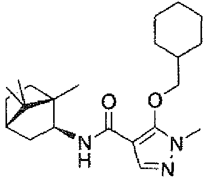 | (DMSO-d6); 0.78 (s, 6H), 0.94 (s, 3H), 0.98-1.80 (m, 18H), 3.60 (s, 3H), 3.89-4.12 (m, 3H), 6.79 (d, J = 8.0Hz, 1H), 7.68 (s, 1H) |
| C-58 | 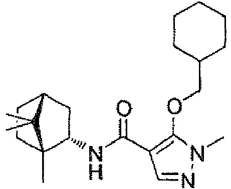 | (DMSO-d6); 0.72 (s, 3H), 0.84 (s, 3H), 0.93 (s, 3H), 0.09-1.80 (m, 17H), 2.14 (brt, J = 10.4Hz, 1H), 3.60 (s, 3H), 4.03 (t, J = 8.0Hz, 1H), 4.12 (t, J = 7.6Hz, 1H), 4.28 (brs, 1H), 7.36 (d, J = 8.0Hz, 1H), 7.76 (s, 1H) |
| C-59 | 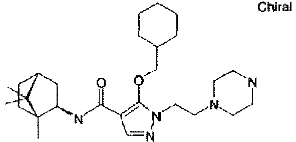 | (DMSO-d6); 0.78 (s, 6H), 0.94 (s, 3H), 0.98-1.80 (m, 18H), 2.32 (brs, 4H), 2.57-2.68 (brm, 6H), 3.89-4.14 (m, 5H), 6.87 (d, J = 7.2Hz, 1H), 7.70 (s, 1H) |
| C-60 | 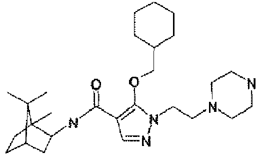 | (DMSO-d6); 0.78 (s, 6H), 0.94 (s, 3H), 0.98-1.80 (m, 18H), 2.31 (brs, 4H), 2.57-2.66 (brm, 6H), 3.89-4.14 (m, 5H), 6.87 (d, J = 7.2Hz, 1H), 7.70 (s, 1H) |

**[Table 23]**

| No. | Structure | NMR(CDCl3 or d6-DMSO) |
|---|---|---|
| C-61 | 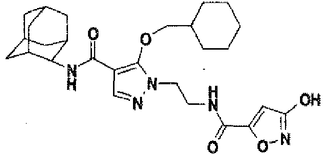 | (DMSO-d6):0.88-2.04(m,25H), 3.50-3.59(m,2H), 3.94-4.12(m,5H), 6.52(s,1H), 7.19(d,J=6.8Hz,1H), 7.84(s, 1H), 8.94(t,J=5.6Hz,1H), 11.66(br.s,1H) |
| C-62 | 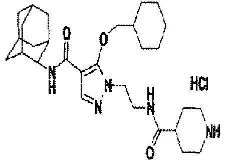 | (DMSO-d6): 0.97-2.04(m,29H), 2.34-2.44(m,1H), 2.77-2.90(m,2H), 3.15-3.26(m,2H), 3.33-3.43(m,2H), 3.93-4.02(m,3H), 4.06(d,J=6.0Hz,2H), 7.23(d,J=6.8Hz,1H), 7.86(s,1H), 8.14(t,J=5.6Hz,1H), 8.96-9.10(br,1H), 9.31-9.44(br,1H) |
| C-63 | 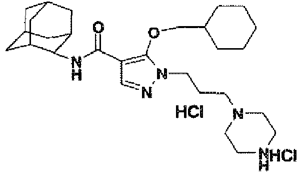 | (DMSO-d6): 0.97-2.31(m,27H), 3.10-4.23(m,14H), 7.29(d,J=6.8Hz,1H), 7.84(s,1H), 9.83-10.12(br,1H) |
| C-64 | 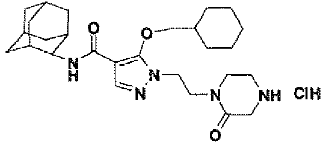 | (DMSO-d6): 0.95-2.07(m,29H), 3.22-3.31 (m,2H), 3.35-3.43(m,2H), 3.93-3.99(m,1H), 4.03-4.14(m,4H), 7.30(d,J=6.0Hz,1H), 7.84(s,1H), 9.89-10.05(br,1H) |
| C-65 | 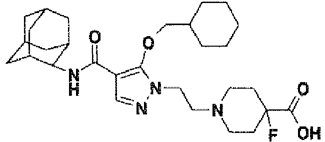 | (CDCl3): 1.05-2.05(m,29H), 2.20-2.33(m,3H), 3.05-3.20(m,2H), 3.50-3.61(m,2H), 4.11 (d,J=6.0Hz,2H), 4.16-4.21(m,1H), 4.56-4.63(m,2H), 6.54(d,J=8.1Hz,1H), 7.71(s,1H) |
| C-66 | 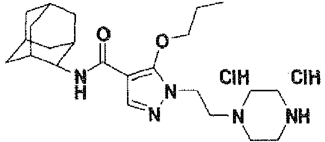 | (DMSO-d6): 0.97(t,J=7.6Hz,3H), 1.48-2.02(m,16H), 2.26-2.74(m,10H), 3.93-4.06(m,3H), 4.22(t,J=6.4Hz,2H), 7.25(d,J=6.4Hz,1H), 7.97(s,1H) |

**[Table 24]**

| No. | Structure | NMR(CDCl3 or d6-DMSO) |
|---|---|---|
| C-67 | 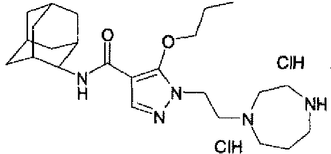 | (DMSO-d6):1.18(t,J=7.2Hz,3H), 1.47-2.04(m,18H), 2.54-2.69(m,6H), 2.72(t,J=6.4Hz,2H), 2.84(t,J=6.4Hz,2H), 3.87-4.04(m,3H), 4.23(t,J=6.4Hz,2H), 7.22(d,J=6.4Hz,1H), 7.78-7.82(br,1H) |
| C-68 | 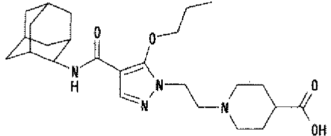 | (DMSO-d6): 0.97(t,J=7.6Hz,3H), 1.43-2.31(m,21H). 2.59-3.44(m,6H), 3.93-4.15(m, 3H), 4.24(t,J=6.4Hz,2H), 7.26(d,J=6.0Hz,1H), 7.83(s,1H) |
| C-69 | 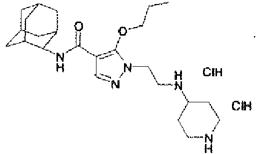 | (DMSO-d6): 0.97(t,J=7.2Hz,3H), 1.44-2.29(m,20H), 2.82-2.98(m,2H), 3.21-3.48(m,5H), 3.93-4.03(m,1H), 4.27(t,J=6.4Hz,2H), 4.34-4.44(m,2H), 7.31(d,J=7.2Hz,1H), 7.93(s,1H), 9.12-9.27(br,1H), 9.49-9.62(br,1H), 9.93-10.05(br,2H) |
| C-70 | 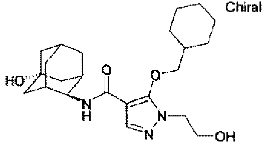 | (CDCl3): 1.01-2.25(m,24H), 3.98-4.05(m,4H), 4.07-4.12(m,2H), 4.14-4.20(m,^{l}H), 6.34(d,J=7.8Hz,1H), 7.76(s,1H) |
| C-71 | 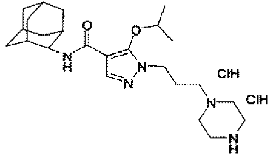 | (CDCl3):1.36(d,J=6.3Hz,6H), 1.65-2.07(m,16H), 2.35(t,J=6.9Hz,2H), 2.37-2.45(m,4H), 2.90(t,J=4.8Hz,4H), 4.01 (t,J=7.2Hz,2H), 4.18-4.23(m, 1H), 4.76-4.84(m,1H), 6.40(d,J=7.5Hz,1H), 7.74(s,1H) |
| C-72 | 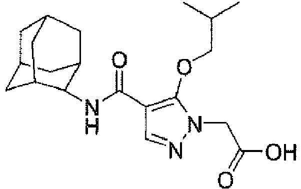 | (DMSO-d6): 0.92 (s, 3H), 0.93 (s, 3H), 1.51 (d, J= 12.4 Hz, 2H), 1.61-2.08 (m, 13H), 3.92-4.00 (m, 1H), 4.06 (d, J= 6.4 Hz, 2H), 4.67-4.76 (m, 2H), 7.35 (d, J= 6.8 Hz, 1H), 7.84 (s, 1H), 13.2 (brs, 1H). |

**[Table 25]**

| No. | Structure | NMR(CDCl3 or d6-DMSO) |
|---|---|---|
| C-73 | 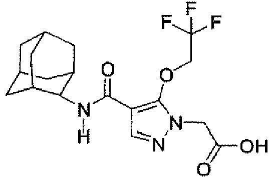 | (DMSO-d6); 1.51 (d, J= 12.4 Hz, 2H), 1.60-2.14 (m, 12H), 3.94-4.02 (m, 1H), 4.77 (s, 2H), 4.94-5.11 (m, 2H), 7.54 (d, J= 6.4 Hz, 1H), 8.08 (s, 1H), 13.3 (brs, 1H). |
| C-74 | 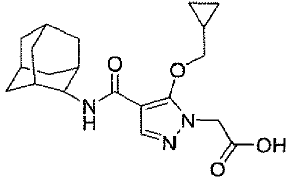 | (DMSO-d6): 0.19-0.31 (m, 2H), 0.44-0.56 (m, 2H), 1.08-1.21 (m, 1H), 1.52 (d, J= 12.8 Hz, 2H), 1.61-2.09 (m, 12H), 3.92-4.01 (m, 1H), 4.12 (d, J= 7.6 Hz, 2H), 4.76 (s, 2H), 7.30 (d, J= 7.2 Hz, 1H), 7.88 (s, 1H), 13.3 (brs, 1H). |
| C-75 | 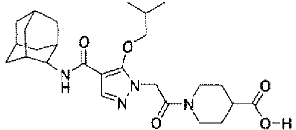 | (DMSO-d6); 0.89 (s, 3H), 0.91 (s, 3H), 1.25-2.10 (m, 16H), 2.67-2.85 (m, 1H), 3.01-3.22 (m, 1H), 3.49-3.66 (m, 2H), 3.73-4.21 (m, 5H), 4.76-5.03 (m, 4H), 7.26 (s, 1H), 7.83 (s, 1H), 11.93 (brs, 1H). |
| C-76 | 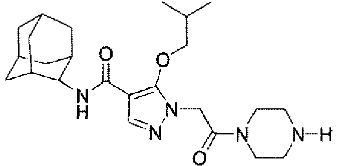 | (DMSO-d6); 0.91 (s, 3H), 0.93 (s, 3H), 1.53 (d, J= 12.8 Hz, 2H), 1.61-2.09 (m, 13H), 2.56-2.75 (m, 5H), 3.28-3.48 (m, 4H), 3.91-4.08 (m, 3H), 4.84-4.96 (m, 2H), 726 (d, J= 6.4 Hz, 1H), 7.83 (s, 1H). |
| C-77 | 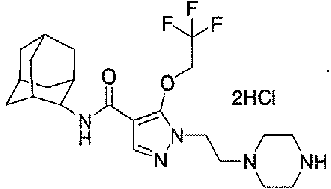 | (DMSO-d6); 1.51 (d, J= 12.4 Hz, 2H), 1.59-2.14 (m, 12H), 3.26-3.71 (m, 11H), 3.92-4.04 (m, 1H), 4.35-4.51 (m, 2H), 5.10-5.26 (m, 2H), 7.57 (d, J= 6.4 Hz, 1H), 8.18 (s, 1H), 10.06 (brs, 2H). |
| C-78 | 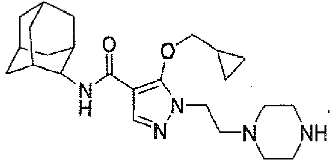 | (DMSO-d6); 0.25-0.30 (m, 4H), 0.49-0.53 (m, 4H), 1.52 (d, J= 11.8 Hz, 2H), 1.63-2.12 (m, 19H), 3.81-4.28 (m, 6H), 7.23 (s, 1H), 7.83 (s, 1H). |

**[Table 26]**

| No. | Structure | NMR(CDCl3 or d6-DMSO) |
|---|---|---|
| C-79 | 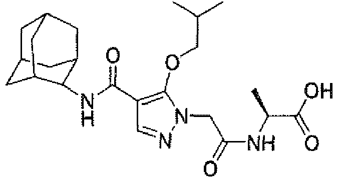 | (DMSO-d6); 0.92 (s, 3H), 0.94 (s, 3H), 1.10-1.21 (m, 1H), 1.23-1.37 (m, 3H), 1.53 (d, J= 11.6 Hz, 2H), 1.61-2.11 (m, 12H), 3.85-4.11 (m, 3H), 4.12-4.33 (m, 1H), 4.53-4.75 (m, 2H), 7.26 (d, J= 6.0 Hz, 1H), 7.83 (s, 1H), 8.46 (d, J= 6.0 Hz, 1H), 12.5 (brs, 1H). |
| C-80 | 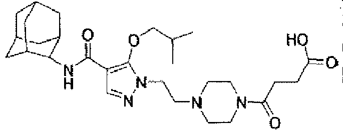 | (DMSO-d6); 0.97 (s, 3H), 0.99 (s, 3H), 1.52 (d, J= 11.6 Hz, 2H), 1.62-2.12 (m, 12H), 2.37-2.62 (m, 6H), 2.85-3.25 (m, 3H), 3.38-3.61 (m, 4H), 3.90-4.01 (m, 1H), 4.02-4.18 (m, 3H), 4.35-4.51 (m, 3H), 7.37 (d, J= 6.8 Hz, 1H), 7.91 (s, 1H), 11.72 (brs, 1H). |
| C-81 | 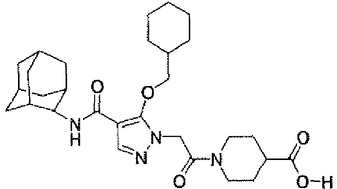 | (DMSO-d6); 0.80-1.03 (m, 2H), 1.05-1.28 (m, 4H), 1.29-1.42 (m, 1H), 1.43-2.07 (m, 22H), 2.48-2.58 (m, 1H), 2.67-2.83 (m, 1H), 3.03-3.19 (m, 1H), 3.77-4.23 (m, 5H), 4.80-5.02 (m, 2H), 7.24 (d, J= 6.8 Hz, 1H), 7.81 (s, 1H), 12.29 (brs, 1H). |
| C-82 | 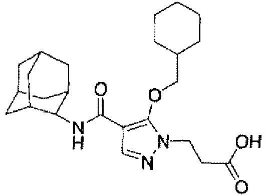 | (DMSO-d6); 0.90-1.31 (m, 6H), 1.52 (d, J= 12.4 Hz, 2H), 1.57-2.07 (m, 17H), 2.69-2.81 (m, 2H), 3.88-4.00 (m, 1H), 4.02-4.18 (m, 4H), 7.25 (d, J= 7.2 Hz, 1H), 7.78 (s, 1H), 12.33 (brs, 1H). |
| C-83 | 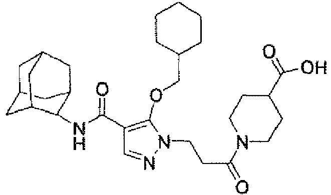 | (DMSO-d6): 0.92-2.05 (m, 28H), 2.40-2.49 (m, 1 H), 2.64-2.78 (m, 1 H), 2.79-2.91 (m, 2H), 2.97-3.12 (m, 1H), 3.65-3.80 (m, 1H), 3.85-4.22 (m, 7H), 7.19 (d, J= 6.8 Hz, 1H), 7.78 (s, 1H), 12.23 (brs, 1H). |
| C-84 | 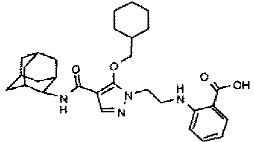 | (CDCl3);0.90-1.28(m,11H),1.58-2.11(m,14H),3.71-3.81 (m,2H),3.94(d,J=5.7Hz,2H),4.12-4.23(m,1H),4.23-4.27(m,2H),6.43(d,J=6.9Hz,1H),6.73-6.78(m,1H),6.88-6.91(m,1H),7.39-7.44(m,1H),7.82(s,1H),7.98-8.01 (m,1H) |

**[Table 27]**

| No. | Structure | NMR(CDCl3 or d6-DMSO) |
|---|---|---|
| C-85 | 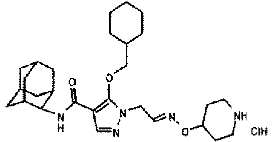 | (DMSO-d6); 0.99-1.25 (m, 11H), 1.50-2.03(m, 18H), 2.94-3.10 (m, 2H), 3.10-3.25(m, 2H), 3.96-3.98 (m, 1H), 4.08-4.12(m, 2H), 4.23-4.36 (m, 1H), 4.71 (d,J=4.8Hz,2H),6.99-7.02 (m, 1H), 7.32-7.36 (m, 1H), 7.84(s, 1H), 8.76-8.85 (m, 2H) |
| C-86 | 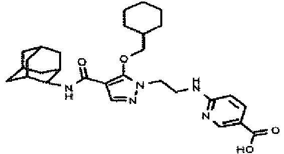 | (CDCl3); 0.86-1.28 (m, 11H), 1.66-1.98(m, 14H), 3.89-4.01 (m, 4H), 4.11 - 4.23 (m, 1H), 4.23-4.35 (m, 2H), 6.47(d, J=8.1 Hz, 1H), 6.65(d, J=8.1 Hz, 1H), 7.76(s, 1H), 8.14(d, J=8.1 Hz, 1H), 8.61 (s , 1H) |
| C-87 | 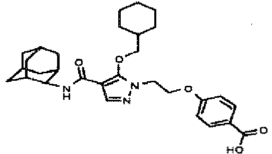 | (CDCl3); 1.04-1.32 (m, 11H), 1.68-2.06(m, 14H), 4.12 (d, J=7.2Hz,2H), 4.19-4.22(m, 1H),4.37-4.40(m,2H),4.45-4.49(m, 2H), 6.41 (d, J=8.1 Hz, 1H), 6.89 (d, J=9.0 Hz, 2H), 7.79(s, 1H), 8.03(d, J=9.0Hz, 2H) |
| C-88 | 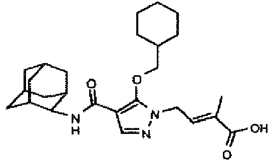 | (CDCl3);0.96-1,33(m, 11H), 1.27(s, 3H), 1.41-2.00(m, 14H), 4.03(d, J=6.3Hz, 2H), 4.18-4.22(m, 1H), 4.78(d, J=6.0Hz, 2H), 6.34 (d, J=8.7 Hz, 1H), 8.84-6.91 (m,1H), 7.74 (s, 1H) |
| C-89 | 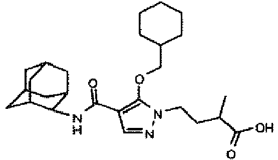 | (CDCl3); 0.86-1.43(m, 14H), 1.61-2.00(m, 14H), 2.01-2.22 (m, 2H), 2.48-2.40(m, 1H),3.95-4.25(m, 3H), 6.37 (bs, 1H), 7.74(s, 1H) |
| C-90 | 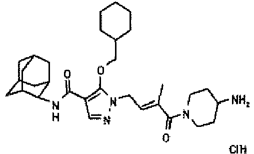 | (CDCl3); 1.00-1.35 (m, 1H),1.59-2.11 (m, 16H) 2.09(s, 3H), 2.20-2.38(m, 2H), 2.77-3.12(m, 4H), 3.41- 3.60(m, 1H),4.12-4.20(m, 1H) 4.21-4.54(m,2H), 4.85-5.11 (m, 2H), 5.63-5.74(m,1H), 7.43(bs, 1H), 8.52-8.74(m,3H) |

**[Table 28]**

| No. | Structure | NMR(CDCl3 or d6-DMSO) |
|---|---|---|
| C-91 | 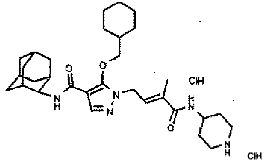 | (CDCl3); 1.02-1.33(m, 11H), 1.66-2.10(m, 18H), 1.88(s, 3H), 2.70-3.09(m, 4H), 3.42-3.60 (m,1H),4.06-4.10(m,5H),4.73(d,J=5.4Hz, 1H), 6.4 - 6.03(m,1H), 6.75(d, J=7.2Hz, 1H), 7.27-7.40(m, 1H),8.91 (s, 1H), 9.36(bs, 1H), 9.57 (bs, 1 H) |
| C-92 | 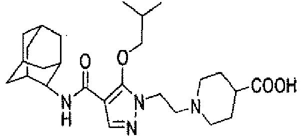 | (DMSO-d6); 0.98 (d, J = 6.6Hz, 6H), 1.50-2.09 (m, 20H), 2.93-3.61 (m, 6H), 3.98 (d, J = 6.3Hz, 1H), 4.10 (d, J = 6.3Hz, 2H), 4.39 (m, 2H), 7.38 (m, 1H), 7.90 (s, 1H), 12.35 (br, 1H) |
| C-93 | 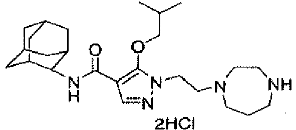 | (DMSO-d6); 0.98 (d, J = 6.6Hz, 6H), 1.50-1.54 (m, 2H), 1.71-2.18 (m, 17H), 3.29 (m, 4H), 3.72 (m, 4H), 3.98 (m, 1H), 4.11 (d, J = 6.6Hz, 2H), 4.44 (t, J = 6.6Hz, 2H), 7.40 (d, J = 6.6Hz, 1H), 7.92 (s, 1H), 9.49-9.71 (m, 2H), 11.79 (br, 1H) |
| C-94 | 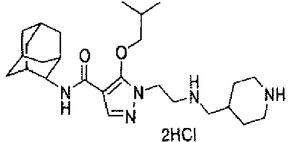 | (DMSO-d6); 0.98 (d, J = 6.3Hz, 6H), 1.36-1.54 (m, 4H), 1.71-2.11 (m, 16H), 2.80-2.90 (m, 4H), 3.24-3.29 (m, 4H), 3.96-3.99 (m, 1H), 4.10 (d, J = 6.6Hz, 2H), 4.34 (t, J = 6.6Hz, 2H), 7.37 (d, J = 6.9Hz, 1H), 7.92 (s, 1H), 8.87-9.01 (m, 2H), 9.33 (br, 2H) |
| C-95 | 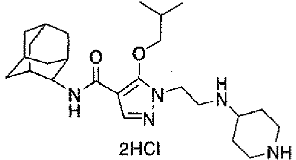 | (DMSO-d6); 0.98 (d, J = 6.6Hz, 6H), 1.50-1.54 (m, 2H), 1.71-2.12 (m, 15H), 2.19-2.23 (m, 2H), 2.83-2.95 (m, 2H), 3.35-3.39 (m, 5H), 3.98 (d, J = 6.9Hz, 1H), 4.10 (d, J = 6.6Hz, 2H), 4.34 (t, J = 6.6Hz, 2H), 7.36 (d, J = 6.9Hz, 1H), 7.92 (s, 1H), 9.09-9.26 (m, 2H), 9.74 (br, 2H) |
| C-96 | 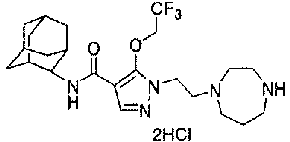 | (DMSO-d6); 1.49-2.16 (m, 16H), 3.21-3.71 (m, 10H), 4.00 (d, J = 6.6Hz, 1H), 4.43 (m, 2H), 5.19 (q, J = 9.0Hz, 2H), 7.58 (d, J = 6.6Hz, 1H), 8.18 (s, 1H), 9.49-9.70 (m, 2H), 11.66 (m, 1H) |

**[Table 29]**

| No. | Structure | NMR(CDCl3 or d6-DMSO) |
|---|---|---|
| C-97 | 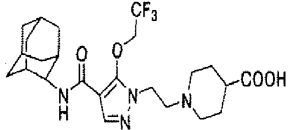 | (DMSO-d6); 1.47-2.07 (m, 21H), 3.17-3.40 (m, 4H), 3.97-4.40 (m, 3H), 5.13 (q, J = 8.4Hz, 2H), 7.51 (d, J = 6.9Hz, 1H), 8.11 (s, 1H), 12.35 (br, 1H) |
| C-98 | 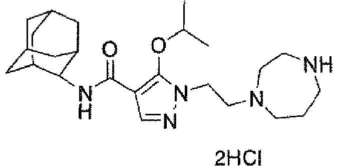 | (DMSO-d6); 1.29 (d, J = 6.0Hz, 6H), 1.51-2.15 (m, 16H), 3.24-3.51 (m, 10H), 3.98 (d, J = 7.2Hz, 1H), 4.40 (m, 2H), 4.93-5.02 (m, 1H), 7.31 (d, J = 7.2Hz, 1H), 7.97 (s, 1H), 9.45 (brs, 2H), 11.77 (brs, 1H) |
| C-99 | 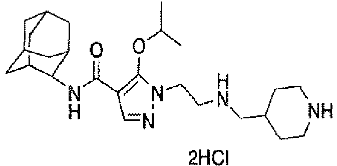 | (DMSO-d6); 1.29 (d, J = 6.3Hz, 6H), 1.40-1.56 (m, 4H), 1.71-2.03 (m, 15H), 2.80-2.91 (m, 4H), 3.26-3.29(m, 4H), 3.99 (d, J = 6.9Hz, 1H), 4.35 (t, J = 6.9Hz, 2H), 4.91-5.00 (m, 1H), 7.30 (d, J = 6.9Hz, 1H), 7.98 (s, 1H), 8.80-8.94 (m, 2H), 9.27 (m, 2H) |
| C-100 | 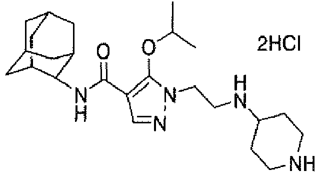 | (DMSO-d6); 1.30 (d, J = 6.0Hz, 6H), 1.52-2.24 (m, 18H), 2.88-2.92 (m, 2H), 3.30-3.41 (m, 5H), 4.01 (m, 1H), 4.35 (t, J = 6.9Hz, 2H), 4.92-5.00 (m, 1H), 7.31 (d, J = 6.6Hz, 1H), 8.00 (s, 1H), 9.05-9.17 (m, 2H), 9.72 (m, 2H) |
| C-101 | 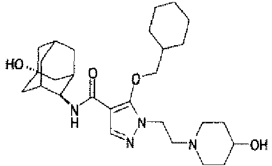 | (DMSO-d6):0.96-2.12(m,30H), 2.57-2.72(m,4H), 3.36-3.47(m,1H), 3.84-3.91 (m,1H), 3.97(t,J=6.4Hz,2H), 4.08(d,J=6.0Hz,2H), 4.43(s,1H), 4.54(d,J=4.0Hz,1H), 7.23(d,J=6.8Hz, 1H), 7.79(s, 1H) |
| C-102 | 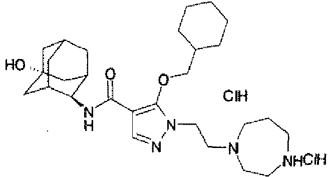 | (DMSO-d6): 0.93-2.30(m,28H), 3.06-3.94(m,9H), 4.07-4.21 (m,2H), 4.36-4.54(m,2H), 7.35(br.s, 1H), 7.90(s, 1H), 9.67-10.19(m, 2H), 11.87-12.18(br, 1H) |

**[Table 30]**

| No. | Structure | NMR(CDCl3 or d6-DMSO) |
|---|---|---|
| C-103 | 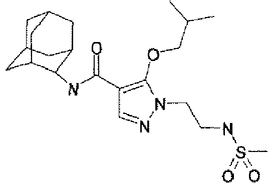 | (CDCl3): 1.05(d,J=6.6Hz,6H). 1.66-2.05(m,15H), 2.94(s,3H), 3.60-3.67(m,2H), 4.08(d,J=6.9Hz,2H), 4.17-4.24(m,3H), 6.41 (d,J=7.8Hz,1H), 7.78(s,1H) |
| C-104 | 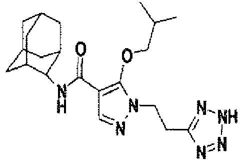 | (CDCl3): 1.04(d,J=6.6Hz,6H), 1.67-2.19(m,15H), 3.59(t,J=6.6Hz,2H), 4.12(d,J=6.6Hz,2H), 4.15-4.21(m.1H), 4.59(t,J=6.6Hz,2H), 7.72(d,J=8.4Hz,1H), 8.06(s,1H) |
| C-105 | 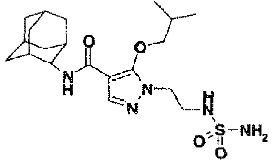 | (CDCl3): 1.05(d,J=6.6Hz,6H). 1.65-2.19(m,15H), 3.59(t,J=5.4Hz,2H), 4.09(d,J=6.6Hz.2H), 4.16-4.28(m.3H), 6.47(d,J=7.2Hz,1H), 7.79(s,1H) |
| C-106 | 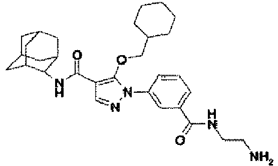 | (DMSO-d6): 0.76-2.11 (m,25H), 3.04-3.40(m,4H), 3.94-4.07(m,3H), 7.45-7.93(m,4H), 8.04(s,1H), 8.14(s,1H), 8.56-8.65(br,1H) |
| C-107 | 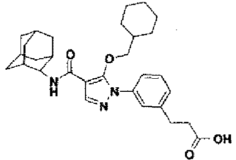 | (DMSO-d6): 0.80-2.08(m,25H), 2.57(t,J=7.6Hz,2H), 2.90(t,J=7.6Hz,2H), 3.93-4.06(m,3H), 7.24-7.31 (m,1H), 7.38-7.51 (m,4H), 7.98(s,1H), 12.15(br.s, 1H) |
| C-108 | 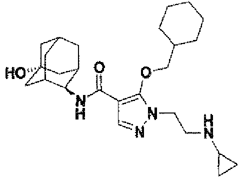 | (CDCl3): 0.42-0.51 (m,4H), 1.01-2.24(m,25H), 3.18(d,J=6.3Hz,2H), 4.02(d,J=6.3Hz,2H), 4.09-4.20(m,3H), 6.28(d,J=7.5Hz,1H), 7.73(s,1H) |

**[Table 31]**

| No. | Structure | NMR(CDCl3 or d6-DMSO) |
|---|---|---|
| C-109 | 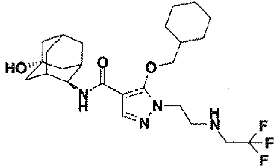 | (DMSO-d6):0.94-2.08 (m, 24H), 2.90-3.01(m,2H), 3.16-3.31 (m,2H), 3.84-4.01(m, 3H), 4.08(d,J=5.2Hz,2H), 4.33-4.46(br,1H), 7.21 (d,J=6.4Hz,1H), 7.80(s, 1H) |
| C-110 | 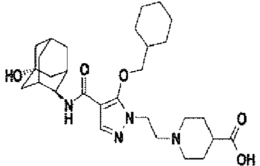 | (DMSO-d6): 0.92-2.27(m,31H), 2.56-2.88(m,4H), 3.79-4.18(m,6H), 7.22(br.s, 1H), 7.80 (s, 1H) |
| C-111 | 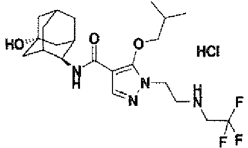 | (CDCl3): 1.04(d, J=6.6Hz,6H), 1.51-2.24(m, 14H), 3.16-3.26(m, 4H), 4.03(d,J=6.6Hz,2H)< 4.10(t,J=5.4Hz.2H), 4.13-4.20(m,1H), 6.23(d,J=6.3Hz,1H), 7.72(s,1H) |
| C-112 | 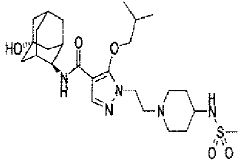 | (CDCl3): 1.06(d,J=6.6Hz,6H), 1.44-2.23(m,22H), 2.39-2.56(m,2H), 3.02-3.53(m,3H), 4.07(d,J=6.6Hz,2H), 4.12-4.19(m, 1H), 4.22-4.40(br, 1H), 6.21 - 6.32(br, 1H), 7.69(s, 1H) |
| C-113 | 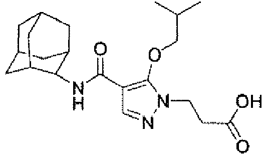 | (DMSO-d6); 0.97 (s, 3H), 0.99 (s, 3H), 1.52 (d, J= 12.0 Hz, 2H), 1.60-2.09 (m, 13H), 2.76 (t, J= 6.8 Hz), 3.88-4.01 (m, 1H), 4.06 (d, J= 6.8 Hz, 2H), 4.13 (t, J= 6.8 Hz, 2H), 7.27 (d, J= 6.8 Hz, 1H), 7.80 (s, 1H), 12.33 (brs, 1H). |
| C-114 | 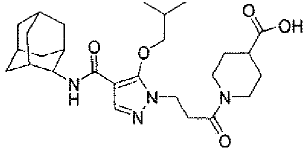 | (DMSO-d6); 0.97 (s, 3H), 0.98 (s, 3H), 1.19-1.57 (m, 5H), 1.61-2.09 (m, 15H), 2.62-2.78 (m, 1H), 2.79-2.90 (m, 2H), 2.96-3.11 (m, 1H), 3.65-3.80 (m, 1H), 3.90-4.22 (m, 6H), 7.22 (d, J= 6.4 Hz, 1H), 7.80 (s, 1H), 12.23 (brs, 1H). |

**[Table 32]**

| No. | Structure | NMR(CDCl3 or d6-DMSO) |
|---|---|---|
| C-115 | 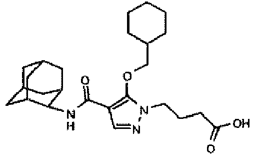 | (CDCl3);1.00-1.35(m,11H),1.67-1.88(m,14H),3.27(d,J=6.0Hz,2H),4.01 (d,J=6. 0Hz,2H),4.18-4.21 (m,1H),6.37(dt, J=6.0, 14.1 Hz,1H), 6.48(d, J=7.8Hz, 1H), 6.86(d, J=14.1Hz, 1H), 7.84(s, 1H ) |
| C-116 | 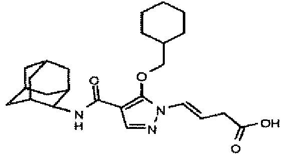 | (CDCl3);1.01-1.39(m,11H),1.35-2.05(m,14H),2.05-2.17(m,2H),2.37-2.42(m,2H),4.02(d,J=6.3Hz,2H),4.06-4.11 (m,2H),4.18-4.21 (m,1H),6.38(d,J=7.8Hz, 1H),7.73(s, 1H) |
| C-117 | 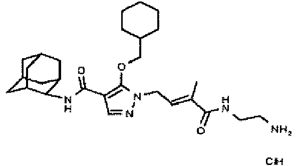 | (DMSO-d6);0.99-1.24(m,11H),1.50-2.03(m, 14H), 1.91 (s,3H),2.84-2.90(m,2H),3.36-3.50(m,2H),3.96-4.00(m, 1H), 4.08(d, J=6.0Hz, 2H), 4,7 (d, J=6.0 Hz,2H), 6.30 (t, J=6.0Hz, 1H), 7.31 (d, J=7.2Hz, 1 H), 7.83(s, 1H), 7.89(bs, 2H), 8.10-8.18(m, 1H) |
| C-118 | 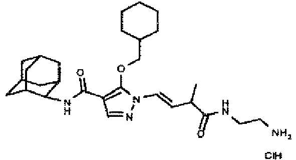 | (DMSO-d6); 1.03-1.22 (m, 1H), 1.50-2.04(m, 17H), 2.81-2.96(m, 2H), 3.19-3.50 (m, 2H), 3.60-3.72 (m, H), 3.94-4.00(m, 1H), 4.12 (d, J=5.1Hz, 2H), 6.18 (dd, J=8. 4, 14.4Hz, 1H), 6.89 (d, J=14.4Hz, 1H), 7.44(d, J= 6.9Hz, 1H), 7.95 (s, 1H),8.01 (bs, 1H), 8.27 (t, J=5. 4Hz, 1H) |
| C-119 | 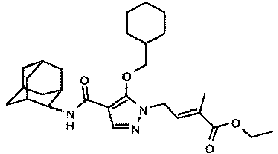 | (CDCl3); 1.02-1.34(m, 14H), 1.67-2.00(m, 14H), 1.98(s., 3H), 4.03(d, J=6.3Hz, 2H), 4.16-4.21 (m, 1H), 4.19(q, J=6.9Hz, 2H), 4.76(d,J=6.3 Hz, 2H), 6.35(d, J=8.1Hz, 1H), 6.76(t, J=6.3Hz, 1 H), 7.73(s, 1H) |
| C-120 | 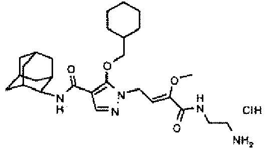 | (DMSO-d6); 1.03-4.26 (m, 11H),1.56-2.02(m, 14H), 2.92-3.00 (m, 2H), 3.24-3.21 (m,2H),3.39(s,3H),3.46-3.57(m, 2H), 3.99-4.02 (m, 1H), 4.48-4.51 (m, 2H), 4.64-4.70 (m, 1H) 7.90 (d,J=7.5Hz, 1H), 8.22(bs, 2H), 9 .08 (t, J=6.0Hz, 1H), 9.20(s, 1H) |

**[Table 33]**

| No. | Structure | NMR(CDCl3 or d6-DMSO) |
|---|---|---|
| C-121 | 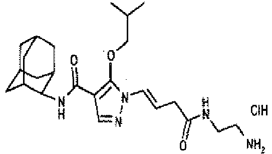 | (DMSO-d6);0.96(d,J=6.6Hz,6H),1.49-2.07(m,15H),2.83-2.91(m,2H),3.07(d,J=6.9Hz,2H),3.28-3.40(m,2H),3.96-4.00(m,1H),4.10(d,J=6.6Hz,2H),6.20(dt,J=6. 9,14.1Hz,1H),6.94(d,J=14.1Hz,1H),7.48(d,J= 6.9Hz,1H),7.92(s,1H),7.96(bs,2H),8.28(t,J=6. 0Hz.1H) |
| C-122 | 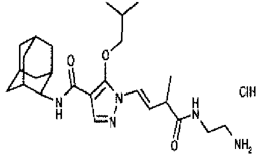 | (DMSO-d6);0.96(d,J=6.6Hz,6H),1.21(d,J=6.9Hz,3H), 1.71-2.04(m,14H),2.82-2.88(m,2H),3.20-3.40(m,4H),3.95-3.98(m,1H),4.10(d,J=6.3Hz,2H),6.19(dd,J=8. 1,14.1Hz,1H),6.90(d,J=14.1Hz,H),7.46(d,J= 7.2Hz,1H),7.97(bs,2H),8.25(t,J=5.5Hz,1H) |
| C-123 | 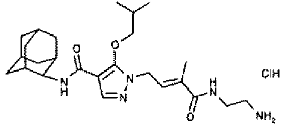 | (DMSO-d6);0.95(d,J=6.9Hz,6H),1.50-2.05(m,18H),2.84-2.90(m,2H),3.32-3.38(m,2H),3.95-3.98(m,1H),4.06(d,J=6.6Hz,2H),4.73(d,J=6.6 Hz,2H),6.31(t,J=6.6Hz,1H),7.34(d,J=6.9Hz,1 H),7.86(s,1H),7.97(bs,2H),8.15(t,J=5.4Hz,1H ) |
| C-124 | 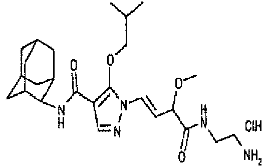 | (DMSO-d6);0.95(d,J=6.9Hz,6H),1.49-2.06(m,15H),2.84-2.97(m,2H),3.19-3.35(m,2H),3.34(s,3H),3.96-3.99(m,1H),4.14(d,J=6.6Hz,2H),4.01-4.20(m,1H),0.06(dd,J=7.6,14.1Hz,1H),7.12(d, J=14.1Hz,1H),7.54(d,J=6.6Hz,1H),7.96(bs,2 H),7.99(s,1H),8.28(t,J=6.0Hz,1H) |
| C-125 | 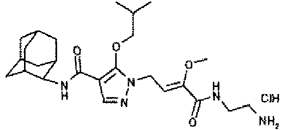 | (DMSO-d6);0.99(d,J=6,6Hz,6H),1.53-2.13(m,15H),2.95-3.00(m,2H),3.15-3.23(m,2H),3.40(s.3H),4.01-4.04(m,1H),4.40-4.81(m,5H),8.02(d,J=6.9Hz,1H),8.28(bs,2H), 9.11(t,J=5.4Hz,1H),9.25(s,1H) |
| C-126 | 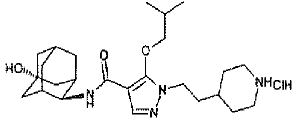 | (DMSO-d6);0.97(d,J=6.9Hz,6H),1.32-2.07(m,21H),2.73-2,89(m,2H),3.19-3.24(m,2H),3.83-3.90(m,1H),3.95(t,J=6.9Hz,2H),4.06(d,J=6.3 Hz,2H),7.27(d,J=6,3Hz,1H),7.84(s,1H),8,70( bs,1H),8.92(bs,1H) |

**[Table 34]**

| No. | Structure | NMR(CDCl3 or d6-DMSO) |
|---|---|---|
| C-127 | 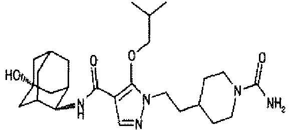 | (CDCl3);1.05(d,J=6.6Hz,6H),1.14-2.20(m,21H),2.75-2.85(m,2H),3.92-4.04(m,4H),4.00(d,J=6.9Hz,2H),6.21(d,J=7.5 Hz,1H),7.68(s,1H) |
| C-128 | 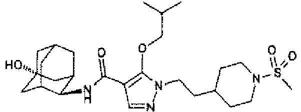 | (CDCl3);1.06(d,J=6.9Hz,6H),1.32-2.20(m,21H),2.59-2.67(m,2H),2.76(s,3H),3.76-3.81(m,2H),4.12(d,J=6.9Hz,2H)4.01-4.06(m,2H),4.13-4.18(m,1H),6.21(d,J=7.5Hz,1H),7.69(s,1H) |
| C-129 | 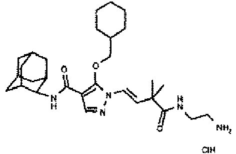 | (DMSO-d6);1.02-1.31(m,11H),1.31(s,6H),1.50-2.04(m,14H)2.83(t,J=6.6Hz,2H),3.25-3.30(m,2H),3,96-3.98(m,11H),4.12(d,J=6.0Hz,2H),6.36(d,J=14. 4Hz,1H),6.79(d,J=14.4Hz,1H),7.38(d,J=7.2H z,1H),7.78(t,J=6.6Hz,1H),7.84(bs,1H),7.97(s, 1H) |
| C-130 | 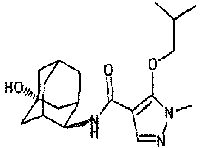 | (CDCl3);1.06(d,J=6.6Hz,6H),1.53-2.20(m,14H),3,72(s,3H),3.98(d,J=6.6Hz,2H), 6.25-6.30(m,1H),7.71(s,1H) |
| C-131 | 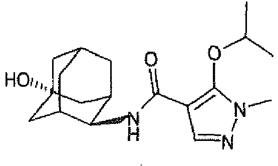 | (CDCl3);1.38(d,J=6.0Hz,6H),1.54-2.20(m,13H),3.71(s,3H),4.12-4,20(m,1H),4.69-4.78(m,1H),6.30-6.38(m,1H),7.75(s,1H) |
| C-132 | 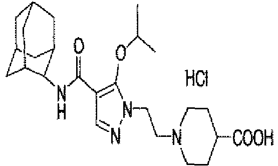 | (DMSO-d6); 1.27 (d, J = 6.3Hz, 6H), 1.51-2.03 (m, 19H), 2.99-3.32 (m, 6H), 3.97-3.99 (m, 1H), 4.34 (m, 2H), 4.93-5.02 (m, 1H), 7.29 (d, J = 6.9Hz, 1H), 7.93 (s, 1H), 10.49 (brs, 1H), 12.32 (brs, 1H) |

**[Table 35]**

| No. | Structure | NMR(CDCl3 or d6-DMSO) |
|---|---|---|
| C-133 | 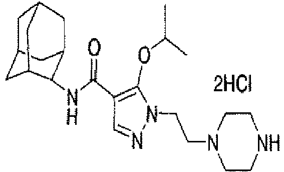 | (DMSO-d6); 1.28 (d, J = 6.3Hz, 6H), 1.51-2.04 (m, 14H), 3.37 (m, 10H), 3.97-3.99 (m, 1H), 4.35 (m, 2H), 4.93-5.01 (m, 1H), 7.31 (d, J = 7.2Hz, 1H), 7.95 (s, 1H), 9.49 (m, 3H) |
| C-134 | 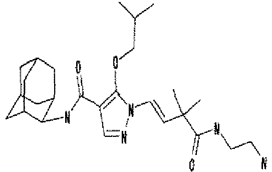 | (CDCl3); 1.05(d,J=6.6Hz,6H) 1.41(s.6H),1.65 -1.99(m, 14H),2.03-2.20(m, H),3.12-3.29(m,2H),3.54-3.70(m,2H),4.06(d,J=5.4Hz,2H),4.11 - 4.20(m,1H),6.52(d,J=13.8Hz, 1H),6.66-6.73(m,1H),6.84(d,J=13.8Hz, 1H), 7.50-7,64(m,1H),7.93(s, 1H),8.32(bs,2H) |
| C-135 | 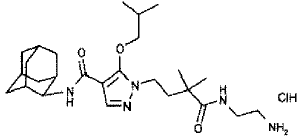 | (DMSO-d6);0.98(d,J=6.6Hz,6H),1.15(s,6H),1.50-2.04(m,17H),2.82-2.88(m,2H),3.26-3.33(m,2H),3.81-3.89(m,2H),3.95-3.98(m,1H),4.03(d,J=6.6Hz,2H),7.25(d,J=6.9 Hz,1H),7.81 (s,1H),7.81-7.87(m,1H),7.87(bs,2H) |
| C-136 | 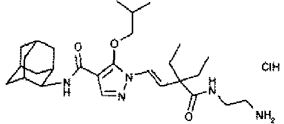 | (DMSO-d6);0.76(t,J=7.2Hz.6H),0.97(d,J=6.6Hz.6H),1 .50-2.04(m, 19H),2.78-2.90(m,2H),3.33-3.41(m,2H),3.96-4.03(m,1H),4.11 (d,J=6.6Hz,2H),6.31 (d,J=11. 7Hz,1H),6.83(d,J=11.7Hz,1H).7.44(d,J=6.9H z,1H), 7.86-7 9A(m 1H) 7.98(s, 1H) 8.07 07(bs 2H) |
| C-137 | 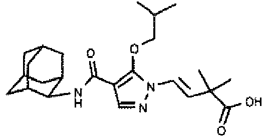 | (CDC3);1.06(d,J=6.6Hz.6H), 1.45(s,6H),1.67 -2.05(m,14H),2.08-2.17(m,1H),3.99(d,J=6,9Hz,2H),4.19-4.22(m, 1H),6.46(d,J=8,1Hz, H),6.54(d,J=14. 4Hz,1H),6.86(d,J=14.4Hz, 1H),7.84(s, 1H) |
| C-138 | 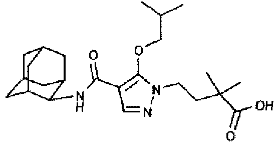 | (CDCl₃);1.05(d,J=6.6Hz,6H), 1.28(s, 6H),1.66 -1.99(m,16H),2.02-2.16(m,1H), 4.00(d,J=6.6Hz,2H), 4.04-4.08(m,2H),4.16-4.20(m, 1H),6.38(d,J=7.8Hz, 1H), 7.73(s,1H) |

**[Table 36]**

| No. | Structure | NMR(CDCl3 or d6-DMSO) |
|---|---|---|
| C-139 | 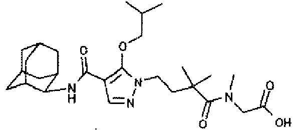 | (CDCl3):1.04(d,J=6.6Hz.6H),1.37(s.6H),1.66 -2.09(m,16H7,2.11-2.18(m, 1H),2.18(s,3H),3.98(d,J=6.6Hz,2H),4. 03-4.07(m,2H),4.17-4.22(m,1H),6.46(d,J=7.8Hz,1H),7.73(s,1H) |
| C-140 | 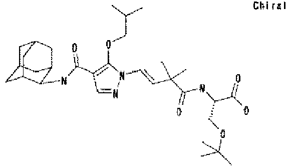 | (CDCl3),1.07(d,J=6.6Hz,6H),1.12(s,9H),1.42( s,3H),1.44(s,3H),1.67-2.10(m,14H), 2.10-2.19(m,1H),3.52-3.57(m,1 H),3.84-3.88(m,1H), 4.00(d,J=6.6Hz,2H), 4.18-4.22(m,1H),4.58-4.64(m, 1H),6.50(d,J=14.1Hz, 1H),6.49-6.52(m,1H),6.62(d,J=7.8Hz,1H),6.94(d, J=14. 1Hz 1H) 7.84 |
| C-141 | 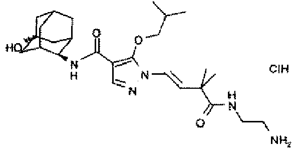 | (DMSO-d6): 0.97(d,J=6.4Hz,6H), 1.31(s,6H), 1.01-2.06(m,14H), 2.79-2.87(m,2H), 3.27-3.35(m,2H), 3.86-3.91 (m, 1H), 4.11 (d,J=5.6Hz,2H), 6.36(d,J=15.2Hz,1H), 6.81 (d,J=15.2Hz, 1H), 7.38(d,J=6.4Hz, 1H), 7.83-7,89(m, 1H), 7.98(s, 1H), 8.02-8.11 (br,2H) |
| C-142 | 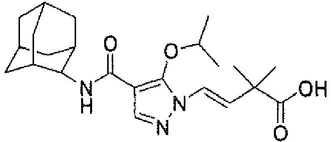 | (DMSO-d6): 1.25(d,J=6.0Hz, 6H), 1.27(s,6H), 1.51-2.04(m, 13H), 3.97-4.02(m,1H), 4.84-4.91 (m, 1H), 6.45(d,J=14.4Hz,1H), 6.76(d,J=14.4Hz, 1H), 7.35(d,J=6.9Hz, 1H), 8.01 (s, 1H) |
| C-143 | 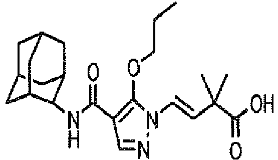 | (DMSO-d6) 0.96(t, J = 7.4 Hz, 3H), 1.32(s, 6H), 1.45-2.05(m, 16H), 3.97(m, 1H), 4.27(t, J = 6.4 Hz, 2H), 6.36(d, J = 14.4 Hz, 1H), 6.82(d, J = 14.4 Hz, 1H), 7.41 (d, J = 7.1 Hz, 1H), 7.98(s, 1H), 12.49(brs, 1H) |
| C-144 | 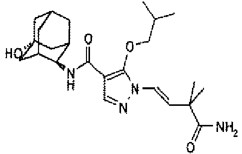 | (DMSO-d6); 0.98 (d, J = 6.8Hz, 6H), 1.27 (s, 6H), 1.34 (d, J = 12.4Hz, 2H), 1.61-2.04 (m, 12H), 3.89 (brs, 1H), 4.10 (d, J = 6.4Hz, 2H), 4.42 (s, 1H), 6.37 (d, J = 14.4Hz, 1H), 6.81 (d, J = 14.0Hz, 1H), 6.92 (s, 1H), 7.14 (s, 1H), 7.37 (d, J = 6.4Hz, 1H), |

**[Table 37]**

| No. | Structure | NMR(CDCl3 or d6-DMSO) |
|---|---|---|
| C-145 | 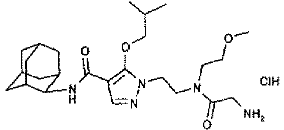 | (DMSO-d6);0.97(d,J=6.9Hz,6H),1.49-2.04(m, 15H),3.45(s,3H),3.35-4.15(m,13H), 7.32(d, J=6.9Hz, 1H), 7.86(s, 1H), 8.12(bs,2H) |
| C-146 | 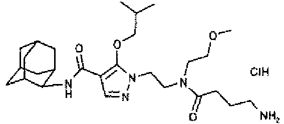 | (DMSO-d6); 1.03(d,J=6.9Hz, 6H), 1.57-2.11 (m, 15H), 2.30-2.51 (m, 2H), 2.71 - 2.90(m,2H),3.29(s,3H),3.57-4.20(m, 13H),7.37(d,J=7.2Hz, 1H), 787(bs,2H), 7.95(s, 1H) |
| C-147 | 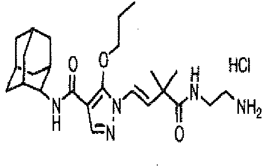 | (DMSO-d6) 0.97(t, J = 7.3 Hz, 3H), 1.31 (s, 6H), 1.45-2.05(m, 14H), 2.50(m, 2H), 2.82(m, 2H), 3.29(m, 2H), 3.99(m, 1H), 4.28(t, J = 6.5 Hz, 2H), 6.36(d, J = 14.3 Hz, 1H), 6.80(d, J = 14.3 Hz, 1H), 7.40(d, J = 6.9 Hz, 1H), 7.80(brs, 1H), 7.89(brs, 2H), 7.99 |
| C-148 | 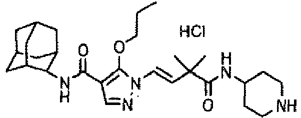 | (DMSO-d6) 0.96(t, J = 7.4 Hz, 3H), 1.29(s, 6H), 1.45-2.05(m, 20H), 2.80-3.00(m, 2H), 3.20-3.30(m, 2H), 3.81 (m, 1H), 3.97(m, 1H), 4.27(t, J = 6.5 Hz, 2H), 6.39(d, J = 14.3 Hz, 1H), 6.76(d, J = 14.3 Hz, 1H), 7.42(d, J = 7.0 Hz, 1H), 7.62(d, J = 7.8 Hz, 1H), |
| C-149 | 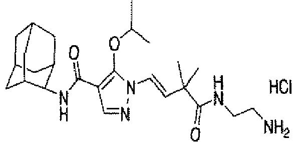 | (DMSO-d6): 1.04(d,J=6.2Hz,6H), 1.09(s,6H), 1.28-1.81(m, 15H), 2.55-2.64(m,2H), 3.04-3.10(m,2H), 3.73-3.77(m,1H), 4.64-4.73(m,1H), 6.15(d,J=14.4Hz,1H), 6.56(d,J=14.4Hz,1H), 7.12(d,J=6.9Hz,1H), 7.59(t,J=5.5Hz,1H), 7.71 (br,s,2H), 7.81 (s,1H) |
| C-150 | 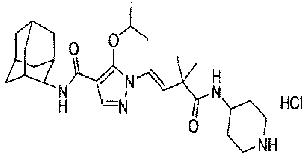 | (DMSO-d6): 1.12(d,J=6.0Hz,6H), 1.15(s,6H), 1.40-1.89(m,17H), 2.73-2.85(m,2H), 3.08-3.21 (m,2H), 3.66-3.70(m,1H), 3.82-3.85(m, 1H), 4.73-4.82(m,1H), 6.25(d,J=14.3Hz, 1H), 6.61(d,J=14.3Hz,1H), 7.22(d,J=6.9Hz,1H), 7.46(d,J=7.6Hz,1H), 7.88(s,1H), 8.63(br.s 2H) |

**[Table 38]**

| No. | Structure | NMR(CDCl3 or d6-DMSO) |
|---|---|---|
| C-151 | 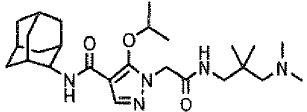 | (DMSO-d6) 0.80(s, 6H), 1.23(d, J = 6.2 Hz, 6H), 1.45-2.13(m, 16H), 2.16(s, 6H), 2.98(d, J = 5.9 Hz, 2H), 3.99(m, 1H), 4.61 (s, 2H), 4.95(m, 1H), 7,26(d, J = 6.7 Hz, 1H), 7.91 (s, 1H), 7.95(brs, 1H) |
| C-152 | 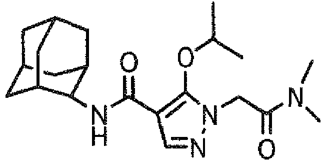 | (DMSO-d6) 1.22(d, J = 6.1 Hz, 6H), 1.45-2.10(m, 14H), 2.84(s, 3H), 3.05(s, 3H), 3.98(m, 1H), 4.87(s, 2H), 4.90(m, 1H), 7.26(d, J = 7.0 Hz, 1H), 7.85(s, 1H) |
| C-153 | 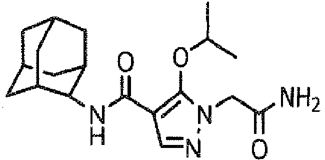 | (DMSO-d6) 1.23(d, J = 6.0 Hz, 6H), 1.45-2.05(m, 14H), 3.98(m, 1H), 4.53(s, 2H), 4.91 (sep, J = 6.1 Hz, 1H), 7.21 (brs, 1H), 7.24(brs, 1H), 7.47(brs, 1H), 7.86(s, 1H) |
| C-154 | 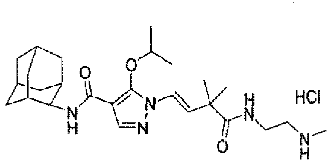 | (DMSO-d6): 1.23(d,J=6.2Hz,6H), 1.28(s,6H), 1.47-2.00 (m,13H), 2.50(t,J=6.0Hz,3H), 2.87-2.91(m,2H), 3.28-3.32(m,2H), 3.94-3.96(m,1H), 4.85-4.91 (m,1H), 6.34(d,J=14.4Hz,1H), 6.76(d, J=14.4Hz,1H), 7.33(d,J=7.0Hz,1H), 7.85(t,J=5.5Hz,1 H), 8.00(s,1H), 8.78(br,s, |
| C-155 | 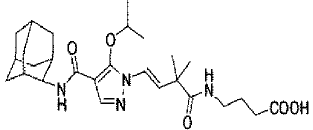 | (DMSO-d6): 1.27(d,J=6.3Hz,6H), 1.28(s,6H), 1.50-3.09(m,19H), 3.97-4.01 (m, 1H), 4.86-4.94(m, 1H), 6.35(d,J=14.3Hz,1H), 6.76(d,J=14.3Hz,1H), 7.36(d,J=6.9Hz,1H), 7.63(t,J=5.6,1H), 8.02 (s, 1H) |
| C-156 | 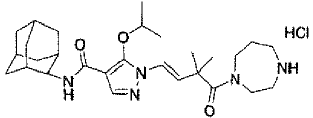 | (DMSO-d6): 1.07(d,J=6.0Hz,6H), 1.16(s,sH), 1.32-1.84(m,15H), 2.94-3.47(m,8H), 3.77-3.81 (m,1H), 4.70-4.74(m,1H), 6.34(d,J=14.4Hz,1H), 6.63(d,J=14.4Hz,1H), 7.17(d,J=6.9Hz,1H), 7.85(s,1H), 8.68(br,s,2H) |

**[Table 39]**

| No. | Structure | NMR(CDCl3 or d6-DMSO) |
|---|---|---|
| C-157 | 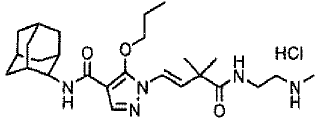 | (DMSO-d6) 0.97(t, J = 7.4 Hz, 3H), 1.32(s, 6H), 1.45-2.10 (m, 17H), 2.54(m, 2H), 2.85-3.00(m, 2H), 3.30-3.40(m, 2H), 3.96(m, 1H), 4.28(t, J = 6.5 Hz, 2H), 6.37(d, J = 14.1 Hz, 1H), 6.80(d, J = 14.1 Hz, 1H), 7.40(d, J = 6.7 Hz, 1H), 7.88(t, J = 5.3 Hz, 1H), 7.99(s, 1H), 8.76(brs, 1H) |
| C-158 | 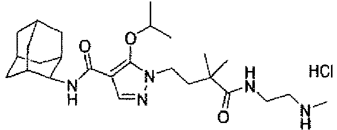 | (DMSO-d6): 1.08(s,6H), 1.16(d,J=6.2Hz,6H), 1.41-1.99(m,15H), 2.45-2.48(t,J=5.5Hz,3H), 2.84-2.88(m,2H), 3.26-3.31(m,2H), 3.72-3.77(m,2H), 3.88-3.89(m,1H), 4.76-4.82(m, 1H), 7.12(d,J=7.1 Hz, 1H), 7.76(s, 1H), 7.86(t,J=6.0Hz,1 H), 8.78(br,s,2H) |
| C-159 | 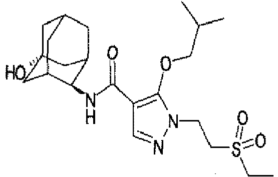 | (DMSO-d6); 0.98 (d, J = 6.8Hz, 6H), 1.19 (t, J = 7.2Hz, 3H), 1.34 (d, J = 12.0Hz, 2H), 1.58-1.72 (m, 6H), 1.88-2.07 (m, 6H), 3.06 (q, J = 7.2Hz, 2H), 3.60 (t, J = 6.8Hz, 2H), 3.88 (brs, 1H), 4.10 (d, J = 6.8Hz, 2H), 4.35 (t, J = 6.8Hz, 2H), 4.43 (s, 1H), 7.30 (d, J = 6.4Hz, 1H), 7.89 (s, 1H) |
| C-160 | 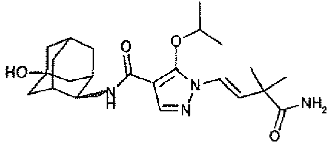 | (CDCl3);1.38(d,J=6.0Hz,6H),1.42(s,6H),1.54 -2.19(m,13H),4.13-4.20(m,1H),4.74(ddd,J=6.0Hz, 1H), 5.52(bs, 1 H), 5.81 (bs, 1H), 6.34(d, J=7.8Hz, 1H),6.49(d,J= 14.4Hz, 1H), 6.87(d, J=14.4Hz, 1H), 7.84(s, 1H) |
| C-161 | 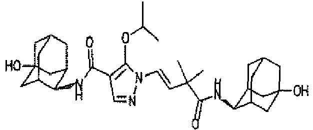 | (CDCl3); 1.39(d, J=6.0Hz, 6H), 1.42(s, 6H), 1.49 -2.20(m, 26H), 3.94-3.97(m, 1H),4.15-4.19(m, 1H), 4.76(ddd,J=6.0Hz,1H),5.94(d,J= 7.8Hz,1H),6.34(d,J=7.8Hz, 1H),6.50(d,J=14.1 Hz,1H),6.89(d,J=14.1 Hz,1H),7.85(s,1H) |
| C-162 | 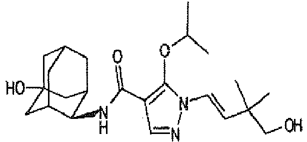 | (CDCl3); 1.15(s,6H), 1.39(d,J=6.0Hz,6H), 1.55 -2.20(m, 13H), 3.45(s,2H), 4.16-4.19(m, 1H), 4.72(ddd,J=6.0Hz, 1H),6.31(d,J= 14.4Hz, 1H),6.39(d,J=6,6Hz,1H),6.74(d,J=14. 4Hz, 1H), 7.85's, 1H) |

**[Table 40]**

| No. | Structure | NMR(CDCl3 or d6-DMSO) |
|---|---|---|
| C-163 | 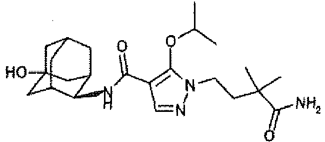 | (CDCl3);1.28(s,6H),1.37(d,J=6.0Hz,6H),1.53 -2.19(m,15H),4.00-4.06(m,2H),4.12-4.20(m,1H),4.78(ddd,J=6.0Hz.1H),5.40(bs,1 H),5.84(bs,1H),6.33(d,J=6.6Hz,1H),7.78(s,1H ) |
| C-164 | 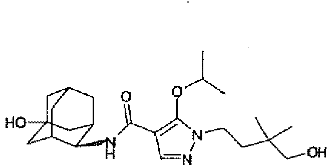 | (CDCl3);0.96(s,6H),1.38(d,J=6.3Hz,6H),1.54 -2.20(m,15H),3.34(s,2H),4.00-4.05(m,2H),4.15-4.18(m,1H),4.81(ddd,J=6.3Hz,1H),6.33(d,J= 7.8Hz,1H),7.78(s,1H) |
| C-165 | 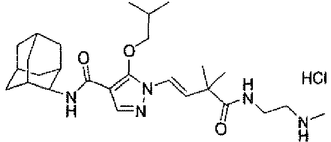 | (DMSO-d6): 1.04(d,J=6.0Hz,6H), 1.31(s,6H), 1.50-2.96(m,21H), 3.95-3.99(m,1H), 4.11 (d,J=6.4Hz,2H), 6.37(d,J=14.3Hz,1H), 6.82(d,J=1 4.3Hz,1H), 7.42(d,J=7.2Hz,1H), 7.86(t,J=5.3Hz,1H), 7.99(s,1H), 8.67(br,s,2H) |
| C-166 | 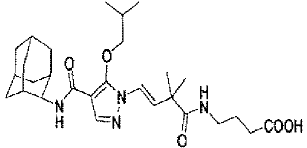 | (DMSO-d6): 0.97(d,J=6.6Hz,6H), 1.27(s,6H), 1.50-3.14(m,20H), 3.97-3.99(m,1H), 4.10(d,J=6.4Hz,2H), 6.35(d,J=14.3Hz,1H), 6.79(d,J=14.3Hz,1H), 7.41(d,J=6.9Hz,1H), 7.66(t,J=6.0Hz,1H), 7.97(s,1H) |
| C-167 | 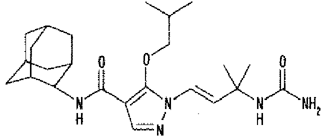 | (DMSO-d6); 0.98 (d, J = 6.4Hz, 6H), 1.38 (s, 6H), 1.52 (d, J = 12.4Hz, 2H), 1.70-2.04 (m, 13H), 3.98 (brs, 1H), 4.07 (d, J = 6.4Hz, 2H), 5.31 (s, 2H), 6.09 (s, 1H), 6.37 (d, J = 14.4Hz, 1H), 6.81 (d, J = 14.0Hz, 1H), 7.40 (d, J = 6.8Hz, 1H), 7.95 (s, 1H) |
| C-168 | 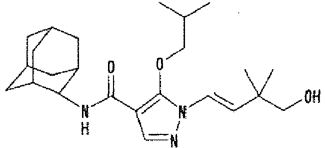 | (DMSO-d6); 0.98 (d, J = 6.8Hz, 6H), 1.03 (s, 6H), 1.52 (d, J = 12.4Hz, 2H), 1.71-2.04 (m, 13H), 3.23 (d, J = 5.2Hz, 2H), 3.98 (brs, 1H), 4.08 (d, J = 6.4Hz, 2H), 4.75 (t, J = 5.6Hz, 1H), 6.22 (d, J = 14.4Hz, 1H), 6.74 (d, J = 14.4Hz, 1H), 7.39 (d, J = 7.2Hz, 1H), 7.95 (s, 1H) |

**[Table 41]**

| No. | Structure | NMR(CDCl3 or d6-DMSO) |
|---|---|---|
| C-169 | 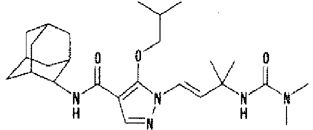 | (DMSO-d6); 0.97 (d, J = 6.8Hz, 6H), 1.42 (s, 6H), 1.52 (d, J = 12.0Hz, 2H), 1.70-2.08 (m, 13H), 2.78 (s, 6H), 3.98 (brs, 1H), 4.06 (d, J = 6.0H), 2H), 5.79 (s, 1H), 6.40 (d, J = 14.4Hz, 1H), 6.80 (d, J = 14.8Hz, 1H), 7.37 (d, J = 6.8Hz, 1H), 7.95 (s, 1H) |
| C-170 | 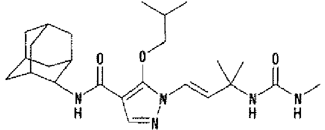 | (DMSO-d6); 0.97 (d, J = 6.8Hz, 6H), 1.38 (s, 6H), 1.52 (d, J = 12.8Hz, 2H), 1.71-2.04 (m, 13H), 2.50 (s, 3H), 3.99 (brs, 1H), 4.06 (d, J = 6.4Hz, 2H), 5.58-5.62 (m, 1H), 5.98 (s, 1H), 6.35 (d, J = 14.0Hz, 1H), 6.79 (d, J = 14.4Hz, 1H), 7.38 (d, J = 6.8Hz, 1H), 7.94 (s, 1H) |
| C-171 | 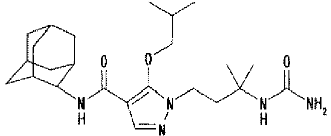 | (DMSO-d6); 0.98 (d, J = 6.4Hz, 6H), 1.20 (s, 6H), 1.52 (d, J = 11.6Hz, 2H), 1.70-2.11 (m, 15H), 3.88-3.93 (m, 2H), 3.98 (brs, 1H), 4.02 (d, J = 6.8Hz, 2H), 5.24 (s, 2H), 5.80 (s, 1H), 7.22 (d, J = 6.4Hz, 1H), 7.79 (s, 1H) |
| C-172 | 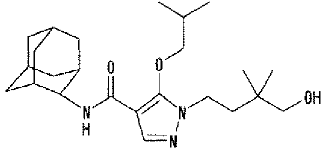 | (DMSO-d6); 0.85 (s, 6H), 0.98 (d, J = 6.4Hz, 6H), 1.52 (d, J = 12.4Hz, 2H), 1.59-2.05 (m, 15H), 3.13 (brs, 2H), 3.91-3.99 (m, 3H), 4.04 (d, J = 6.4Hz, 2H), 4.60 (brs, 1H), 7.23 (d, J = 6.4Hz, 1H), 7.79 (s, 1H) |
| C-173 | 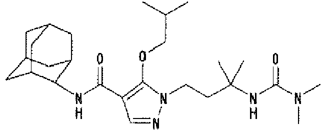 | (DMSO-d6); 0.97 (d, J = 6.8Hz, 6H), 1.26 (s, 6H), 1.53 (d, J = 12.8Hz, 2H), 1.70-2.04 (m, 13H), 2.13 (t, J = 7.6Hz, 2H), 2.76 (s, 6H), 3.89 (t, J = 7.6Hz, 2H), 3.98-4.02 (m, 3H), 5.41 (s, 1H), 7.21 (d, J = 6.4Hz, 1H), 7.80 (s, 1H) |
| C-174 | 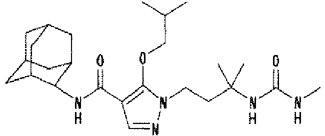 | (DMSO-d6); 0.98 (d, J = 6.4Hz, 6H), 1.20 (s, 6H), 1.53 (d, J = 12.4Hz, 2H), 1.70-2.04 (m, 13H), 2.09 (t, J = 8.0Hz, 2H), 2.51 (s, 3H), 3.90 (t, J = 7.6Hz, 2H), 3.96-4.02 (m, 3H), 5.51-5.57 (brm, 1H), 5.66 (s, 1H), 7.21 (d, J = 6.4Hz, 1H), 7.79 (s, 1H) |

**[Table 42]**

| No. | Structure | NMR(CDCl3 or d6-DMSO) |
|---|---|---|
| C-175 | 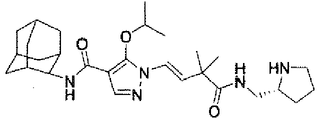 | (DMSO-d6): 1.26(d,J=6.0Hz,6H), 1.29(s,6H), 1.51-3.11(m,21H), 3.97-3.99(m,1H), 4.87-4.93(m,1H), 6.36(d,J=14.4Hz,1H), 6.79(J=14.4Hz,1H), 7.35(d,J=7.1Hz,1H), 7.44(t,J=5.5Hz,1 H), 8.0(s,1H) |
| C-176 | 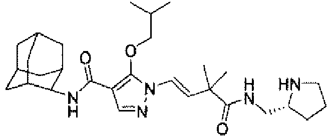 | (DMSO-d6): 097(d,J=6.7Hz,6H), 1.28(s,6H), 1.50-3.23(m,22H), 3.96-3.98(m,1H), 4.10(d,J=6.4Hz,2H), 6.35(d,J=14.3Hz,1H), 6.80(d,J=14.3Hz,1H), 7.41-7.45(m,2H), 7.97(s,1H) |
| C-177 | 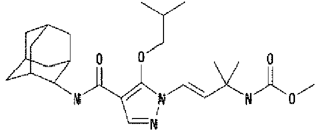 | (DMSO-d6); 0.97 (d, J = 6.8Hz, 6H), 1.39 (s, 6H), 1.52 (d, J = 13.2Hz, 2H), 1.70-2.04 (m, 13H), 3.49 (s, 3H), 3.97 (brs, 1H), 4.08 (d, J = 6.4Hz, 2H), 6.30 (d, J = 14.0Hz, 1H), 6.79 (d, J = 14.0Hz, 1H), 7.25 (s, 1H), 7.41 (d, J = 6.8Hz, 1H), 7.95 (s, 1H) |
| C-178 | 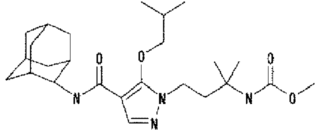 | (DMSO-d6); 0.97 (d, J = 6.8Hz, 6H), 1.22 (s, 6H), 1.52 (d, J = 12.0Hz, 2H), 1.70-2.08 (m, 15H), 3.49 (s, 3H), 3.89 (t, J = 8.0Hz, 2H), 3.97 (brs, 1H), 4.02 (d, J = 6.4Hz, 2H), 6.93 (s, 1H), 7.24 (d, J = 6.8Hz, 1H), 7.80 (s, 1H) |
| C-179 | 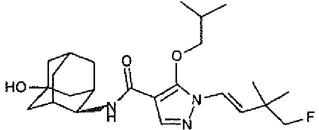 | (CDCl3);1.06(d,J=6.6Hz,6H),1.37(s,3H),1.44( s,3H),1.54-2.20(m,14H),2.45-2.54(m,2H),3.99(d,J=6.6Hz,2H),4.18-4.20(m,1H),6.30(d,J=14.4Hz,1H),6.25-6.35(m,1H),6.79(d,J=14.4Hz,1H),7.80(s,1H) |
| C-180 | 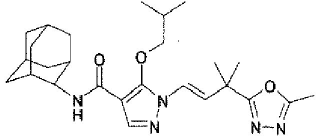 | (DMSO-d6); 0.94 (d, J = 6.8Hz, 6H), 1.50-1.56 (m, 8H), 1.70-2.04 (m, 13H), 2.48 (s, 3H), 3.97 (brs, 1H), 4.10 (d, J = 6.4Hz, 2H), 6.40 (d, J = 14.0Hz, 1H), 6.79 (d, J = 14.4Hz, 1H), 7.44 (d, J = 6.4Hz, 1H), 7.99 (s, 1H) |

**[Table 43]**

| No. | Structure | NMR(CDCl3 or d6-DMSO) |
|---|---|---|
| C-181 | 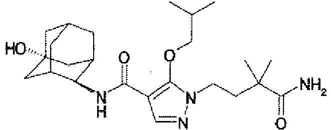 | (DMSO-d6); 0.97 (d, J = 6.8Hz, 6H), 1.12 (s, 6H), 1.34 (d, J = 12.0Hz, 2H), 1.61-2.03 (m, 14H), 3.84-3.90 (m, 3H), 4.02 (d, J = 6.4Hz, 2H), 4.41 (s, 1H), 6.87 (s, 1H), 7.14 (s, 1H), 7.20 (d, J = 6.4Hz, 1H), 7.79 (s, 1H) |
| C-182 | 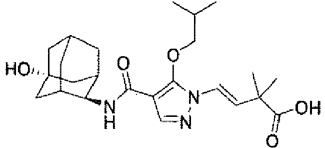 | (DMSO-d6): 0.97(d,J=6.7Hz,6H), 1.09(s,6H), 1.40-2.10(m,14H), 3.79(s,2H), 3.93-3.96(m,1H), 4.09(d,J=6.4Hz,2H), 6.22(br,d,J=14.4Hz,3H), 6.48(br,s,2H), 6.76(d,J=14.4Hz,1H), 7.43(d,J=6.7Hz, 1H), 8.00(s,1H) |
| C-183 | 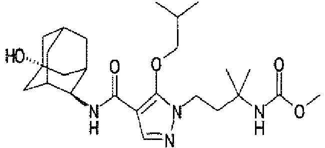 | (DMSO-d6); 0.97 (d, J = 6.8Hz, 6H), 1.22 (s, 6H), 1.34 (d, J = 11.6Hz, 2H), 1.61-2.06 (m, 14H), 3.48 (s, 3H), 3.88 (brs, 3H), 4.01 (d, J = 6.8Hz, 2H), 4.42 (s, 1H), 6.92 (s, 1H), 7.20 (d, J = 6.4Hz, 1H), 7.79 (s, 1H) |
| C-184 | 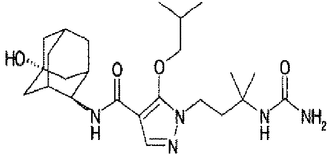 | (DMSO-d6); 0.98 (d, J = 6.4Hz, 6H), 1.20 (s, 6H), 1.34 (d, J = 12.4Hz, 2H), 1.61-2.11 (m, 14H), 3.89 (brs, 3H), 4.02 (d, J = 6.4Hz, 2H), 4.42 (s, 1H), 5.24 (s, 2H), 5.81 (s, 1H), 7.19 (d, J = 6.4Hz, 1H), 7.79 (s, 1H) |
| C-185 | 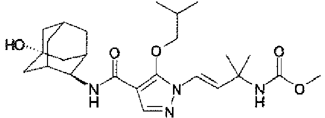 | (DMSO-d6); 0.97 (d, J = 6.8Hz, 6H), 1.34 (d, J = 13.2Hz, 2H), 1.38 (s, 6H), 1.61-2.04 (m, 12H), 3.49 (s, 3H), 3.90 (brs, 1H), 4.08 (d, J = 6.4Hz, 2H), 4.42 (s, 1H), 6.30 (d, J = 14.4Hz, 1H), 6.79 (d, J = 14.4Hz, 1H), 7.25 (s, 1H), 7.37 (d, J = 6.4Hz, 1H), 7.95 (s, 1H) |
| C-186 | 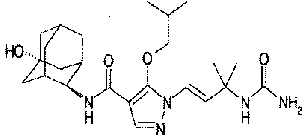 | (DMSO-d6); 0.98 (d, J = 6.8Hz, 6H), 1.32-1.41 (m, 8H), 1.61-2.04 (m, 12H), 3.88 (brs, 1H), 4.07 (d, J = 6.4Hz, 2H), 4.42 (s, 1H), 5.29 (s, 2H), 6.08 (s, 1H), 6.37 (d, J = 14.0Hz, 1H), 6.81 (d, J = 14.4Hz, 1H), 7.35 (d, J = 6.0Hz, 1H), 7.95 (s, 1H) |

**[Table 44]**

| No. | Structure | NMR(CDCl3 or d6-DMSO) |
|---|---|---|
| C-187 | 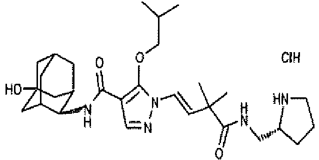 | (DMSO-d6);0.98(d,J=6.6Hz,6H),1.33(s,6H),1.18-2.03(m,18H),3.11-3.23(m,2H),3.12-3.50(m,3H),3.88-3.95(m,1H),4,12(d,J=6.3Hz,2H),6.36(d,J=14. 4Hz,1H),6.84(d,J=14.4Hz,1H),7.40(d,J=6.6H z,1H),8.00(s,1H),8.00-8.05(m 1H), 8 84(bs, 1H), 9.26(bs, 1H) |
| C-188 | 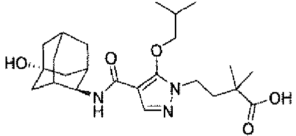 | (DMSO-d6): 097(d,J=6.7Hz,6H), 1.14(s,6H), 1.32-2.01 (m,14H), 3.86-3.99(m,3H), 4.03(d,J=6.5Hz,2H), 4.41 (s,1H), 7.22(d,J=6.7Hz,1H), 7.80(s,1H) |
| C-189 | 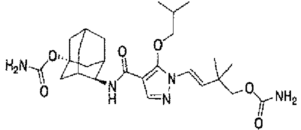 | (DMSO-d6): 0.97(d,J=6.7Hz,6H), 1.09(s,6H), 1.40-2.01 (m,14H), 3.79(s, 2H), 3.93-3.96(m,1H), 4.09(d,J=6.4Hz,2H), 6.22(br,d,J=14.4Hz,3H), 6.48(br,s,2H), 6.76(d,J=14.4Hz,1H), 7.43(d,J=6.7Hz,1H), 8.00(s,1H) |
| C-190 | 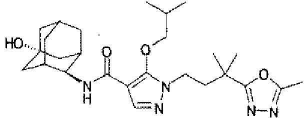 | (DMSO-d6); 0.94 (d, J = 6.8Hz, 6H), 1.31-1.37 (m, 8H), 1.61-2.02 (m, 12H), 2.11 (t, J = 7.6Hz, 2H), 2.42 (s, 3H), 3.89-3.95 (m, 3H), 4.01 (d, J = 6.4Hz, 2H), 4.42 (s, 1H), 7.18 (d, J = 7.2Hz, 1H), 7.78 (s, 1H) |
| C-191 | 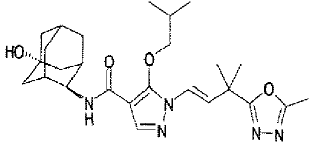 | (DMSO-d6); 0.94 (d, J = 6.8Hz, 6H), 1.34 (d, J = 12.8Hz, 2H), 1.55 (s, 6H), 1.61-2.04 (m, 12H), 2.47 (s, 3H), 3.89 (brs, 1H), 4.10 (d, J = 6.0Hz, 2H), 4.42 (s, 1H), 6.40 (d, J = 14.0Hz, 1H), 6.79 (d, J = 14.4Hz, 1H), 7.41 (d, J = 6.4Hz, 1H), 7.99 (s, 1H) |
| C-192 | 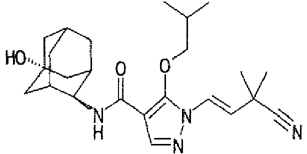 | (DMSO-d6); 0.98 (d, J = 6.8Hz, 6H), 1.34 (d, J = 11.6Hz, 2H), 1.52 (s, 6H), 1.61-2.04 (m, 12H), 3.89 (brs, 1H), 4.15 (d, J = 6.4Hz, 2H), 4.42 (s, 1H), 6.24 (d, J = 14.0Hz, 1H), 7.10 (d, J = 14.4Hz, 1H), 7.46 (d, J = 6.8Hz, 1H), 8.02 (s, 1H) |

**[Table 45]**

| No. | Structure | NMR(CDCl3 or d6-DMSO) |
|---|---|---|
| C-193 | 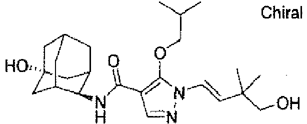 | (DMSO-d6): 0.97(d,J=6.6Hz,6H), 1.02(s,6H), 1.25-2.02(m,14H), 3.22(d,J=5.5Hz,2H), 3.84-3.90(m, 1H), 4.08(d,J=6.6Hz,2H), 4.43(s,1H), -4.74(t,J=5.3Hz,1H), 6.21(d,J=14.3Hz,1H), 6.74(d,J=14.3Hz,1H), 7.36(d,J=6.9Hz,1H), 7.95(s,1H) |
| C-194 | 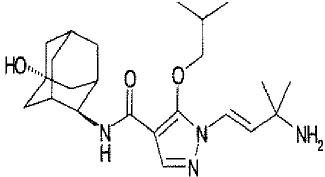 | (DMSO-d6); 0.98 (d, J = 6.8Hz, 6H), 1.20 (s, 6H), 1.34 (d, J = 12.4Hz, 2H), 1.61-2.04 (m, 12H), 3.88 (brs, 1H), 4.08 (d, J = 6.4Hz, 2H), 4.42 (brs, 1H), 6.30 (d, J = 14.0Hz, 1H), 6.91 (d, J = 14.0Hz, 1H), 7.34 (d, J = 6.8Hz, 1H), 7.93 (s, 1H) |
| C-195 | 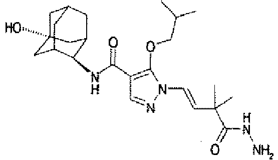 | (DMSO-d6); 0.98 (d, J = 6.8Hz, 6H), 1.29 (s, 6H), 1.34 (d, J = 12.4Hz, 2H), 1.61-2.04 (m, 12H), 3.88 (brs, 1H), 4.10 (d, J = 6.4Hz, 2H), 4.20 (brs, 2H), 4.42 (brs, 1H), 6.33 (d, J = 14.4Hz, 1H), 6.79 (d, J = 14.4Hz, 1H), 7.36 (d, J = 6.4Hz, 1H), 7.97 (s, 1H), 8.90 (s, 1H) |
| C-196 | 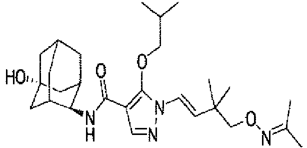 | (CDCl3-d1): 1.07(d,J=6.9Hz,6H), 1.17(s,6H), 1.56-2.20(m,20H), 3.92(s,2H), 3.96(d,J=6.5Hz,2H), 4.15-4.19(m,1H), 6.34(d,J=8,4,1H), 6.38(d,J=14.3Hz,1H), 6.76(d,J=14.3Hz,1H), 7.80(s,1H) |
| C-197 | 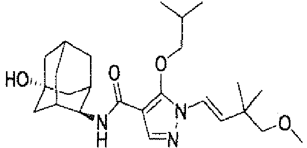 | (CDCl3-d1): 1.08(d,J=6.7Hz,6H), 1.14(s,6H), 1.54-2.20(m,14H), 3.21(s,2H), 3.35(s,3H), 3.97(d,J=6.5Hz,2H), 4.15-4.20(m,1H), 6.35(d,J=14.3Hz,1H), 6.33(d,J=6.0,1H), 6.76(d,J=14.3Hz, 1H), 7.81(s,1H) |
| C-198 | 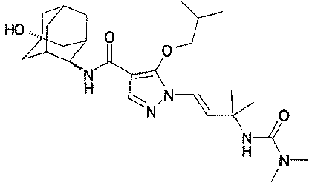 | (DMSO-d6); 0.97 (d, J = 6.4Hz, 6H), 1.34 (d, 14.0Hz, 2H), 1.42 (s, 6H), 1.61-2.05 (m, 12H), 2.77 (s, 6H), 3.89 (brs, 1H), 4.06 (d, J = 6.4Hz, 2H), 4.43 (s, 1H), 5.79 (s, 1H), 6.39 (d, J = 14.4Hz, 1H), 6.76 (d, J = 14.4Hz, 1H), 7.34 (d, J = 6.4Hz, 1H), 7.94 (s, 1H) |

**[Table 46]**

| No. | Structure | NMR(CDCl3 or d6-DMSO) |
|---|---|---|
| C-199 | 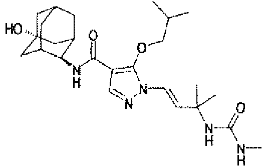 | (DMSO-d6); 0.97 (d, J = 6.8Hz, 6H), 1.32-1.38 (m, 8H), 1.61-2.05 (m, 12H), 2.50 (s, 3H), 3.88 (brs, 1H), 4.06 (d, J = 6.4Hz, 2H), 4.42 (s, 1H), 5.56-5.61 (brm, 1H), 5.97 (s, 1H), 6.35 (d, J = 14.4Hz, 1H), 6.79 (d, J = 14.4Hz, 1H), 7.35 (d, J = 6.8Hz, 1H), 7.94 (s, 1H) |
| C-200 | 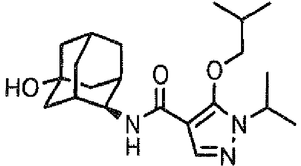 | (DMSO-d6) 0.95(d, J= 6.7 Hz, 6H), 1.32(d, J = 6.6 Hz, 6H), 1.55-2.05(m, 14H), 3.85(brs, 1H), 4.01 (d, J = 6.7 Hz, 2H), 4.43(s, 1H), 4.48(sep, J = 6.7 Hz, 1H), 7.25(d, J = 6.7 Hz, 1H), 7.77(s, 1H) |
| C-201 | 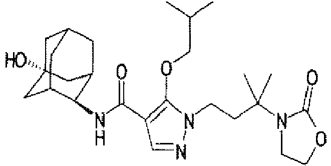 | (DMSO-d6); 0.98 (d, J = 6.8Hz, 6H), 1.30-1.36 (m, 8H), 1.61-2.03 (m, 12H), 2.19 (t, J = 8.0Hz, 2H), 3.60 (t, J = 7.6Hz, 2H), 3.88 (brs, 1H), 3.95 (t, J = 8.4Hz, 2H), 4.03 (d, J = 6.4Hz, 2H), 4.16 (t, J = 8.0Hz, 2H), 4.40 (s, 1H), 7.21 (d, J = 6.4Hz, 1H), 7.81 (s, 1H) |
| C-202 | 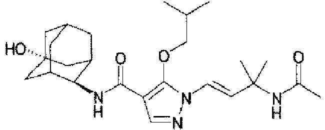 | (DMSO-d6); 0.97 (d, J = 6.8Hz, 6H), 1.34 (d, 11.6Hz, 2H), 1.40 (s, 6H), 1.61-2.04 (m, 15H), 3.89 (brs, 1H), 4.07 (d, J = 6.0Hz, 2H), 4.41 (s, 1H), 6.36 (d, J = 14.4Hz, 1H), 6.77 (d, J = 14.4Hz, 1H), 7.36 (d, J = 6.0Hz, 1H), 7.77 (s, 1H), 7.95 (s, 1H) |
| C-203 | 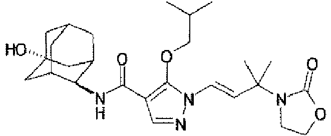 | (DMSO-d6); 0.97 (d, J = 6.4Hz, 6H), 1.34 (d, 13.2Hz, 2H), 1.51 (s, 6H), 1.61-2.04 (m, 12H), 3.61 (t, J = 8.0Hz, 2H), 3.88 (brs, 1H), 4.07 (d, J = 6.0Hz, 2H), 4.18 (t, J = 7.6Hz, 2H), 4.41 (s, 1H), 6.37 (d, J = 14.8Hz, 1H), 6.87 (d, J = 14.0Hz, 1H), 7.39 (d, J = 6.8Hz, 1H), 7.98 (s, 1H) |
| C-204 | 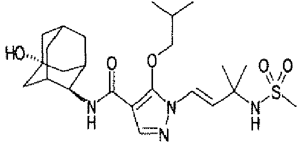 | (DMSO-d6); 0.97 (d, J = 6.8Hz, 6H), 1.34 (d, 12.0Hz, 2H), 1.44 (s, 6H), 1.61-2.04 (m, 12H), 2.90 (s, 3H), 3.89 (brs, 1H), 4.10 (d, J = 6.4Hz, 2H), 4.41 (s, 1H), 6.33 (d, J = 14.4Hz, 1H), 6.92 (d, J = 13.2Hz, 1H), 7.22 (s, 1H), 7.41 (d, J = 6.0Hz, 1H), 7.96 (s, 1H) |

**[Table 47]**

| No. | Structure | NMR(CDCl3 or d6-DMSO) |
|---|---|---|
| C-205 | 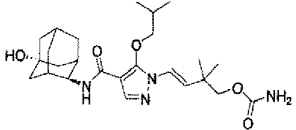 | (CDCl3-d1): 1.08(d,J=6.7Hz,6H), 1.17(s,6H), 1.59-2.20(m,14H), 3.95(s,2H), 3.99(d,J=6.5Hz,2H), 4.17-4.19(m,1H), 4.62(br,s,2H), 6.33(d,J=5.4Hz, H), 6.36(d,J=14.4Hz,1H), 6.76(d,J=14.4Hz,1H), 7.80(s,1H) |
| C-206 | 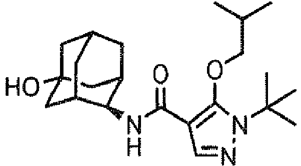 | (DMSO-d6) 0.80(d, J = 6.5 Hz, 6H), 1.20-1,50(m, 2H), 1.35(s, 9H), 1.40-1.90(m, 12H), 3.69(brs, 1H), 3.88(d, J = 6.5 H, 2H), 4.26(s, 1H), 7.20(d, J = 6.0 Hz, 1H), 7.49(s, 1H) |
| C-207 | 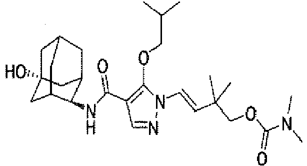 | (ODCl3-d1): 1.07(d,J=6.5Hz,6H), 1.18(s,6H),1.55-2.20(m,14H), 2.91(s,6H), 3.94(s,2H), 3.98(d,J=6.7Hz,2H), 4.14-4.19(m,1H), 6.31(d,J=7.7Hz,1H), 6.36(d,J=14.3Hz,1H), 6.76(d,J=14.3Hz,1H), 7.79(s,1H) |
| C-208 | 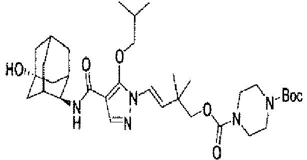 | (CDCl3-d1): 1.08(d,J=6.6Hz,6H), 1.18(s,6H), 1.46(s,9H), 1.57-2.21(m,14H), 3.40-3.44(m,8H), 3.97(s,2H), 4.00(d,J=6.0Hz,2H), 4.13-4.21 (m,1H), 6.30(d,J=8.2Hz,1H), 6.36(d,J=14.3Hz,1H), 6.76(d,J=14.3Hz,1H), 7.80(s,1H) |
| C-209 | 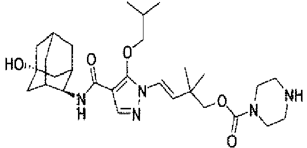 | (CDCl3-d1): 1.07(d,J=6.7Hz,6H), 1.18(s,6H), 1.54-3.67(m,23H), 3.98(s,2H), 4.00(d,J=6.0Hz,2H), 4.14-4.19(m,1H), 6.23(d,J=8.2Hz,1H), 6.33(d,J=14.4Hz,1H), 6.75(d,J=14.4Hz,1H), 7.79(s,1H) |
| C210 | 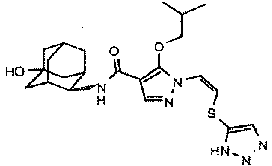 | (CDCl3);1.06(d,J=6.6Hz,6H),1.55-2.22(m,14H),4.03(d,J=6.6Hz,2H),4.17-4.20(m,1H),6.34(d,J=7.2Hz,1H),6.40(d,J=8.4 Hz,1H).6.89(d,J=8.4Hz,1H),7.72(s,1H),7.95(s ,1H) |

**[Table 48]**

| No. | Structure | NMR(CDCl3 or d6-DMSO) |
|---|---|---|
| C-211 | 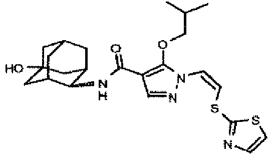 | (CDCl3);1.07(d,J=6.6Hz,6H),1.58-2.21 (m,14H),4.03(d,J=6.6Hz,2H),4.18-4.23(m,1H),6.32(d,J=7.5Hz, 1H),6.97(s,2H),7. 30(d,J=3.3Hz,1H),7.74(d,J=3.3Hz,1H),7.95(s ,1H) |
| C-212 | 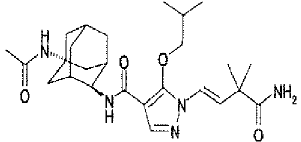 | (DMSO-d6); 0.98(d, J = 6.8Hz, 6H), 1.27(s, 6H), 1.42(d, J = 12.4Hz, 2H), 1.75(s, 3H), 1.93-2.03(m, 12H), 3.93(brs, 1H), 4.10(d, J = 6.4Hz, 2H), 6.37(d, J = 14.4Hz, 1H), 6.81 (d, J = 14.0Hz, 1H), 6.92 (brs, 1H), 7.14(brs, 1H), 7.35(s, 1H), 7.41(d, J = 6.4Hz |
| C-213 | 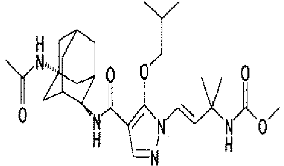 | (DMSO-d6); 0.97(d, J = 6.8Hz, 6H), 1.38-1.43(m, 8H), 1.75(s, 3H), 1.93-2.02(m, 12H), 3.49(s, 3H), 3.92-3.94(brm, 1H), 4.08(d, J = 6.4Hz, 2H), 6.30(d, J = 14.4Hz, 1H), 6.79(d, J = 14.0Hz, 1H), 7.25(brs, 1H), 7.35(s, 1H), 7.42(d, J 6.8Hz, 1H), 7.96(s, 1H) |
| C-214 | 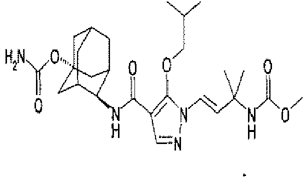 | (DMSO-d6); 0.97(d, J = 6.8Hz, 6H), 1.38-1.44(m, 8H), 1.93-2.12(m, 12H), 3.49(s, 3H), 3.94-3.96(brm, 1H), 7.44(d, J = 6.8Hz, 2H), 6.19(brs, 2H), 6.30(d, J = 14.0Hz, 1H), 6.79(d, J = 14.4Hz, 1H), 7.25(s, 1H), 7.44(d, 6.8Hz, 1H), 7.96(s, 1H) |
| C-215 | 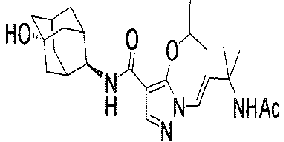 | (DMSO-d6); 1.24 (d, J = 6.1Hz, 6H), 1.25-1.35 (m, 2H), 1.36 (s, 6H) 1.60-1.75 (m, 8H), 1.80-1.94 (m, 4H), 2.03 (s, 3H), 3.87 (brs, 1H), 4.43 (s, 1H), 4.85 (hept, J = 6.1Hz, 1H), 6.35 (d, J = 14.3Hz, 1H), 6.75 (d, J = 14.3Hz, 1H), 7.29 (d, J = 6.6Hz, 1H), |
| C-216 | 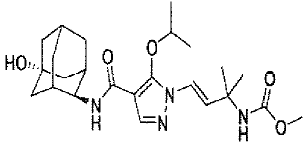 | (CDCl3); 1.38 (d, J=6.1Hz, 6H), 1.51 (s, 6H), 1.54-2.20 (m, 14H), 3.62 (s, 3H), 4.15-4.19 (m, 1H), 4.66-4.75 (m, 1H), 4.83 (s, 1H), 6.36 (d, J=14.2Hz, 1H), 6.44 (d, J=7.3Hz, 1H), 6.82 (d, J=14.2Hz, 1H), 7.85 (s, 1H) |

**[Table 49]**

| No. | Structure | NMR(CDCl3 or d6-DMSO) |
|---|---|---|
| C-217 | 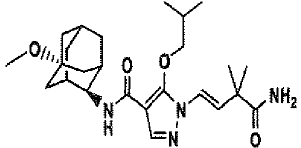 | (DMSO-d6); 0.98(d, J = 6.7Hz, 6H), 1.28(s, 6H), 1.30-1.42(m, 2H), 1.62-2.18(m, 12H), 3.12(s, 3H), 3.90(m, 1H), 4.10(d, J = 6.4Hz, 2H), 6.36(d, J = 14.3Hz, 1H), 6.80(d, J = 14.3Hz, 1H), 6.96(brs, 1H), 7.16(brs, 1H), 7.43(d, J = 6.6Hz, 1H), 7.99(s, 1H) |
| C-218 | 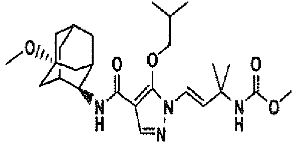 | (DMSO-d6); 0.97(d, J = 6.7Hz, 6H), 1.35-1.37(m, 2H), 1.38(s, 6H), 1.65-2.17(m, 12H), 3.11(s, 3H), 3.49(s, 3H), 3.90(m, 1H), 4.08(d, J = 6.4Hz, 2H), 6.29(d, J = 14.3Hz, 1H), 6.79(d. J = 14.3Hz, 1H), 7.26(brs, 1H), 7.42(d, J = 6.7Hz, 1H), 7.96(s, 1H) |
| C-219 | 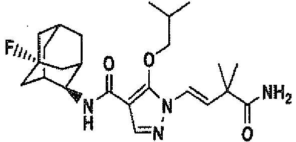 | (DMSO-d6); 0.97(d, J = 6.9Hz, 6H), 1.27(s, 6H), 1.35-1.50(m, 2H), 1.80-2.22(m, 12H), 3.95(m, 1H), 4.10(d, J = 6.3Hz, 2H), 6.37(d, J = 14.3Hz, 1H), 6.80(d, J = 14.3Hz, 1H), 6.95(brs, 1H), 7.16(brs, 1H), 7.47(d, J = 6.3Hz, 1H), 7.99(s, 1H) |
| C-220 | 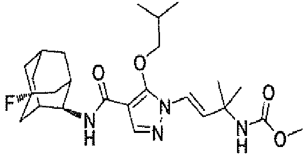 | (CDCl3); 1.08(d,J=6.9Hz,6H), 1.51 (s,6H), 1.53-2.32(m,14H), 3.62(s,3H), 3.99(d,J=6.6Hz,3H), 4.19(m,1H), 4.83(br.s,1H), 6.34(d,J=7.5Hz), 6.38(d,J=14.1Hz,1H), 6.87(d,J=14.1Hz,1H), 7.82(s,1H) |
| C-221 | 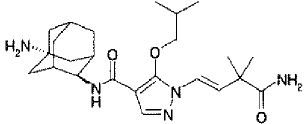 | (DMSO-d6); 0.98(d, J = 6.8Hz, 6H), 1.27(s, 6H), 1.34(d, J = 12.0Hz, 2H), 1.52-1.63(m, 6H), 1.90-1.99(m, 6H), 3.88(brs, 1H), 4.10(d, J = 6.0Hz, 2H), 6.37(d, J = 14.4Hz, 1H), 6.81(d, J = 14.4Hz, 1H), 6.93(brs, 1H), 7.14(brs, 1H), 7.36(d, J = 6.8Hz, 1H), 7.9 |
| C-222 | 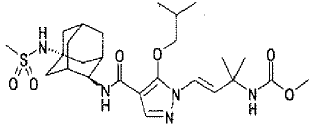 | (DMSO-d6); 0.97(d, J = 6.4Hz, 6H), 1.38-1.42(m, 8H), 1.89-2.05(m, 12H), 2.92(s, 3H), 3.49(s, 3H), 3.91(brs, 1H), 4.08(d, J = 6.4Hz, 2H), 6.30(d, J = 14.4Hz, 1H), 6.79(d, J = 14.0Hz, 1 H), 6.87(s, 1H), 7.25(s, 1H), 7.44(d, J = 6.0Hz, 1H), 7.96(s, 1H) |

**[Table50]**

| No. | Structure | NMR(CDCl3 or d6-DMSO) |
|---|---|---|
| C-223 | 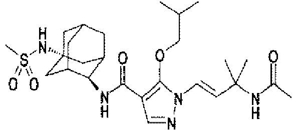 | (DMSO-d6); 0.97(d, J = 6.8Hz, 6H), 1.40(s, 8H), 1.79(s, 3H), 1.90-2.06(m, 12H), 2.95(s, 3H), 3.91(brs, 1H), 4.08(d, J = 6.4Hz, 2H), 6.36(d, J = 14.4Hz, 1H), 6.78(d, J = 14.4Hz, 1H), 6.87(s, 1H), 7.43(d, J = 6.8Hz, 1H), 7.77(s, 1H), 7.96(s, 1H) |
| C-224 | 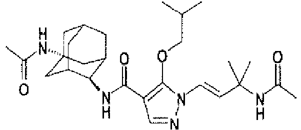 | (DMSO-d6); 0.97(d, J = 6.8Hz, 6H), 1.40-1.44(m, 8H), 1.75(s, 3H), 1.79(s, 3H), 1.93-2.09(m, 12H), 3.93(brs, 1H), 4.08(d, J = 6.0Hz, 2H), 6.36(d, J = 14.4Hz, 1H), 6.78(d, J = 14.4Hz, 1H), 7.36(s, 1H), 7.40(d, J = 6.4Hz, 1H), 7.77(s, 1H), 7.96(s, 1H) |
| C-225 | 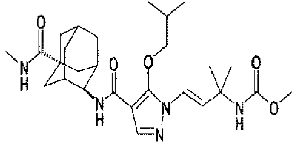 | (DMSO-d6); 0.97(d, J = 6.4Hz, 6H), 1.29(s, 6H), 1.44(d, J = 12.4Hz, 2H), 1.75-2.02(m, 12H), 2.56(d, J = 4.0Hz, 3H), 3.49(s, 3H), 3.93(brs, 1H), 4.08(d, J = 6.0Hz, 2H), 6.30(d, J = 14.0Hz, 1H), 6.79(d, J = 14.4Hz, 1H), 7.25(s, 1H), 7.38(d, J = 4.4Hz, 1H), |
| C-226 | 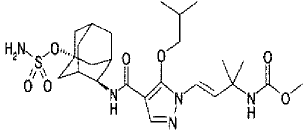 | (DMSO-d6); 0.97(d, J = 6.8Hz, 6H), 1.38(s, 6H), 1.43(d, J = 13.2Hz, 2H), 1.93-2.20(m, 12H), 3.49(s, 3H), 3.95(brs, 1H), 4.09(d, J = 6.4Hz, 2H), 6.30(d, J = 14.4Hz, 1H), 6.79(d, J = 14.4Hz, 1 H), 7.25(s, 1H), 7.35(s, 2H), 7.48(d, J = 6.0Hz, 1H), 7.96(s, 1H) |
| C-227 | 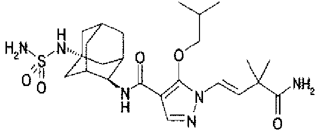 | (DMSO-d6); 0.98(d, J = 6.4Hz, 6H), 1.27(s, 6H), 1.39(d, J = 11.7Hz, 2H), 1.90-2.03(m, 12H), 3.92(brs, 1H), 4.10(d, J = 6.4Hz, 2H), 6.35-6.39(m, 2H), 6.46(s, 2H), 6.81(d, J = 14.4Hz, 1H), 6.92(s, 1H), 7.14(s, 1H), 7.14(d, J = 7.2Hz, 1H), 7.97(s, 1H) |
| C-228 | 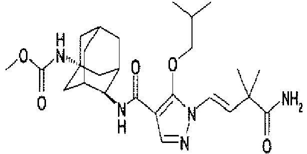 | (DMSO-d6); 0.98(d, J = 6.8Hz, 6H), 1.27(s, 6H), 1.40(d, J = 11.6Hz, 2H), 1.86-2.10(m, 12H), 3.47(s, 3H), 3.93(brs, 1H), 4.10(d, J = 6.4Hz, 2H), 6.37(d, J = 14.4Hz, 1H), 6.81(d, J = 14.4Hz, 1H), 6.84(s, 1H), 6.92(s, 1H), 7.14(s, 1H), 7.42(d, J = 6.4Hz, 1H) |

**[Table51]**

| **No.** | **Structure** | **NMR(CDCl3 or d6-DMSO)** |
|---|---|---|
| C-229 | 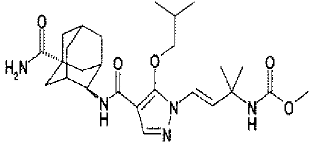 | (DMSO-d6); 0.97(d, J = 6,4Hz, 6H), 1.39(s, 6H), 1.44(d, J = 12.0Hz, 2H), 1.76-2.08(m, 12H), 3.49(s, 3H), 3.93(brs, 1H), 4.08(d, J = 6.4Hz, 2H), 6.30(d, J = 14.4Hz, 1H), 6.71 (s, 1H), 6.79(d, J = 14.4Hz, 1H), 7.00(s, 1H), 7.25(s, 1H), 7.43(d, J = 7.2Hz, 1H) |
| C-230 | 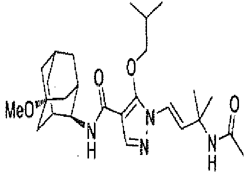 | (CDCl3); 1.09(d,J=6.6Hz,6H), 1.54(s,6H), 1.57-2.26(m,14H), 1.95(s,3H), 3.25(s,3H), 3.96(d,J=6.9Hz,2H), 4.16(m,1H), 5.47(br.s,1H), 6.39(d,J=14.1Hz,1H), 6.46(d,J=7.5Hz,1H), 6.87(d,J=14.1Hz,1H), 7.82(s,1H) |
| C-231 | 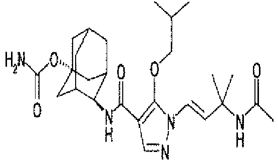 | (DMSO-d6); 0.97(d, J = 6.8Hz, 6H), 1.40-1.44(m, 8H), 1.79(s, 3H), 1.93-2.12(m, 12H), 3.95(brs, 1 H), 4.08(d, J = 6.4Hz, 2H), 6.20(brs, 2H), 6.36(d, J = 14.0Hz, 1H), 6.77(d, J = 14.4Hz, 1H), 7.43(d, J = 6.4Hz, 1H), 7.77(s, 1H), 7.96(s, 1H) |
| C-232 | 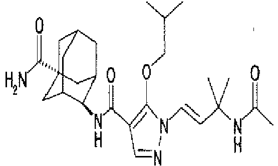 | (DMSO-d6); 0.98(d, J = 6.8Hz, 6H), 1.40-1.46(m, 8H), 1.75-2.02(m, 15H), 3.93(brs, 1H), 4.08(d, J = 6.4Hz, 2H), 6.36(d, J = 14.4Hz, 1H), 6.71 (s, 1H), 6.77(d, J = 14.0Hz, 1H), 6.99(s, 1H), 7.42(d, J = 6.4Hz, 1H), 7.77(s, 1H), 7,96(s, 1H) |
| C-233 | 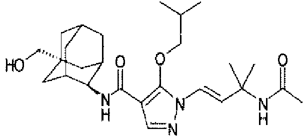 | (DMSO-d6); 0.98(d, J = 6.8Hz, 6H), 1.40-1.51(m, 14H), 1.78(s, 3H), 1.87-2.04(m, 6H), 3.00(d, J = 5.6Hz, 2H), 3.87(brs, 1H), 4.08(d, J = 6.4Hz, 2H), 4.38(t, J = 5.2Hz, 1 H), 6.36(d, J = 14.4Hz, 1H), 6.77(d, J = 14.4Hz, 1H), 7.40(d, J = 6.4Hz, 1H), 7.79(s, 1 |
| C-234 | 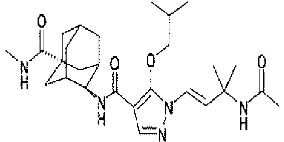 | (DMSO-d6); 0.97(d, J = 6.8Hz, 6H), 1.40-1.45(m, 8H), 1.74-2.03(m, 12H), 2.56(d, J = 4.0Hz, 3H), 3.92(brs, 1H), 4.08(d, J = 6.4Hz, 2H), 6.35(d, J=14.4Hz, 1H), 6.77(d, J = 14.4Hz, 1H), 7.39(d, J = 4.4Hz, 1H), 7.44(d, J = 6.4Hz, 1H), 7.79(s, 1H), 7.96(s, 1 |

**[Table 52]**

| No. | Structure | NMR(CDCl3 or d6-DMSO) |
|---|---|---|
| C-235 | 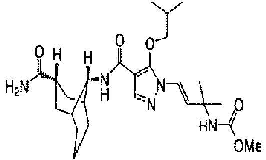 | (DMSO-d6); 0.96(s, 3H), 0.99(s, 3H), 1.15-1.20(m, 2H), 1.30-1.32(m, 1H), 1.38(s, 6H), 1.53-1.60(m, 2H), 1.74-1.78(m, 3H), 1.96-2.06(m, 5H), 2.52-2.61(m, 1H), 3.49(s, 3H), 4.03-4.08 (m, 1H), 4.07(d, 2H, J=6.0Hz), 6.30(d, J=14.1Hz, 1H), 6.73(br s, 1H), 6.78 |
| C-236 | 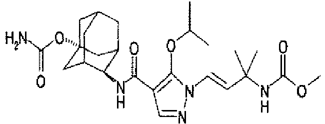 | (DMSO-d6); 1.25(d, J= 6.0Hz, 6H), 1.38-1.44(m, 8H), 1.92-2.10(m, 11H), 3.49(s, 3H), 3.95(brs, 1H), 4.82-4.90(m, 1H), 6.20(brs, 2H), 6.31(d, J=14.0Hz, 1H), 6.78(d, J = 14.4Hz, 1H), 7.27(s, 1H), 7.38(d, J = 6.0Hz, 1H), 8.02(s, 1H) |

**[Table53]**

| No. | Structure | retention time | LC-MS |
|---|---|---|---|
| D-1 | 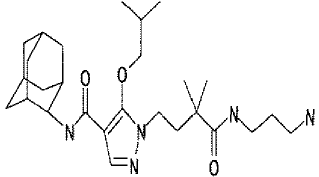 | 1.48 | 489.4 |
| D-2 | 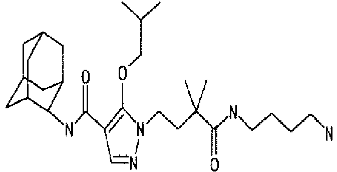 | 1.44 | 503.5 |
| D-3 | 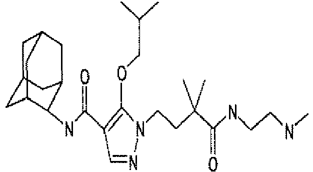 | 1.49 | 489.7 |
| D-4 | 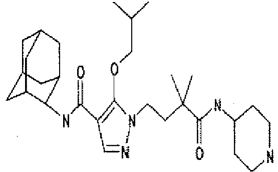 | 1.45 | 515.2 |
| D-5 | 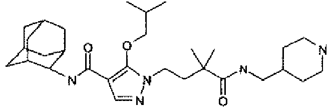 | 1.45 | 529.5 |
| D-6 | 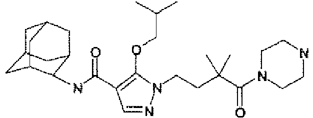 | 1.56 | 500 |

**[Table54]**

| No. | Structure | retention time | LC-MS |
|---|---|---|---|
| D-7 | 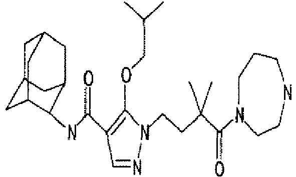 | 1.5 | 515.3 |
| D-8 | 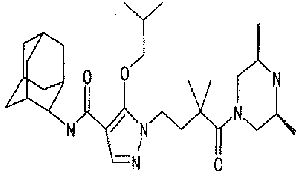 | 1.52 | 529.2 |
| D-9 | 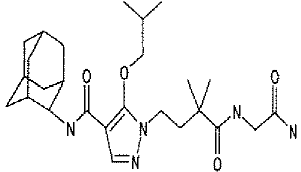 | 1.99 | 489.2 |
| D-10 | 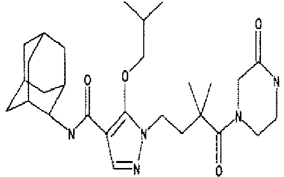 | 2.06 | 515.2 |
| D-11 | 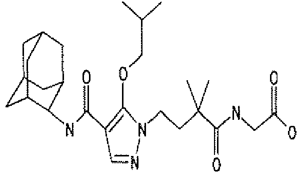 | 2.11 | 490.1 |
| D-12 | 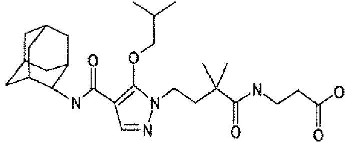 | 2.12 | 504 |

**[Table55]**

| No. | Structure | retention time | LC-MS |
|---|---|---|---|
| D-13 | 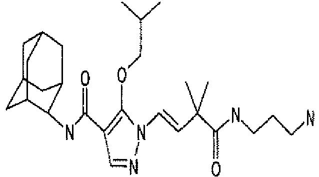 | 1.56 | 486.1 |
| D-14 | 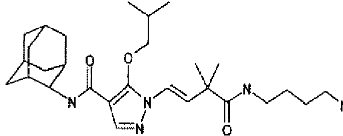 | 1.56 | 500.7 |
| D-15 | 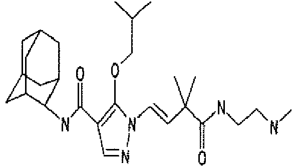 | 1.57 | 486.6 |
| D-16 | 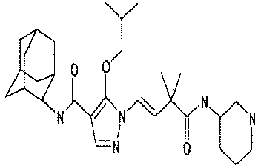 | 1.63 | 512.8 |
| D-17 | 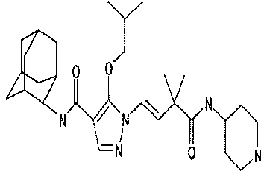 | 1.6 | 512.7 |
| D-18 | 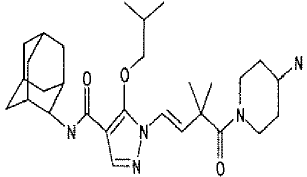 | 1.57 | 513.3 |

**[Table56]**

| No. | Structure | retention time | LC-MS |
|---|---|---|---|
| D-19 | 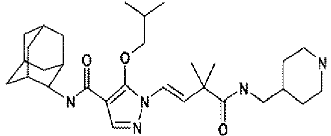 | 1.65 | 527.3 |
| D-20 | 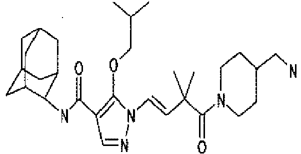 | 1.56 | 527.3 |
| D-21 | 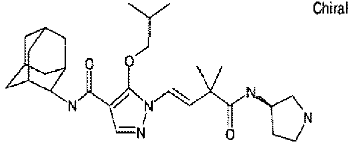 | 1.68 | 499.3 |
| D-22 | 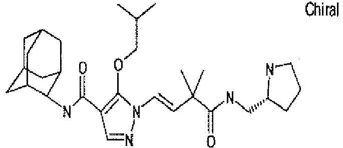 | 1.6 | 513.3 |
| D-23 | 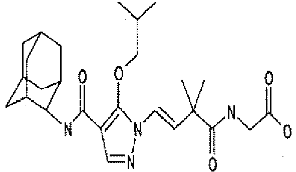 | 2.23 | 487.6 |
| D-24 | 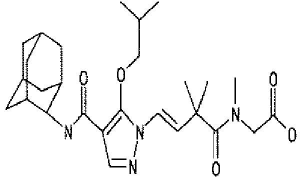 | 2.3 | 501.6 |

**[Table 57]**

| No. | Structure | retention time | LC-MS |
|---|---|---|---|
| D-25 | 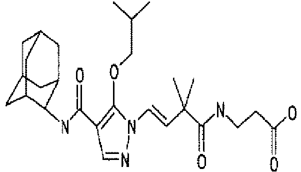 | 2.25 | 501.1 |
| D-26 | 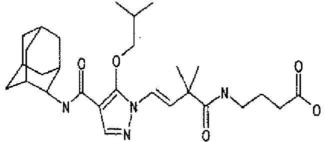 | 2.31 | 515.6 |
| D-27 | 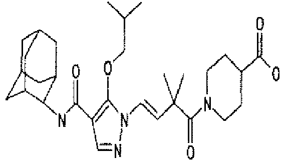 | 2.33 | 541.6 |
| D-28 | 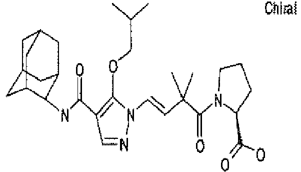 | 2.37 | 527.7 |
| D-29 | 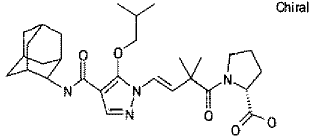 | 2.39 | 527.7 |
| D-30 | 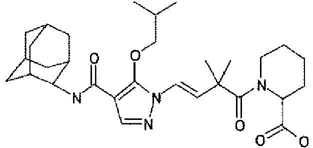 | 2.53 | 541.1 |

**[Table 58]**

| No. | Structure | retention time | LC-MS |
|---|---|---|---|
| D-31 | 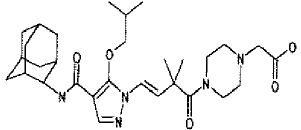 | 1.84 | 556 |
| D-32 | 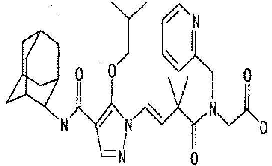 | 2.26 | 578.1 |
| D-33 | 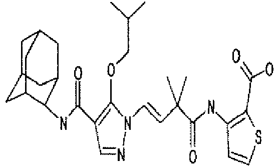 | 2.81 | 555 |
| D-34 | 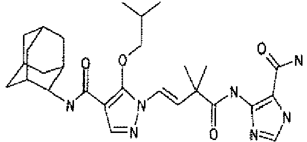 | 2.29 | 539.2 |
| D-35 | 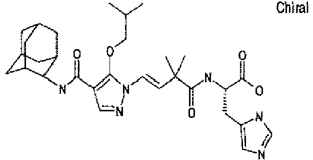 | 1.58 | 567.6 |

**[Table 59]**

| No. | Structure | No. | Structure |
|---|---|---|---|
| E-1 | 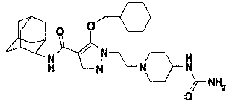 | E-2 | 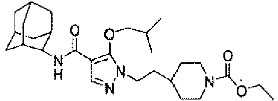 |
| E-3 | 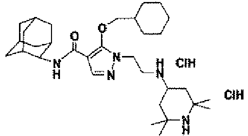 | E-4 | 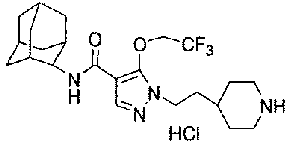 |
| E-5 | 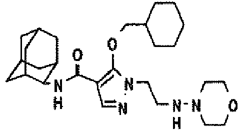 | E-6 | 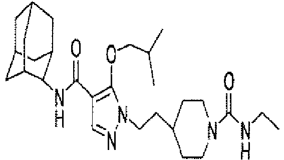 |
| E-7 | 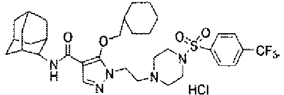 | E-8 | 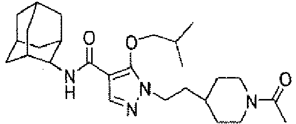 |
| E-9 | 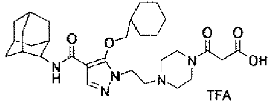 | E-10 | 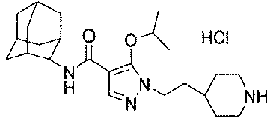 |
| Z-11 | 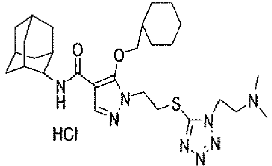 | Z-12 | 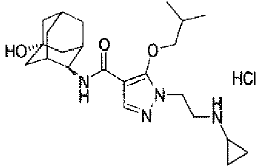 |

**[Table60]**

| **No.** | **Structure** | **No.** | **Structure** |
|---|---|---|---|
| E-13 | 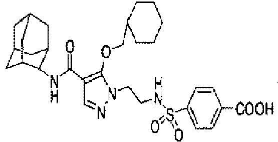 | E-14 | 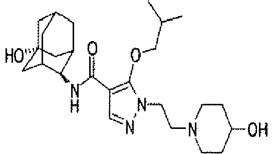 |
| E-15 | 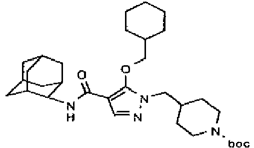 | E-16 | 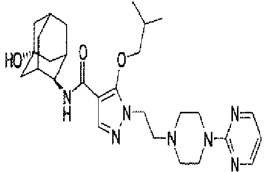 |
| E-17 | 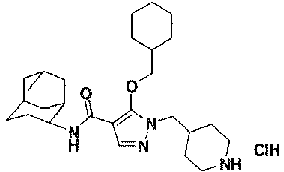 | E-18 | 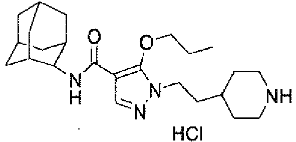 |
| E-19 | 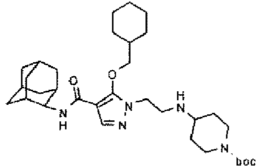 | E-20 | 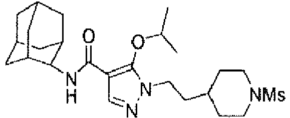 |
| E-21 | 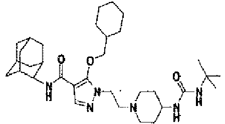 | E-22 | 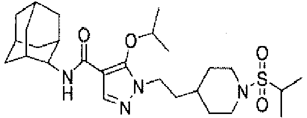 |
| E-23 | 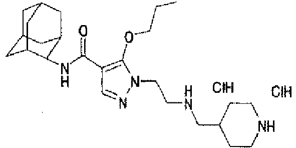 | E-24 | 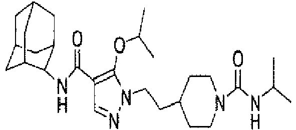 |

**[Table 61]**

| No. | Structure | No. | Structure |
|---|---|---|---|
| E-25 | 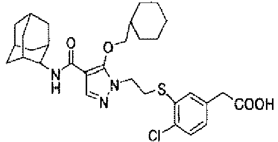 | E-26 | 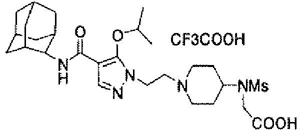 |
| E-27 | 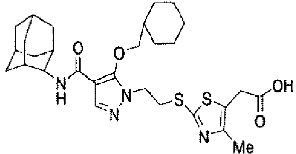 | E-28 | 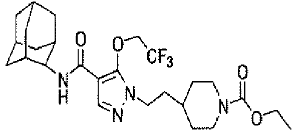 |
| E-29 | 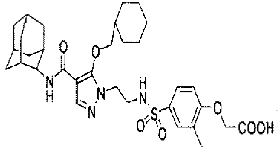 | E-30 | 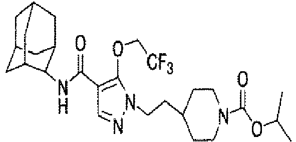 |
| E-31 | 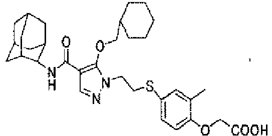 | E-32 | 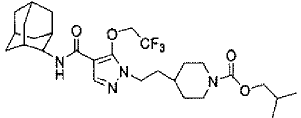 |
| E-33 | 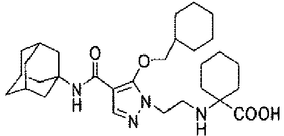 | E-34 | 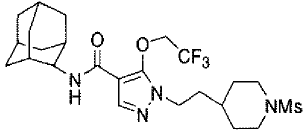 |
| E-35 | 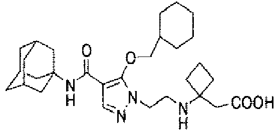 | E-36 | 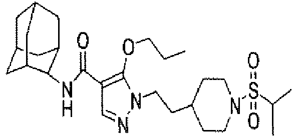 |

**[Table62]**

| **No.** | **Structure** | **No.** | **Structure** |
|---|---|---|---|
| E-37 | 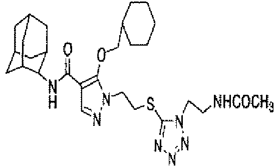 | E-38 | 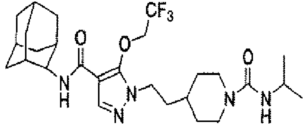 |
| E-39 | 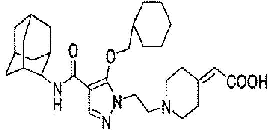 | E-40 | 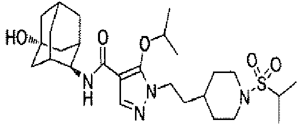 |
| E-41 | 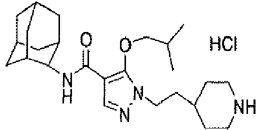 | E-42 | 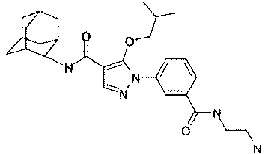 |
| E-43 | 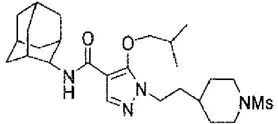 | E-44 | 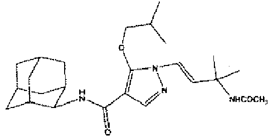 |
| E-45 | 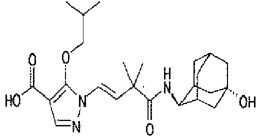 | E-46 | 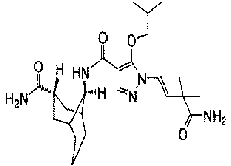 |
| E-47 | 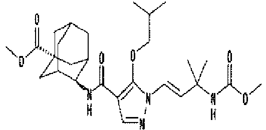 | E-48 | 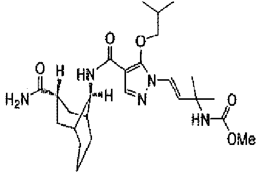 |

**[Table63]**

| No. | Structure | No. | Structure |
|---|---|---|---|
| E-49 | 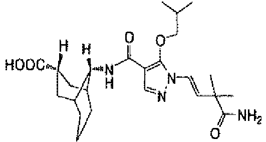 | E-50 | 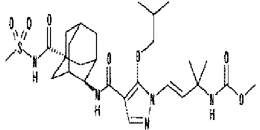 |
| E-51 | 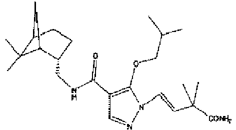 | E-52 | 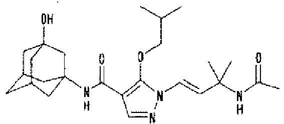 |
| E-53 | 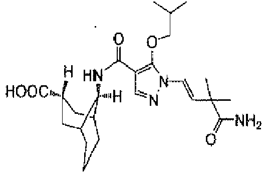 | E-54 | 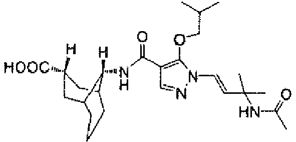 |
| E-55 | 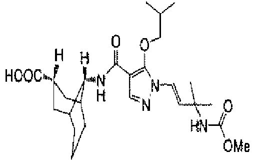 | E-56 | 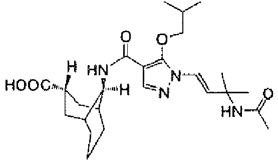 |
| E-57 | 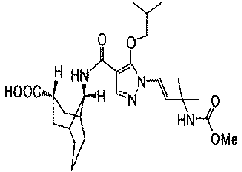 | E-58 | 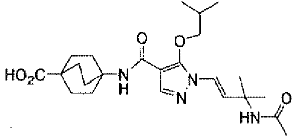 |
| E-59 | 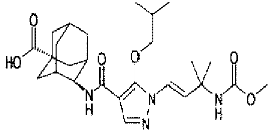 | E-60 | 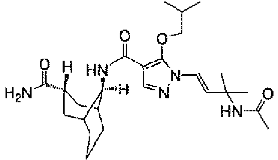 |

**[Table 64]**

| **No.** | **Structure** | **No.** | **Structure** |
|---|---|---|---|
| E-61 | 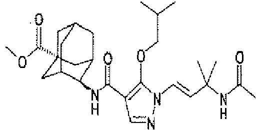 | E-62 | 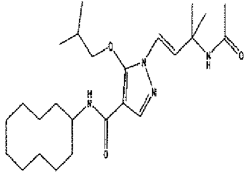 |
| E-63 | 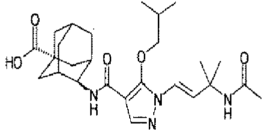 | E-64 | 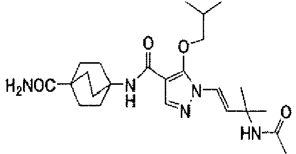 |
| E-65 | 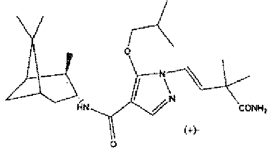 | E-66 | 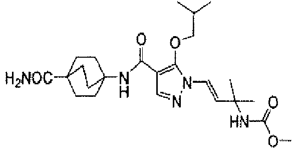 |
| E-67 | 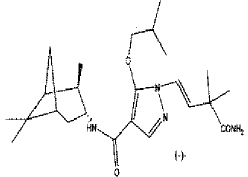 | | |

### Experimental Example 1

### Evaluation of 11ß-HSD1 inhibitors (enzyme activity assay on human 11ß-HSD1)

Enzymatic activity for human 11β-HSD1 was determined in a 10 µl final volume of assay mixture containing 50 mM sodium phosphate buffer (pH 7.6), 1 mg/ml bovine serum albumin, 0.42 mg/ml NADPH, 1.26 mg/ml glucose-6-phosphate, glucose-6-phosphate dehydrogenase, test compound, recombinant human 11β-HSD1, and 5 µM cortisone as substrate. The reaction was started with the addition of cortisone. After incubation for 2 hours at 37 °C, 5 µl of europium cryptate-labelled anti-cortisol antibody and 5 µl of XL665-labeled cortisol were added. After further incubation for 2 hours at room temperature, the homogeneous time-resolved fluorescence (HTRF) signal was measured. The cortisol production was quantitated by a standard curve generated with several known concentrations of cortisol in each assay.

The amount of cortisol production without compounds was served as control, and the percent inhibition by test compound at each concentration was calculated. The IC50 value of the compound for 11β-HSD1 was obtained using the inhibition curve generated by plotting the percent inhibition versus the concentration of test compound.

### Experimental Example 2

### Evaluation of 11B-HSD1 inhibitors (enzyme activity assay on mouse 11β-HSD1)

Enzymatic activity for mouse 11β-HSD1 activity was determined in a 10 µl final volume of assay mixture containing 50 mM sodium phosphate buffer (pH 7.6), 1 mg/ml bovine serum albumin, 0.42 mg/ml NADPH, 1.26 mg/ml glucose-6-phosphate, glucose-6-phosphate dehydrogenase, test compound, recombinant mouse 11β-HSD1, and 10 µM 11-dehydrocorticosterone as substrate. The reaction was started with the addition of 11-dehydrocorticosterone. After incubation for 2 hours at 37 °C, 5 µl of europium cryptate-labelled anti- corticosterone antibody and 5 µl of XL665-labeled corticosterone were added. After further incubation for 2 hours at room temperature, the HTRF signal was measured. The corticosterone production was quantitated by a standard curve generated with several known concentrations of corticosterone in each assay.

The amount of corticosterone production without compounds was served as control, and the percent inhibition by compound at each concentration was calculated. The IC50 value of the compound for 11β-HSD1 was obtained using the inhibition curve generated by plotting the percent inhibition versus the concentration of test compound.

The results of experimental example 1 and 2 are shown in the following table.

**[Table 65]**

| No. | humanIC50(µM) | mouseIC50(µM) |
|---|---|---|
| **A-1** | **0.19** | **5.9** |
| **A-2** | **0.27** | **1.1** |
| **A-3** | **0.3** | **24.1** |
| **A-4** | **0.037** | **1.6** |
| **A-5** | **0.71** | **>30** |
| **A-6** | **0.0083** | **0.94** |
| **A-7** | **0.018** | **0.71** |

### Experimental Example 3

### Materials and methods in oral absorption of 11β-HSD1 inhibitor

### (1) Animals

Male C57BL/6J Jcl mice were purchased from CLEA Japan at the age of 6 weeks. After 1-week preliminary rearing, the mice were used for this study at the age of 7 weeks

### (2) Rearing conditions

The mice were placed at an animal room, where was set at room temperature of 23 ± 2°C and humidity of 55 ± 10%, and lighting cycle time was 12 hours [light (8:00 - 20:00)/dark (20:00 - 8:00)]. The mice were allowed free access to solid laboratory food (CE-2, CLEA Japan) and sterile tap water through the preliminary rearing and experimental periods.

### (3) Identification of animals and cages

The mice were identified by tail marking with an oil marker pen.

Labels identifying the study director, purchased date, strain, sex and supplier were placed on each cage. The mice were housed by 20 mice/cage in the preliminary rearing period, and 3 mice/cage in the experimental period.

### (4) Group composition

Oral administration: 20 mg/kg (n=3)
Intravenous administration: 5 mg/kg (n=3)

### (5) Preparation of dosing formulation

Dosing suspension for oral administration was prepared using 0.5% methyl cellulose (1500 cP) aqueous solution. Dosing solution for intravenous administration was prepared using N-dimethylacetamide / polyethyleneglycol 400 (1/2).

### (6) Dosing method

As to oral administration, the dosing suspension at 10 mL/kg was administered into the stomach using a feeding tube. As to intravenous administration, the dosing solution at 2.5 mL/kg was administered into the caudal vein using a glass syringe.

### (7) Evaluation items

The blood samples were collected from the heart at each sampling point. The drug concentration in plasma was measured using HPLC or LC/MS/MS.

### (8) Statistical analysis

The area under the plasma concentration-time curve (AUC) was calculated by WinNonlin^{®}, and the bioavailability was calculated by the AUC values after oral and intravenous administration.

The following formulation examples 1 to 8 are provided to further illustrate the present invention and are not intended to limit the scope of the present invention. The term of "active ingredient" means a compound of the present invention, a pharmaceutical acceptable salt, or a hydrate thereof.

### (Formulation Example 1)

Hard gelatin capsules are prepared with the following ingredients:

| | Dose (mg/capsule) |
|---|---|
| Active ingredient | 250 |
| Starch (dried) | 200 |
| Magnesium stearate | 10 |
| Total | 460 mg |

### (Formulation Example 2)

Tablets are prepared with the following ingredients:

| | Dose (mg/tablet) |
|---|---|
| Active ingredient | 250 |
| Cellulose (microcrystal) | 400 |
| Silicon dioxide, fumed | 10 |
| Stearic acid | 5 |
| | 161 |
| Total | 665 mg |

The ingredients are blended and compressed to form tablets each weighing 665 mg.

### (Formulation Example 3)

Tablets, each containing 60 mg of active ingredient, are made as follows.

| | |
|---|---|
| Active ingredient | 60 mg |
| Starch | 45 mg |
| Microcrystals cellulose | 35 mg |
| Polyvinylpyrrolidone (as 10% solution in water) | 4 mg |
| Sodium carboxymethyl starch | 4.5 mg |
| Magnesium stearate | 0.5 mg |
| Talc | 1 mg |
| Total | 150 mg |

The active ingredient, starch, and cellulose are passed through a No. 45 mesh U.S. sieve, and the mixed thoroughly. The aqueous solution containing polyvinylpyrrolidone is mixed with the obtained powder, and then the admixture is passed through a No. 14 mesh U.S. sieve. The granules so produced are dried at 50 °C and passed through a No. 18 mesh U.S. sieve. The sodium carboxymethyl starch, magnesium stearate, and talc, previously passed through No. 60 mesh U.S. sieve, are added to the granules, mixed, and then compressed on a tablet machine to yield tablets each weighing 150 mg.

### (Formulation Example 4)

Capsules, each containing 80 mg of active ingredient, are made as follows:

| | |
|---|---|
| Active ingredient | 80 mg |
| Starch | 59 mg |
| Microcrystals cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

### (Formulation Example 5)

Suppositories, each containing 225 mg of active ingredient, are made as follows:

| | |
|---|---|
| Active ingredient | 225 mg |
| Saturated fatty acid glycerides | 2000 mg |
| Total | 2225 mg |

The active ingredient is passed through a No. 60 mesh U.S. sieve and suspended in the saturated fatty acid glycerides previously melted using the minimum heat necessary. The mixture is then poured into a suppository mold of nominal 2g capacity and allowed to cool.

### (Formulation Example 6)

Suspensions, each containing 50 mg of active ingredient, are made as follows:

| | |
|---|---|
| Active ingredient | 50 mg |
| Sodium carboxymethyl cellulose | 50 mg |
| Syrup | 1.25 mL |
| Benzoic acid solution | 0.10 mL |
| Flavor | q.v. |
| Color | q.v. |
| Purified water to total | 5 mL |

The active ingredient is passed through a No. 45 U.S. sieve, and mixed with the sodium carboxymethyl cellulose and syrup to form a smooth paste. The benzoic acid solution and flavor are diluted with a portion of the water, added and stirred. Then sufficient water is added to produce the required volume.

### (Formulation Example 7)

An intravenous formulation may be prepared as follows:

| | |
|---|---|
| Active ingredient | 100 mg |
| Saturated fatty acid glycerides | 1000 mL |

The solution of the above ingredients is generally administered intravenously to a patient at a rate of 1 mL per minute.

## Claims

1. A compound represented by formula (I): 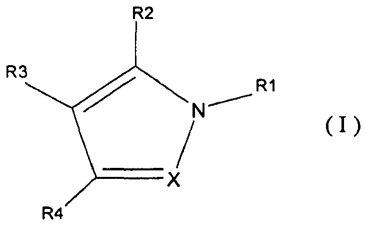 a pharmaceutically acceptable salt or a solvate thereof,
wherein
R¹ is optionally substituted alkyl, optionally substituted alkenyl, optionally substituted alkynyl, optionally substituted cycloalkyl, optionally substituted cycloalkenyl, optionally substituted aryl, optionally substituted heteroaryl or optionally substituted heterocycle,
one of R² and R⁴ is a group of formula: -Y-R⁵,
wherein Y is -O-or -S -, and
R⁵ is substituted straight alkyl wherein the substituent of said substituted straight alkyl is cycloalkyl or cycloalkenyl,
branched alkyl optionally substituted with hydroxy, carboxy or halogen, alkenyl optionally substituted with hydroxy, carboxy or halogen, alkynyl optionally substituted with hydroxy, carboxy or halogen, cycloalkyl optionally substituted with hydroxy, carboxy or halogen, cycloalkenyl optionally substituted with hydroxy, carboxy or halogen, aryl optionally substituted with hydroxy, carboxy or halogen, heteroaryl optionally substituted with hydroxy, carboxy or halogen or heterocycle optionally substituted with hydroxy, carboxy or halogen,
the other of R² and R⁴ is hydrogen, optionally substituted alkyl, optionally substituted alkenyl, optionally substituted alkynyl, optionally substituted cycloalkyl, optionally substituted cycloalkenyl, optionally substituted aryl, optionally substituted heteroaryl or optionally substituted heterocycle,
R³ is a group of formula: -C(=O)-Z-R⁶,
wherein Z is -NR⁷- or -NR⁷-W-, and
R⁶ is cycloalkyl optionally substituted with hydroxy, carboxy, halogen, cyano, -CH₃, - CH₂OH, -CH₂OCONH₂, -CH₂NH₂, -OCONH₂, -NHCOCH₃, -OCH₃, -NH₂, - NHSO₂CH₃, -CONHCH₃, -OSO₂NH₂, -NHSO₂NH₂, -NHCOOCH₃, -CONH₂, -COOCH₃ or -CONHSO₂CH₃, said cycloalkyl is selected from 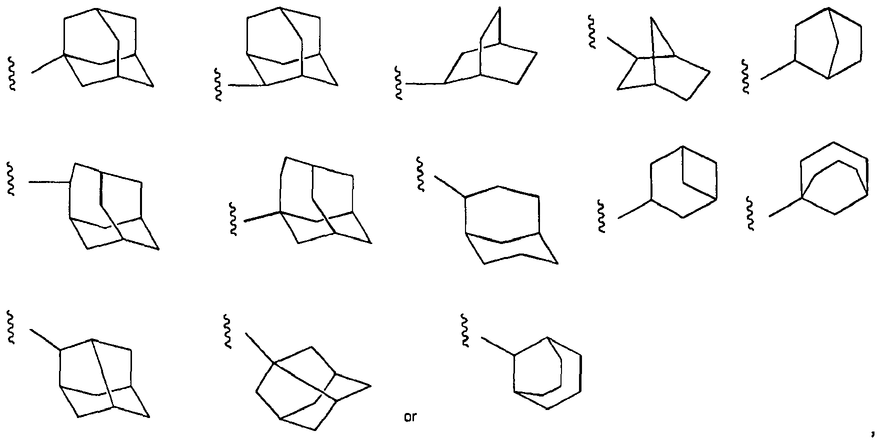 R⁷ is hydrogen or optionally substituted alkyl,
W is optionally substituted alkylene,
X is =N- or =CR⁸-, and
R⁸ is hydrogen or optionally substituted alkyl,
wherein "alkyl" means a C1 to C10 straight or branched alkyl group;
"alkenyl" means a C2 to C8 straight or branched alkenyl group;
"alkynyl" means a C2 to C8 straight or branched alkynyl group;
"cycloalkyl" means a C3 to C15 saturated cyclic hydrocarbon group;
"cycloalkenyl" means a C3 to C7 unsaturated aliphatic hydrocarbon group; "aryl" means phenyl or naphthyl;
"heteroaryl" means furyl, thienyl, pyrrolyl, imidazolyl, pyrazolyl, triazolyl, tetraz olyl, oxazolyl, isoxazolyl, thiazolyl, thiadiazolyl, isothiazolyl, pyridyl, pyridazinyl, pyrimidinyl, furazanyl, pyrazinyl, oxadiazolyl, benzofuryl, benzothienyl, benzimi dazolyl, dibenzofuryl, benzoxazolyl, quinoxalyl, cinnolyl, quinazolyl, quinolyl, pht halazinyl, isoquinolyl, puryl, pteridinyl, carbazolyl, phenanthridinyl, acridinyl, in dolyl, isoindolyl, phenazinyl or phenothiazinyl;
"heterocycle" means 1-pyrrolinyl, 2-pyrrolinyl, 3-pyrrolinyl, 1-pyrrolidinyl, 2-pyrr olidinyl, 3-pyrrolidinyl, 1-imidazolinyl, 2-imidazolinyl, 4-imidazolinyl, 1-imidazoli dinyl, 2-imidazolidinyl, 4-imidazolidinyl, 1-pyrazolinyl, 3-pyrazolinyl, 4-pyrazoliny 1,1-pyrazolidinyl, 3-pyrazolidinyl, 4-pyrazolidinyl, piperidino, 2-piperidinyl, 3-pip eridinyl, 4-piperidinyl, 1-piperazinyl, 2-piperazinyl, 2-morpholinyl, 3-morpholinyl, morpholino, tetrahydropyranyl or the following groups

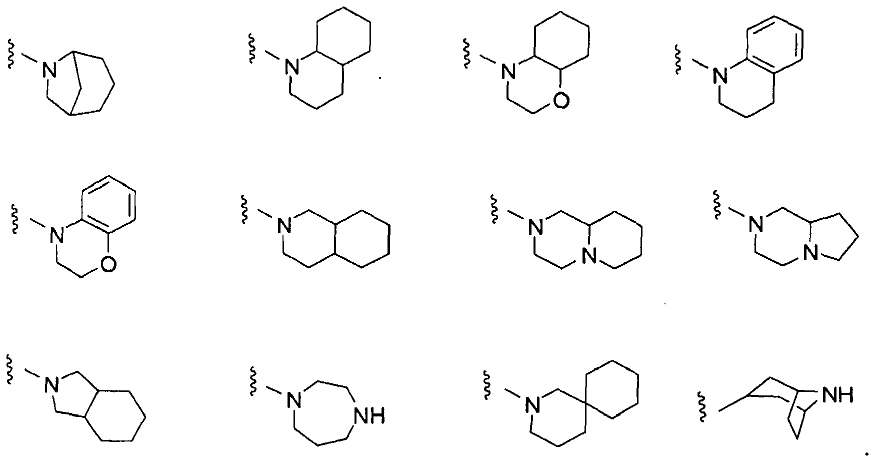

2. The compound according to claim 1, a pharmaceutically acceptable salt or a solvate thereof, wherein R¹ is substituted alkyl wherein the substituent of said substituted alkyl is optionally substituted amino or optionally substituted heterocycle.

3. The compound according to claim 1, a pharmaceutically acceptable salt or a solvate thereof, wherein R¹ is substituted ethyl wherein the substituent of said substituted ethyl is optionally substituted amino or optionally substituted heterocycle.

4. The compound according to claim 1, a pharmaceutically acceptable salt or a solvate thereof, wherein R¹ is unsubstituted alkyl.

5. The compound according to claim 1, a pharmaceutically acceptable salt or a solvate thereof, wherein R² is a group of formula: -Y-R⁵,
wherein Y and R⁵ have the same meaning as defined in claim 1.

6. The compound according to claim 5, a pharmaceutically acceptable salt or a solvate thereof, wherein Y is -O-.

7. The compound according to claim 5 or 6, a pharmaceutically acceptable salt or a solvate thereof, wherein R⁵ is substituted straight alkyl wherein the substituent of said substituted straight alkyl is optionally substituted cycloalkyl.

8. The compound according to claim 7, a pharmaceutically acceptable salt or a solvate thereof, wherein R³ is substituted methyl wherein the substituent of said substituted methyl is optionally substituted cycloalkyl.

9. The compound according to claim 7 or 8, a pharmaceutically acceptable salt or a solvate thereof, wherein said optionally substituted cycloalkyl is optionally substituted cyclohexyl.

10. The compound according to claim 5 or 6, a pharmaceutically acceptable salt or a solvate thereof, wherein R⁵ is branched alkyl.

11. The compound according to claim 1, a pharmaceutically acceptable salt or a solvate thereof, wherein Z is -NR⁷-, and R⁷ has the same meaning as defined in claim 1.

12. The compound according to claim 11, a pharmaceutically acceptable salt or a solvate thereof, wherein R⁷ is hydrogen.

13. The compound according to claim 1, a pharmaceutically acceptable salt or a solvate thereof, wherein R⁶ is adamantyl.

14. The compound according to claim 1, a pharmaceutically acceptable salt or a solvate thereof,
wherein R³ is a group of formula (II):

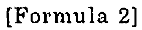

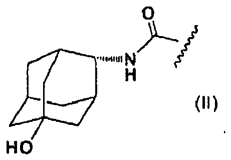

15. The compound according to any one of claims 1 to 14, a pharmaceutically acceptable salt or a solvate thereof, wherein X is =N-.

16. The compound according to claim 1, a pharmaceutically acceptable salt or a solvate thereof, wherein R¹ is a group of formula: -CH=CH-C(R⁹R¹⁰)-R¹¹-R¹²,
wherein R⁹ and R¹⁰ are each independently hydrogen, optionally substituted alkyl or halogen, or R⁹ and R¹⁰ taken together with the carbon atom to which they are attached may form an optionally substituted ring,
R¹¹ is -(CH₂)ₙ-, wherein n is an integer of 0 to 3, and
R¹² is hydrogen, hydroxy, carboxy, cyano, optionally substituted alkyl, optionally substituted alkenyl, optionally substituted alkynyl, optionally substituted cycloalkyl, optionally substituted cycloalkenyl, optionally substituted aryl, optionally substituted heteroaryl, optionally substituted heterocycle, optionally substituted alkyloxycarbonyl, optionally substituted arylalkylcarbonyl, optionally substituted carbamoyl, optionally substituted thiocarbamoyl, optionally substituted alkylsulfonyl, optionally substituted arylsulfonyl, optionally substituted sulfamoyl, optionally substituted amino, optionally substituted carbamoyloxy, optionally substituted alkyloxy or optionally substituted alkylthio,
a group of formula: -C(=O)-NR¹³R¹⁴,
wherein R¹³ and R¹⁴ are each independently hydrogen, optionally substituted amino, optionally substituted alkyl, optionally substituted alkynyl, optionally substituted alkynyl, optionally substituted cycloalkyl, optionally substituted cycloalkenyl, optionally substituted aryl, optionally substituted heteroaryl, optionally substituted heterocycle, optionally substituted alkylsulfonyl, optionally substituted arylsulfonyl, optionally substituted heteroarylsulfonyl or optionally substituted heterocyclesulfonyl, or R¹³ and R¹⁴ taken together with the nitrogen atom to which they are attached may form an optionally substituted ring or,
a group of formula: -NR¹⁵R¹⁶,
wherein R¹⁶ and R¹⁶ are each independently hydrogen, carboxy, hydroxy, optionally substituted alkyl, optionally substituted alkenyl, optionally substituted alkynyl, optionally substituted cycloalkyl, optionally substituted cycloalkenyl, optionally substituted aryl, optionally substituted heteroaryl, optiona.lly substituted heterocycle, optionally substituted acyl, optionally substituted carbamoyl, optionally substituted thiocarbamoyl, optionally substituted alkylsulfonyl, optionally substituted arylsulfonyl, optionally substituted alkyloxycarbonyl or optionally substituted sulfamoyl, or R¹⁵ and R¹⁶ taken together with the nitrogen atom to which they are attached may form optionally substituted ring.

17. The compound according to claim 1, a pharmaceutically acceptable salt or a solvate thereof, wherein R¹ is a group of formula: -CH₂-CH₂-C(R⁹R¹⁰)-R¹¹-R¹²,
wherein R⁹, R¹⁰, R¹¹ and R¹² have the same meaning as defined in claim 16.

18. The compound according to claim 16 or 17, a pharmaceutically acceptable salt or a solvate thereof, wherein R⁹ and R¹⁰ are each independently optionally substituted alkyl or R⁹ and R¹⁰ taken together with the carbon atom to which they are attached may form optionally substituted ring.

19. The compound according to any one of claims 16 to 18, a pharmaceutically acceptable salt or a solvate thereof, wherein R¹¹ is -(CH₂)ₙ-, wherein n is an integer of 0 to 1.

20. The compound according to any one of claims 16 to 19, a pharmaceutically acceptable salt or a solvate thereof, wherein R¹² is carboxy, cyano or heterocycle.

21. The compound according to any one of claims 16 tO 19, a pharmaceutically acceptable salt or a solvate thereof, wherein R¹² is a group of formula: C(=O)-NR¹³R¹⁴,
wherein R¹³ and R¹⁴ are each independently hydrogen, optionally substituted alkyl, optionally substituted aryl, optionally substituted cycloalkyl, optionally substituted alkylsulfonyl, optionally substituted arylsulfonyl, optionally substituted heteroarylsulfonyl, optionally substituted heterocyclesulfonyl or optionally substituted heterocycle, or R¹³ and R¹⁴ taken together with the nitrogen atom to which they are attached may form an optionally substituted ring.

22. The compound according to any one of claims 16 to 19, a pharmaceutically acceptable salt or a solvate thereof, wherein R¹² is a group of formula: -NR¹⁵R¹⁶,
wherein R¹⁵ and R¹⁶ have the same meaning as defined in claim 16.

23. The compound according to claim 22, a pharmaceutically acceptable salt or a solvate thereof, wherein R¹⁵ is a group of formula: -C(=O)R', wherein R' is optionally substituted alkyl, optionally substituted alkenyl, optionally substituted cycloalkyl, optionally substituted aryl, optionally substituted amino or optionally substituted alkyloxy.

24. A pharmaceutical composition which comprises the compound according to any one of claims 1 to 23, a pharmaceutically acceptable salt or a solvate thereof as an active ingredient.

25. The pharmaceutical composition according to claim 24 for use in treating and/or preventing diabetes.

## Patentansprüche

1. Verbindung der Formel (I): 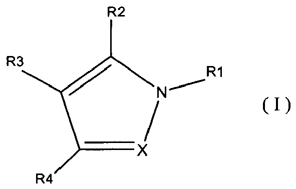 ein pharmazeutisch annehmbares Salz oder ein Solvat davon,
worin:
R¹ gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Aryl, gegebenenfalls substituiertes Heteroaryl oder ein gegebenenfalls substituierter Heterocyclus ist,
eines von R² und R⁴ eine Gruppe der Formel: -Y-R⁵ ist,
worin
Y -O- oder -S- ist, und
R⁵ substituiertes geradkettiges Alkyl, wobei der Substituent dieses substituierten geradkettigen Alkyls Cycloalkyl oder Cycloalkenyl ist,
verzweigtes Alkyl, gegebenenfalls substituiert mit Hydroxy, Carboxy oder Halogen,
Alkenyl, gegebenenfalls substituiert mit Hydroxy, Carboxy oder Halogen,
Alkinyl, gegebenenfalls substituiert mit Hydroxy, Carboxy oder Halogen,
Cycloalkyl, gegebenenfalls substituiert mit Hydroxy, Carboxy oder Halogen,
Cycloalkenyl, gegebenenfalls substituiert mit Hydroxy, Carboxy oder Halogen,
Aryl, gegebenenfalls substituiert mit Hydroxy, Carboxy oder Halogen,
Heteroaryl, gegebenenfalls substituiert mit Hydroxy, Carboxy oder Halogen, oder
ein Heterocyclus, gegebenenfalls substituiert mit Hydroxy, Carboxy oder Halogen,
ist,
das andere von R² und R⁴ Wasserstoff, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Aryl, gegebenenfalls substituiertes Heteroaryl oder ein gegebenenfalls substituierter Heterocyclus ist,
R³ eine Gruppe der Formel: -C(=O)-Z-R⁶ ist,
worin
Z -NR⁷- oder -NR⁷-W- ist und
R⁶ Cycloalkyl, gegebenenfalls substituiert mit Hydroxy, Carboxy, Halogen, Cyano, -CH₃, -CH₂OH -CH₂OCONH₂, -CH₂NH₂, -OCONH₂, -NHCOCH₃, -OCH₃, -NH₂, -NHSO₂CH₃, -CONHCH₃, -OSO₂NH₂, -NHSO₂NH₂, -NHCOOCH₃, -CONH₂, -COOCH₃ oder -CONHSO₂CH₃ ist, wobei das Cycloalkyl ausgewählt ist aus: 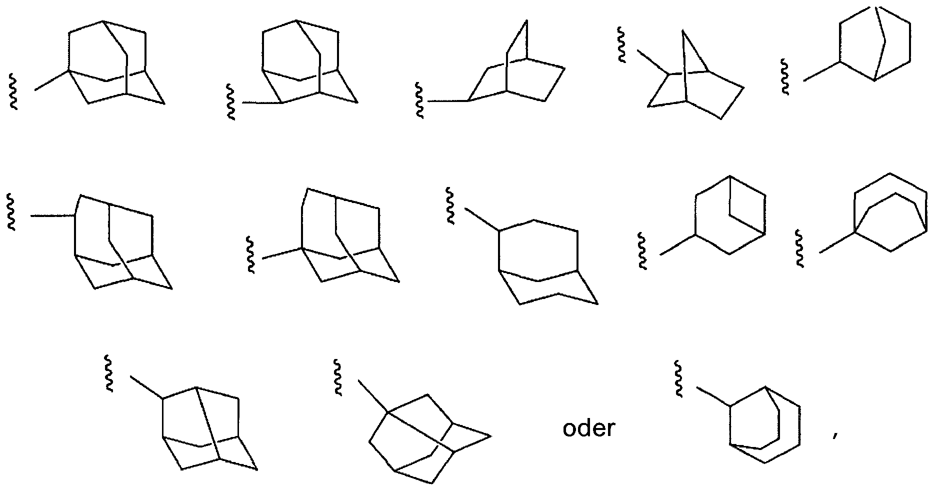
R⁷ Wasserstoff oder gegebenenfalls substituiertes Alkyl ist,
W gegebenenfalls substituiertes Alkylen ist,
X =N- oder =CR⁸- ist und
R⁸ Wasserstoff oder gegebenenfalls substituiertes Alkyl ist,
worin
"Alkyl" eine geradkettige oder verzweigte C₁₋₁₀-Alkylgruppe bedeutet;
"Alkenyl" eine geradkettige oder verzweigte C₂₋₈-Alkenylgruppe bedeutet;
"Alkinyl" eine geradkettige oder verzweigte C₂₋₈-Alkinylgruppe bedeutet;
"Cycloalkyl" eine gesättigte cyclische C₃₋₁₅-Kohlenwasserstoffgruppe bedeutet;
"Cycloalkenyl" eine ungesättigte aliphatische C₃₋₇-Kohlenwasserstoffgruppe bedeutet;
"Aryl" Phenyl oder Naphthyl bedeutet;
"Heteroaryl" Furyl, Thienyl, Pyrrolyl, Imidazolyl, Pyrazolyl, Triazolyl, Tetrazolyl, Oxazolyl, Isoxazolyl, Thiazolyl, Thiadiazolyl, Isothiazolyl, Pyridyl, Pyridazinyl, Pyrimidinyl, Furazanyl, Pyrazinyl, Oxadiazolyl, Benzofuryl, Benzothienyl, Benzimidazolyl, Dibenzofuryl, Benzoxazolyl, Chinoxalyl, Cinnolyl, Chinazolyl, Chinolyl, Phthalazinyl, Isochinolyl, Puryl, Pteridinyl, Carbazolyl, Phenanthridinyl, Acridinyl, Indolyl, Isoindolyl, Phenazinyl oder Phenothiazinyl bedeutet;
"Heterocyclus" 1-Pyrrolinyl, 2-Pyrrolinyl, 3-Pyrrolinyl, 1-Pyrrolidinyl, 2-Pyrrolidinyl, 3-Pyrrolidinyl, 1-Imidazolinyl, 2-Imidazolinyl, 4-Imidazolinyl, 1-Imidazolidinyl, 2-Imidazolidinyl, 4-Imidazolidinyl, 1-Pyrazolinyl, 3-Pyrazolinyl, 4-Pyrazolinyl, 1-Pyrazolidinyl, 3-Pyrazolidinyl, 4-Pyrazolidinyl, Piperidino, 2-Piperidinyl, 3-Piperidinyl, 4-Piperidinyl, 1-Piperazinyl, 2-Piperazinyl, 2-Morpholinyl, 3-morpholinyl,, Morpholino, Tetrahydropyranyl oder die nachstehenden Gruppen bedeutet:

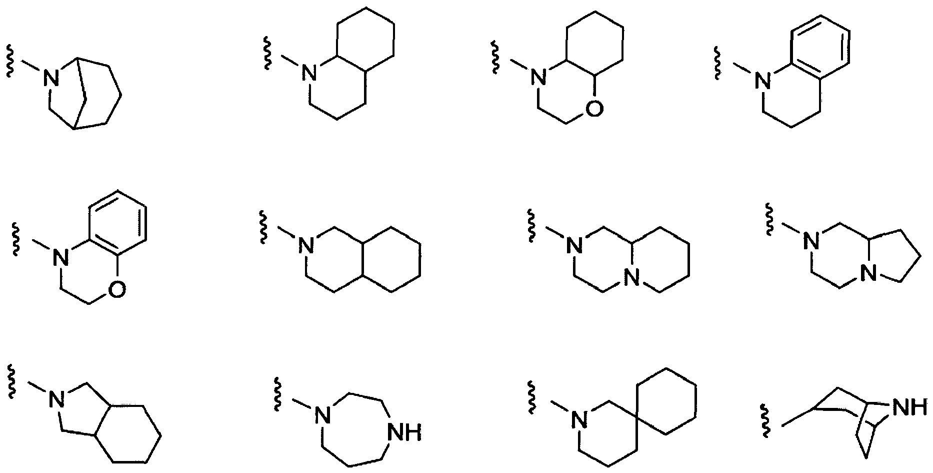

2. Verbindung gemäss Anspruch 1, ein pharmazeutisch annehmbares Salz oder ein Solvat davon, worin R^{l} substituiertes Alkyl ist, wobei der Substituent dieses substituierten Alkyls gegebenenfalls substituiertes Amino oder ein gegebenenfalls substituierter Heterocyclus ist.

3. Verbindung gemäss Anspruch 1, ein pharmazeutisch annehmbares Salz oder ein Solvat davon, worin R¹ substituiertes Ethyl ist, wobei der Substituent dieses substituierten Ethyls gegebenenfalls substituiertes Amino oder ein gegebenenfalls substituierter Heterocyclus ist.

4. Verbindung gemäss Anspruch 1, ein pharmazeutisch annehmbares Salz oder ein Solvat davon, worin R¹ unsubstituiertes Alkyl ist.

5. Verbindung gemäss Anspruch 1, ein pharmazeutisch annehmbares Salz oder ein Solvat davon, worin R² eine Gruppe der Formel: -Y-R⁵ ist, worin Y und R⁵ die gleichen Bedeutungen haben, wie in Anspruch 1 definiert.

6. Verbindung gemäss Anspruch 5, ein pharmazeutisch annehmbares Salz oder ein Solvat davon, worin Y -O- ist.

7. Verbindung gemäss Anspruch 5 oder 6, ein pharmazeutisch annehmbares Salz oder ein Solvat davon, worin R⁵ substituiertes geradkettiges Alkyl ist, wobei der Substituent dieses substituierten geradkettigen Alkyls gegebenenfalls substituiertes Cycloalkyl ist.

8. Verbindung gemäss Anspruch 7, ein pharmazeutisch annehmbares Salz oder ein Solvat davon, worin R⁵ substituiertes Methyl ist, wobei der Substituent dieses substituierten Methyls gegebenenfalls substituiertes Cycloalkyl ist.

9. Verbindung gemäss Anspruch 7 oder 8, ein pharmazeutisch annehmbares Salz oder ein Solvat davon, worin dieses gegebenenfalls substituierte Cycloalkyl gegebenenfalls substituiertes Cyclohexyl ist.

10. Verbindung gemäss Anspruch 5 oder 6, ein pharmazeutisch annehmbares Salz oder ein Solvat davon, worin R⁵ verzweigtes Alkyl ist.

11. Verbindung gemäss Anspruch 1, ein pharmazeutisch annehmbares Salz oder ein Solvat davon, worin Z -NR⁷-ist und R⁷ die gleiche Bedeutung hat wie in Anspruch 1 definiert.

12. Verbindung gemäss Anspruch 11, ein pharmazeutisch annehmbares Salz oder ein Solvat davon, worin R⁷ Wasserstoff ist.

13. Verbindung gemäss Anspruch 1, ein pharmazeutisch annehmbares Salz oder ein Solvat davon, worin R⁶ Adamantyl ist.

14. Verbindung gemäss Anspruch 1, ein pharmazeutisch annehmbares Salz oder ein Solvat davon,
worin
R³ eine Gruppe der Formel (II) ist:

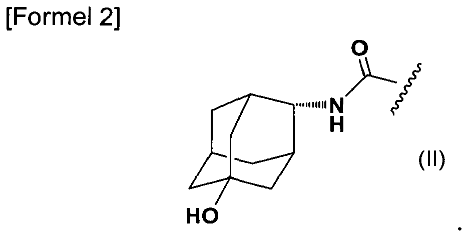

15. Verbindung gemäss irgendeinem der Ansprüche 1 bis 14, ein pharmazeutisch annehmbares Salz oder ein Solvat davon, worin X =N- ist.

16. Verbindung gemäss Anspruch 1, ein pharmazeutisch annehmbares Salz oder ein Solvat davon, worin R¹ eine Gruppe der Formel -CH=CH-C(R⁹R¹⁰)-R¹¹-R¹² ist,
worin
R⁹ und R¹⁰ jeweils unabhängig Wasserstoff, gegebenenfalls substituiertes Alkyl oder Halogen sind oder R⁹ und R¹⁰ zusammen mit dem Kohlenstoffatom, an das sie gebunden sind, einen gegebenenfalls substituierten Ring bilden können,
R¹¹ -(CH₂)ₙ- ist, worin n eine ganze Zahl von 0 bis 3 ist, und
R¹² Wasserstoff, Hydroxy, Carboxy, Cyano, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Aryl, gegebenenfalls substituiertes Heteroaryl, ein gegebenenfalls substituierter Heterocyclus, gegebenenfalls substituiertes Alkyloxycarbonyl, gegebenenfalls substituiertes Arylalkylcarbonyl, gegebenenfalls substituiertes Carbamoyl, gegebenenfalls substituiertes Thiocarbamoyl, gegebenenfalls substituiertes Alkylsulfonyl, gegebenenfalls substituiertes Arylsulfonyl, gegebenenfalls substituiertes Sulfamoyl, gegebenenfalls substituiertes Amino, gegebenenfalls substituiertes Carbamoyloxy, gegebenenfalls substituiertes Alkyloxy oder gegebenenfalls substituiertes Alkylthio ist,
eine Gruppe der Formel -C(=O)-NR¹³R¹⁴ ist,
worin R¹³ und R¹⁴ jeweils unabhängig Wasserstoff, gegebenenfalls substituiertes Amino, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Aryl, gegebenenfalls substituiertes Heteroaryl, ein gegebenenfalls substituierter Heterocyclus, gegebenenfalls substituiertes Alkylsulfonyl, gegebenenfalls substituiertes Arylsulfonyl, gegebenenfalls substituiertes Heteroarylsulfonyl oder gegebenenfalls substituiertes Heterocyclylsulfonyl sind, oder R¹³ und R¹⁴ zusammen mit dem Stickstoffatom, an das sie gebunden sind, einen gegebenenfalls substituierten Ring bilden können oder, eine Gruppe der Formel -NR¹⁵R¹⁶ ist,
worin R¹⁵ und R¹⁶ jeweils unabhängig Wasserstoff, Carboxy, Hydroxy, gegebenenfalls substituiertes Alkyl, gegebenenfalls substituiertes Alkenyl, gegebenenfalls substituiertes Alkinyl, gegebenenfalls substituiertes Cycloalkyl, gegebenenfalls substituiertes Cycloalkenyl, gegebenenfalls substituiertes Aryl, gegebenenfalls substituiertes Heteroaryl, ein gegebenenfalls substituierter Heterocyclus, gegebenenfalls substituiertes Acyl, gegebenenfalls substituiertes Carbamoyl, gegebenenfalls substituiertes Thiocarbamoyl, gegebenenfalls substituiertes Alkylsulfonyl, gegebenenfalls substituiertes Arylsulfonyl, gegebenenfalls substituiertes Alkyloxycarbonyl oder gegebenenfalls substituiertes Sulfamoyl sind, oder R¹⁵ und R¹⁶ zusammen mit dem Stickstoffatom, an das sie gebunden sind, einen gegebenenfalls substituierten Ring bilden können.

17. Verbindung gemäss Anspruch 1, ein pharmazeutisch annehmbares Salz oder ein Solvat davon, worin R¹ eine Gruppe der Formel -CH₂-CH₂-C(R⁹R¹⁰)-R¹¹-R¹² ist, worin R⁹, R¹⁰, R¹¹ und R¹² die gleichen Bedeutungen haben wie in Anspruch 16 definiert.

18. Verbindung gemäss Anspruch 16 oder 17, ein pharmazeutisch annehmbares Salz oder ein Solvat davon, worin R⁹ und R¹⁰ jeweils unabhängig gegebenenfalls substituiertes Alkyl sind oder R⁹ und R¹⁰ zusammen mit dem Kohlenstoffatom, an das sie gebunden sind, einen gegebenenfalls substituierten Ring bilden können.

19. Verbindung gemäss irgendeinem der Ansprüche 16 bis 18, ein pharmazeutisch annehmbares Salz oder ein Solvat davon, worin R¹¹ -(CH₂)ₙ- ist, worin n eine ganze Zahl von 0 bis 1 ist.

20. Verbindung gemäss irgendeinem der Ansprüche 16 bis 19, ein pharmazeutisch annehmbares Salz oder ein Solvat davon, worin R¹² Carboxy, Cyano oder ein Heterocyclus ist.

21. Verbindung gemäss irgendeinem der Ansprüche 16 bis 19, ein pharmazeutisch annehmbares Salz oder ein Solvat davon, worin R¹² eine Gruppe der Formel -C(=O)-NR¹³R¹⁴ ist, worin R¹³ und R¹⁴ jeweils unabhängig Wasserstoff, gegebenenfalls substituiertes Alkyl, gegebenenfalls substituiertes Aryl, gegebenenfalls substituiertes Cycloalkyl, gegebenenfalls substituiertes Alkylsulfonyl, gegebenenfalls substituiertes Arylsulfonyl, gegebenenfalls substituiertes Heteroarylsulfonyl, gegebenenfalls substituiertes Heterocyclylsulfonyl oder ein gegebenenfalls substituierter Heterocyclus sind oder R¹³ und R¹⁴ zusammen mit dem Stickstoffatom, an das sie gebunden sind, einen gegebenenfalls substituierten Ring bilden können.

22. Verbindung gemäss irgendeinem der Ansprüche 16 bis 19, ein pharmazeutisch annehmbares Salz oder ein Solvat davon, worin R¹² eine Gruppe der Formel -NR¹⁵R¹⁶ ist, worin R¹⁵ und R¹⁶ die gleichen Bedeutungen haben wie in Anspruch 16 definiert.

23. Verbindung gemäss Anspruch 22, ein pharmazeutisch annehmbares Salz oder ein Solvat davon, worin R¹⁵ eine Gruppe der Formel -C(=O)R' ist, worin R' gegebenenfalls substituiertes Alkyl, gegebenenfalls substituiertes Alkenyl, gegebenenfalls substituiertes Cycloalkyl, gegebenenfalls substituiertes Aryl, gegebenenfalls substituiertes Amino oder gegebenenfalls substituiertes Alkyloxy ist.

24. Pharmazeutische Zusammensetzung, die eine Verbindung gemäss irgendeinem der Ansprüche 1 bis 23, ein pharmazeutisch annehmbares Salz oder ein Solvat davon als Wirkstoff umfasst.

25. Pharmazeutische Zusammensetzung gemäss Anspruch 24 zur Verwendung bei der Behandlung und/oder Prävention von Diabetes.

## Revendications

1. Composé représenté par formule (I) : 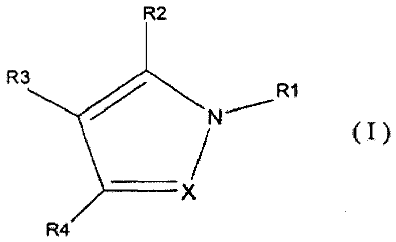 un sel ou un solvate de celui-ci acceptable d'un point de vue pharmaceutique, dans lequel
R¹ est alkyle substitué optionnellement, alcényle substitué optionnellement, alcynyle substitué optionnellement, cycloalkyle substitué optionnellement, cycloalcényle substitué optionnellement, aryle substitué optionnellement, hétéroaryle substitué optionnellement ou hétérocycle substitué optionnellement,
un de R² et R⁴ est un groupe de formule : -Y-R⁵,
dans lequel Y est -O- ou -S-, et
R⁵ est alkyle linéaire substitué dans lequel le substituant dudit alkyle droit substitué est cycloalkyle ou cycloalcényle,
alkyle ramifié substitué optionnellement par hydroxy, carboxy ou halogène,
alcényle substitué optionnellement par hydroxy, carboxy ou halogène, alcynyle substitué optionnellement par hydroxy, carboxy ou halogène, cycloalkyle substitué optionnellement par hydroxy, carboxy ou halogène, cycloalcényle substitué optionnellement par hydroxy, carboxy ou halogène,
aryle substitué optionnellement par hydroxy, carboxy ou halogène, hétéroaryle substitué optionnellement par hydroxy, carboxy ou halogène ou
hétérocycle substitué optionnellement par hydroxy, carboxy ou halogène,
l'autre de R² et R⁴ est hydrogène, alkyle substitué optionnellement, alcényle substitué optionnellement, alcynyle substitué optionnellement, cycloalkyle substitué optionnellement, cycloalcényle substitué optionnellement, aryle substitué optionnellement, hétéroaryle substitué optionnellement ou hétérocyclique substitué optionnellement,
R³ est un groupe de formule : -C(=O)-Z-R⁶,
dans laquelle Z est -NR⁷- ou -NR⁷-W-, et
R⁶ est cycloalkyle substitué optionnellement par hydroxy, carboxy, halogène, cyano, -CH₃, -CH₂OH, -CH₂OCONH₂, -CH₂NH₂, -OCONH₂, -NHCOCH₃, -OCH₃, -NH₂, -NHSO₂CH₃, -CONHCH₃, -OSO₂NH₂, -NHSO₂NH₂, -NHCOOCH₃, -CONH₂, -COOCH₃ ou -CONHSO₂CH₃, ledit cycloalkyle est sélectionné à partir de 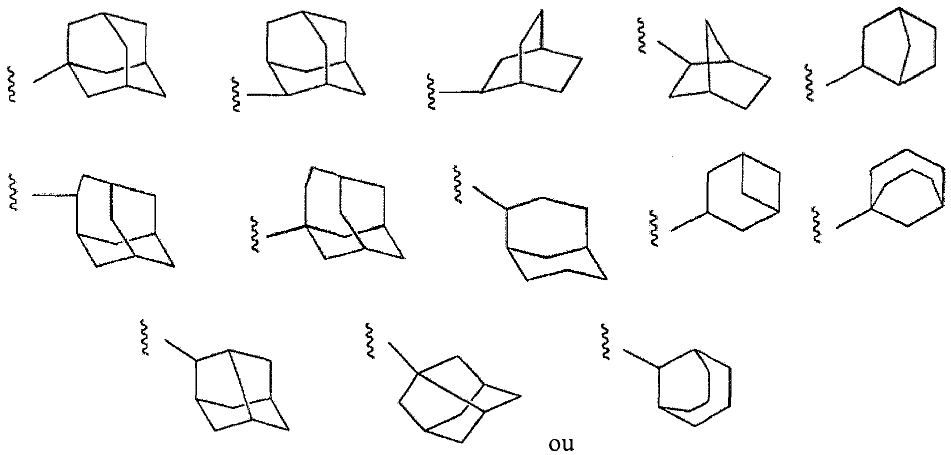 R⁷ est hydrogène ou alkyle substitué optionnellement,
W est alkylène substitué optionnellement,
X est =N- ou =CR⁸-, et
R⁸ est hydrogène ou alkyle substitué optionnellement,
dans lequel « alkyle » signifie un groupe alkyle linéaire ou ramifié en C1 à C10 ; « alcényle » signifie un groupe alcényle linéaire ou ramifié en C2 à C8 ;
« alcynyle » signifie un groupe alcynyle linéaire ou ramifié en C2 à C8 ;
« cycloalkyle » signifie un groupe hydrocarbure cyclique saturé en C3 à C15 ;
« cycloalcényle » signifie un groupe hydrocarbure aliphatique non saturé en C3 à C7;
« aryle » signifie phényle ou naphthyle ;
« hétéroaryle » signifie furyle, thiényle, pyrrolyle, imidazolyle, pyrazolyle, triazolyle, tétrazolyle, oxazolyle, isoxazolyle, thiazolyle, thiadiazolyle, isothiazolyle, pyridyle, pyridazinyle, pyrimidinyle, furazanyle, pyrazinyle, oxadiazolyle, benzofuryle, benzothiényle, benzimidazolyle, dibenzofuryle, benzoxazolyle, quinoxalyle, cinnolyle, quinazolyle, quinolyle, phthalazinyle, isoquinolyle, puryle, ptéridinyle, carbazolyle, phénanthridinyle, acridinyle, indolyle, iso-indolyle, phénazinyle ou phénothiazinyle ;
« hétérocycle » signifie 1-pyrrolinyle, 2-pyrrolinyle, 3-pyrrolinyle, 1-pyrrolidinyle, 2-pyrrolidinyle, 3-pyrrolidinyle, 1-imidazolinyle, 2-imidazolinyle, 4-imidazolinyle, 1-imidazolidinyle, 2-imidazolidinyle, 4-imidazolidinyle, 1-pyrazolinyle, 3-pyrazolinyle, 4-pyrazolinyle, 1-pyrazolidinyle, 3-pyrazolidinyle, 4-pyrazolidinyle, pipéridino, 2-pipéridinyle, 3-pipéridinyle, 4-pipéridinyle, 1-pipérazinyle, 2-pipérazinyle, 2-morpholinyle, 3-morpholinyle, morpholino, tétrahydropyranyle ou les groupes suivants

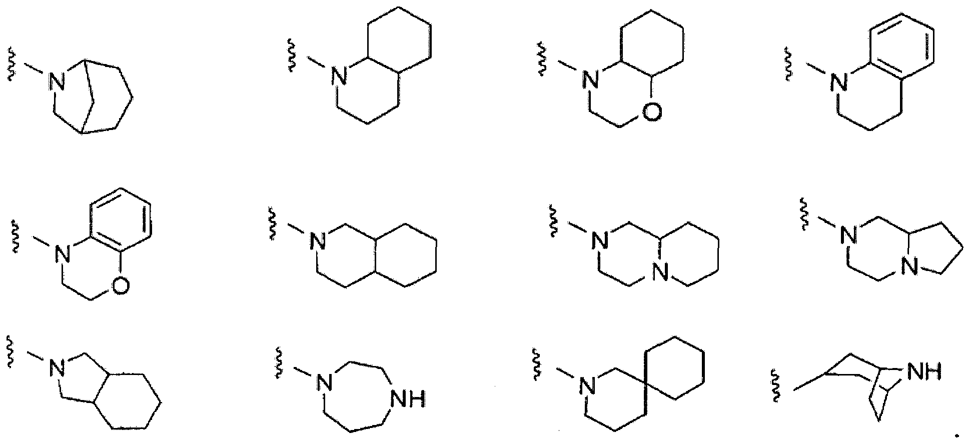

2. Composé selon la revendication 1, un solvate ou un sel de celui-ci acceptable d'un point de vue pharmaceutique, dans lequel R¹ est alkyle substitué dans lequel le substituant dudit alkyle substitué est amino substitué optionnellement ou hétérocycle substitué optionnellement.

3. Composé selon la revendication 1, un solvate ou un sel de celui-ci acceptable d'un point de vue pharmaceutique, dans lequel. R¹ est éthyle substitué dans lequel le substituant dudit éthyle substitué est amino substitué optionnellement ou hétérocycle substitué optionnellement.

4. Composé selon la revendication 1, un solvate ou un sel de celui-ci acceptable d'un point de vue pharmaceutique, dans lequel R¹ est de l'alkyle non substitué.

5. Composé selon la revendication 1, un solvate ou un sel de celui-ci acceptable d'un point de vue pharmaceutique, dans lequel R² est un groupe de formule : -Y-R⁵,
dans lequel Y et R⁵ ont la même signification que celle définie dans la revendication 1.

6. Composé selon la revendication 5, un sel ou un solvate de celui-ci acceptable d'un point de vue pharmaceutique, dans lequel Y est -O-.

7. Composé selon la revendication 5 ou 6, un solvate ou un sel de celui-ci acceptable d'un point de vue pharmaceutique, dans lequel R⁵ est alkyle linéaire substitué dans lequel le substituant dudit alkyle linéaire substitué est du cycloalkyle substitué optionnellement.

8. Composé selon la revendication 7, un solvate ou un sel de celui-ci acceptable d'un point de vue pharmaceutique, dans lequel R⁵ est méthyle substitué dans lequel le substituant dudit méthyle substitué est du cycloalkyle substitué optionnellement.

9. Composé selon la revendication 7 ou 8, un solvate ou un sel de celui-ci acceptable d'un point de vue pharmaceutique, dans lequel ledit cycloalkyle substitué optionnellement est du cyclohexyle substitué optionnellement.

10. Composé selon la revendication 5 ou 6, un solvate ou un sel de celui-ci acceptable d'un point de vue pharmaceutique, dans lequel R⁵ est de l'alkyle ramifié.

11. Composé selon la revendication 1, un solvate ou un sel de celui-ci acceptable d'un point de vue pharmaceutique, dans lequel Z est -NR⁷-, et R⁷ a la même signification que celle définie dans la revendication 1.

12. Composé selon la revendication 11, un solvate ou un sel de celui-ci acceptable d'un point de vue pharmaceutique, dans lequel R⁷ est de l'hydrogène.

13. Composé selon la revendication 1, un solvate ou un sel de celui-ci acceptable d'un point de vue pharmaceutique, dans lequel R⁶ est de l'adamantyle.

14. Composé selon la revendication 1, un solvate ou un sel de celui-ci acceptable d'un point de vue pharmaceutique,
dans lequel R³ est un groupe de formule (II) :

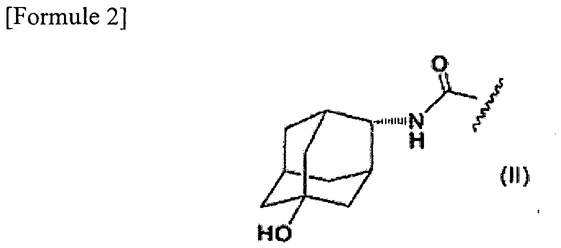

15. Composé selon l'une quelconque des revendications 1 à 14, un solvate ou un sel de celui-ci acceptable d'un point de vue pharmaceutique, dans lequel X est =N-.

16. Composé selon la revendication 1, un solvate ou un sel de celui-ci acceptable d'un point de vue pharmaceutique, dans lequel R¹ est un groupe de formule :
-CH=CH-C(R⁹R¹⁰)-R¹¹-R¹²,
dans laquelle R⁹ et R¹⁰ sont chacun indépendamment de l'hydrogène, alkyle substitué optionnellement ou halogène, ou R⁹ et R¹⁰ pris ensemble avec l'atome de carbone auquel ils sont attachés peuvent former un anneau substitué optionnellement,
R¹¹ est -(CH₂)n-, dans lequel n est un entier de 0 à 3, et
R¹² est hydrogène, hydroxy, carboxy, cyano, alkyle substitué optionnellement, alcényle substitué optionnellement, alcynyle substitué optionnellement, cycloalkyle substitué optionnellement, cycloalcényle substitué optionnellement, aryle substitué optionnellement, hétéroaryle substitué optionnellement, hétérocycle substitué optionnellement, alkyloxycarbonyle substitué optionnellement, arylalkylcarbonyle substitué optionnellement, carbamoyle substitué optionnellement, thiocarbamoyle substitué optionnellement, alkylsulfonyle substitué optionnellement, arylsulfonyle substitué optionnellement, sulfamoyle substitué optionnellement, amino substitué optionnellement, carbamoyloxy substitué optionnellement, alkyloxy substitué optionnellement ou alkylthio substitué optionnellement,
un groupe de formule : -C(=O)-NR¹³R¹⁴,
dans lequel R¹³ et R¹⁴ sont chacun indépendamment de l'hydrogène, amino substitué optionnellement, alkyle substitué optionnellement, alcényle substitué optionnellement, alcynyle substitué optionnellement., cycloalkyle substitué optionnellement, cycloalcényle substitué optionnellement, aryle substitué optionnellement, hétéroaryle substitué optionnellement, hétérocycle substitué optionnellement, alkylsulfonyle substitué optionnellement, arylsulfonyle substitué optionnellement, hétéroarylsulfonyle substitué optionnellement ou hétérocyclosulfonyle substitué optionnellement, ou R¹³ et R¹⁴ pris ensemble avec l'atome d'azote auquel ils sont attachés peuvent former un anneau substitué optionnellement ou,
un groupe de formule : -NR¹⁵R¹⁶,
dans lequel R¹⁵ et R¹⁶ sont chacun indépendamment de l'hydrogène, carboxy, hydroxy, alkyle substitué optionnellement, alcényle substitué optionnellement, alcynyle substitué optionnellement, cycloalkyle substitué optionnellement, cycloalcényle substitué optionnellement, aryle substitué optionnellement, hétéroaryle substitué optionnellement, hétérocycle substitué optionnellement, acyle substitué optionnellement, carbamoyle substitué optionnellement, thiocarbamoyle substitué optionnellement, alkylsulfonyle substitué optionnellement, arylsulfonyle substitué optionnellement, alkyloxycarbonyle substitué optionnellement ou sulfamoyle substitué optionnellement, ou R¹⁵ et R¹⁶ pris ensemble avec l'atome d'azote auquel ils sont attachés peuvent former un anneau substitué optionnellement.

17. Composé selon la revendication 1, un solvate ou un sel de celui-ci acceptable d'un point de vue pharmaceutique, dans lequel R¹ est un groupe de formule : -CH₂-CH₂-C(R⁹R¹⁰)-R¹¹-R¹²,
dans lequel R⁹, R¹⁰, R¹¹ et R¹² ont la même signification que celle définie dans la revendication 16.

18. Composé selon la revendication 16 ou 17, un solvate ou un sel de celui-ci acceptable d'un point de vue pharmaceutique, dans lequel R⁹ et R¹⁰ sont chacun indépendamment un alkyle substitué optionnellement ou R⁹ et R¹⁰ pris ensemble avec l'atome de carbone auquel ils sont attachés peuvent former un anneau substitué optionnellement.

19. Composé selon l'une quelconque des revendications 16 à 18, un solvate ou un sel de celui-ci acceptable d'un point de vue pharmaceutique, dans lequel R¹¹ est -(CH₂)n-, dans lequel n est un entier de 0 à 1.

20. Composé selon l'une quelconque des revendications 16 à 19, un solvate ou un sel de celui-ci acceptable d'un point de vue pharmaceutique, dans lequel R¹² est carboxy, cyano ou un composé hétérocyclique.

21. Composé selon l'une quelconque des revendications 16 à 19, ou un solvate un sel de celui-ci acceptable d'un point de vue pharmaceutique, dans lequel R¹² est un groupe de formule : -C(=O)-NR¹³R¹⁴,
dans lequel R¹³ et R¹⁴ sont chacun indépendamment de l'hydrogène, alkyle substitué optionnellement, aryle substitué optionnellement, cycloalkyle substitué optionnellement, alkylsulfonyle substitué optionnellement, arylsulfonyle substitué optionnellement, hétéroarylsulfonyle substitué optionnellement, hétérocyclosulfonyle substitué optionnellement ou hétérocycle substitué optionnellement, ou R¹³ et R¹⁴ pris ensemble avec l'atome d'azote auquel ils sont attachés peuvent former un anneau substitué optionnellement.

22. Composé selon l'une quelconque des revendications 16 à 19, un solvate ou un sel de celui-ci acceptable d'un point de vue pharmaceutique, dans lequel R¹² est un groupe de formule : -NR¹⁵R¹⁶,
dans lequel R¹⁵ et R¹⁶ ont la même signification que celle définie dans la revendication 16.

23. Composé selon la revendication 22, un sel ou un solvate de celui-ci acceptable d'un point de vue pharmaceutique, dans lequel R¹⁵ est un groupe de formule : -C(=O)R',
dans lequel R' est un alkyle substitué optionnellement, alcényle substitué optionnellement, cycloalkyle substitué optionnellement, aryle substitué optionnellement, amino substitué optionnellement ou alkyloxy substitué optionnellement.

24. Composition pharmaceutique qui comprend le composé selon l'une quelconque des revendications 1 à 23, un solvate ou un sel de celui-ci acceptable d'un point de vue pharmaceutique en tant qu'un ingrédient actif.

25. Composition pharmaceutique selon la revendication 24 pour utilisation dans du traitement et/ou de la prévention de diabète.
